(19) Europäisches Patentamt / European Patent Office / Office européen des brevets

(11) **EP 2 699 260 B1**

(12) **EUROPEAN PATENT SPECIFICATION**

(45) Date of publication and mention
of the grant of the patent:
**20.11.2024 Bulletin 2024/47**

(21) Application number: **12715407.8**

(22) Date of filing: **20.04.2012**

(51) International Patent Classification (IPC):
**A61K 39/395** (2006.01)   **A61P 35/00** (2006.01)
**C07K 16/32** (2006.01)   **C07K 16/10** (2006.01)
**C07K 16/28** (2006.01)   **C07K 16/46** (2006.01)

(52) Cooperative Patent Classification (CPC):
**C07K 16/1063; C07K 16/2809; C07K 16/2863;**
**C07K 16/2887; C07K 16/32; C07K 16/468;**
C07K 2317/21; C07K 2317/24; C07K 2317/31;
C07K 2317/41; C07K 2317/526; C07K 2317/53;
C07K 2317/55; C07K 2317/73; C07K 2317/732;

(Cont.)

(86) International application number:
**PCT/EP2012/057303**

(87) International publication number:
**WO 2012/143523 (26.10.2012 Gazette 2012/43)**

(54) **BISPECIFC ANTIBODIES AGAINST HER2**

BISPEZIFISCHE ANTIKÖRPER GEGEN HER2

ANTICORPS BISPÉCIFIQUES CONTRE HER2

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**

(30) Priority: **20.04.2011 PCT/EP2011/056388**
**20.04.2011 DK 201100312**
**27.05.2011 PCT/EP2011/058772**
**27.05.2011 PCT/EP2011/058779**
**27.10.2011 DK 201100822**
**27.10.2011 US 201161552267 P**

(43) Date of publication of application:
**26.02.2014 Bulletin 2014/09**

(73) Proprietor: **Genmab A/S**
**2500 Valby (DK)**

(72) Inventors:
(72) Inventors:
• **DE GOEIJ, Bart**
**NL-3584 CT Utrecht (NL)**
• **VAN BERKEL, Patrick**
**NL-3544 NH Utrecht (NL)**

• **STRUMANE, Kristin**
**NL-3584 CT Utrecht (NL)**
• **LABRIJN, Aran Frank**
**NL-3584 CT Utrecht (NL)**
• **NEIJSSEN, Joost J.**
**NL-3584 CT Utrecht (NL)**
• **MEESTERS, Joyce I.**
**3522 EC CM Utrecht (NL)**
• **PARREN, Paul**
**NL-3984 PR Odijk (NL)**
• **SCHUURMAN, Janine**
**1111 CH Diemen (NL)**

(74) Representative: **Genmab A/S**
**Carl Jacobsens Vej 30**
**2500 Valby (DK)**

(56) References cited:
**WO-A1-2008/031531   WO-A1-2009/068628**
**WO-A1-2009/154651   WO-A1-2011/147986**
**WO-A2-2011/131746**

- **VAN SPRIEL A B ET AL: "Immunotherapeutic perspective for bispecific antibodies", IMMUNOLOGY TODAY, ELSEVIER PUBLICATIONS, CAMBRIDGE, GB, vol. 21, no. 8, 1 August 2000 (2000-08-01), pages 391 - 397, XP004215167, ISSN: 0167-5699, DOI: 10.1016/S0167-5699(00)01659-5**
- **A EMDE ET AL: "Combining epitope-distinct antibodies to HER2: cooperative inhibitory effects on invasive growth", ONCOGENE, vol. 30, no. 14, 7 April 2011 (2011-04-07), pages 1631 - 1642, XP055029581, ISSN: 0950-9232, DOI: 10.1038/onc.2010.547**
- **W. SCHEUER ET AL: "Strongly Enhanced Antitumor Activity of Trastuzumab and Pertuzumab Combination Treatment on HER2-Positive Human Xenograft Tumor Models", CANCER RESEARCH, vol. 69, no. 24, 15 December 2009 (2009-12-15), pages 9330 - 9336, XP055029437, ISSN: 0008-5472, DOI: 10.1158/0008-5472.CAN-08-4597**

Remarks:
   The complete document including Reference Table(s) and the Sequence Listing(s) can be downloaded from the EPO website

(52) Cooperative Patent Classification (CPC): (Cont.)
   C07K 2317/734; C07K 2317/77; C07K 2317/94

**Description**

**FIELD OF THE INVENTION**

The present invention relates to bispecific antibodies directed to human epidermal growth factor receptor 2 (HER2) and to uses of such antibodies, in particular their use in the treatment of cancer.

**BACKGROUND OF THE INVENTION**

HER2 is a 185-kDa cell surface receptor tyrosine kinase and member of the epidermal growth factor receptor (EGFR) family that comprises four distinct receptors: EGFR/ErbB-1, HER2/ErbB-2, HER3/ErbB-3, and HER4/ErbB-4. Both homo- and heterodimers are formed by the four members of the EGFR family, with HER2 being the preferred and most potent dimerization partner for other ErbB receptors (Graus-Porta et al., Embo J 1997;16:1647-1655; Tao et al., J Cell Sci 2008;121:3207-3217). HER2 can be activated by overexpression or by heterodimerization with other ErbBs that can be activated by ligand binding (Riese and Stern, Bioessays 1998;20:41-48). For HER2, no ligand has been identified. HER2 activation leads to receptor phosphorylation, which triggers a cascade of downstream signals through multiple signaling pathways, such as MAPK, phosphoinositol 3-kinase/AKT, JAK/STAT and PKC, which ultimately results in the regulation of multiple cellular functions, such as growth, survival and differentiation (Huang et al., Expert Opin Biol Ther 2009;9:97-110).

Much of the attention on HER2 in tumors has been focused on its role in breast cancer, in which HER2 overexpression is reported in approximately 20% of the cases and is correlated with poor prognosis (Reese et al., Stem Cells 1997;15:1-8; Andrechek et al., Proc Natl Acad Sci U S A 2000;97:3444-3449; and Slamon et al., Science 1987;235:177-182). Besides breast cancer, HER2 expression has also been associated with other human carcinoma types, including prostate cancer, non-small cell lung cancer, bladder cancer, ovarian cancer, gastric cancer, colon cancer, esophageal cancer and squamous cell carcinoma of the head & neck (Garcia de Palazzo et al., Int J Biol Markers 1993;8:233-239; Ross et al., Oncologist 2003;8:307-325; Osman et al., J Urol 2005;174:2174-2177; Kapitanovic et al., Gastroenterology 1997;112:1103-1113; Turken et al., Neoplasma 2003;50:257-261; and Oshima et al., Int J Biol Markers 2001;16:250-254).

Trastuzumab (Herceptin®) is a recombinant, humanized monoclonal antibody directed against domain IV of the HER2 protein, thereby blocking ligand-independent HER2 homodimerization, and to a lesser extend heterodimerization of HER2 with other family members in cells with high HER2 overexpression (Cho et al., Nature 2003;421:756-760 and Wehrman et al., Proc Natl Acad Sci U S A 2006;103:19063-19068). In cells with modest HER2 expressing levels, trastuzumab was found to inhibit the formation of HER2/EGFR heterodimers (Wehrman *et al.,* (2006), *supra*; Schmitz et al., Exp Cell Res 2009;315:659-670). Trastuzumab mediates antibody-dependent cellular cytotoxicity (ADCC) and prevents ectodomain shedding, which would otherwise result in the formation of a truncated constitutively active protein in HER2 overexpressing cells. Also inhibition of both *in vitro* and *in vivo* proliferation of tumor cells expressing high levels of HER2 has been reported for trastuzumab (reviewed in Nahta and Esteva, Oncogene 2007;26:3637-3643). Herceptin® has been approved both for first-line and adjuvant treatment of HER2 overexpressing metastatic breast cancer, either in combination with chemotherapy, or as a single agent following one or more chemotherapy regimens. Trastuzumab has been found to be effective only in 20-50% of HER2 overexpressing breast tumor patients and many of the initial responders show relapse after a few months (Dinh et al., Clin Adv Hematol Oncol 2007;5:707-717). Herceptin® is also approved, in combination with cisplatin and a fluoropyrimidine (either capecitabine or 5-fluorouracil), for the treatment of patients with HER2-overexpressing metastatic gastric or gastroesophageal (GE) junction adenocarcinoma who have not received prior treatment for metastatic disease.

Pertuzumab (Omnitarg™) is another humanized monoclonal antibody. It is directed against domain II of the HER2 protein, resulting in inhibition of ligand-induced heterodimerization (*i.e.*, HER2 dimerizing with another member of the ErbB family to which a ligand has bound); a mechanism reported to not strictly require high HER2 expression levels (Franklin et al., Cancer Cell 2004;5:317-328.). Although pertuzumab also mediates ADCC, the main mechanism of action of pertuzumab relies on its dimerization blockade (Hughes et al., Mol Cancer Ther 2009;8:1885-1892). Moreover, pertuzumab was found to enhance EGFR internalization and downregulation by inhibiting the formation of EGFR/HER2 heterodimers, which otherwise tethers EGFR at the plasma membrane (Hughes *et al.,* 2009, *supra*). This correlates with the observation that EGFR homodimers internalize more efficient than EGFR/HER2 dimers (Pedersen et al., Mol Cancer Res 2009;7:275-284).

Another suggested HER2-based therapeutic approach is the combination of HER2 antibodies against different HER2 epitopes, which was reported to be more effective than individual HER2 antibodies in reducing tumor growth in *in vitro* and *in vivo* tumor models (Emde et al., Oncogene 2011;30:1631-1642; Spiridon et al., Clin Cancer res 2002;8:1720-1730). For example, the complementary mechanisms of action of pertuzumab and trastuzumab reportedly results in enhanced anti-tumor effects and efficacy when combined in patients who progressed during prior trastuzumab therapy (Baselga et al., J Clin Oncol 2010;28:1138-1144). It was shown in a phase III trial (CLEOPATRA) that the antibody combination pertuzumab plus trastuzumab together with Docetaxel results in prolonged progression-free survival compared to trastuzumab with Docetaxel in patients with previously untreated HER2-positive metastatic breast cancer.

An alternative approach to improve targeted antibody therapy is by delivering cytotoxic cells or drugs specifically to the antigen-expressing cancer cells.

A HER2 antibody drug conjugate (ADC) is currently in clinical development. T-DM1 consists of trastuzumab conjugated to the fungal toxin maytansine. In Phase II trials, responses in a heavily pretreated patient cohort including prior trastuzumab and/or lapatinib therapy were reported (Burris et al, 2011, J Clin Oncol 29: 398-405 and Lewis Phillips et al., Cancer Res 2008;68:9280-9290). Preliminary data from a Phase II trial determining efficacy and safety of T-DM1 versus trastuzumab plus docetaxel in HER2-positive metastatic breast cancer patients with no prior chemotherapy for metastatic disease were reported (Perez et al, Abstract BA3, European Society for Medical Oncology meeting 2010). A Phase III trial (EMILIA) to evaluate T-DM1 efficacy and safety versus capecitabine + lapatinib in patients with HER2-positive locally advanced or metastatic breast cancer who received prior trastuzumab therapy is ongoing. A Phase III trial (MARIANNE) to evaluate T-DM1 for first line treatment in patients with advanced HER2-positive breast cancer has started in July 2010.

While many factors are involved in selecting a suitable antibody for HER2 targeted therapy, it is typically an advantage for an ADC approach if the HER2-antibody complex efficiently internalizes upon antibody binding. Studies on murine HER2 antibodies have shown that certain combinations of antibodies instigate HER2 endocytosis (Ben-Kasus et al., PNAS 2009;106:3294-9). Human HER2 antibodies F5 and C1 have been reported to internalize relatively rapidly when bound to HER2 antigen and to bind the same epitope (WO 99/55367 and WO 2006/116107). As compared to EGFR, however, internalization of HER2 is impaired. Indeed, EGFR homodimers internalize much more efficiently than HER2 homodimers (Dinh et al., Clin Adv Hematol Oncol 2007;5:707-717). EGFR, and also HER3, can increase endocytosis of HER2 by the formation of EGFR/HER2 and HER3/HER2 heterodimers, respectively (Baulida et al., J Biol Chem 1996;271:5251-5257; Pedersen NM, et al., Mol Cancer Res 2009;7:275-84).

Alternatively, bispecific antibodies can be applied to mediate killing of target cells by combining two different Fab arms in one molecule: one Fab arm that binds the antigen on the tumor cell, and one Fab arm that binds CD3 on cytotoxic T cells (CTL). For example, the so-called trifunctional antibodies provide bispecific antigen binding by the Fab arms in addition to Fc receptor binding by the Fc region. Upon bispecific antigen binding, T cells (CD3) are recruited to tumor cells (tumor antigen) and, additionally, effector cells bind the Fc domain of the trifunctional antibody. The formed complexes lead to killing of the tumor cells (Muller and Kontermann, BioDrugs 2010;24:89-98). Ertumaxomab is one such HER2xCD3 trifunctional antibody, which induces cytotoxicity in cell lines with low HER2 expression (Jones et al., Lancet Oncol 2009;10: 1179-1187 and Kiewe et al., Clin Cancer Res 2006;12:3085-3091). Alternatively, a complex of T cells and tumor cells can be formed, leading to killing of the tumor cells (Muller and Kontermann, BioDrugs 2010;24:89-98, Baeuerle and Reinhardt 2009, Cancer Research 96: 4941) by an dual targeting antibody fragment (*e.g.* dual targeting single chain antibodies). Blinatumomab (Bargou et al, Science 2008, 321:974-976) is a single chain antibody construct named BiTE which induces cytotoxicity by targeting CD19 and CD3. Other antibody fragment based T-cell engaging bispecifics have been described (Moore et al. 2011, Blood 117:4542-4551, Baeuerle et al., Current opinion in Molecular Therapeutics 2009, 11:22-30).

Other bispecific constructs, simulatanously targeting HER2 and a second member of the EGFR family, have also been discussed as a potential strategy to increase efficiency and selectivity of HER2-targeted therapy. Examples of such constructs are HER2xEGFR affibody (Friedman et al., Biotechnol Appl Biochem. 2009 Aug 21;54(2):121-31) and HER2xHER3 tandem single chain Fv's MM-111 (Robinson et al., Br.J.Cancer 2008;99:1415-25; WO 2005/117973).

In WO 2008/031531 it is concluded that the the combined use of trastuzumab and pertuzumab leads to improve tumor growth inhibition and survival when compared to the respective monotherapies.

WO 2009/154651 provides a method for the treatment of breast cancer, comprising administering to a HER2 positive metastatic breast cancer patient an effective amount of a growth inhibitory HER2 antibody, a HER2 dimeriation inhibitor antibody, and a taxane, wherein the growth inhibitory HER2 antibody binds within Domain IV of the HER2 amino acid sequence; e.g. the growth inhibitory HER2 antibody binds essentially to epitope 4D5 of HER2, and wherein the HER2 dimirization inhibitory antibody binds essentially to epitope 2C4; and bispecific antibodies which bind to two different epitopes of the HER2 protein.

WO 2009/068628 discloses three biparatopi HER2xHER2 antibodies. It is further disclosed that these antibodies are to be conjugated with cytotoxic drugs or with cytokines. Further disclosed are expression vetors, host cells, pharmaceutical sompositions, first and second medical uses, combination uses amd methods of production.

The complex mechanisms regulating the function of HER2 warrant further research on new and optimized therapeutic strategies against this proto-oncogene. Accordingly, there remains a need for effective and safe products for treating HER2-related diseases, such as cancer.

## SUMMARY OF THE INVENTION

It is an object of the present invention to provide bispecific antibodies comprising a first antigen-binding region and a second antigen-binding region, which first and second antigen-binding regions bind different epitopes on human epidermal growth factor receptor 2 (HER2), and wherein the first antigen-binding region is selected from the group consisting of:

a) an antibody comprising a VH region comprising the sequence of SEQ ID NO:63 and a VL region comprising the sequence of SEQ ID NO:67 (**153**),

b) an antibody comprising a variable heavy (VH) region comprising the sequence of SEQ ID NO:165 and a variable light (VL) region comprising the sequence of SEQ ID NO:169 (**005**), and

c) an antibody comprising a VH region comprising the sequence of SEQ ID NO:22 and a VL region comprising the sequence of SEQ ID NO:26 (**025**),

wherein the second antigen binding region comprises a VH region comprising SEQ ID NO:1, and a VL region comprising SEQ ID NO:5 (**169**).

The bispecific antibodies may be prepared from HER2 antibodies that are fully human or humanized, bind to novel epitopes, and/or have favorable properties for therapeutic use in human patients. Each Fab-arm of the bispecific antibodies may further include an Fc-region, optionally comprising modifications promoting the formation of the bispecific antibody, modifications affecting Fc-mediated effector functions, conjugated drugs, or any combination of these and/or other features described herein.

## BRIEF DESCRIPTION OF THE DRAWINGS

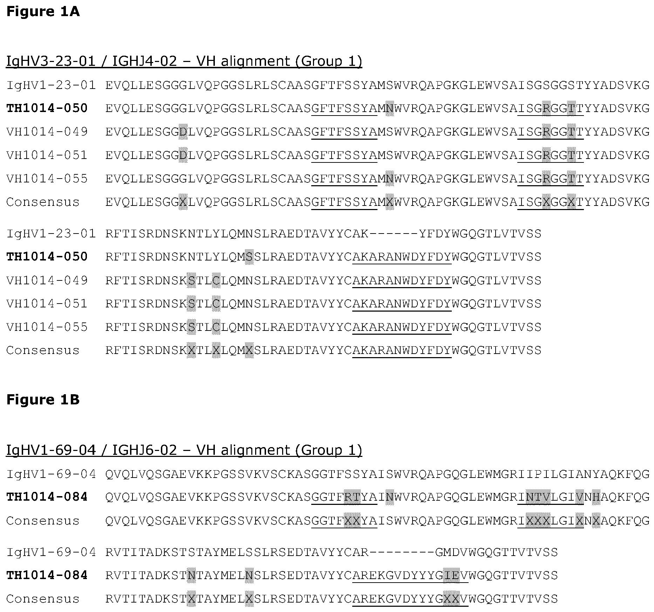

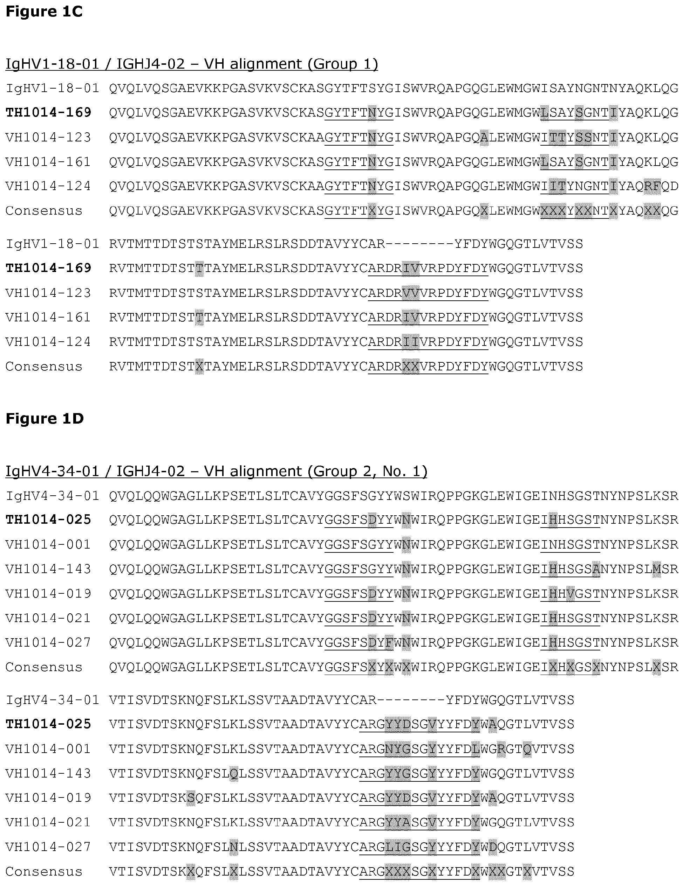

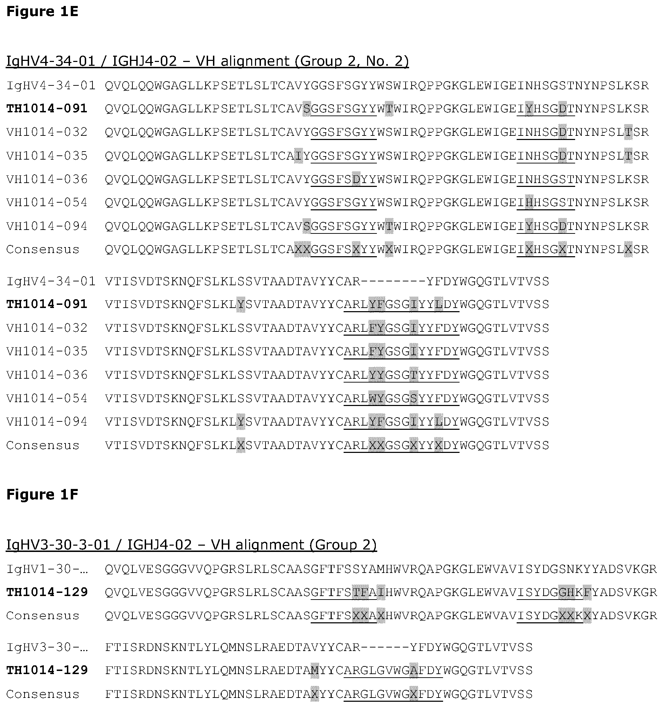

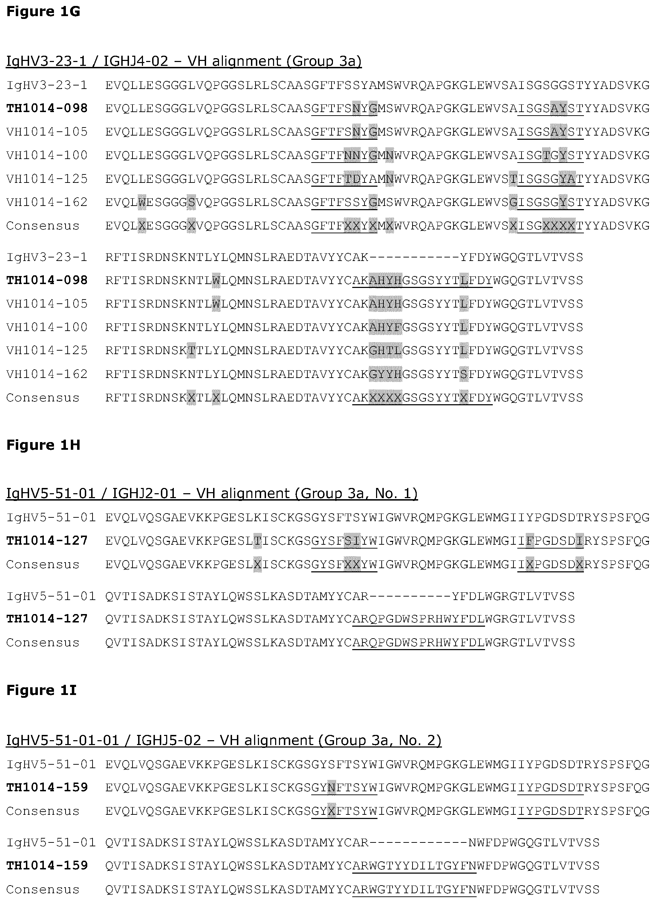

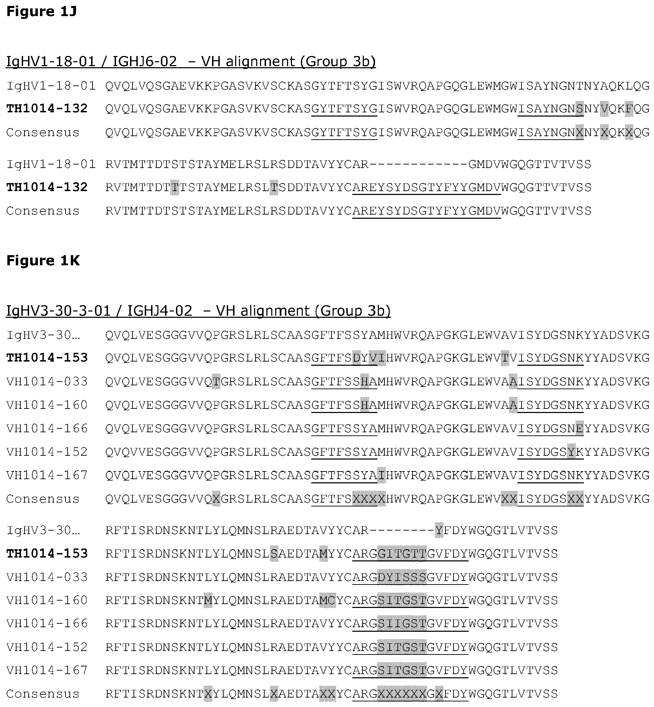

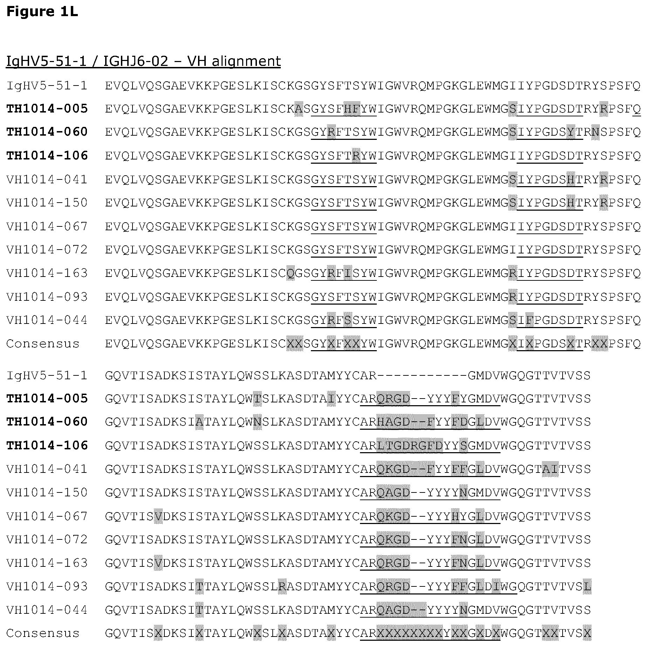

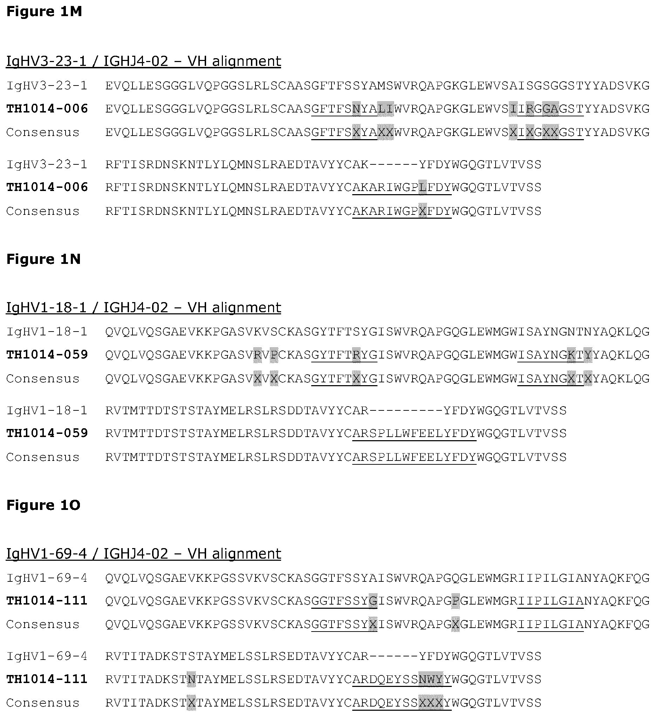

**Figure 1:** Alignment of HuMab heavy chain variable region (VH) sequences with germline (reference) sequences (A-O). In each VH sequence, the amino acids that differ from those of the germline (reference) at specific positions are highlighted. Consensus VH sequences are shown, where "X" indicates positions at which alternative amino acids (selected from those aligned at each position) are possible. The CDR1, CDR2, and CDR3 sequences are underlined in each VH sequence. The consensus CDR sequences are further defined in Table 4.

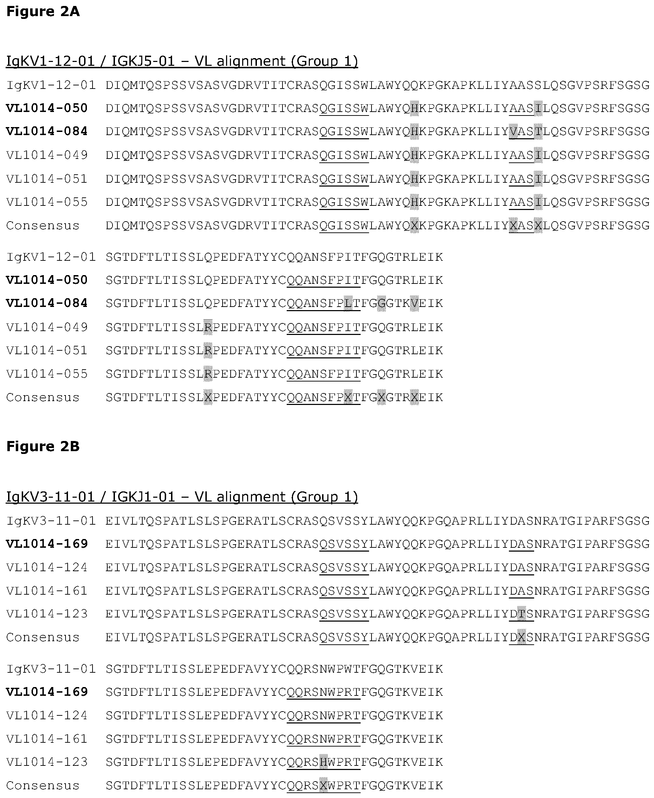

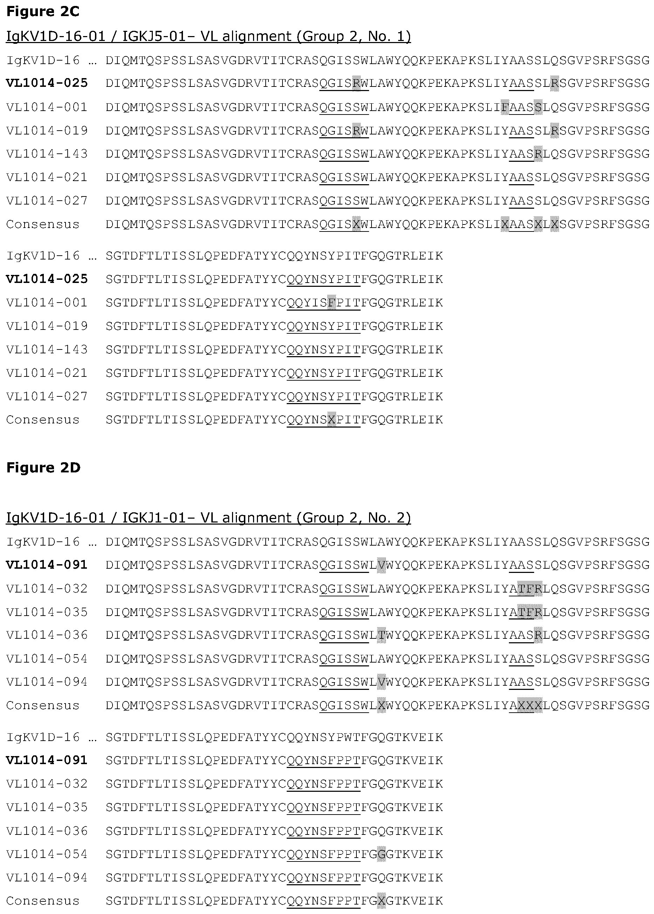

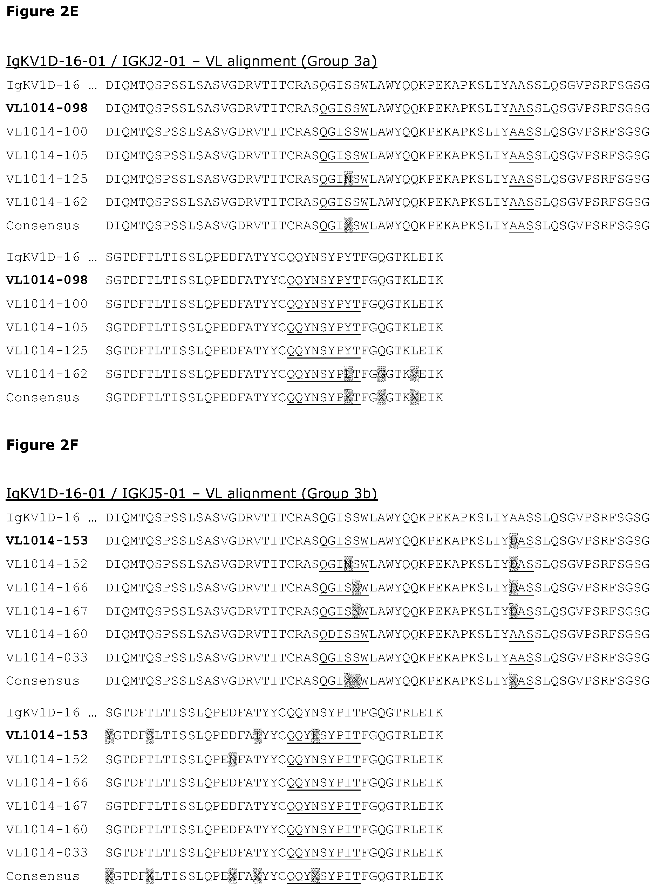

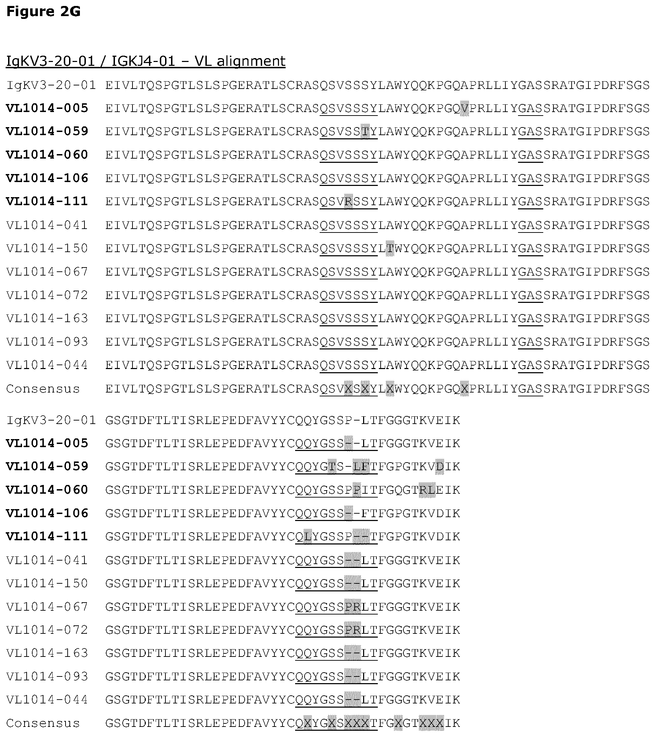

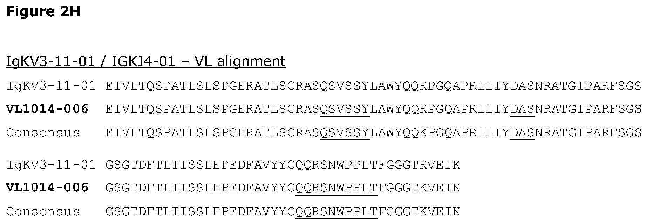

**Figure 2:** Alignment of HuMab light chain variable region (VL) sequences with germline (reference) sequences (panels A-H). In each VL sequence, the amino acids that differ from those of the germline (reference) at specific positions are highlighted. In Figure 2A, all VL sequences derived from the same V-segment (IgKV1-12-01), but the closest J-segment differed between antibodies. Consensus VL sequences are shown, where "X" indicates positions at which alternative amino acids (selected from those aligned at the indicated position) are possible. The CDR1, CDR2, and CDR3 sequences are underlined in each VL sequence. The consensus CDR sequences are further defined in Table 4.

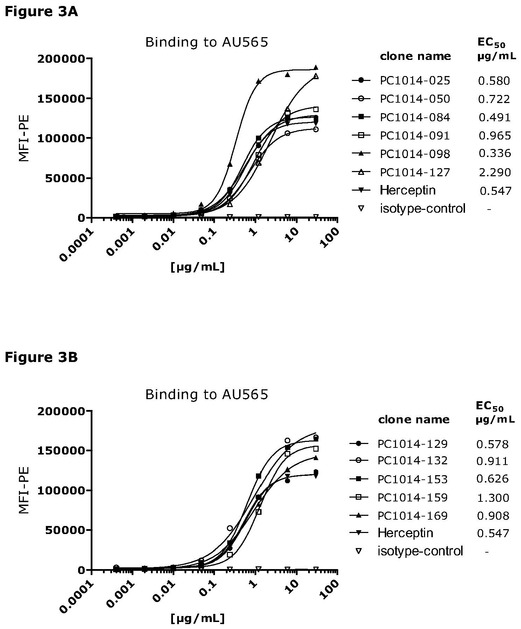

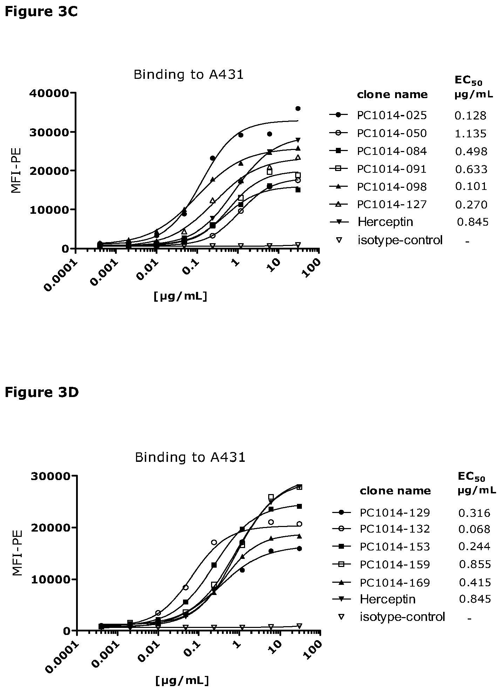

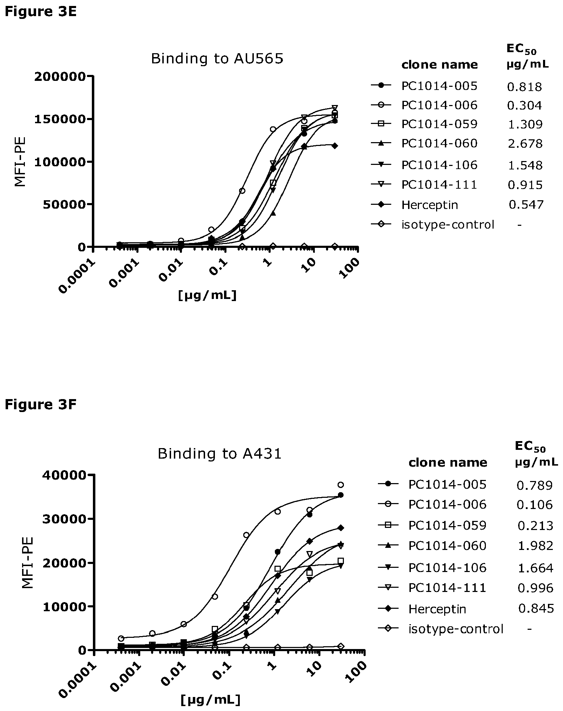

**Figure 3:** Binding curves of HER2 antibodies to (A, B, E) high (AU565) and (C, D, F) low (A431) HER2 expressing cell lines, determined as described in Example 12. Data shown are mean fluorescence intensities (MFI) of one representative experiment for each cell line. The $EC_{50}$ values indicate the apparent affinities.

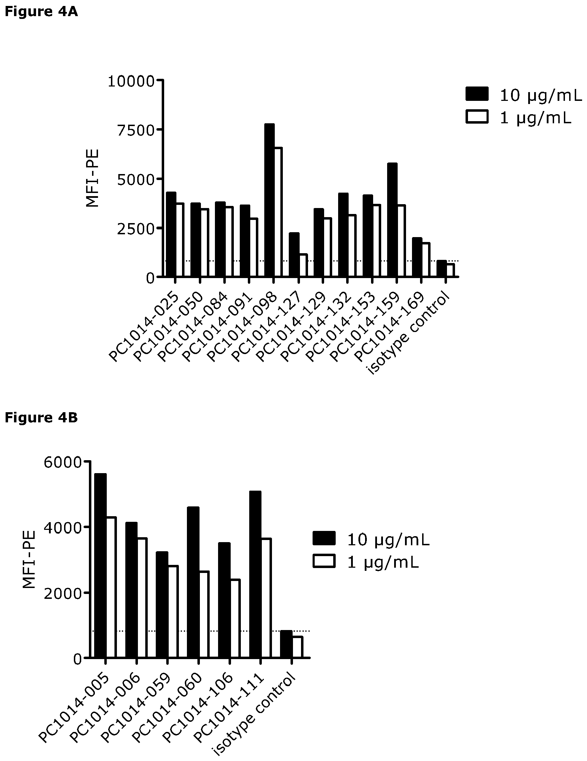

**Figure 4:** Binding of HER2 antibodies to HER2 expressed on monkey Rhesus epithelial cells. Data shown are mean fluorescence intensities (MFI) of one experiment, described in Example 13.

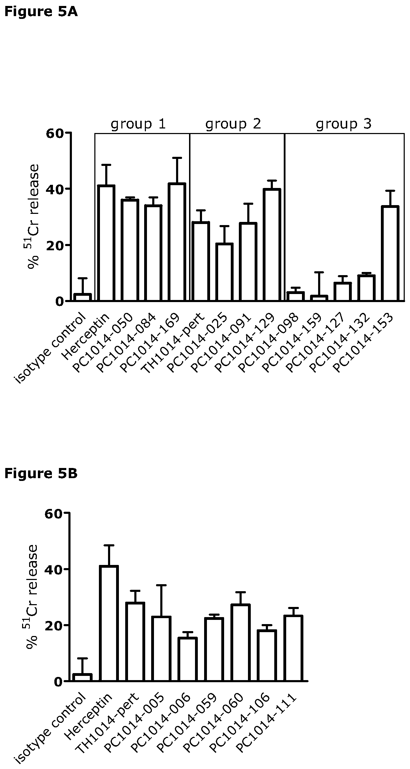

**Figure 5:** Chromium-release (ADCC) assay of HER2 antibodies, showing PBMC-mediated lysis of $^{51}$Cr-labeled SK-BR-3 cells after incubation with HER2 antibody. Values depicted are the mean maximum percentages $^{51}$Cr-release $\pm$ the standard deviation from one representative *in vitro* ADCC experiment with SK-BR-3 cells. See Example 15 for details.

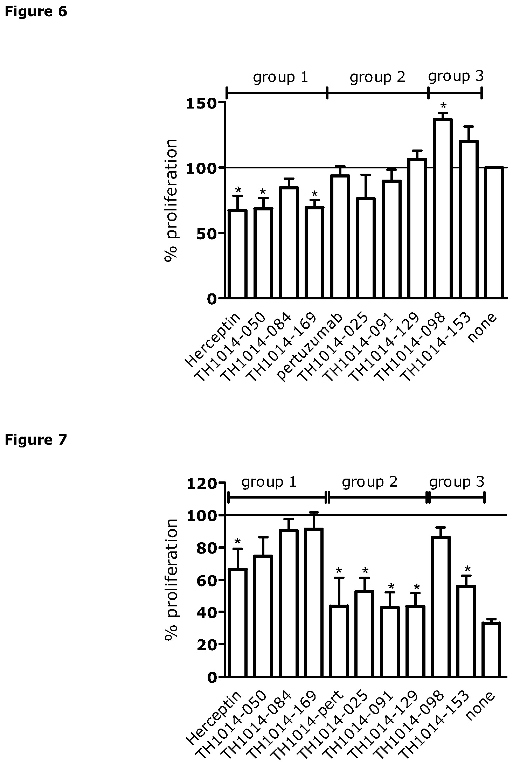

**Figure 6:** Effect of HER2 antibodies on the proliferation of AU565 cells, as compared to untreated cells (set to 100%). Data shown are percentages proliferation of AU565 cells compared to untreated cells measured in three independent experiments $\pm$ the standard deviation. * Significant (P<0.05). See Example 16 for details.

**Figure 7:** Percentage of viable MCF7 cells stimulated with Heregulin-$\beta$1 and treated with the indicated HER2 antibodies, relative to cells stimulated with Heregulin-$\beta$1 only. As a control, the percentage proliferation of unstimulated cells is shown (none). Data was obtained from three independent experiments $\pm$ the stdev. * Significant inhibition of Heregulin-$\beta$1-induced proliferation (P<0.05). See Example 17 for details.

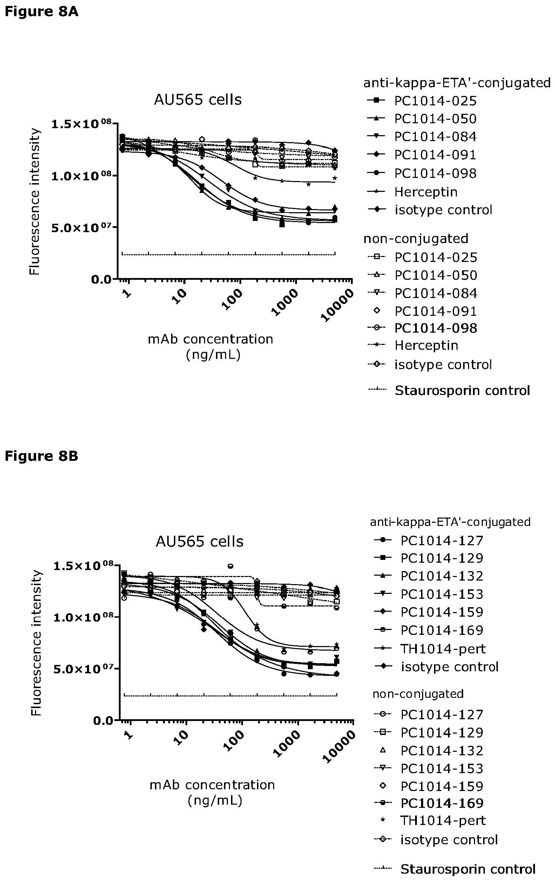

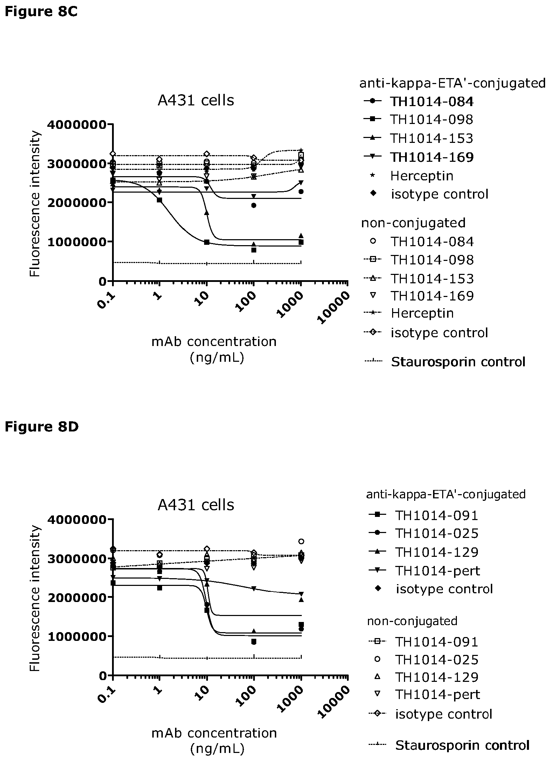

**Figure 8:** ADC assay, showing killing of AU565 cells (A, B) or A431 cells (C, D) via anti-kappa-ETA'-conjugated HER2 antibodies. (A, B) Data shown are fluorescence intensities (FI) of one representative experiment with AU565 cells treated with non-conjugated and anti-kappa-ETA'-conjugated HER2 antibodies. (C, D) Data shown are mean fluorescence intensities (MFI) of one representative experiment with A431 cells treated with non-conjugated and anti-kappa-ETA'-conjugated HER2 antibodies. See Example 18 for details.

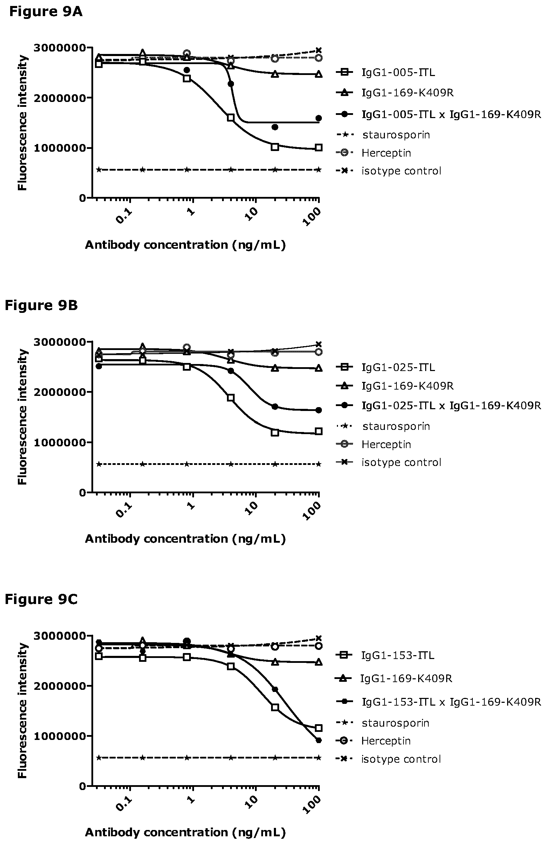

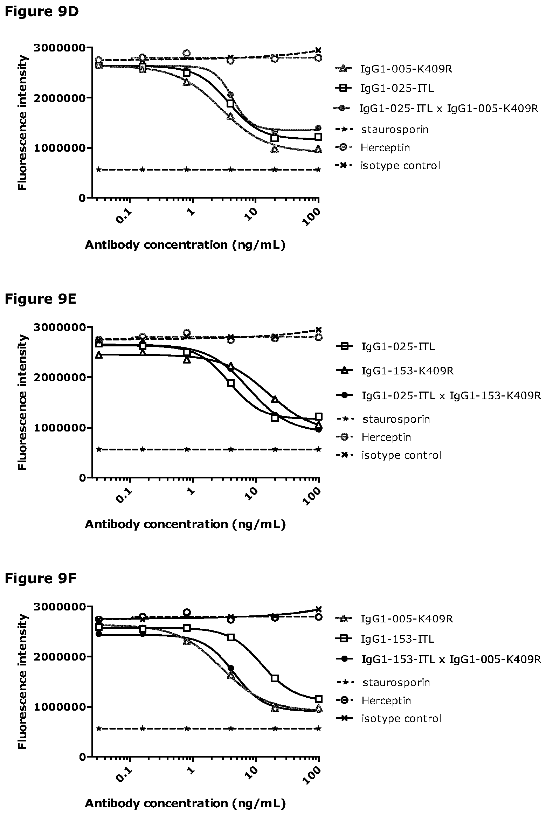

**Figure 9:** Killing of A431 cells induced by anti-kappa-ETA' pre-incubated HER2 x HER2 bispecific antibodies. The viability of A431 cells after 3 days incubation with HER2 antibodies, pre-incubated with anti-kappa-ETA'. Cell viability was quantified using Alamarblue. Data shown are fluorescence intensities (FI) of one experiment with A431 cells treated with anti-kappa-ETA'-conjugated HER2 antibodies and HER2 x HER2 bispecific antibodies. Staurosporin was used as positive control, whereas an isotype control antibody was used as negative control.

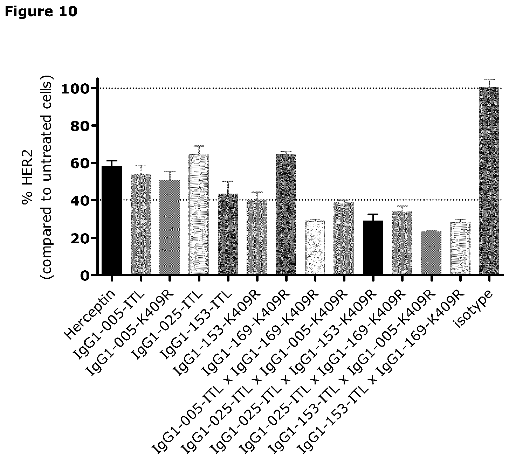

**Figure 10:** HER2 x HER2 bispecific molecules induced downmodulation of HER2 receptor. Relative percentage of HER2 expression levels in AU565 cell lysates after 3 days incubation with 10 μg/mL mAb. The amount of HER2 was quantified using a HER2-specific capture ELISA and depicted as percentage inhibition compared to untreated cells. Isotype control was IgG1-3G8-QITL. Data shown is the mean of two experiments plus standard deviation, except for combinations of monospecific IgG1 antibodies which were tested once.

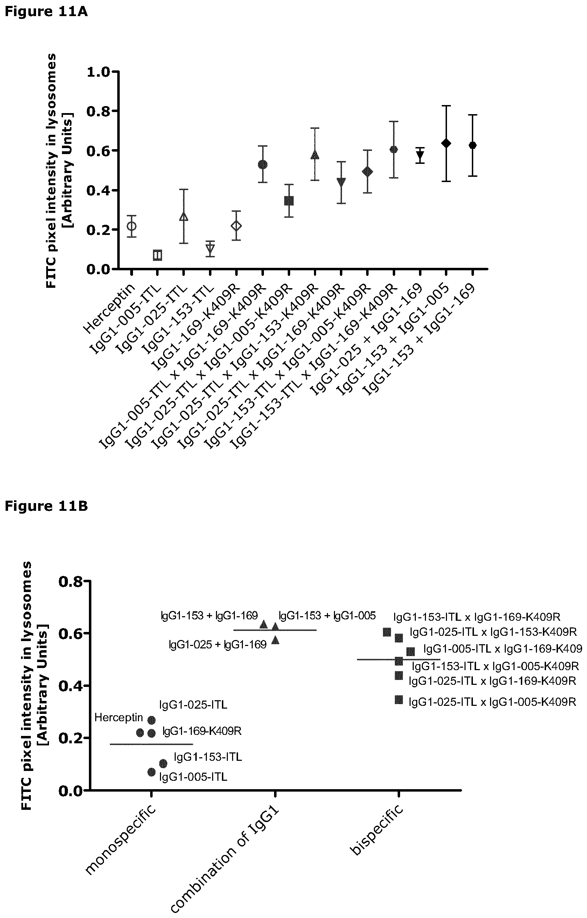

**Figure 11:** Colocalization analysis of HER2 x HER2 bispecific antibodies (FITC) with lysosomal marker LAMP1 (Cy5). FITC pixel intensity overlapping with Cy5 for various monospecific HER2 antibodies and HER2 x HER2 bispecific antibodies (A). FITC pixel intensity in LAMP1/Cy5 positive pixels of three different images is plotted for each antibody tested. Monospecifics show lower FITC pixel intensities in the LAMP1/Cy5 positive pixels compared to bispecifics. (B) Mean value of FITC pixel intensity per LAMP1/Cy5 positive pixel calculated from the three different images. Together these results indicate that after internalization higher levels of bispecific antibodies, compared to monospecifics antibodies, localize to Lamp1/Cy5 positive vesicles.

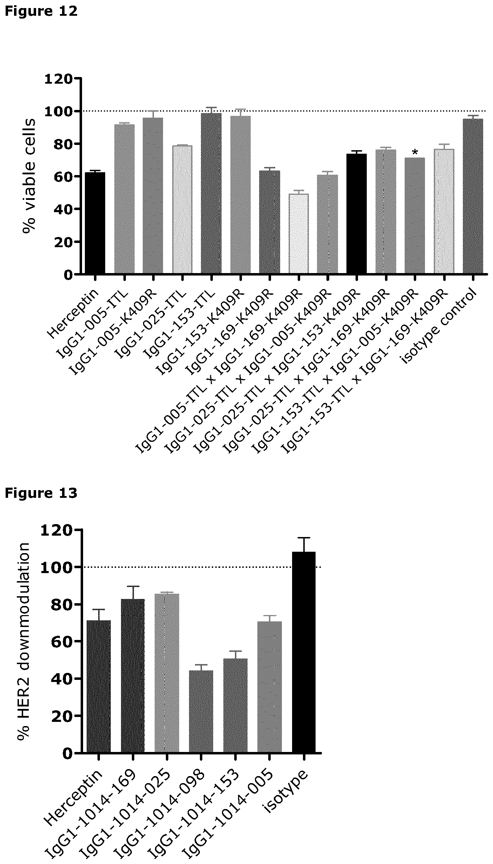

**Figure 12:** Inhibition of proliferation by HER2 mono- and bispecific antibodies. AU565 cells were seeded in the presence of 10 μg/mL HER2 antibody or HER2 x HER2 bispecific antibody in serum-free cell culture medium. After three days, the amount of viable cells was quantified with Alamarblue and cell viability was presented as a percentage relative to untreated cells. An isotype control antibody (IgG1-b12) was used as negative control. Data shown are percentage viable AU565 cells compared to untreated cells measured in five-fold ± the standard deviation. * indicates only one data point was depicted.

**Figure 13:** Antibody induced downmodulation of HER2. Relative percentage of HER2 expressed in AU565 cell lysate after 3 days incubation with 10 μg/mL antibody. The amount of HER2 was quantified using a HER2-specific capture ELISA and plotted as a percentage relative to untreated cells. Data shown are mean of three experiments ± standard deviation.

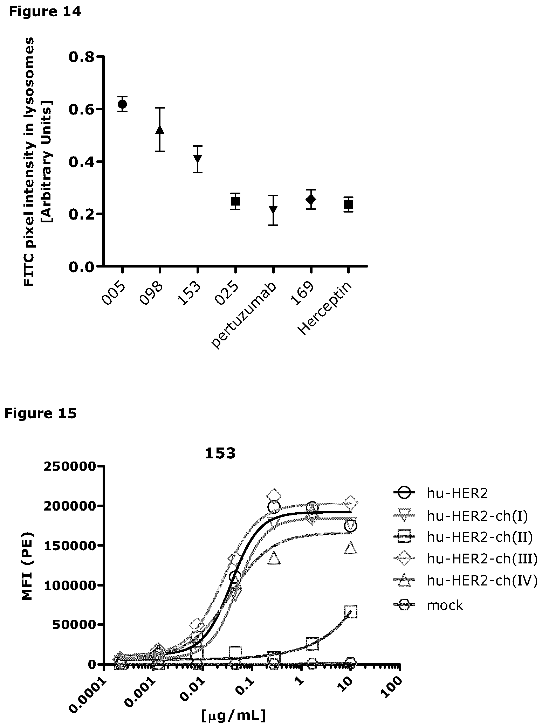

**Figure 14:** Colocalization analysis of HER2 antibodies (FITC) with lysosomal marker LAMP1 (Cy5). FITC pixel intensity overlapping with Cy5 for various monospecific HER2 antibodies. FITC pixel intensity in LAMP1/Cy5 positive pixels of three different images is plotted for each antibody. Group 3 antibodies 098 and 153 show higher FITC pixel intensities in the LAMP1/Cy5 positive compartments compared to antibodies 025 and pertuzumab from Group 2 and 169 and Herceptin from Group 1.

**Figure 15:** HER2 antibody binding to CHO-S cells transfected with different HER2 ECD construct analyzed by means of flow cytometry. Hu-HER2 = fully human HER2, Hu-HER2-ch(I) CR1 = hu-HER2 with chicken domain I, Hu-HER2-ch(II) = hu-HER2 with chicken domain II, hu-HER2-ch(III) = hu-HER2 with chicken domain III and Hu-HER2-ch(IV) = hu-HER2 with chicken domain IV. Data shown are mean fluorescence intensities (MFI) of one representative antibody, TH1014-153. See Example 27 for details.

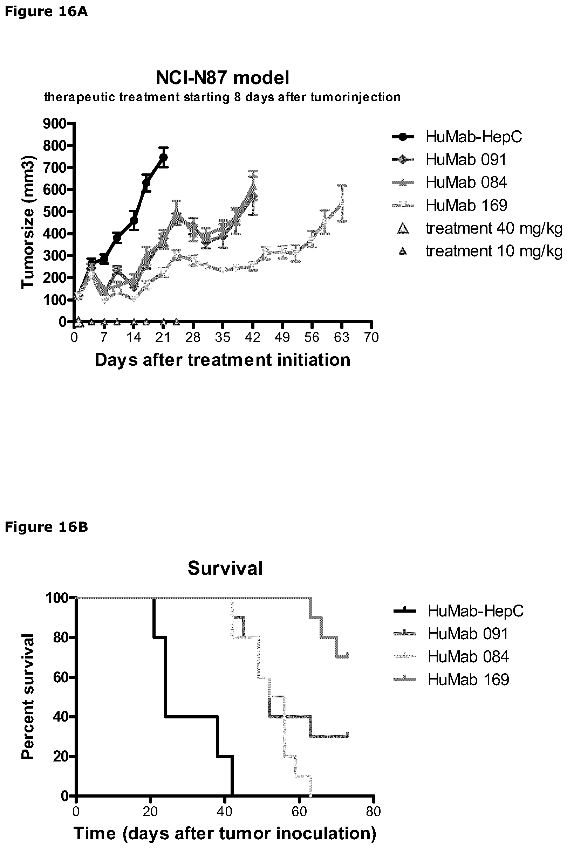

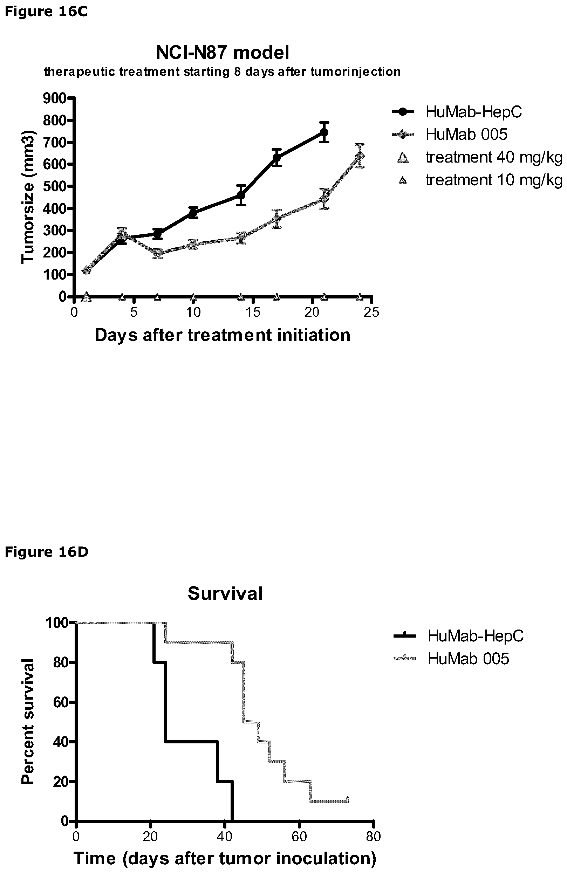

**Figure 16:** *In vivo* effect of HER2-HuMabs in the NCI-N87 human gastric carcinoma xenograft model in female CB.17 severe combined immunodeficiency (SCID) mice. Data shown are mean tumorsize ± S.E.M. per group (n = 10 mice per group) (A, C) and survival (B, D). See Example 28 for details.

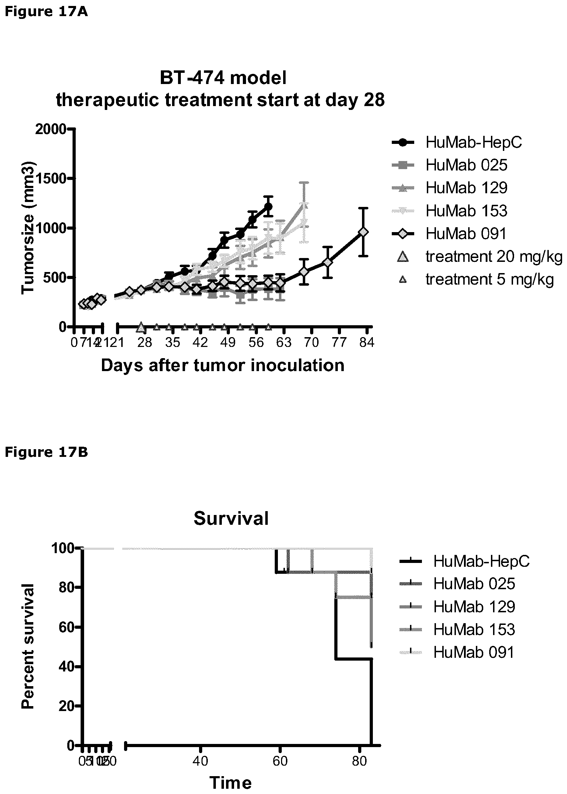

**Figure 17:** *In vivo* effect of HER2 HuMabs in BT-474 breast tumor xenografts in Balb/C nude mice. Data shown are mean tumorsize ± S.E.M. per group (n = 8 mice per group) (A) and survival (B). See Example 29 for details.

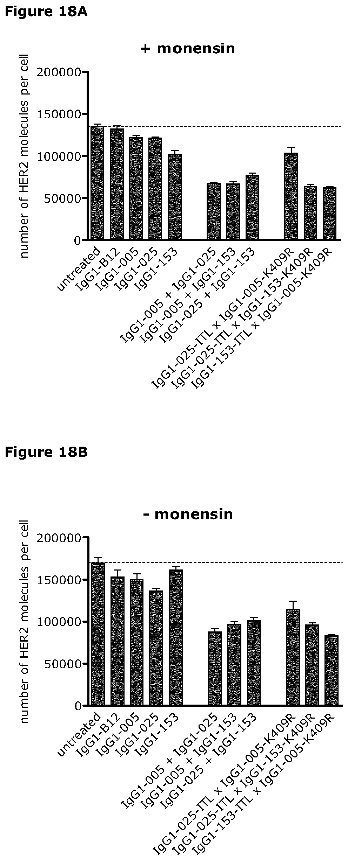

**Figure 18:** Antibody-induced downmodulation of HER2 surface expression. HER2 surface expression was determined after 3 hours incubation with the indicated antibodies at a final concentration of 10 μg/mL, with are without monensis to block recycling. Receptor surface expression was quantified by QIFIKIT® analysis. Monospecific HER2 antibodies did not influence the number of HER2 molecules present on the cell surface compared to untreated cells. Bispecific HER2 x HER2 antibodies resulted in HER2 downmodulation from the surface, comparable to the combination of the two corresponding monospecific parental antibodies. Monensin had only a minor effect on the surface expression in all samples, suggesting that only a minority of the internalized HER2 molecules is recycled back to the surface. Graphs present mean +/- standard deviation.

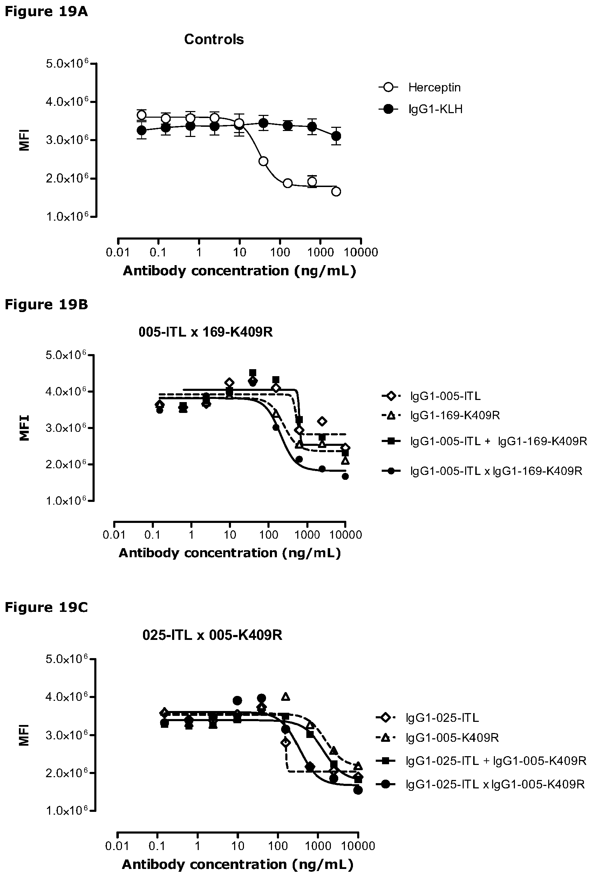

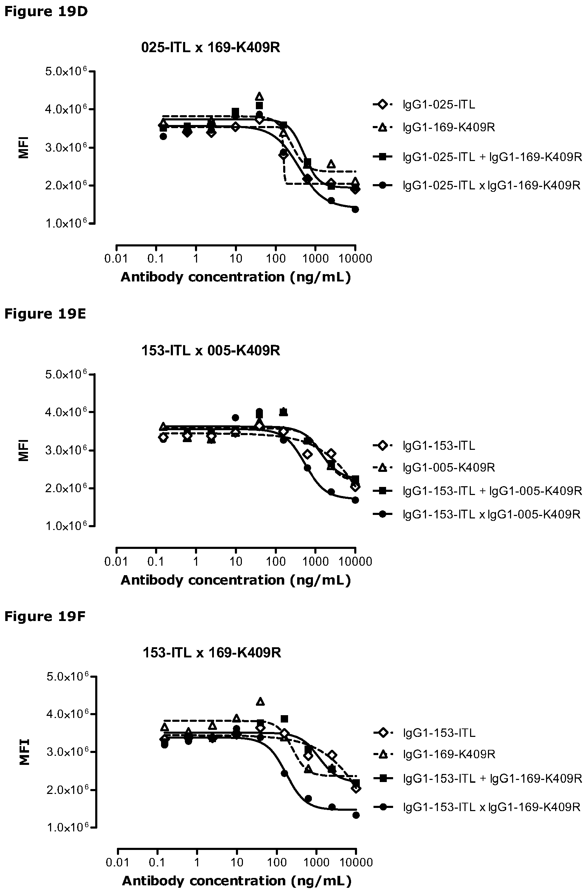

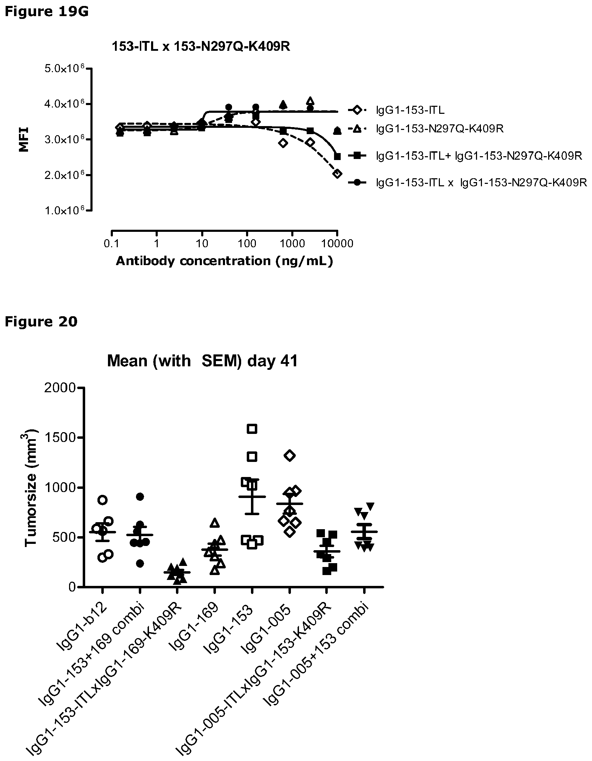

**Figure 19:** PBMC-mediated cytotoxicity by HER2 x HER2 bispecific antibodies on AU565 cells. Killing activity of bispecific antibodies (indicated by x in the legend) was compared to that of the parental monospecific antibodies and the combination thereor (indicated by + in the legend). Dose-dependent killing of AU565 cells by HER2 antibodies in a PBMC-mediated cytotoxicity assay was retained in bispecific HER2 x HER2 antibodies. Herceptin and IgG1-KLH (irrelevant antibody) were used as positive and negative control antibodies, respectively. Inactivating one of the two Fc-domains by introduction of the N297Q mutation, resulted in loss of ADCC for IgG1-153-ITLxIgG1-153-K409R-N297Q.

**Figure 20:** Efficacy of HER2 x HER2 bispecific antibodies to inhibit tumor growth in an NCI-N87 xenograft model in SCID mice. Mice were treated with saturating antibody doses on day 7, 14 and 21 after tumor inoculation. Mean tumor sizes at day 41 per treatment group are shown. Both tested HER2 x HER2 bispecific antibodies demonstrated better *in vivo* efficacy compared to their monospecific counterparts and the combination of these two monospecific antibodies.

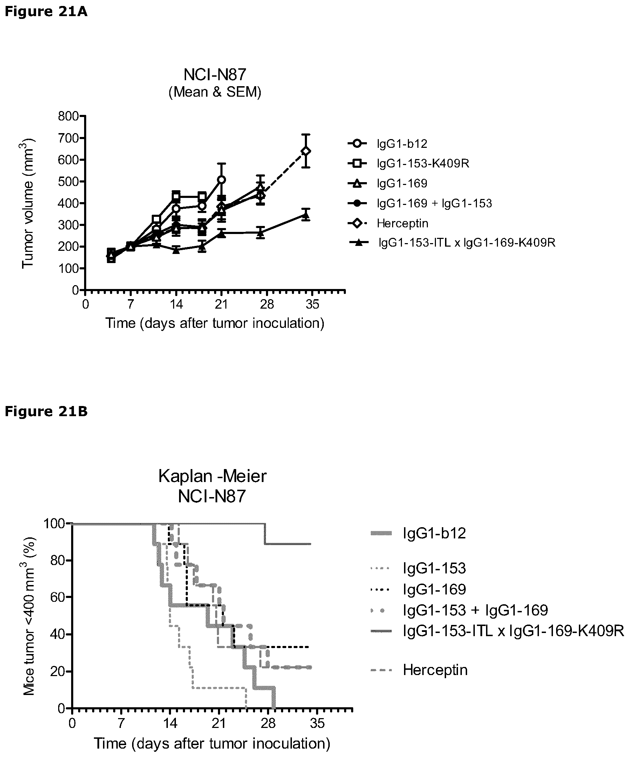

**Figure 21:** Efficacy of Her2 x Her2 bispecific antibodies to inhibit tumor growth in an NCI-N87 xenograft model in SCID mice. In (A), tumor development (Mean & SEM) in mice with NCI-N87 s.c. xenografts treated with saturating antibody doses on day 7 and 14 after tumor inoculation is shown. The Her2 x Her2 bispecific IgG1-153-ITL x IgG1-169-K409R antibody demonstrated better *in vivo* efficacy compared to their monospecific counterparts and the combination of these two monospecific antibodies. In (B) the percentage mice with tumor sizes smaller than 400 mm$^3$ is shown in a Kaplan-Meier plot.

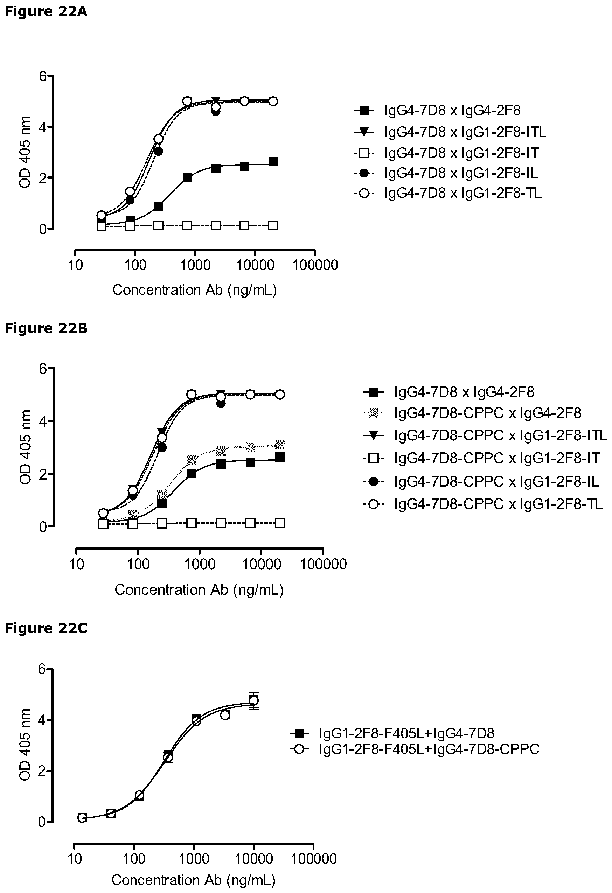

**Figure 22:** Comparison between triple mutant (ITL), double mutants (IT, IL, TL) and single mutant (L) human IgG1-2F8 in the generation of bispecific antibodies by Fab-arm exchange with human IgG4-7D8. The generation of bispecific antibodies after 2-MEA-induced *in vitro* Fab-arm exchange between the human IgG1-2F8 triple and double mutants and wild type IgG4-7D8 with a CPSC hinge (A) or mutant IgG4-7D8-CPPC with a stabilized hinge (B), or the single mutant IgG1-2F8-F405L and IgG4-7D8 with a wild type CPSC or stabilized CPPC hinge (C), was determined by an ELISA. A concentration series (total antibody) of 0-20 μg/mL or 0-10 μg/mL was analyzed in the ELISA for the experiments including the double and single mutants, respectively. Combinations with the double mutants IgG1-2F8-IL and - TL result in bispecific EGFR/CD20 binding similar as the triple mutant IgG1-ITL. Combinations with the IgG1-2F8-IT do not result in a bispecific product. Combinations with the single mutant IgG1-2F8-F405L result in bispecific EGFR/CD20 binding.

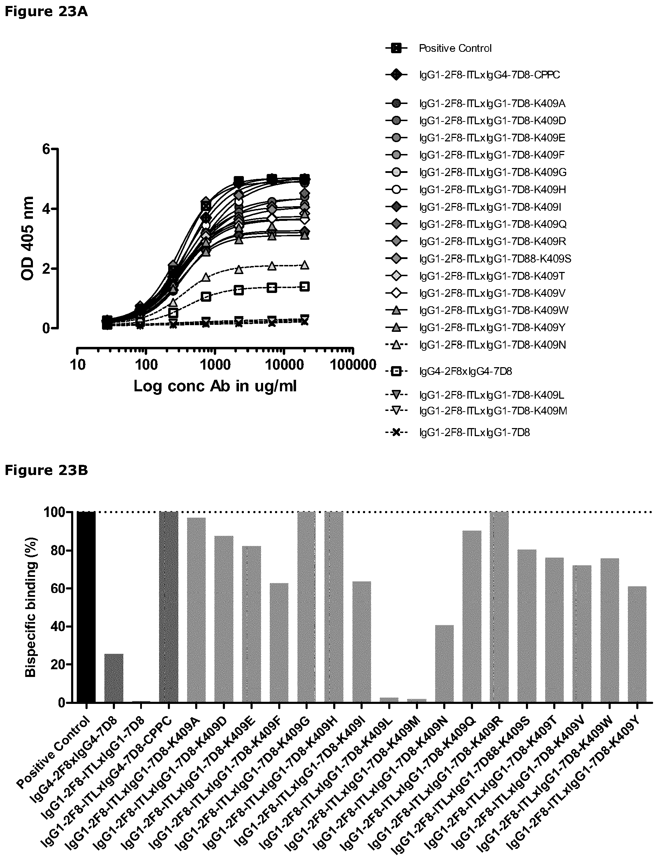

**Figure 23:** 2-MEA-induced Fab-arm exchange between IgG1-2F8-ITL and IgG1-7D8-K409X mutants. The generation of bispecific antibodies after 2-MEA-induced *in vitro* Fab-arm exchange between IgG1-2F8-ITL and the indicated IgG1-7D8-K409X mutants was determined by an ELISA. (A) A concentration series (total antibody) of 0-20 μg/mL was analyzed. The positive control is a purified batch of bispecific antibody, derived from IgG1-2F8-ITL x IgG4-7D8-CPPC. (B) The exchange is presented as bispecific binding at 20 μg/mL relative to the positive control (black bar). Dark grey bars represents the bispecific binding between the IgG4 control (IgG4-7D8 x IgG4-2F8), the negative control (IgG1-2F8 x IgG1-7D8-K409R) and between IgG1-2F8-ITL and IgG4-7D8-CPPC. Light grey bars represent results from simultaneously performed Fab-arm-exchange reactions between the indicated IgG1-7D8-K409X mutants and IgG1-2F8-ITL.

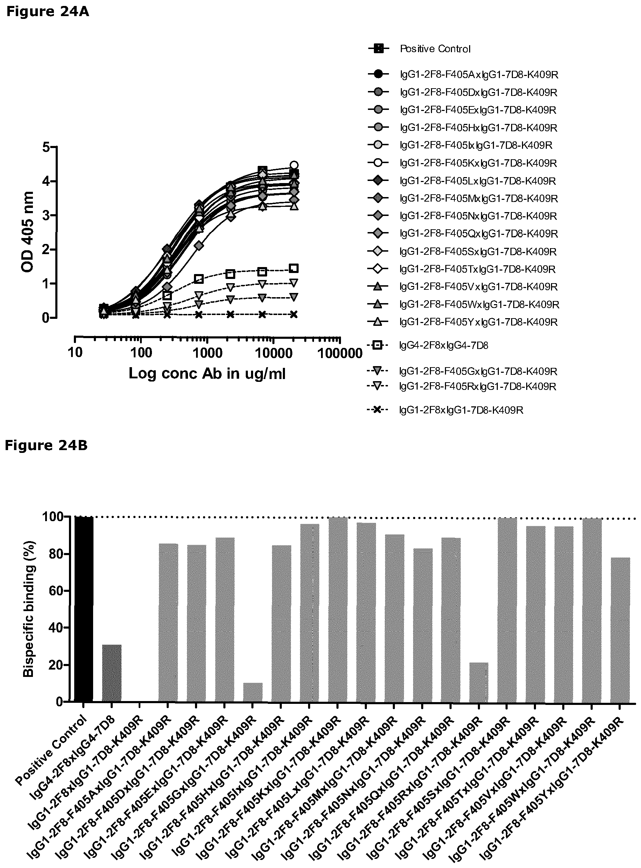

**Figure 24:** 2-MEA-induced Fab-arm-exchange between IgG1-2F8-F405X mutants and IgG1-7D8-K409R. The generation of bispecific antibodies after 2-MEA-induced *in vitro* Fab-arm-exchange between the indicated IgG1-2F8-F405X mutants and IgG1-7D8-K409R was determined by an ELISA. (A) A concentration series (total antibody) of 0-20 μg/mL was analyzed in the ELISA. The positive control is a purified batch of bispecific antibody, derived from IgG1-2F8-F405L x IgG1-7D8-K409R. (B) The exchange is presented as bispecific binding at 20 μg/mL antibody concentration relative to the positive control (black bar). Dark grey bars represents the bispecific binding between the IgG4 control (IgG4-7D8 x IgG4-2F8) and the negative control (IgG1-2F8 x IgG1-7D8-K409R). Light grey bars represent results from simultaneously performed Fab-arm-exchange reactions between the indicatedIgG1-2F8-F405X mutants and IgG1-7D8-K409R or controls.

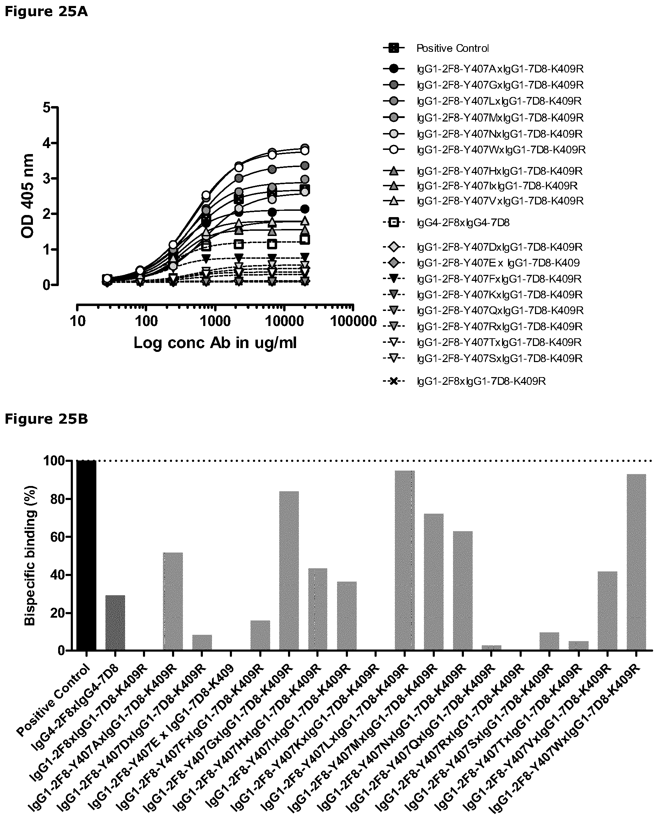

**Figure 25:** 2-MEA-induced Fab-arm-exchange between IgG1-2F8-Y407X mutants and IgG1-7D8-K409R. The generation of bispecific antibodies after 2-MEA-induced *in vitro* Fab-arm-exchange between the indicated IgG1-2F8-Y407X mutants and IgG1-7D8-K409R was determined by an ELISA. (A) A concentration series (total antibody) of 0-20 μg/mL was analyzed in the ELISA. The positive control is a purified batch of bispecific antibody, derived from IgG1-2F8-F405L x IgG1-7D8-K409R. (B) The exchange is presented as bispecific binding at 20 μg/mL antibody concentration relative to the positive control (black bar). Dark grey bars represents the bispecific binding between the IgG4 control (IgG4-7D8 x IgG4-2F8) and the negative control (IgG1-2F8 x IgG1-7D8-K409R). Light grey bars represent results from simultaneously performed Fab-arm-exchange reactions between the indicated IgG1-2F8-Y407X mutants and IgG1-7D8-K409R or controls.

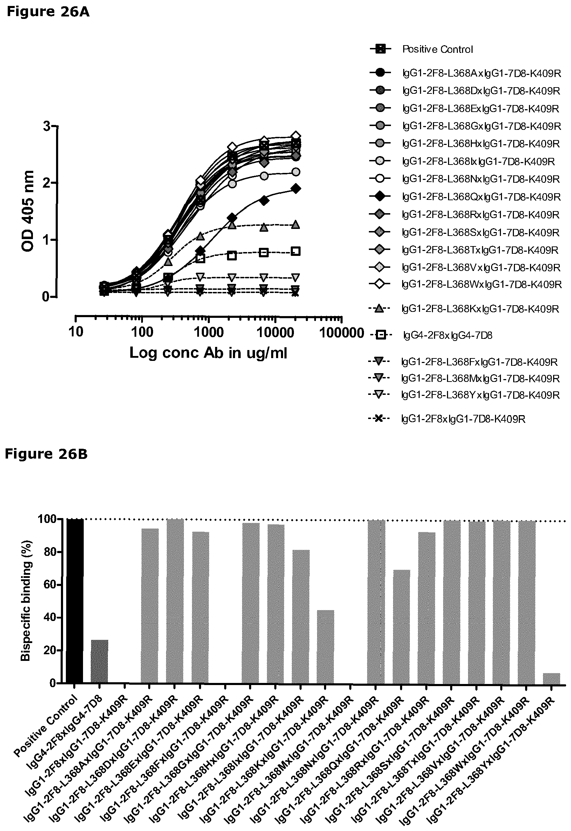

**Figure 26:** Generation of bispecific antibodies after 2-MEA-induced *in vitro* Fab-arm exchange between the indicated IgG1-2F8-L368X mutants and IgG1-7D8-K409R was determined by an ELISA using a concentration series (total antibody) of 0-20 μg/mL (A). The positive control is a purified batch of bispecific antibody, derived from IgG1-2F8-F405L x IgG1-7D8-K409R. (B) The bispecific binding at 20 μg/mL relative to the positive control (black bar). Dark grey bars represents the bispecific binding between the IgG4 control (IgG4-7D8 x IgG4-2F8) and the negative control (IgG1-2F8 x IgG1-7D8-K409R). Light grey bars represent results from simultaneously performed Fab-arm-exchange reactions between the indicated IgG1-2F8-L368X mutants and IgG1-7D8-K409R.

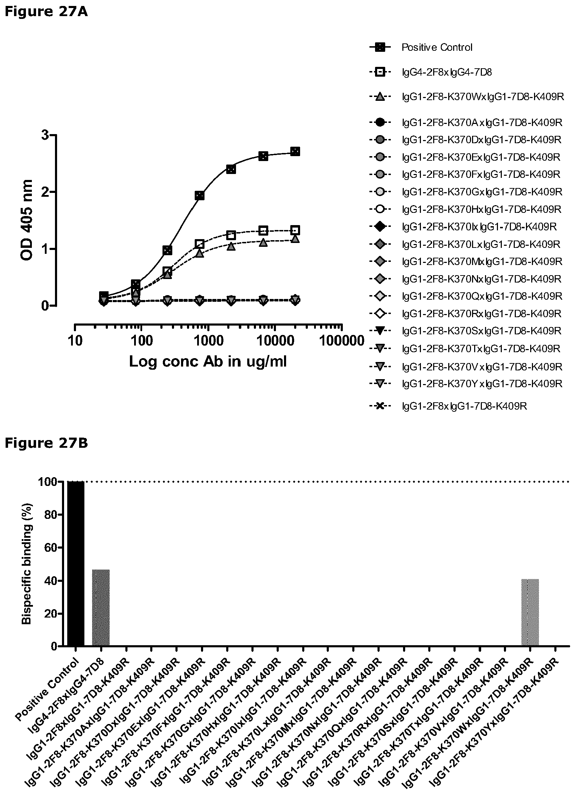

**Figure 27:** Generation of bispecific antibodies after 2-MEA-induced *in vitro* Fab-arm exchange between the indicated IgG1-2F8-K370X mutants and IgG1-7D8-K409R was determined by an ELISA using a concentration series (total antibody) of 0-20 μg/mL (A). The positive control is a purified batch of bispecific antibody, derived from IgG1-2F8-F405L x IgG1-7D8-K409R. (B) The bispecific binding at 20 μg/mL relative to the positive control (black bar). Dark grey bars represents the bispecific binding between the IgG4 control (IgG4-7D8 x IgG4-2F8) and the negative control (IgG1-2F8 x IgG1-7D8-K409R). Light grey bars represent results from simultaneously performed Fab-arm-exchange reactions between the indicated IgG1-2F8-D370X mutants and IgG1-7D8-K409R.

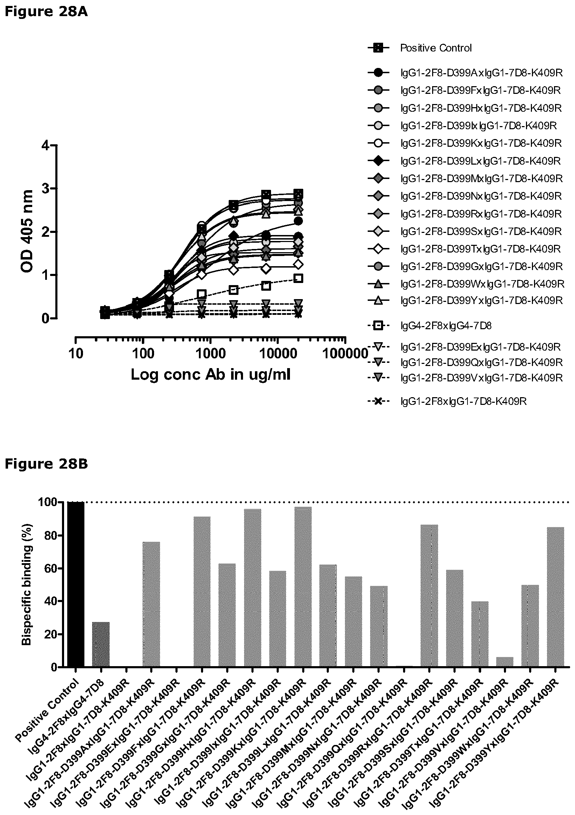

**Figure 28:** Generation of bispecific antibodies after 2-MEA-induced *in vitro* Fab-arm exchange between the indicated IgG1-2F8-D399X mutants and IgG1-7D8-K409R was determined by an ELISA using a concentration series (total antibody) of 0-20 μg/mL (A). (B) The bispecific binding at 20 μg/mL antibody concentration relative to the positive control (black bar). Dark grey bars represents the bispecific binding between the IgG4 control (IgG4-7D8 x IgG4-2F8) and the negative control (IgG1-2F8 x IgG1-7D8-K409R). Light grey bars represent results from simultaneously performed Fab-arm-exchange reactions between the indicated IgG1-2F8-D399X mutants and IgG1-7D8-K409R.

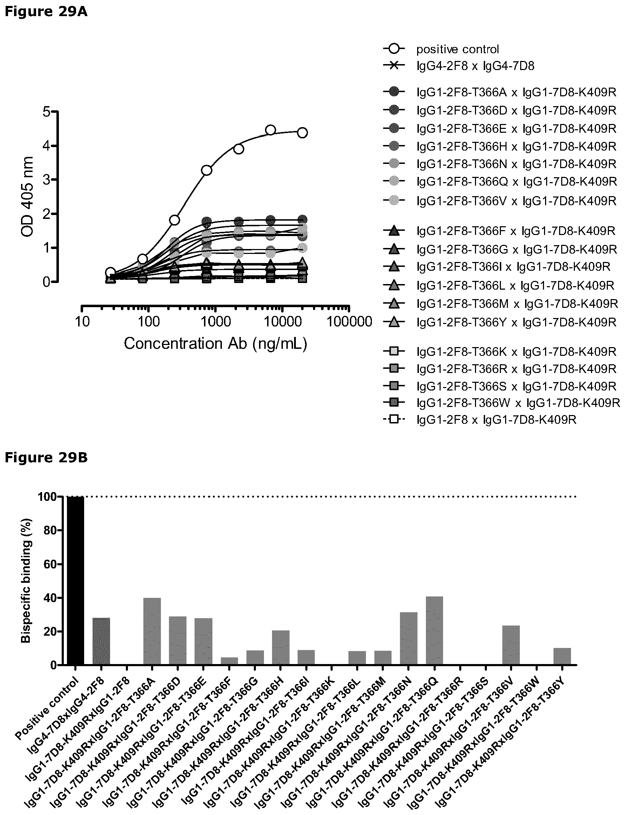

**Figure 29:** Generation of bispecific antibodies after 2-MEA-induced *in vitro* Fab-arm exchange between the indicated IgG1-2F8-T366X mutants and IgG1-7D8-K409R was determined by an ELISA using a concentration series (total antibody) of 0-20 μg/mL (A). (B) The bispecific binding at 20 μg/mL antibody concentration relative to the positive control (black bar). Dark grey bars represents the bispecific binding between the IgG4 control (IgG4-7D8 x IgG4-2F8) and the negative control (IgG1-2F8 x IgG1-7D8-K409R). Light grey bars represent results from simultaneously performed Fab-arm-exchange reactions between the indicated IgG1-2F8-T366X mutants and IgG1-7D8-K409R.

## DETAILED DESCRIPTION

### A) *Definitions*

The term "HER2" (also known as ErbB-2, NEU, HER-2, and CD340), when used herein, refers to human epidermal growth factor receptor 2 (SwissProt P04626) and includes any variants, isoforms and species homologs of HER2 which are naturally expressed by cells, including tumor cells, or are expressed on cells transfected with the HER2 gene or cDNA. Species homologs include rhesus monkey HER2 (macaca mulatta; Genbank accession No. GI:109114897).

The term "immunoglobulin" refers to a class of structurally related glycoproteins consisting of two pairs of polypeptide chains, one pair of light (L) low molecular weight chains and one pair of heavy (H) chains, all four inter-connected by disulfide bonds. The structure of immunoglobulins has been well characterized. See for instance Fundamental Immunology Ch. 7 (Paul, W., ed., 2nd ed. Raven Press, N.Y. (1989)). Briefly, each heavy chain typically is comprised of a heavy chain variable region (abbreviated herein as $V_H$ or VH) and a heavy chain constant region. The heavy chain constant region typically is comprised of three domains, $C_H1$, $C_H2$, and $C_H3$. Each light chain typically is comprised of a light chain variable region (abbreviated herein as $V_L$ or VL) and a light chain constant region. The light chain constant region typically is comprised of one domain, $C_L$. The $V_H$ and $V_L$ regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs). Each $V_H$ and $V_L$ is typically composed of three CDRs and four FRs, arranged from amino-terminus to carboxy-terminus in the following order: FR1, CDR1, FR2, CDR2, FR3, CDR3, FR4 (see also Chothia and Lesk J. Mol. Biol. 196, 901-917 (1987)). Unless otherwise stated or contradicted by context, CDR sequences herein are identified according to IMGT rules (Brochet X., Nucl Acids Res. 2008;36:W503-508 and Lefranc MP., Nucleic Acids Research 1999;27:209-212; see also internet http address imgt.cines.fr/IMGT_vquest/vquest?livret=0&Option=humanIg). However, the numbering of amino acid residues in an antibody sequence can also be performed by the method described in Kabat et al., Sequences of Proteins of Immunological Interest, 5th Ed. Public Health Service, National Institutes of Health, Bethesda, MD. (1991) (phrases such as "variable domain residue numbering as in Kabat", "Kabat position" or "according to Kabat" herein refer to this numbering system). Particularly, for numbering of amino acids in the constant region, the EU index numbering system (Kabat *et al*, supra), can be used. The Kabat numbering of residues may be determined for a given antibody as described in Kabat *et al.*, supra.

In the present invention reference to amino acid positions is, unless contradicted by the context, according to the EU-index as described in Kabat et al., Sequences of Proteins of Immunological Interest, 5th Ed. Public Health Service, National Institutes of Health, Bethesda, MD. (1991).

The term "antibody" (Ab) in the context of the present invention refers to an immunoglobulin molecule, a fragment of an immunoglobulin molecule, or a derivative of either thereof, which has the ability to specifically bind to an antigen under typical physiological conditions with a half life of significant periods of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). The variable regions of the heavy and light chains of the immunoglobulin molecule contain a binding domain that interacts with an antigen. The constant regions of the antibodies (Abs) may mediate the binding of the immunoglobulin to host tissues or factors, including various cells of the immune system (such as effector cells) and components of the complement system such as C1q, the first component in the classical pathway of complement activation. A HER2 antibody may also be a multispecific antibody, such as a bispecific antibody, diabody, or similar molecule (see for instance PNAS USA 90(14), 6444-8 (1993) for a description of diabodies). Indeed, bispecific antibodies, diabodies, and the like, provided by the present invention may bind any suitable target in addition to a portion of HER2. As indicated above, the term antibody herein, unless otherwise stated or clearly contradicted by context, includes fragments of an antibody that are antigen-binding fragments, *i.e.,* retain the ability to specifically bind to the antigen. It has been shown that the antigen-binding function of an antibody may be performed by fragments of a full-length antibody. Examples of antigen-binding fragments encompassed within the term "antibody" include (i) a Fab' or Fab fragment, a monovalent fragment consisting of the $V_L$, $V_H$, $C_L$ and $C_H1$ domains, or a monovalent antibody as described in WO2007059782 (Genmab); (ii) F(ab')$_2$ fragments, bivalent fragments comprising two Fab fragments linked by a disulfide bridge at the hinge region; (iii) a Fd fragment consisting essentially of the $V_H$ and $C_H1$ domains; (iv) a Fv fragment consisting essentially of the $V_L$ and $V_H$ domains of a single arm of an antibody, (v) a dAb fragment (Ward et al., Nature 341, 544-546 (1989)), which consists essentially of a $V_H$ domain and also called domain antibodies (Holt et al; Trends Biotechnol. 2003 Nov;21(11):484-90); (vi) camelid or nanobodies (Revets et al; Expert Opin Biol Ther. 2005 Jan;5(1):111-24) and (vii) an isolated complementarity determining region (CDR). Furthermore, although the two domains of the Fv fragment, $V_L$ and $V_H$, are coded for by separate genes, they may be joined, using recombinant methods, by a synthetic linker that enables them to be made as a single protein chain in which the $V_L$ and $V_H$ regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single chain antibodies are encompassed within the term antibody unless otherwise noted or clearly indicated by context. Although such fragments are generally included within the meaning of antibody, they collectively and each independently are unique features of the present invention, exhibiting different biological properties and utility. These and other useful antibody fragments in the context of the present invention, as well as bispecific formats of such fragments, are discussed further herein. It also should be understood that the term antibody, unless specified otherwise, also includes polyclonal antibodies, monoclonal antibodies (mAbs), antibody-like polypeptides, such as chimeric antibodies and humanized antibodies, and antibody fragments retaining the ability to specifically bind to the antigen (antigen-binding fragments) provided by any known technique, such as enzymatic cleavage, peptide synthesis, and recombinant techniques. An antibody as generated can possess any isotype.

The term "bispecific antibody" is in the context of the present invention to be understood as an antibody with two different antigen-binding regions (based on sequence information). This can mean different target binding but includes as well binding to different epitopes in one target.

When used herein, unless contradicted by context, the term "Fab-arm" or "arm" refers to one heavy chain-light chain pair.

When used herein, unless contradicted by context, the term "Fc region" refers to an antibody region comprising at least a hinge region, CH2 domain, and a CH3 domain.

As used herein, "isotype" refers to the immunoglobulin class (for instance IgG1, IgG2, IgG3, IgG4, IgD, IgA, IgE, or IgM) that is encoded by heavy chain constant region genes.

The term "monovalent antibody" means in the context of the present invention that an antibody molecule is capable of binding a single molecule of the antigen, and thus is not able of antigen crosslinking.

An "antibody deficient in effector function" or an "effector-function-deficient antibody" refers to an antibody which has a significantly reduced or no ability to activate one or more effector mechanisms, such as complement activation or Fc receptor binding. Thus, effector-function deficient antibodies have significantly reduced or no ability to mediate antibody-dependent cell-mediated cytotoxicity (ADCC) and/or complement-dependent cytotoxicity (CDC). An example of such an antibody is IgG4. Another example is the introduction of mutations in Fc-region which can strongly reduce the interaction with complement proteins and Fc-receptors. See, for example, Bolt S et al., Eur J Immunol 1993, 23:403-411; Oganesyan, Acta Crys. 2008, D64, 700-704; and Shields et al., JBC 2001, 276: 6591-6604.

A "HER2 antibody" or "anti-HER2 antibody" is an antibody as described above, which binds specifically to the antigen HER2.

A "HER2xHER2 antibody" or "anti-HER2xHER2 antibody" is a multispecific antibody, optionally a bispecific antibody, which comprises two different antigen-binding regions, both of which bind specifically to the antigen HER2, optionally to different HER2 epitopes.

The term "human antibody", as used herein, is intended to include antibodies having variable and constant regions derived from human germline immunoglobulin sequences. The human antibodies of the invention may include amino acid residues not encoded by human germline immunoglobulin sequences (*e.g.*, mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted onto human framework sequences.

As used herein, a human antibody is "derived from" a particular germline sequence if the antibody is obtained from a system using human immunoglobulin sequences, for instance by immunizing a transgenic mouse carrying human immunoglobulin genes or by screening a human immunoglobulin gene library, and wherein the selected human antibody is at least 90%, such as at least 95%, for instance at least 96%, such as at least 97%, for instance at least 98%, or such as at least 99% identical in amino acid sequence to the amino acid sequence encoded by the germline immunoglobulin gene. Typically, outside the heavy chain CDR3, a human antibody derived from a particular human germline sequence will display no more than 20 amino acid differences, *e.g.* no more than 10 amino acid differences, such as no more than 9, 8, 7, 6 or 5, for instance no more than 4, 3, 2, or 1 amino acid difference from the amino acid sequence encoded by the germline immunoglobulin gene.

When used herein, the term "heavy chain antibody" or "heavy-chain antibody" refers to an antibody which consists only of two heavy chains and lacks the two light chains usually found in antibodies. Heavy chain antibodies, which naturally occur in *e.g.* camelids, can bind antigens despite their lack of VL domains.

The antibody of the invention may be isolated. An "isolated antibody," as used herein, is intended to refer to an antibody which is substantially free of other antibodies having different antigenic specificities (for instance an isolated antibody that specifically binds to HER2 is substantially free of antibodies that specifically bind antigens other than HER2). An isolated antibody that specifically binds to an epitope, isoform or variant of HER2 may, however, have cross-reactivity to other related antigens, for instance from other species (such as HER2 species homologs). Moreover, an isolated antibody may be substantially free of other cellular material and/or chemicals. In one embodiment of the present invention, two or more "isolated" monoclonal antibodies having different antigen-binding specificities are combined in a well-defined composition.

When used herein in the context of two or more antibodies, the term "competes with" or "cross-competes with" indicates that the two or more antibodies compete for binding to HER2, *e.g.* compete for HER2 binding in the assay described in Example 14. An antibody "blocks" or "cross-blocks" one or more other antibodies from binding to HER2 if the antibody competes with the one or more other antibodies 25% or more, with 25%-74% representing "partial block" and 75%-100% representing "full block", preferably as determined using the assay of Example 14. For some pairs of antibodies, competition or blocking in the assay of the Examples is only observed when one antibody is coated on the plate and the other is used to compete, and not vice versa. Unless otherwise defined or negated by context, the terms "competes with", "cross-competes with", "blocks" or "cross-blocks" when used herein is also intended to cover such pairs of antibodies.

The term "epitope" means a protein determinant capable of specific binding to an antibody. Epitopes usually consist of surface groupings of molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents. The epitope may comprise amino acid residues directly involved in the binding and other amino acid residues, which are not directly involved in the binding, such as amino acid residues which are effectively blocked or covered by the specifically antigen binding peptide (in other words, the amino acid residue is within the footprint of the specifically antigen binding peptide).

The term "monoclonal antibody" as used herein refers to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human monoclonal antibodies may be generated by a hybridoma which includes a B cell obtained from a transgenic or transchromosomal nonhuman animal, such as a transgenic mouse, having a genome comprising a human heavy chain transgene and a light chain transgene, fused to an immortalized cell.

As used herein, the term "binding" in the context of the binding of an antibody to a predetermined antigen or epitope typically is a binding with an affinity corresponding to a $K_D$ of about $10^{-7}$ M or less, such as about $10^{-8}$ M or less, such as about $10^{-9}$ M or less, about $10^{-10}$ M or less, or about $10^{-11}$ M or even less when determined by for instance surface plasmon resonance (SPR) technology in a BIAcore 3000 instrument using the antigen as the ligand and the antibody as the analyte, and binds to the predetermined antigen with an affinity corresponding to a $K_D$ that is at least ten-fold lower, such as at least 100 fold lower, for instance at least 1,000 fold lower, such as at least 10,000 fold lower, for instance at least 100,000 fold lower than its affinity for binding to a non-specific antigen (*e.g.*, BSA, casein) other than the predetermined antigen or a closely-related antigen. The amount with which the affinity is lower is dependent on the $K_D$ of the antibody, so that when the $K_D$ of the antibody is very low (that is, the antibody is highly specific), then the amount with which the affinity for the antigen is lower than the affinity for a non-specific antigen may be at least 10,000 fold.

The term "$k_d$" ($sec^{-1}$), as used herein, refers to the dissociation rate constant of a particular antibody-antigen interaction. Said value is also referred to as the $k_{off}$ value.

The term "$k_a$" ($M^{-1}$ x $sec^{-1}$), as used herein, refers to the association rate constant of a particular antibody-antigen interaction.

The term "$K_D$" (M), as used herein, refers to the dissociation equilibrium constant of a particular antibody-antigen interaction.

The term "KA" ($M^{-1}$), as used herein, refers to the association equilibrium constant of a particular antibody-antigen interaction and is obtained by dividing the $k_a$ by the $k_d$.

When used herein the term "heterodimeric interaction between the first and second CH3 regions" refers to the interaction between the first CH3 region and the second CH3 region in a first-CH3/second-CH3 heterodimeric protein.

When used herein the term "homodimeric interactions of the first and second CH3 regions" refers to the interaction between a first CH3 region and another first CH3 region in a first-CH3/first-CH3 homodimeric protein and the interaction between a second CH3 region and another second CH3 region in a second-CH3/second-CH3 homodimeric protein.

The term "reducing conditions" or "reducing environment" refers to a condition or an environment in which a substrate, here a cysteine residue in the hinge region of an antibody, is more likely to become reduced than oxidized.

As used herein, the term "inhibits proliferation" (*e.g.* referring to cells, such as tumor cells) is intended to include any substantial decrease in the cell proliferation when contacted with a HER2 antibody as compared to the proliferation of the same cells not in contact with a HER2 antibody, *e.g.*, the inhibition of proliferation of a cell culture by at least about 10%, at least about 20% or at least about 30%, or at least as much as a reference antibody such as trastuzumab, *e.g.,* as determined by an assay in the Examples, *e.g.* Example 16.

As used herein, the term "promotes proliferation" (*e.g.* referring to cells, such as tumor cells) is intended to include any substantial increase in the cell proliferation when contacted with a HER2 antibody as compared to the proliferation of the same cells not in contact with a HER2 antibody, *e.g.*, the promotion of proliferation of a cell culture by at least about 10%, at least about 20% or at least about 30%, or at least as much as a reference antibody as F5, *e.g.*, as determined by an assay in the Examples.

As used herein, the term "internalization", when used in the context of a HER2 antibody includes any mechanism by which the antibody is internalized into a HER2-expressing cell from the cell-surface and/or from surrounding medium, *e.g.*, via endocytosis. The internalization of an antibody can be evaluated using a direct assay measuring the amount of internalized antibody (such as, *e.g.*, the fab-CypHer5E assay described in Example 19), or an indirect assay where the effect of an internalized antibody-toxin conjugate is measured (such as, *e.g.*, the anti-kappa-ETA'assay of Example 18).

The present invention also provides antibodies comprising functional variants of the $V_L$ region, $V_H$ region, or one or more CDRs of the antibodies of the examples. A functional variant of a $V_L$, $V_H$, or CDR used in the context of a HER2 antibody still allows the antibody to retain at least a substantial proportion (at least about 50%, 60%, 70%, 80%, 90%, 95% or more) of the affinity/avidity and/or the specificity/selectivity of the parent antibody and in some cases such a HER2 antibody may be associated with greater affinity, selectivity and/or specificity than the parent antibody.

Such functional variants typically retain significant sequence identity to the parent antibody. The percent identity between two sequences is a function of the number of identical positions shared by the sequences (*i.e.*, % homology = # of identical positions/total # of positions x 100), taking into account the number of gaps, and the length of each gap, which need to be introduced for optimal alignment of the two sequences. The percent identity between two nucleotide or amino acid sequences may *e.g.* be determined using the algorithm of E. Meyers and W. Miller, Comput. Appl. Biosci 4, 11-17 (1988) which has been incorporated into the ALIGN program (version 2.0), using a PAM120 weight residue table, a gap length penalty of 12 and a gap penalty of 4. In addition, the percent identity between two amino acid sequences may be determined using the Needleman and Wunsch, J. Mol. Biol. 48, 444-453 (1970) algorithm.

Exemplary variants include those which differ from a parent antibody VH and/or VL sequence shown in Figures 1 and 2 at one or more "variant" amino acid positions, denoted "X" in the corresponding consensus sequence. Preferred variants are those in which the new amino acid is selected from those at the corresponding position in one of the aligned sequences in Figure 1 or 2 (for details on CDR sequence variants, see Table 4). Alternatively or additionally, the sequence of VH, VL or CDR variants may differ from the sequence of the VH, VL or CDR of the parent antibody sequences mainly by conservative substitutions; for instance at least 10, such as at least 9, 8, 7, 6, 5, 4, 3, 2 or 1 of the substitutions in the variant are conservative amino acid residue replacements.

In the context of the present invention, conservative substitutions may be defined by substitutions within the classes of amino acids reflected in the following table:

| Amino acid residue classes for conservative substitutions | |
|---|---|
| Acidic Residues | Asp (D) and Glu (E) |
| Basic Residues | Lys (K), Arg (R), and His (H) |

(continued)

| Amino acid residue classes for conservative substitutions | |
| --- | --- |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

In the context of the present invention the following notations are, unless otherwise indicated used to describe a mutation; i) substitution of an amino acid in a given position is written as *e.g.* K405R which means a substitution of a lysine in position 405 with an arginine; and ii) for specific variants the specific three or one letter codes are used, including the codes Xaa and X to indicate any amino acid residue. Thus, the substitution of Arginine for Lysine in position 405 is designated as: K405R, or the substitution of any amino acid residue for Lysine in position 405 is designated as K405X. In case of deletion of Lysine in position 405 it is indicated by K405*.
The term "recombinant host cell" (or simply "host cell"), as used herein, is intended to refer to a cell into which an expression vector has been introduced, *e.g.* an expression vector encoding an antibody of the invention. Recombinant host cells include, for example, transfectomas, such as CHO cells, HEK293 cells, NS/0 cells, and lymphocytic cells.

The term "transgenic non-human animal" refers to a non-human animal having a genome comprising one or more human heavy and/or light chain transgenes or transchromosomes (either integrated or non-integrated into the animal's natural genomic DNA) and which is capable of expressing fully human antibodies. For example, a transgenic mouse can have a human light chain transgene and either a human heavy chain transgene or human heavy chain transchromosome, such that the mouse produces human HER2 antibodies when immunized with HER2 antigen and/or cells expressing HER2. The human heavy chain transgene may be integrated into the chromosomal DNA of the mouse, as is the case for transgenic mice, for instance HuMAb® mice, such as HCo7, HCo12, or HCo17 mice, or the human heavy chain transgene may be maintained extrachromosomally, as is the case for transchromosomal KM mice as described in WO02/43478. Similar mice, having a larger human Ab gene repertoire, include HCo7 and HCo20 (see *e.g.* WO2009097006). Such transgenic and transchromosomal mice (collectively referred to herein as "transgenic mice") are capable of producing multiple isotypes of human monoclonal antibodies to a given antigen (such as IgG, IgA, IgM, IgD and/or IgE) by undergoing V-D-J recombination and isotype switching. Transgenic, nonhuman animal can also be used for production of antibodies against a specific antigen by introducing genes encoding such specific antibody, for example by operatively linking the genes to a gene which is expressed in the milk of the animal.

"Treatment" refers to the administration of an effective amount of a therapeutically active compound of the present invention with the purpose of easing, ameliorating, arresting or eradicating (curing) symptoms or disease states.

An "effective amount" or "therapeutically effective amount" refers to an amount effective, at dosages and for periods of time necessary, to achieve a desired therapeutic result. A therapeutically effective amount of a HER2 antibody may vary according to factors such as the disease state, age, sex, and weight of the individual, and the ability of the HER2 antibody to elicit a desired response in the individual. A therapeutically effective amount is also one in which any toxic or detrimental effects of the antibody or antibody portion are outweighed by the therapeutically beneficial effects.

An "anti-idiotypic" antibody is an antibody which recognizes unique determinants generally associated with the antigen-binding site of an antibody.

## B) *Technical teachings*

The technical information set out below may in some respects go beyond the disclosure of the invention, which is defined exclusively by the appended claims. The additional technical information is provided to place the actual invention in a broader technical context and to illustrate possible related technical developments. Such additional technical information which does not fall within the scope of the appended claims, is not part of the invention.

The present disclosure relates to a bispecific antibody comprising two different antigen-binding regions which bind HER2.

The disclosure relates to a bispecific molecule comprising a first antigen binding site from a HER2 antibody described herein and a second antigen binding site from a HER2 antibody described herein with a different binding specificity, such as a binding specificity for a non-overlapping epitope of HER2, *i.e.* a bispecific antibody wherein the first and second antigen binding regions do not cross-block each other for binding to HER2, *e.g.* when tested as described in Example 14.

The bispecific antibody may comprise at least one antigen-binding region from an antibody of cross-block group 1, 2, 3 or 4, described below. The bispecific antibody may comprise at least one antigen-binding region from an antibody cross-blocking or binding to the same epitope as a reference antibody selected from cross-block groups 1, 2, 3 and 4, *e.g.*, cross-block group 4. The bispecific antibody may comprise two different antigen-binding regions from the antibodies of cross-block groups 1, 2, 3 and 4, optionally from different cross-block groups. The bispecific antibody may comprise two different antigen-binding regions from antibodies which each cross-block or bind to the same epitope as a reference antibody of cross-block groups 1, 2, 3 and 4, optionally from different cross-block groups. For example, the bispecific antibody may comprise one antigen-binding region from an antibody of cross-block group 1, 2, 3 or 4, and one antigen-binding region from trastuzumab or pertuzumab.

Thus the bispecific antibody of the present disclosure may comprise a first antigen-binding region and a second antigen-binding region, which first and second antigen-binding regions bind different epitopes on human epidermal growth factor receptor 2 (HER2).

The first and second antigen-binding region of the bispecific antibody of the present disclosurei may be an antigen-binding region from any of cross-block groups 1, 2, 3, and 4. The bispecific antibody of the present disclosurei comprises two different antigen-binding regions which bind HER2. Furthermore, as described below one method of producing a

bispecific antibody of the present invention is based on incubating a first and a second HER2 antibody under reducing conditions.

The antigen-binding regions of a bispecific antibody of the present disclosurei and the antigen-binding region of a first or second HER2 antibody of the present disclosurei may belong to any of cross-block groups 1, 2, 3 and 4 described herein. Thus a first or second HER2 antibody of the present disclosure may comprise an antigen-binding region of any of the HER2 antibodies of cross-block groups 1, 2, 3 and 4, which are described below.

The bispecific antibody may comprise an antigen-binding region of one or more of the human antibodies of cross-blocks 1, 2, 3, or 4, which blocks the binding to HER2.

The bispecific antibody may comprise an antigen-binding region which blocks binding to the same epitope on soluble HER2 as one or more of the human antibodies of cross-blocks 1, 2, 3, or 4.

The bispecific antibody may comprise an antigen-binding region which binds to the same epitope on HER2 as one or more of the human antibodies of cross-blocks 1, 2, 3, or 4.

HER2 antibodies and/or antigen binding regions

*Cross-block group 1*

The bispecific antibody of the disclosurei may comprise one antigen-binding region which blocks the binding to HER2, e.g. soluble HER2, of one or more of the human antibodies of cross-block group 1 described herein, or binds the same epitope on HER2 as one or more of the human antibodies of cross-block group 1 described herein.

The bispecific antibody may then comprise a second antigen-binding region which cross-blocks or binds to the same epitope as an antibody of cross-block groups 2, 3, or 4.

The antigen-binding region may cross-block the binding to soluble HER2 of trastuzumab, when determined as described in Example 14.

The antigen-binding region may block the binding to HER2, e.g soluble HER2, or may bind the same epitope as a reference antibody comprising a VH region comprising the sequence of SEQ ID NO:1 and a VL region comprising the sequence of SEQ ID NO:5 (**169**).

The antigen-binding region may block the binding to HER2, e.g soluble HER2, or bind the same epitope as a reference antibody comprising a VH region comprising the sequence of SEQ ID NO:8 and a VL region comprising the sequence of SEQ ID NO:12 (**050**).

The antigen-binding region may block the binding to HER2, e.g soluble HER2, or bind the same epitope as a reference antibody comprising a VH region comprising the sequence of SEQ ID NO:15 and a VL region comprising the sequence of SEQ ID NO:19 **(084).**

The antigen-binding region may block the binding to HER2, e.g soluble HER2, or binds to the same epitope as a reference antibody comprising VH and VL regions selected from the group consisting of:

a) a VH region comprising the sequence of SEQ ID NO:77 and a VL region comprising the sequence of SEQ ID NO:78 (**049**);
b) a VH region comprising the sequence of SEQ ID NO:79 and a VL region comprising the sequence of SEQ ID NO:80 (**051**);
c) a VH region comprising the sequence of SEQ ID NO:81 and a VL region comprising the sequence of SEQ ID NO:82 (**055**);
d) a VH region comprising the sequence of SEQ ID NO:83 and a VL region comprising the sequence of SEQ ID NO:84 (**123**);

e) a VH region comprising the sequence of SEQ ID NO:85 and a VL region comprising the sequence of SEQ ID NO:86 (**161**); and

f) a VH region comprising the sequence of SEQ ID NO:87 and a VL region comprising the sequence of SEQ ID NO:88 (**124**).

In the bispecific antibody of the disclosurei, one antigen-binding region may bind to HER2 and comprises a VH CDR3, VH region and/or VL region sequence similar or identical to such a sequence of an antibody described herein.

The antigen-binding region may comprise a VH CDR3 region having a sequence selected from the group consisting of

SEQ ID NO:11 (**050, 049, 051, 055**), optionally wherein the VH region is derived from the IgHV3-21-1 germline sequence;

SEQ ID No: 130, such as the sequence of SEQ ID NO:18 (**084**), optionally wherein the VH region is derived from the IgHV1-69-04 germline sequence;

SEQ ID NO:133 (**169, 123, 161, 124**), such as the sequence of SEQ ID NO:4 (**169**), optionally wherein the VH region is derived from the IgHV1-18-1 germline sequence; or

The antigen-binding region may comprise a VH CDR3 region of one of antibodies 123, 161, or 124, as shown in Figure 1, optionally wherein the VH region is derived from an IgHV1-18-1 germline.

The bispecific antibody or antigen-binding region may comprise a VH region selected from the group consisting of

a) a VH region comprising the CDR1, CDR2 and CDR3 sequences of SEQ ID NOs:9, 127 and 11, such as the CDR1, CDR2 and CDR3 sequences of SEQ ID NOS: 9, 10 and 11 (**050**); optionally where the VH region is derived from an IgHV3-23-1 germline;

b) a VH region comprising the CDR1, CDR2 and CDR3 sequences of SEQ ID NOs:128, 129 and 130, such the CDR1, CDR2 and CDR3 sequences of SEQ ID NOs: 16, 17 and 18, respectively (**084**), optionally where the VH region is derived from an IgHV1-69-04 germline; and

c) a VH region comprising the CDR1, CDR2, and CDR3 sequences of SEQ ID NOs:131, 132, and 133, such as the CDR1, CDR2, and CDR3 sequences of SEQ ID NOs: 2, 3 and 4 (**169**), respectively, optionally where the VH region is derived from an IgHV1-18-1 germline.

The bispecific antibody or antigen-binding region may comprise a VH region selected from the preceding embodiments (a) or (b) and a VL region comprising the CDR1, CDR2, and CDR3 sequences of SEQ ID NO:13, XAS (wherein X is A or V), and SEQ ID No: 155, respectively, such as a CDR1 sequence selected from SEQ ID Nos: 13 or 20, a CDR2 which is AAS or VAS, and a CDR3 sequence selected from SEQ ID NOs:14 and 21 (**050, 084**); respectively, optionally where the VL region is derived from an IgKV1-12-01 germline.

The bispecific antibody or antigen-binding region may comprise a VH region which is the preceding embodiment (c) and a VL region comprising the CDR1, CDR2, and CDR3 sequences of SEQ ID NO:6, DXS (wherein X=A or T), and SEQ ID NO:156 (**169**), respectively, optionally wherein the VL region is derived from IgKV3-11-01.

The bispecific antibody or antigen-binding region may comprise a VH region comprising the CDR1, CDR2 and CDR3 sequences of SEQ ID NOs:2, 3 and 4, respectively; and a VL region comprising the CDR1, CDR2 and CDR3 sequences of SEQ ID NOs:6, DAS, and SEQ ID NO:7, respectively (**169**).

The bispecific antibody or antigen-binding region may comprise a VH region comprising the CDR1, CDR2 and CDR3 sequences of SEQ ID NOs:9, 10 and 11, respectively; and a VL region comprising the CDR1, CDR2 and CDR3 sequences of SEQ ID NOs:13, AAS, and SEQ ID NO:14, respectively (**050**).

The bispecific antibody or antigen-binding region may comprise a VH region comprising the CDR1, CDR2 and CDR3 sequences of SEQ ID NOs:16, 17 and 18, respectively; and a VL region comprising the CDR1, CDR2 and CDR3 sequences of SEQ ID NOs:20, VAS, and SEQ ID NO:21, respectively (**084**).

The bispecific antibody or antigen-binding region may comprise:

a) a VH region comprising the sequence of SEQ ID NO:1 and, preferably, a VL region comprising the sequence of SEQ ID NO:5 (**169**);

b) a VH region comprising the sequence of SEQ ID NO:8 and, preferably, a VL region comprising the sequence of SEQ ID NO:12 (**050**);

c) a VH region comprising the sequence of SEQ ID NO:15 and, preferably, a VL region comprising the sequence of SEQ ID NO:19 (**084**);

d) a VH region comprising the sequence of SEQ ID NO:77 and, preferably, a VL region comprising the sequence of SEQ ID NO:78 (**049**);

e) a VH region comprising the sequence of SEQ ID NO:79 and, preferably, a VL region comprising the sequence of SEQ ID NO:80 (**051**);

f) a VH region comprising the sequence of SEQ ID NO:81 and, preferably, a VL region comprising the sequence of SEQ ID NO:82 (**055**);

g) a VH region comprising the sequence of SEQ ID NO:83 and, preferably, a VL region comprising the sequence of SEQ ID NO:84 (**123**);

h) a VH region comprising the sequence of SEQ ID NO:85 and, preferably, a VL region comprising the sequence of SEQ ID NO:86 (**161**);

i) a VH region comprising the sequence of SEQ ID NO:87 and, preferably, a VL region comprising the sequence of SEQ ID NO:88 (**124**); and/or

j) a variant of any of said antibodies or antigen-binding regions, wherein said variant preferably has at most 1, 2 or 3 amino-acid modifications, more preferably amino-acid substitutions, such as conservative amino acid substitutions and substitutions where the new amino acid is one at the same position in an aligned sequence in Figures 1 or 2, particularly at positions indicated by "X" in the corresponding consensus sequence.

*Cross-block group 2*

The bispecific antibody of the disclosure may comprise an antigen-binding region which blocks the binding to HER2 of one or more of the human antibodies of cross-block group 2 described herein, or binds the same epitope on HER2 as one or more of the human antibodies of cross-block group 2 described herein.

The bispecific antibody may then comprise a second antigen-binding region which cross-blocks, blocks the binding to HER2, e.g soluble HER2, or binds to the same epitope as an antibody of cross-block groups 1, 3, or 4.

The antigen-binding region may cross-block the binding to soluble HER2 of pertuzumab, when determined as described in Example 14.

The antigen-binding region may block the binding to soluble HER2, e.g soluble HER2, or bind the same epitope as a reference antibody comprising a VH region comprising the sequence of SEQ ID NO:22 and a VL region comprising the sequence of SEQ ID NO:26 (**025**).

The antigen-binding region may block the binding to soluble HER2, e.g soluble HER2, or bind the same epitope as a reference antibody comprising a VH region comprising the sequence of SEQ ID NO:29 and a VL region comprising the sequence of SEQ ID NO:32 (**091**).

The antigen-binding region may block the binding to soluble HER2, e.g soluble HER2, or bind the same epitope as a reference antibody comprising a VH region comprising the sequence of SEQ ID NO:35 and a VL region comprising the sequence of SEQ ID NO:39 (**129**).

The antigen-binding region may block the binding to soluble HER2, e.g soluble HER2, or bind to the same epitope as a reference antibody comprising VH and VL regions selected from the group consisting of:

a) a VH region comprising the sequence of SEQ ID NO:89 and a VL region comprising the sequence of SEQ ID NO:90 (**001**);

b) a VH region comprising the sequence of SEQ ID NO:91 and a VL region comprising the sequence of SEQ ID NO:92 (**143**);

c) a VH region comprising the sequence of SEQ ID NO:93 and a VL region comprising the sequence of SEQ ID NO:94 (**019**);

d) a VH region comprising the sequence of SEQ ID NO:95 and a VL region comprising the sequence of SEQ ID NO:96 (**021**);

e) a VH region comprising the sequence of SEQ ID NO:97 and a VL region comprising the sequence of SEQ ID NO:98 (**027**);

f) a VH region comprising the sequence of SEQ ID NO:99 and a VL region comprising the sequence of SEQ ID NO:100 (**032**)

g) a VH region comprising the sequence of SEQ ID NO:101 and a VL region comprising the sequence of SEQ ID NO:102 (**035**);

h) a VH region comprising the sequence of SEQ ID NO:103 and a VL region comprising the sequence of SEQ ID NO:104 (**036**);

i) a VH region comprising the sequence of SEQ ID NO:105 and a VL region comprising the sequence of SEQ ID NO:106 (**054**); and

j) a VH region comprising the sequence of SEQ ID NO:107 and a VL region comprising the sequence of SEQ ID NO:108 (**094**).

Alternatively or additionally, the bispecific antibody or antigen-binding region may comprise a VH CDR3, VH region and/or VL region sequence similar or identical to a sequence of the novel antibodies described herein.

The bispecific antibody or antigen-binding region may comprise a VH CDR3 region having a sequence selected from the group consisting of

SEQ ID NO:136, such as the sequence of SEQ ID NO:25 (**025**), optionally wherein the VH region is derived from the IgHV4-34-1 germline sequence;
SEQ ID NO:139, such as the sequence of SEQ ID NO:31 (**091**), optionally wherein the VH region is derived from the IgHV4-34-01 germline sequence; and
SEQ ID NO:142, such as the sequence of SEQ ID NO:38 (**129**), optionally wherein the VH region is derived from the IgHV3-30-01 germline sequence.

The bispecific antibody or antigen-binding region may comprise a VH CDR3 region of one of antibodies 001, 143, 019, 021, 027, 032, 035, 036, 054 or 094 as shown in Figure 1, optionally wherein the VH region is derived from an IgHV4-34-1 germline.

The bispecific antibody or antigen-binding region may comprise a VH region selected from the group consisting of

a) a VH region comprising the CDR1, CDR2 and CDR3 sequences of SEQ ID NOs:134, 135 and 136, such as the CDR1, CDR2 and CDR3 sequences of SEQ ID NOS: 23, 24 and 25 (**025**); optionally where the VH region is derived from an IgHV4-34-1 germline;
b) a VH region comprising the CDR1, CDR2 and CDR3 sequences of SEQ ID NOs:137, 138 and 139, such the CDR1, CDR2 and CDR3 sequences of SEQ ID NOs:30, 163, and 31, respectively (**091**), optionally where the VH region is derived from an IgHV4-34-01 germline; and
c) a VH region comprising the CDR1, CDR2, and CDR3 sequences of SEQ ID NOs:140, 141 and 142, such as the CDR1, CDR2, and CDR3 sequences of SEQ ID NOs: 36, 37 and 38 (**129**), respectively, optionally where the VH region is derived from an IgHV3-30-01 germline.

The bispecific antibody or antigen-binding region may comprise a VH region selected from the preceding embodiment (a) and a VL region comprising the CDR1, CDR2, and CDR3 sequences of SEQ ID NO:157, AAS, and SEQ ID No: 164, respectively, such as the CDR1, CDR2, and CDR3 sequences of SEQ ID Nos:27, AAS, and SEQ ID NO:28 (**025**);
respectively, optionally where the VL region is derived from an IgKV1D-16-01 germline.

The bispecific antibody or antigen-binding region may comprise a VH region selected from the preceding embodiment (b) and a VL region comprising the CDR1, CDR2, and CDR3 sequences of SEQ ID NO:33, $AX_1X_2$ (wherein $X_1$ is A or T, preferably A; and $X_2$ is S or F, preferably S), and SEQ ID No: 158, respectively, such as the CDR1, CDR2 and CDR3 sequences of SEQ ID Nos:33, AAS, and SEQ ID NO:34 (**091**); respectively, optionally where the VL region is derived from an IgKV1D-16-01 germline.

The bispecific antibody or antigen-binding region may comprise a VH region which is the preceding embodiment (c) and a VL region comprising the CDR1, CDR2, and CDR3 sequences of SEQ ID NO:40, DAS and SEQ ID NO:41 (**129**), respectively, optionally wherein the VL region is derived from IgKV3-11-01.

The bispecific antibody or antigen-binding region may comprise a VH region comprising the CDR1, CDR2 and CDR3 sequences of SEQ ID NOs:23, 24 and 25, respectively; and a VL region comprising the CDR1, CDR2 and CDR3 sequences of SEQ ID NOs:27, AAS, and SEQ ID NO:28, respectively (**025**).

The bispecific antibody or antigen-binding region may comprise a VH region comprising the CDR1, CDR2 and CDR3 sequences of SEQ ID NOs:30, 163 and 31, respectively; and a VL region comprising the CDR1, CDR2 and CDR3 sequences of SEQ ID NOs:33, AAS, and SEQ ID NO:34, respectively (**091**).

The bispecific antibody or antigen-binding region may comprise a VH region comprising the CDR1, CDR2 and CDR3 sequences of SEQ ID NOs:36, 37 and 38, respectively; and a VL region comprising the CDR1, CDR2 and CDR3 sequences of SEQ ID NOs:40, DAS, and SEQ ID NO:41, respectively (**129**).

The bispecific antibody or antigen-binding region may comprises:

a) a VH region comprising the sequence of SEQ ID NO:22 and, preferably, a VL region comprising the sequence of SEQ ID NO:26 (**025**);
b) a VH region comprising the sequence of SEQ ID NO:29 and, preferably, a VL region comprising the sequence of SEQ ID NO:32 (**091**);
c) a VH region comprising the sequence of SEQ ID NO:35 and, preferably, a VL region comprising the sequence of SEQ ID NO:39 (**129**);
d) a VH region comprising the sequence of SEQ ID NO:89 and, preferably, a VL region comprising the sequence of SEQ ID NO:90 (**001**);

e) a VH region comprising the sequence of SEQ ID NO:91 and, preferably, a VL region comprising the sequence of SEQ ID NO:92 (**143**);

f) a VH region comprising the sequence of SEQ ID NO:93 and, preferably, a VL region comprising the sequence of SEQ ID NO:94 (**019**);

g) a VH region comprising the sequence of SEQ ID NO:95 and, preferably, a VL region comprising the sequence of SEQ ID NO:96 (**021**);

h) a VH region comprising the sequence of SEQ ID NO:97 and, preferably, a VL region comprising the sequence of SEQ ID NO:98 (**027**);

i) a VH region comprising the sequence of SEQ ID NO:99 and, preferably, a VL region comprising the sequence of SEQ ID NO:100 (**032**);

j) a VH region comprising the sequence of SEQ ID NO:101 and, preferably, a VL region comprising the sequence of SEQ ID NO:102 (**035**);

k) a VH region comprising the sequence of SEQ ID NO:103 and, preferably, a VL region comprising the sequence of SEQ ID NO:104 (**036**);

l) a VH region comprising the sequence of SEQ ID NO:105 and, preferably, a VL region comprising the sequence of SEQ ID NO:106 (**054**);

m) a VH region comprising the sequence of SEQ ID NO:106 and, preferably, a VL region comprising the sequence of SEQ ID NO:108 (**094**); and/or

n) a variant of any of said antibodies, wherein said variant preferably has at most 1, 2 or 3 amino-acid modifications, more preferably amino-acid substitutions, such as conservative amino acid substitutions and substitutions where the new amino acid is one at the same position in an aligned sequence in Figures 1 or 2, particularly at positions indicated by "X" in the corresponding consensus sequence.

*Cross-block group 3*

IThe bispecific antibody may comprise an antigen-binding region which blocks the binding to HER2 of one or more of the human antibodies of cross-block group 3 described herein or binds the same epitope on HER2 as one or more of the human antibodies of cross-block group 3 described herein. The bispecific antibody may then comprise a second antigen-binding region which cross-blocks, blocks the binding to HER2, e.g soluble HER2, or binds to the same epitope as an antibody of cross-block groups 1, 2, or 4.

The antigen-binding region may cross-block the binding to soluble HER2 of F5 and/or C1, when determined as described in Example 14.

The antigen-binding region may block the binding to HER2, e.g soluble HER2, or bind the same epitope as a reference antibody comprising a VH region comprising the sequence of SEQ ID NO:46 and a VL region comprising the sequence of SEQ ID NO:49 (**127**).

The antigen-binding region may block the binding to HER2, e.g soluble HER2, or bind the same epitope as a reference antibody comprising a VH region comprising the sequence of SEQ ID NO:49 and a VL region comprising the sequence of SEQ ID NO:53 (**159**).

The antigen-binding region may block the binding to HER2, e.g soluble HER2, or bind the same epitope as a reference antibody comprising a VH region comprising the sequence of SEQ ID NO:56 and a VL region comprising the sequence of SEQ ID NO:60 (**098**).

The antigen-binding region may block the binding to HER2, e.g soluble HER2, or bind the same epitope as a reference antibody comprising a VH region comprising the sequence of SEQ ID NO:63 and a VL region comprising the sequence of SEQ ID NO:67 (**153**).

The antigen-binding region may block the binding to HER2, e.g soluble HER2, or binds the same epitope as a reference antibody comprising a VH region comprising the sequence of SEQ ID NO:70 and a VL region comprising the sequence of SEQ ID NO:74 (**132**).

The antigen-binding region may block the binding to HER2, e.g soluble HER2, or bind to the same epitope as a reference antibody comprising VH and VL regions selected from the group consisting of:

a) a VH region comprising the sequence of SEQ ID NO:109 and a VL region comprising the sequence of SEQ ID NO:110 (**105**);

b) a VH region comprising the sequence of SEQ ID NO:111 and a VL region comprising the sequence of SEQ ID NO:112 (**100**);

c) a VH region comprising the sequence of SEQ ID NO:113 and a VL region comprising the sequence of SEQ ID NO:114 (**125**);

d) a VH region comprising the sequence of SEQ ID NO:115 and a VL region comprising the sequence of SEQ ID NO:116 (**162**);

e) a VH region comprising the sequence of SEQ ID NO:117 and a VL region comprising the sequence of SEQ ID NO:118 (**033**);

f) a VH region comprising the sequence of SEQ ID NO:119 and a VL region comprising the sequence of SEQ ID NO:120 (**160**)

g) a VH region comprising the sequence of SEQ ID NO:121 and a VL region comprising the sequence of SEQ ID NO:122 (**166**);

h) a VH region comprising the sequence of SEQ ID NO:123 and a VL region comprising the sequence of SEQ ID NO:124 (**152**); and

i) a VH region comprising the sequence of SEQ ID NO:125 and a VL region comprising the sequence of SEQ ID NO:126 (**167**).

Aditionally or alternatively, the bispecific antibody of the disclosure or antigen-binding region may comprise a VH CDR3, VH region and/or VL region sequence similar or identical to a sequence of the novel antibodies described herein.

The bispecific antibody or antigen-binding region may comprise a VH CDR3 region having a sequence selected from the group consisting of

SEQ ID NO:148, such as the sequence of SEQ ID NO:48 (**127**), optionally wherein the VH region is derived from the IgHV5-51-01 germline sequence;

SEQ ID NO:52 (**159**), optionally wherein the VH region is derived from the IgHV5-51-01 germline sequence;

SEQ ID NO:145, such as the sequence of SEQ ID NO:59 (**098**), optionally wherein the VH region is derived from the IgHV3-23-01 germline sequence;

SEQ ID NO:154, such as the sequence of SEQ ID NO:66 (**153**), optionally wherein the VH region is derived from the IgHV3-30-03-01 germline sequence; and

SEQ ID NO:151, such as the sequence of SEQ ID NO:73 (**132**), optionally wherein the VH region is derived from the IgHV1-18-01 germline sequence.

The bispecific antibody or antigen-binding region may comprise a VH CDR3 region of one of antibodies 105, 100, 125 or 162 as shown in Figure 1, optionally wherein the VH region is derived from an IgHV3-23-1 germline.

The bispecific antibody or antigen-binding region may comprise a VH CDR3 region of one of antibodies 033, 160, 166, 152 or 167 as shown in Figure 1, optionally wherein the VH region is derived from an IgHV3-30-3-01 germline.

The bispecific antibody or antigen-binding region may comprise a VH region selected from the group consisting of

a) a VH region comprising the CDR1, CDR2 and CDR3 sequences of SEQ ID NOs:146, 147 and 148, such as the CDR1, CDR2 and CDR3 sequences of SEQ ID NOS: 43, 44 and 45 (**127**); optionally where the VH region is derived from an IgHV5-51-01 germline;

b) a VH region comprising the CDR1, CDR2 and CDR3 sequences of SEQ ID NOs:149, 51 and 52, such as the CDR1, CDR2 and CDR3 sequences of SEQ ID NOs:50, 51 and 52, respectively (**159**), optionally where the VH region is derived from an IgHV5-51-01 germline;

c) a VH region comprising the CDR1, CDR2, and CDR3 sequences of SEQ ID NOs:143, 144 and 145, such as the CDR1, CDR2, and CDR3 sequences of SEQ ID NOs: 57, 58 and 59 (**098**), respectively, optionally where the VH region is derived from an IgHV3-23-01 germline;

d) a VH region comprising the CDR1, CDR2 and CDR3 sequences of SEQ ID NOs:152, 153 and 154, such as the CDR1, CDR2 and CDR3 sequences of SEQ ID NOs:64, 65 and 66, respectively (**153**), optionally where the VH region is derived from an IgHV3-30-03-01 germline; and

e) a VH region comprising the CDR1, CDR2, and CDR3 sequences of SEQ ID NOs:71, 150 and 151, such as the CDR1, CDR2, and CDR3 sequences of SEQ ID NOs: 71, 72 and 73 (**132**), respectively, optionally where the VH region is derived from an IgHV1-18-01 germline.

The bispecific antibody or antigen-binding region may comprise a VH region selected from the preceding embodiment (a) and a VL region comprising the CDR1, CDR2, and CDR3 sequences of SEQ ID NO:47, AAS and SEQ ID NO:48, respectively (**127**); respectively, optionally where the VL region is derived from an IgKV1D-8-01 germline.

The bispecific antibody or antigen-binding region may comprise a VH region selected from the preceding embodiment (b) and a VL region comprising the CDR1, CDR2, and CDR3 sequences of SEQ ID NO:54, AAS, and SEQ ID No:55 (**159**); respectively, optionally where the VL region is derived from an IgKV1D-16-01 germline.

The bispecific antibody or antigen-binding region may comprise a VH region which is the preceding embodiment (c) and a VL region comprising the CDR1, CDR2, and CDR3 sequences of SEQ ID NO:159, AAS and SEQ ID NO:160, respectively, such as the VL CDR1, CDR2 and CDR3 sequences of SEQ ID NOS: 61, AAS and SEQ ID NO:62 (**098**), optionally wherein the VL region is derived from IgKV1D-16-01.

The bispecific antibody or antigen-binding region may comprise a VH region which is the preceding embodiment (d) and a VL region comprising the CDR1, CDR2, and CDR3 sequences of SEQ ID NO:161, XAS (wherein X=D or A, preferably D), and SEQ ID NO:162 (**153**), respectively, such as the VL CDR sequences of SEQ ID NO:68, DAS, and 69, optionally wherein the VL region is derived from IgKV1D-16-01.

The bispecific antibody or antigen-binding region may comprise a VH region which is the preceding embodiment (e) and a VL region comprising the CDR1, CDR2, and CDR3 sequences of SEQ ID NO:75, DAS and SEQ ID NO:76 (**132**), respectively, optionally wherein the VL region is derived from IgKV3-11-01.

The bispecific antibody or antigen-binding region may comprise a VH region comprising the CDR1, CDR2 and CDR3 sequences of SEQ ID NOs:43, 44 and 45, respectively; and a VL region comprising the CDR1, CDR2 and CDR3 sequences of SEQ ID NOs:47, AAS, and SEQ ID NO:48, respectively (**127**).

The bispecific antibody or antigen-binding region may comprise a VH region comprising the CDR1, CDR2 and CDR3 sequences of SEQ ID NOs:50, 51 and 52, respectively; and a VL region comprising the CDR1, CDR2 and CDR3 sequences of SEQ ID NOs:54, AAS, and SEQ ID NO:55, respectively (**159**).

The bispecific antibody or antigen-binding region may comprise a VH region comprising the CDR1, CDR2 and CDR3 sequences of SEQ ID NOs:57, 58 and 59, respectively; and a VL region comprising the CDR1, CDR2 and CDR3 sequences of SEQ ID NOs:60, AAS, and SEQ ID NO:61, respectively (**098**).

The bispecific antibody or antigen-binding region may comprise a VH region comprising the CDR1, CDR2 and CDR3 sequences of SEQ ID NOs:64, 65 and 66, respectively; and a VL region comprising the CDR1, CDR2 and CDR3 sequences of SEQ ID NOs:68, DAS, and SEQ ID NO:69, respectively (**153**).

The bispecific antibody or antigen-binding region may comprise a VH region comprising the CDR1, CDR2 and CDR3 sequences of SEQ ID NOs:71, 72 and 73, respectively; and a VL region comprising the CDR1, CDR2 and CDR3 sequences of SEQ ID NOs:75, DAS, and SEQ ID NO:76, respectively (**132**).

The bispecific antibody or antigen-binding region may comprises:

a) a VH region comprising the sequence of SEQ ID NO:46 and, preferably, a VL region comprising the sequence of SEQ ID NO:49 (**127**);

b) a VH region comprising the sequence of SEQ ID NO:49 and, preferably, a VL region comprising the sequence of SEQ ID NO:53 (**159**);

c) a VH region comprising the sequence of SEQ ID NO:56 and, preferably, a VL region comprising the sequence of SEQ ID NO:60 (**098**);

d) a VH region comprising the sequence of SEQ ID NO:63 an, preferably, a VL region comprising the sequence of SEQ ID NO:67 (**153**);

e) a VH region comprising the sequence of SEQ ID NO:70 and, preferably, a VL region comprising the sequence of SEQ ID NO:74 (**132**);

f) a VH region comprising the sequence of SEQ ID NO:109 and, preferably, a VL region comprising the sequence of SEQ ID NO:110 (**105**);

g) a VH region comprising the sequence of SEQ ID NO:111 and, preferably, a VL region comprising the sequence of SEQ ID NO:112 (**100**);

h) a VH region comprising the sequence of SEQ ID NO:113 and, preferably, a VL region comprising the sequence of SEQ ID NO:114 (**125**);

i) a VH region comprising the sequence of SEQ ID NO:115 and, preferably, a VL region comprising the sequence of SEQ ID NO:116 (**162**);

j) a VH region comprising the sequence of SEQ ID NO:117 and, preferably, a VL region comprising the sequence of SEQ ID NO:118 (**033**);

k) a VH region comprising the sequence of SEQ ID NO:119 and, preferably, a VL region comprising the sequence of SEQ ID NO:120 (**160**)

l) a VH region comprising the sequence of SEQ ID NO:121 and, preferably, a VL region comprising the sequence of SEQ ID NO:122 (**166**);

m) a VH region comprising the sequence of SEQ ID NO:123 and, preferably, a VL region comprising the sequence of SEQ ID NO:124 (**152**);

o) a VH region comprising the sequence of SEQ ID NO:125 and, preferably, a VL region comprising the sequence of SEQ ID NO:126 (**167**); and/or

p) a variant of any of said antibodies, wherein said variant preferably has at most 1, 2 or 3 amino-acid modifications, more preferably amino-acid substitutions, such as conservative amino acid substitutions and substitutions where the new amino acid is one at the same position in an aligned sequence in Figures 1 or 2, particularly at positions indicated by "X" in the corresponding consensus sequence.

## EP 2 699 260 B1

*Cross-block group 4*

The bispecific antibody may comprise an antigen-binding region which binds HER2 but which does not block the binding to soluble HER2 of a second antibody, optionally in immobilized form, comprising the VH and VL sequences of any of trastuzumab, pertuzumab, F5, and C1, when determined as described in Example 14.

Additionally or alternatively the antibody of the disclosureiinvention, the antigen-binding region may block or cross-block the binding to soluble HER2 of one or more of the human antibodies of cross-block group 4. The bispecific antibody may then comprise a second antigen-binding region which cross-blocks or binds to the same epitope as an antibody of cross-block groups 1, 2, or 3.

The antigen-binding region may block the binding to soluble HER2 of a reference antibody, optionally immobilized, wherein the reference antibody comprises a VH region comprising the sequence of SEQ ID NO:165 and a VL region comprising the sequence of SEQ ID NO:5 (**005**), preferably wherein the antibody is fully blocking when determined as described in Example 14.

The antigen-binding region may block the binding to soluble HER2 of a reference antibody, optionally immobilized, wherein the reference antibody comprises a VH region comprising the sequence of SEQ ID NO:172 and a VL region comprising the sequence of SEQ ID NO:176 (**006**), preferably wherein the antibody is fully-blocking when determined as described in Example 14.

The antigen-binding region may block the binding to soluble HER2 of a reference antibody, optionally immobilized, wherein the reference antibody comprises a VH region comprising the sequence of SEQ ID NO:179 and a VL region comprising the sequence of SEQ ID NO:183 (**059**), preferably wherein the antibody is fully-blocking when determined as described in Example 14.

The antigen-binding region may block the binding to soluble HER2 of a reference antibody, optionally immobilized, wherein the reference antibody comprises a VH region comprising the sequence of SEQ ID NO:186 and a VL region comprising the sequence of SEQ ID NO:190 (**060**), preferably wherein the antibody is fully-blocking when determined as described in Example 14.

The antigen-binding region may block the binding to soluble HER2 of a reference antibody, optionally immobilized, wherein the reference antibody comprises a VH region comprising the sequence of SEQ ID NO:193 and a VL region comprising the sequence of SEQ ID NO:197 (**106**), preferably wherein the antibody is fully-blocking when determined as described in Example 14.

The antigen-binding region may block the binding to soluble HER2 of a reference antibody, optionally immobilized, wherein the reference antibody comprises a VH region comprising the sequence of SEQ ID NO:200 and a VL region comprising the sequence of SEQ ID NO:204 (**111**), preferably wherein the antibody is fully-blocking when determined as described in Example 14.

The antigen-binding region may block the binding of two, three, four, five, or six reference antibodies of the preceding embodiment, such as, e.g., antibodies 005 and 111, antibodies 005 and 006; antibodies 059 and 106; antibodies 006 and 059; antibodies 059, 106, 005 and 060; antibodies 006, 59, 060, and 111; or antibodies 059, 106, 005, 060, 111 and 006.

The antibody, when immobilized, may compete for binding to soluble HER2 with all antibodies defined in the preceding embodiment for 25% or more, preferably 50% or more, when determined as described in Example 14.

The antibody may bind the same epitope on HER2 as one or more of the novel human antibodies described herein.

The antigen-binding region may bind the same epitope as an antibody comprising a VH region comprising the sequence of SEQ ID NO:165 and a VL region comprising the sequence of SEQ ID NO:169 (**005**).

The antigen-binding region may bind the same epitope as an antibody comprising a VH region comprising the sequence of SEQ ID NO:172 and a VL region comprising the sequence of SEQ ID NO:176 (**006**).

The antigen-binding region may bind the same epitope as an antibody comprising a VH region comprising the sequence of SEQ ID NO:179 and a VL region comprising the sequence of SEQ ID NO:183 (**059**).

The antigen-binding region may bind the same epitope as an antibody comprising a VH region comprising the sequence of SEQ ID NO:186 and a VL region comprising the sequence of SEQ ID NO:190 (**060**).

The antigen-binding region may bind the same epitope as an antibody comprising a VH region comprising the sequence of SEQ ID NO:193 and a VL region comprising the sequence of SEQ ID NO:197 (**106**).

The antigen-binding region may bind the same epitope as an antibody comprising a VH region comprising the sequence of SEQ ID NO:200 and a VL region comprising the sequence of SEQ ID NO:204 (**111**).

The antigen-binding region may bind to the same epitope as at least one antibody selected from the group consisting of:

a) an antibody comprising a VH region comprising the sequence of SEQ ID NO:207 and a VL region comprising the sequence of SEQ ID NO:208 (**041**)

b) an antibody comprising a VH region comprising the sequence of SEQ ID NO:209 and a VL region comprising the sequence of SEQ ID NO:210 (**150**), and

c) an antibody comprising a VH region comprising the sequence of SEQ ID NO:211 and a VL region comprising the sequence of SEQ ID NO:212 (**067**);

d) an antibody comprising a VH region comprising the sequence of SEQ ID NO:213 and a VL region comprising the sequence of SEQ ID NO:214 (**072**);

e) an antibody comprising a VH region comprising the sequence of SEQ ID NO:215 and a VL region comprising the sequence of SEQ ID NO:216 (**163**);

f) an antibody comprising a VH region comprising the sequence of SEQ ID NO:217 and a VL region comprising the sequence of SEQ ID NO:218 (**093**);

g) an antibody comprising a VH region comprising the sequence of SEQ ID NO:219 and a VL region comprising the sequence of SEQ ID NO:220 (**044**).

Additionally or alternatively , the bispecific antibody or antigen-binding region may comprise a VH CDR3, VH region and/or VL region sequence similar or identical to a sequence of the HER2 antibodies described herein.

The bispecific antibody or antigen-binding region may comprise a VH CDR3 region having an amino acid sequence selected from the group consisting of

SEQ ID No:223, such as the sequence of SEQ ID No: 168, 189, 196 (**005, 060, 106**), optionally wherein the VH region is derived from the IgHV5-51-1 germline;

SEQ ID No:226, such as the sequence of SEQ ID NO:175 (**006**), optionally wherein the VH region is derived from the IgHV3-23-1 germline sequence;

SEQ ID NO:229, such as the sequence of SEQ ID NO:182 (**059**), optionally wherein the VH region is derived from the IgHV1-18-1 germline sequence; or

SEQ ID NO:231, such as the sequence of SEQ ID NO:203 (**111**), optionally wherein the VH region is derived from the IgHV1-69-4 germline sequence.

The bispecific antibody or antigen-binding region may comprise a VH CDR3 region comprising the amino acid sequence of SEQ ID NO: 223, wherein X1=Q, H, or L; X2 = R, A, T, or K; X3=G; X4=D; X5=R or none; X6=G or none; X7=Y or F; X8=Y or D; X9=Y, F, or H; X10=Y, D, S, F, or N; X11=M or L; and X12=V or I; preferably, wherein X1=Q, X2=R or A; X5=X6=none; X7=Y or F; X8=Y; X9=F; X10=Y; and X12=V. The antibody may comprise a VH CDR3 region comprising the amino acid sequence of SEQ ID NO: 223, wherein X1=Q, X2=R or A; X3=G; X4=D, X5=X6=none; X7=Y or F; X8=Y; X9=F; X10=Y; and X12=V. The antibody may comprise a VH CDR3 region comprising the amino acid sequence of SEQ ID NO:223, wherein X1=Q, X2=K; X3=G; X4=D, X5=X6=none; X7=F; X8=Y; X9=X10=F; X11=L; and X12=V; or wherein X1=Q, X2=A; X3=G; X4=D, X5=X6=none; X7=X8=Y; X9=Y; X10=N; X11=M; and X12=V; or wherein X1=Q, X2=K; X3=G; X4=D, X5=X6=none; X7=X8=Y; X9=H; X10=Y; X11=L; and X12=V; or wherein X1=Q, X2=K; X3=G; X4=D, X5=X6=none; X7=Y; X8=Y; X9=F; X10=N; X11=L; and X12=V; or wherein X1=Q, X2=R; X3=G; X4=D, X5=X6=none; X7=Y; X8=Y; X9=F; X10=N; X11=L; and X12=V; or wherein X1=Q, X2=R; X3=G; X4=D, X5=X6=none; X7=Y; X8=Y; X9=X10=F; X11=L; and X12=I; or wherein X1=Q, X2=A; X3=G; X4=D, X5=X6=none; X7=X8=Y; X9=Y; X10=N; X11=M; and X12=V.

The bispecific antibody or antigen-binding region may comprise a VH CDR3 region of one of antibodies 041, 150, 067, 072, 163, or 093, as shown in Figure 1, optionally wherein the VH region is derived from an IgHV5-51-1 germline.

The bispecific antibody or antigen-binding region may comprise a VH region selected from the group consisting of

a) a VH region comprising the CDR1, CDR2 and CDR3 sequences of SEQ ID NOs:221, 222 and 223, such as

a. a CDR1 sequence selected from SEQ ID NOs:166, 187, and 194; a CDR2 sequence selected from 167, 188, and 195; and a CDR3 sequence selected from 168, 189, and 196 (**005, 060, 106**),

b. the CDR1, CDR2, and CDR3 sequences of SEQ ID NOs:166, 167 and 168, respectively (**005**),

c. the CDR1, CDR2, and CDR3 sequences of SEQ ID NOs:187, 188 and 189, respectively (**060**),

d. the CDR1, CDR2, and CDR3 sequences of SEQ ID NOs:196, 197 and 198, respectively (**106**),

optionally where the VH region is derived from an IgHV5-51-1 germline;

b) a VH region comprising the CDR1, CDR2 and CDR3 sequences of SEQ ID NOs:224, 225 and 226, such the CDR1, CDR2 and CDR3 sequences of SEQ ID NOs:173, 174, and 175, respectively (**006**), optionally where the VH region is derived from an IgHV3-23-1 germline; and

c) a VH region comprising the CDR1, CDR2, and CDR3 sequences of SEQ ID NOs:227, 228, and 229, such as the CDR1, CDR2, and CDR3 sequences of SEQ ID NOs: 180, 181 and 182 (**059**), respectively, optionally where the VH region is derived from an IgHV1-18-1 germline; and

d) a VH region comprising the CDR1, CDR2, and CDR3 sequences of SEQ ID NOs:230, 202 and 231, such as the CDR1, CDR2, and CDR3 sequences of SEQ ID NOs: 201, 202 and 203 (**111**), respectively, optionally where the VH region is derived from an IgHV1-69-4 germline.

The bispecific antibody or antigen-binding region may comprise a VH region selected from the preceding embodiments (a), (c) or (d) and a VL region comprising the CDR1, CDR2, and CDR3 sequences of SEQ ID NO:232, GAS, and SEQ ID No:233, respectively, such as a CDR1 sequence selected from SEQ ID Nos: 170, 184, 191, 198 and 205, a CDR2 which is GAS, and a CDR3 sequence selected from 171, 85, 192, 199 and 206 (**005, 059, 060, 106, 111**); respectively, optionally where the VL region is derived from an IgKV3-20-01 germline.

The bispecific antibody or antigen-binding region may comprise a VH region which is the preceding embodiment (b) and a VL region comprising the CDR1, CDR2, and CDR3 sequences of SEQ ID NO:177, DAS, and SEQ ID NO:178 (**006**), respectively, optionally where the VL region is derived from IgKV3-11-01.

The bispecific antibody or antigen-binding region may comprise a VH region comprising the CDR1, CDR2 and CDR3 sequences of SEQ ID NOs:166, 167 and 168, respectively; and a VL region comprising the CDR1, CDR2 and CDR3 sequences of SEQ ID NOs:170, GAS, and SEQ ID NO:171, respectively (**005**).

The bispecific antibody or antigen-binding region may comprise a VH region comprising the CDR1, CDR2 and CDR3 sequences of SEQ ID NOs:173, 174 and 175, respectively; and a VL region comprising the CDR1, CDR2 and CDR3 sequences of SEQ ID NOs:177, DAS, and SEQ ID NO:178, respectively (**006**).

The bispecific antibody or antigen-binding region may comprise a VH region comprising the CDR1, CDR2 and CDR3 sequences of SEQ ID NOs:180, 181 and 182, respectively; and a VL region comprising the CDR1, CDR2 and CDR3 sequences of SEQ ID NOs:184, GAS, and SEQ ID NO:185, respectively (**059**).

The bispecific antibody or antigen-binding region may comprise a VH region comprising the CDR1, CDR2 and CDR3 sequences of SEQ ID NOs:187, 188 and 189, respectively; and a VL region comprising the CDR1, CDR2 and CDR3 sequences of SEQ ID NOs: 191, GAS, and SEQ ID NO:192, respectively (**060**).

The bispecific antibody or antigen-binding region may comprise a VH region comprising the CDR1, CDR2 and CDR3 sequences of SEQ ID NOs:194, 195 and 196, respectively; and a VL region comprising the CDR1, CDR2 and CDR3 sequences of SEQ ID NOs: 198, GAS, and SEQ ID NO:199, respectively (**106**).

The bispecific antibody or antigen-binding region may comprise a VH region comprising the CDR1, CDR2 and CDR3 sequences of SEQ ID NOs:201, 202 and 203, respectively; and a VL region comprising the CDR1, CDR2 and CDR3 sequences of SEQ ID NOs:205, GAS, and SEQ ID NO:206, respectively (**111**).

The bispecific antibody or antigen-binding region may comprise a VH region comprising the CDR1 sequence of SEQ ID NO:221, wherein $X_1$=S; $X_2$=T and $X_3$=S; the CDR2 sequence of SEQ ID NO:226, wherein $X_1$=Y and $X_2$=H and the CDR3 sequence of SEQ ID NO:227, wherein $X_1$=Q, $X_2$=K; $X_3$=G; $X_4$=D, $X_5$=$X_6$=none; $X_7$=F; $X_8$=Y; $X_9$=$X_{10}$=F; $X_{11}$=L; and $X_{12}$=V; and a VL region comprising the CDR1 sequence of SEQ ID NO:232, wherein $X_1$=$X_2$=S; the CDR2 sequence GAS; and the CDR3 sequence of SEQ ID NO: 233, wherein $X_1$=Q, $X_2$=S, $X_3$=$X_4$=none and $X_5$=L (**041**).

The bispecific antibody or antigen-binding region may comprise a VH region comprising the CDR1 sequence of SEQ ID NO:221, wherein $X_1$=S; $X_2$=T and $X_3$=S; the CDR2 sequence of SEQ ID NO:222, wherein $X_1$=Y and $X_2$=H, and the CDR3 sequence of SEQ ID NO:223, wherein $X_1$=Q, $X_2$=A; $X_3$=G; $X_4$=D, $X_5$=$X_6$=none; $X_7$=$X_8$=Y; $X_9$=Y; $X_{10}$=N; $X_{11}$=M; and $X_{12}$=V; and a VL region comprising the CDR1 sequence of SEQ ID NO:232, wherein $X_1$=$X_2$=S; the CDR2 sequence GAS; and the CDR3 sequence of SEQ ID NO: 233, wherein $X_1$=Q, $X_2$=S, $X_3$=$X_4$=none and $X_5$=L (**150**).

The bispecific antibody or antigen-binding region may comprise a VH region comprising the CDR1 sequence of SEQ ID NO:221, wherein $X_1$=S; $X_2$=T and $X_3$=S; the CDR2 sequence of SEQ ID NO:222, wherein $X_1$=Y and $X_2$=D, and the CDR3 sequence of SEQ ID NO:223, $X_1$=Q, $X_2$=K; $X_3$=G; $X_4$=D, $X_5$=$X_6$=none; $X_7$=$X_8$=Y; $X_9$=H; $X_{10}$=Y; $X_{11}$=L; and $X_{12}$=V; and a VL region comprising the CDR1 sequence of SEQ ID NO:232, wherein $X_1$=$X_2$=S; the CDR2 sequence GAS; and the CDR3 sequence of SEQ ID NO: 233, wherein $X_1$=Q, $X_2$=S, $X_3$=P, $X_4$=R and $X_5$=L (**067**).

The bispecific antibody or antigen-binding region may comprise a VH region comprising the CDR1 sequence of SEQ ID NO:221, wherein $X_1$=S; $X_2$=T and $X_3$=S; the CDR2 sequence of SEQ ID NO:222, wherein $X_1$=Y and $X_2$=D, and the CDR3 sequence of SEQ ID NO:223, wherein $X_1$=Q, $X_2$=K; $X_3$=G; $X_4$=D, $X_5$=$X_6$=none; $X_7$=Y; $X_8$=Y; $X_9$=F; $X_{10}$=N; $X_{11}$=L; and $X_{12}$=V; and a VL region comprising the CDR1 sequence of SEQ ID NO:232, wherein $X_1$=$X_2$=S; the CDR2 sequence GAS; and the CDR3 sequence of SEQ ID NO: 233, wherein $X_1$=Q, $X_2$=S, $X_3$=P, $X_4$=R and $X_5$=L (**072**).

The bispecific antibody or antigen-binding region may comprise a VH region comprising the CDR1 sequence of SEQ ID NO:221, wherein $X_1$=R; $X_2$=I and $X_3$=S; the CDR2 sequence of SEQ ID NO:222, wherein $X_1$=Y and $X_2$=D, and the CDR3 sequence of SEQ ID NO:223, wherein $X_1$=Q, $X_2$=R; $X_3$=G; $X_4$=D, $X_5$=$X_6$=none; $X_7$=Y; $X_8$=Y; $X_9$=F; $X_{10}$=N; $X_{11}$=L; and $X_{12}$=V; and a VL region comprising the CDR1 sequence of SEQ ID NO:232, wherein $X_1$=$X_2$=S; the CDR2 sequence GAS; and the CDR3 sequence of SEQ ID NO: 233, wherein $X_1$=Q, $X_2$=S, $X_3$=$X_4$=none and $X_5$=L (**163**).

The bispecific antibody or antigen-binding region may comprise a VH region comprising the CDR1 sequence of SEQ ID NO:221, wherein X1=S; X2=T and X3=S; the CDR2 sequence of SEQ ID NO:222, wherein X1=Y and X2=D, and the CDR3 sequence of SEQ ID NO:223, wherein X1=Q; X2=R; X3=G; X4=D, X5=X6=none; X7=Y; X8=Y; X9=X10=F; X11=L; and X12=I; and a VL region comprising the CDR1 sequence of SEQ ID NO:232, wherein X1=X2=S; the CDR2 sequence GAS; and the CDR3 sequence of SEQ ID NO: 233, wherein X1=Q, X2=S, X3=X4=none and X5=L (**093**).

The bispecific antibody or antigen-binding region may comprise a VH region comprising the CDR1 sequence of SEQ ID NO:221, wherein X1=R; X2=S and X3=S; the CDR2 sequence of SEQ ID NO:222, wherein X1=F and X2=D, and the CDR3 sequence of SEQ ID NO:223, wherein X1=Q, X2=A; X3=G; X4=D, X5=X6=none; X7=X8=Y; X9=Y; X10=N; X11=M; and X12=V; and a VL region comprising the CDR1 sequence of SEQ ID NO:232, wherein X1=X2=S; the CDR2 sequence GAS; and the CDR3 sequence of SEQ ID NO: 233, wherein X1=Q, X2=S, X3=X4=none and X5=L (**044**).

The bispecific antibody or antigen-binding region may comprise:

a) a VH region comprising the sequence of SEQ ID NO:165 and, preferably, a VL region comprising the sequence of SEQ ID NO:169 (**005**)

b) a VH region comprising the sequence of SEQ ID NO:172 and, preferably, a VL region comprising the sequence of SEQ ID NO:176 (**006**)

c) a VH region comprising the sequence of SEQ ID NO:179 and, preferably, a VL region comprising the sequence of SEQ ID NO:183 (**059**)

d) a VH region comprising the sequence of SEQ ID NO:186 and, preferably, a VL region comprising the sequence of SEQ ID NO:190 (**060**)

e) a VH region comprising the sequence of SEQ ID NO:193 and, preferably, a VL region comprising the sequence of SEQ ID NO:197 (**106**)

f) a VH region comprising the sequence of SEQ ID NO:200 and, preferably, a VL region comprising the sequence of SEQ ID NO:204 (**111**)

g) a VH region comprising the sequence of SEQ ID NO:297 and, preferably, a VL region comprising the sequence of SEQ ID NO:208 (**041**)

h) a VH region comprising the sequence of SEQ ID NO:209 and, preferably, a VL region comprising the sequence of SEQ ID NO:210 (**150**),

i) a VH region comprising the sequence of SEQ ID NO:211 and, preferably, a VL region comprising the sequence of SEQ ID NO:212 (**067**),

j) a VH region comprising the sequence of SEQ ID NO:213 and, preferably, a VL region comprising the sequence of SEQ ID NO:214 (**072**),

k) a VH region comprising the sequence of SEQ ID NO:215 and, preferably, a VL region comprising the sequence of SEQ ID NO:216 (**163**),

l) a VH region comprising the sequence of SEQ ID NO:217 and, preferably, a VL region comprising the sequence of SEQ ID NO:218 (**093**),

m) a VH region comprising the sequence of SEQ ID NO:219 and, preferably, a VL region comprising the sequence of SEQ ID NO:220 (**044**), and/or

n) a variant of any of said antibodies, wherein said variant preferably has at most 1,2 or 3 amino-acid modifications, more preferably amino-acid substitutions, such as conservative amino acid substitutions and substitutions where the new amino acid is one at the same position in an aligned sequence in Figures 1 or 2, particularly at positions indicated by "X" in the corresponding consensus sequence.

*Functional properties of antigen-binding regions or HER2 antibodies of cross-block groups 1, 2, 3 and 4*

The antigen-binding region, e.g. first or second antigen-binding region of a bispecific antibody of the present invention, or a first or second HER2 antibody disclosed herein, may block binding to HER2 of or binds to the same HER2 epitope as, one or more of the antingen-binding regions or antibodies of cross-block group 1, 2, 3 or 4 described herein, preferably when determined as described in Example 14; and is further characterized by one or more properties described below or determined as described in Examples 12, 13, 15, 16, 17, 18 and 19.

Thus the first and/or second antigen-binding region of the bispecific antibody of the present disclosure may be the same as the antigen-binding region of an antibody or anti-HER2 antibody having one of the following characteristics. The first and/or second HER2 antibody of the present disclosure may have one or more of the following characteristics.

The anti-HER2 antibody may have a lower $EC_{50}$ value (half maximal effective concentration) than trastuzumab in binding to A431 cells, preferably an $EC_{50}$ value lower than 0.80 $\mu$g/ml, 0.50 $\mu$g/ml, or 0.30 $\mu$g/ml, when determined as described in Example 12, and preferably binds the same epitope as at least one reference antibody comprising the VH and VL regions selected from the group consisting of

a) a VH region comprising the sequence of SEQ ID NO:1 and a VL region comprising the sequence of SEQ ID NO:5 (**169**);

b) a VH region comprising the sequence of SEQ ID NO:15 and a VL region comprising the sequence of SEQ ID NO:19 (**084**);

c) a VH region comprising the sequence of SEQ ID NO:22 and a VL region comprising the sequence of SEQ ID NO:26 (**025**);

d) a VH region comprising the sequence of SEQ ID NO:29 and a VL region comprising the sequence of SEQ ID NO:32 (**091**);

e) a VH region comprising the sequence of SEQ ID NO:46 and a VL region comprising the sequence of SEQ ID NO:49 (**127**);

f) a VH region comprising the sequence of SEQ ID NO:49 and a VL region comprising the sequence of SEQ ID NO:53 (**159**);

g) a VH region comprising the sequence of SEQ ID NO:56 and a VL region comprising the sequence of SEQ ID NO:60 (**098**);

h) a VH region comprising the sequence of SEQ ID NO:63 and a VL region comprising the sequence of SEQ ID NO:67 (**153**);

i) a VH region comprising the sequence of SEQ ID NO:70 and a VL region comprising the sequence of SEQ ID NO:74 (**132**);

j) a VH region comprising the sequence of SEQ ID NO:1 and a VL region comprising the sequence of SEQ ID NO:5 (**005**);

k) a VH region comprising the sequence of SEQ ID NO:8 and a VL region comprising the sequence of SEQ ID NO:11 (**006**); and

l) a VH region comprising the sequence of SEQ ID NO:15 and a VL region comprising the sequence of SEQ ID NO:19 (**059**).

The anti-HER2 antibody may specifically bind HER2-positive Rhesus monkey epithelial cells, when determined as described in Example 13, and may preferably bind the same epitope as at least one reference antibody comprising the VH and VL regions selected from the group consisting of the VH and VL regions of any of antibodies 169, 050, 084, 025, 091, 129, 127, 159, 098, 153, 132, 005, 006, 059, 060, 106 and 111.

The anti-HER2 antibody may efficiently induce ADCC (antibody-dependent cell-mediated cytotoxicity), preferably achieving a specific $^{51}$Cr-release of at least 30%, more preferably at least 40%, when determined as described in Example 15, and may preferably bind the same epitope as at least one reference antibody comprising the VH and VL regions selected from the group consisting of:

a) a VH region comprising the sequence of SEQ ID NO:1 and a VL region comprising the sequence of SEQ ID NO:5 (**169**);

b) a VH region comprising the sequence of SEQ ID NO:8 and a VL region comprising the sequence of SEQ ID NO:12 (**050**);

c) a VH region comprising the sequence of SEQ ID NO:15 and a VL region comprising the sequence of SEQ ID NO:19 (**084**);

d) a VH region comprising the sequence of SEQ ID NO:22 and a VL region comprising the sequence of SEQ ID NO:26 (**025**);

e) a VH region comprising the sequence of SEQ ID NO:29 and a VL region comprising the sequence of SEQ ID NO:32 (**091**);

f) a VH region comprising the sequence of SEQ ID NO:35 and a VL region comprising the sequence of SEQ ID NO:39 (**129**); and

g) a VH region comprising the sequence of SEQ ID NO:63 an, preferably, a VL region comprising the sequence of SEQ ID NO:67 (**153**).

The anti-HER2 antibody may specifically bind HER2-expressing AU565 cells but promotes ligand-independent proliferation of the cells less than any of F5 and C1 when determined as described in Example 16, and may preferably bind the same epitope as at least one reference antibody comprising the VH and VL regions selected from the group consisting of

a) a VH region comprising the sequence of SEQ ID NO:1 and a VL region comprising the sequence of SEQ ID NO:5 (**169**);

b) a VH region comprising the sequence of SEQ ID NO:8 and a VL region comprising the sequence of SEQ ID NO:12 (**050**);

c) a VH region comprising the sequence of SEQ ID NO:15 and a VL region comprising the sequence of SEQ ID NO:19 (**084**);

d) a VH region comprising the sequence of SEQ ID NO:22 and a VL region comprising the sequence of SEQ ID NO:26 (**025**);

e) a VH region comprising the sequence of SEQ ID NO:29 and a VL region comprising the sequence of SEQ ID NO:32 (**091**);

f) a VH region comprising the sequence of SEQ ID NO:35 and a VL region comprising the sequence of SEQ ID NO:39 (**129**);

g) a VH region comprising the sequence of SEQ ID NO:46 and a VL region comprising the sequence of SEQ ID NO:49 (**127**);

h) a VH region comprising the sequence of SEQ ID NO:49 and a VL region comprising the sequence of SEQ ID NO:53 (**159**);

i) a VH region comprising the sequence of SEQ ID NO:56 and a VL region comprising the sequence of SEQ ID NO:60 (**098**);

j) a VH region comprising the sequence of SEQ ID NO:63 and a VL region comprising the sequence of SEQ ID NO:67 (**153**);

k) a VH region comprising the sequence of SEQ ID NO:70 and a VL region comprising the sequence of SEQ ID NO:74 (**132**),

l) a VH region comprising the sequence of SEQ ID NO:1 and a VL region comprising the sequence of SEQ ID NO:5 (**005**); and

m) a VH region comprising the sequence of SEQ ID NO:22 and a VL region comprising the sequence of SEQ ID NO:26 (**060**).

The anti-HER2 antibody may specifically bind HER2-expressing AU565 cells and inhibits ligand-independent proliferation of the cells, preferably inhibiting proliferation by at least 20%, more preferably at least 25%, when determined as described in Example 16, and may preferably bind the same epitope as at least one reference antibody comprising the VH and VL regions selected from the group consisting of:

a) a VH region comprising the sequence of SEQ ID NO:1 and a VL region comprising the sequence of SEQ ID NO:5 (**169**); and

b) a VH region comprising the sequence of SEQ ID NO:8 and a VL region comprising the sequence of SEQ ID NO:12 (**050**).

The anti-HER2 antibody may specifically bind HER2-expressing AU565 cells but may have no significant effect on, or may not promote, ligand-induced proliferation of the cells, preferably inhibiting proliferation by no more than 25%, more preferably by no more than 15%, when determined as described in Example 17, and may bind the same epitope as at least one reference antibody comprising the VH and VL regions selected from the group consisting of:

a) a VH region comprising the sequence of SEQ ID NO:1 and a VL region comprising the sequence of SEQ ID NO:5 (**169**);

b) a VH region comprising the sequence of SEQ ID NO:8 and a VL region comprising the sequence of SEQ ID NO:12 (**050**);

a) a VH region comprising the sequence of SEQ ID NO:15 and a VL region comprising the sequence of SEQ ID NO:19 (**084**); and

b) a VH region comprising the sequence of SEQ ID NO:56 and a VL region comprising the sequence of SEQ ID NO:60 (**098**).

The anti-HER2 antibody may specifically bind HER2-expressing MCF-7 cells and may inhibit ligand-induced proliferation, e.g. it may completely inhibit the ligand-induced effect or inhibit the total proliferation by 50%, *e.g.* 60% or 70% or 80%, of the cells when determined as described in Example 17, and may bind the same epitope as at least one reference antibody comprising the VH and VL regions selected from the group consisting of:

a) a VH region comprising the sequence of SEQ ID NO:22 and a VL region comprising the sequence of SEQ ID NO:26 (**025**);

b) a VH region comprising the sequence of SEQ ID NO:29 and a VL region comprising the sequence of SEQ ID NO:32 (**091**);

c) a VH region comprising the sequence of SEQ ID NO:35 and a VL region comprising the sequence of SEQ ID NO:39 (**129**); and
d) a VH region comprising the sequence of SEQ ID NO:63 an, preferably, a VL region comprising the sequence of SEQ ID NO:67 (**153**).

The anti-HER2 antibody, when conjugated directly or indirectly to a therapeutic moiety such as a truncated form of the pseudomonas-exotoxin A, may be more effective than trastuzumab in killing AU565 cells, A431 cells, or both AU565 and A431 cells, when determined as described in Example 18.

The conjugated anti-HER2 antibody may have an $EC_{50}$ value of less than 70 ng/ml, less than 50 ng/ml, or less than 30 ng/ml in killing AU565 cells and/or A431 cells, when determined as described in Example 18, and may bind the same epitope as at least one reference antibody comprising the VH and VL regions of an antibody selected from the group consisting of 169, 091, 050, 084, 098, 05, 153, 129, 132, 127 and 159; preferably selected from antibodies 153, 129, 098, 091 and 025.

The conjugated anti-HER2 antibody may result in a higher percentage of killed AU565 cells than trastuzumab and pertuzumab when determined as described in Example 18, preferably killing at least 49%, more preferably at least 60% of the AU565 cells, and may bind the same epitope as at least one reference antibody comprising the VH and VL regions of an antibody selected from the group consisting of 169, 091, 050, 084, 098, 025, 153, 129, 132, 127 and 159; preferably selected from antibodies 153, 132, 127, 129, 159 and 025.

The conjugated anti-HER2 antibody may bind to the same epitope as a reference antibody comprising a VH region comprising the sequence of SEQ ID NO:49 and a VL region comprising the sequence of SEQ ID NO:53 (**159**).

The conjugated anti-HER2 antibody may have a higher percentage of killed AU431 cells than trastuzumab and pertuzumab when determined as described in Example 18, preferably killing at least 50%, more preferably at least 70%, and binds the same epitope as at least one reference antibody comprising the VH and VL regions of an antibody selected from the group consisting of 025, 084, 091, 098, 129 and 153; preferably selected from antibodies 025, 091, 098, 129 and 153.

The anti-HER2 conjugated antibody may bind to the same epitope as a reference antibody comprising a VH region comprising the sequence of SEQ ID NO:56 and a VL region comprising the sequence of SEQ ID NO:60 (**098**).

The first or second HER2 antibody may be internalized by tumor cells expressing HER2, such as AU565 cells, to a higher degree than trastuzumab and pertuzumab, preferably more than twice or three times the amount of internalized trastuzumab, preferably when determined according to Example 18, and may bind to the same epitope as an antibody comprising VH and VL regions selected from the group consisting of:

a) a VH region comprising the sequence of SEQ ID NO:46 and a VL region comprising the sequence of SEQ ID NO:49 (**127**);
b) a VH region comprising the sequence of SEQ ID NO:49 and a VL region comprising the sequence of SEQ ID NO:53 (**159**);
c) a VH region comprising the sequence of SEQ ID NO:56 and a VL region comprising the sequence of SEQ ID NO:60 (**098**);
d) a VH region comprising the sequence of SEQ ID NO:63 and a VL region comprising the sequence of SEQ ID NO:67 (**153**); and
e) a VH region comprising the sequence of SEQ ID NO:70 and a VL region comprising the sequence of SEQ ID NO:74 (**132**).

Preferably, the antibody may bind to the same epitope as an antibody comprising VH and VL regions selected from

a) a VH region comprising the sequence of SEQ ID NO:46 and a VL region comprising the sequence of SEQ ID NO:49 (**127**) and
b) a VH region comprising the sequence of SEQ ID NO:56 and a VL region comprising the sequence of SEQ ID NO:60 (**098**).

The anti-HER2 antibody may bind to Domain II or IV of HER2, preferably wherein the antibody does not significantly promote proliferation of HER2 expressing cells, and is more efficiently internalized, or is internalized to a higher degree, than trastuzumab or pertuzumab into HER2-expressing tumor cells, preferably when determined as described in the Examples, *e.g.* examples 16 and 19, respectively.

The anti-HER2 antibody may enhance HER2 downmodulation more than trastuzumab, *e.g.* the antibody may enhance HER2 downmodulation by more 30%, such as more than 40% or more than 50% when determined as described in Example 22, preferably wherein the antibody binds to the same epitope as an antibody of cross-block group 3 of the present invention, *e.g.* an antibody binding to the same epitope as an antibody comprising VH and VL regions selected from the group consisting of:

> a) a VH region comprising the sequence of SEQ ID NO:56 and a VL region comprising the sequence of SEQ ID NO:60 (**098**);
> b) a VH region comprising the sequence of SEQ ID NO:63 and a VL region comprising the sequence of SEQ ID NO:67 (**153**).

The anti-HER2 antibody may decrease tumour growth and improve survival *in vivo* more than trastuzumab, when determined as described in Example 28, preferably wherein the antibody binds to the same epitope as an antibody of cross-block 1 or cross-block 2 of the present invention, *e.g.* an antibody binding to the same epitope as an antibody comprising VH and VL regions selected from the group consisting of:

> a) a VH region comprising the sequence of SEQ ID NO:1 and a VL region comprising the sequence of SEQ ID NO:5 (**169**);
> b) a VH region comprising the sequence of SEQ ID NO:15 and a VL region comprising the sequence of SEQ ID NO:19 (**084**); and
> c) a VH region comprising the sequence of SEQ ID NO:29 and a VL region comprising the sequence of SEQ ID NO:32 (**091**).

The anti-HER2 antibody may decrease tumour growth and improved survival *in vivo* more than trastuzumab, when determined as described in Example 29, preferably wherein the antibody binds to the same epitope as an antibody of cross-block 2 or cross-block 3 of the present invention, *e.g.* an antibody binding to the same epitope as an antibody comprising VH and VL regions selected from the group consisting of:

> a) a VH region comprising the sequence of SEQ ID NO:22 and a VL region comprising the sequence of SEQ ID NO:26 (**025**);
> b) a VH region comprising the sequence of SEQ ID NO:29 and a VL region comprising the sequence of SEQ ID NO:32 (**091**);
> c) a VH region comprising the sequence of SEQ ID NO:35 and a VL region comprising the sequence of SEQ ID NO:39 (**129**); and
> d) a VH region comprising the sequence of SEQ ID NO:63 and a VL region comprising the sequence of SEQ ID NO:67 (**153**).

More particularly, wherein the anti-HER2 antibody binds to the same epitope as an antibody comprising VH and VL regions selected from the group consisting of:

> a) a VH region comprising the sequence of SEQ ID NO:22 and a VL region comprising the sequence of SEQ ID NO:26 (**025**); and
> b) a VH region comprising the sequence of SEQ ID NO:29 and a VL region comprising the sequence of SEQ ID NO:32 (**091**).

The conjugated anti-HER2 antibody may kill at least 60%, preferably at least 70% AU565 cells or A431 cells, when determined as described in Example 18, and may cross-block at least one antibody selected from

> a) an antibody comprising a VH region comprising the sequence of SEQ ID NO:1 and a VL region comprising the sequence of SEQ ID NO:5 (**005**)
> b) an antibody comprising a VH region comprising the sequence of SEQ ID NO:22 and a VL region comprising the sequence of SEQ ID NO:26 (**060**)
> c) an antibody comprising a VH region comprising the sequence of SEQ ID NO:15 and a VL region comprising the sequence of SEQ ID NO:19 (**059**), and
> d) an antibody comprising a VH region comprising the sequence of SEQ ID NO:36 and a VL region comprising the sequence of SEQ ID NO:40 (**111**).

In separate and specific embodiments, the anti-HER2 antibody of the preceding embodiment fully cross-blocks, preferably bind to the same epitope as, antibody 005, 060, 059, 111, or a combination thereof.

The anti-HER2 antibody, when conjugated directly or indirectly to a therapeutic moiety, may be capable of killing tumor cells expressing a lower average amount of HER2 copies per cell than AU565 cells, such as an average of about 500,000 or less, 100,000 or less, or 30,000 or less copies of HER2 per cell (when determined, e.g., as referred to in Example 12), at concentrations where non-conjugated antibody does not induce killing of the cells, preferably when determined as described in Example 17.

The antibody of the preceding embodiment kills at least 80% of A431 cells when determined as described in Example 18, and cross-blocks at least one antibody selected from

> a) an antibody comprising a VH region comprising the sequence of SEQ ID NO:1 and a VL region comprising the sequence of SEQ ID NO:5 (**005**), and
> b) an antibody comprising a VH region comprising the sequence of SEQ ID NO:22 and a VL region comprising the sequence of SEQ ID NO:26 (**060**).

In separate and specific embodiments, the antibody of the preceding embodiment fully cross-blocks, preferably bind to the same epitope as, antibody 005, 060, or a combination thereof.

The anti-HER2 antibody may be internalized by tumor cells expressing HER2, such as AU565 cells, more than trastuzumab is, preferably more than twice or three times the amount of internalized trastuzumab, preferably when determined according to Example 19, and may cross-block at least one antibody selected from the group consisting of:

> a) an antibody comprising a VH region comprising the sequence of SEQ ID NO:1 and a VL region comprising the sequence of SEQ ID NO:5 (**005**)
> b) an antibody comprising a VH region comprising the sequence of SEQ ID NO:8 and a VL region comprising the sequence of SEQ ID NO:11 (**006**)
> c) an antibody comprising a VH region comprising the sequence of SEQ ID NO:15 and a VL region comprising the sequence of SEQ ID NO:19 (**059**)
> d) an antibody comprising a VH region comprising the sequence of SEQ ID NO:22 and a VL region comprising the sequence of SEQ ID NO:26 (**060**)
> e) an antibody comprising a VH region comprising the sequence of SEQ ID NO:29 and a VL region comprising the sequence of SEQ ID NO:33 (**106**)
> f) an antibody comprising a VH region comprising the sequence of SEQ ID NO:36 and a VL region comprising the sequence of SEQ ID NO:40 (**111**).

In separate and specific embodiments, the antibody of the preceding embodiment fully cross-blocks, preferably bind to the same epitope as, antibody 005, 006, 059, 060, 106, 111, or a combination thereof.

Exemplary bispecific antibodies

The antibody may be a bispecific antibody, comprising (i) a first antigen-binding region of a first HER2 antibody as defined herein, and (ii) a second antigen-binding region of a second HER2 antibody as defined herein, wherein the first antigen-binding region binds to a different epitope than the second antigen-binding region.

The first antigen-binding region may comprise a VH region comprising a CDR3 sequence of an antibody of cross-block 1, 2, 3 or 4 as defined herein, such as SEQ ID NO: 4, 25, 66 or 168 (**169, 025, 153, or 005**).

The first antigen-binding region may comprise a VH region comprising CDR1, CDR2 and CDR3 sequences of an antibody of cross-block 1, 2, 3 or 4 as defined herein, such as CDR1, CDR2, and CDR3 sequences SEQ ID NOs: 2, 3 and 4 (**169**), or CDR1, CDR2 and CDR3 sequences of SEQ ID NOs:23, 24 and 25 (**025**), or CDR1, CDR2 and CDR3 sequences of SEQ ID NOs: 64, 65 and 66 (**153**), or CDR1, CDR2 CDR3 sequence of SEQ ID NOs: 166, 167 and 168 (**005**).

The first antigen-binding region may comprise a VH region comprising a CDR3 sequence of an antibody of cross-block 1, 2, 3 or 4 as defined herein, such as CDR3 sequence an antibody of cross-block 1 of SEQ ID NO: 11 (**050**), or SEQ ID NO: 18 (**084**); or a CDR3 sequence of an antibody of cross-block 2 of SEQ ID NO: 31 (**091**), or SEQ ID NO: 38 (**129**), or a CDR3 sequence of an antibody of cross-block 3 of SEQ ID NO: 45 (**127**), or SEQ ID NO:52 (**159**), or SEQ ID NO:59 (**098**), or SEQ ID NO:73 (**132**), or a CDR3 sequence of an antibody of cross-block 4 of SEQ ID NO:175 (**006**), SEQ ID NO: 182 (**059**), SEQ ID NO:189 (**060**), SEQ ID NO:196 (**106**), or SEQ ID NO:203 (**111**).

The first antigen-binding region may comprise a VH region comprising CDR1, CDR2 and CDR3 sequences of an antibody of cross-block 1, 2 or 3 as defined herein, such as CDR1, CDR2, and CDR3 sequences SEQ ID NOs: 2, 3 and 4 (**169**), or CDR1, CDR2 and CDR3 sequences of SEQ ID NOs:23, 24 and 25 (**025**), or CDR1, CDR2 and CDR3 sequences of SEQ ID NOs: 64, 65 and 66 (**153**), or CDR1, CDR2 CDR3 sequence of SEQ ID NOs: 170, GAS and 171 (**005**).

The first antigen-binding region may comprise a VH region comprising CDR1, CDR2 and CDR3 sequences of an antibody of cross-block 1, 2, 3 or 4 as defined herein a VL region comprising CDR1, CDR2 and CDR3 sequences of an antibody of cross-block 1, 2, 3 or 4 as defined herein.

The first antigen-binding region may comprise a VH region comprising CDR1, CDR2 and CDR3 sequences of an antibody of cross-block 1, 2, 3 or 4 as defined herein, such as CDR1, CDR2, and CDR3 sequences of an antibody of cross-block 1 of SEQ ID NOs: 9, 10 and 11 (**050**), or SEQ ID NOs: 16, 17 and 18 (**084**); or CDR1, CDR2, and CDR3 sequences of an antibody of cross-block 2 of SEQ ID NOs: 30, 163 and 31 (**091**), or SEQ ID NOs: 36, 37 and 38 (**129**), or CDR1, CDR2, and CDR3 sequences of an antibody of cross-block 3 SEQ ID NOs: 43, 44 and 45 (**127**), or SEQ ID NOs:50, 51 and 52 (**159**), or SEQ ID NOs:57, 58 and 59 (**098**), or SEQ ID NOs:71, 72 and 73 (**132**), or CDR1, CDR2 and CDR3 sequences of an antibody of cross-block 4 such as SEQ ID NOS: 173, 174,_and 175 (**006**), SEQ ID NOS: 180, 181 , and 182 (**059**), SEQ ID NOS: 187, 188, and 189 (**060**), SEQ ID NOS: 194, 195, and 196 (**106**), or SEQ ID NOS:201, 202, and 203 (**111**).

The first antigen-binding region may comprise a VH region and a VL region selected from the group consisting of:

a) a VH region comprising the CDR1, CDR2, and CDR3 sequences of SEQ ID NOs: 2, 3 and 4; and a VL region comprising the CDR1, CDR2 and CDR3 sequences of SEQ ID: 6, DAS and SEQ ID NO:7, respectively (**169**);
b) a VH region comprising the CDR1, CDR2, and CDR3 sequences of SEQ ID NOs: 23, 24 and 25; and a VL region comprising the CDR1, CDR2 and CDR3 sequences of SEQ ID NO: 27, AAS and SEQ ID NO:28, respectively (**025**);
c) a VH region comprising the CDR1, CDR2 and CDR3 sequences of SEQ ID NOs:64, 65 and 66; and a VL region comprising the CDR1, CDR2 and CDR3 sequences of SEQ ID NO: 68, DAS and SEQ ID NO:69 (**153**); and
d) a VH region comprising the CDR1, CDR2 and CDR3 sequences of SEQ ID NOs:166, 167 and 168; and a VL region comprising the CDR1, CDR2 and CDR3 sequences of SEQ ID NO: 170, GAS and SEQ ID NO:171 (**005**).

The first antigen-binding region comprises a VH region and a VL region selected from the group consisting of:

a) a VH region comprising the CDR1, CDR2 and CDR3 sequences of SEQ ID NOs:9, 127 and 11, such as the CDR1, CDR2 and CDR3 sequences of SEQ ID NOS: 9, 10 and 11 (**050**); optionally where the VH region is derived from an IgHV3-23-1 germline;
b) a VH region comprising the CDR1, CDR2 and CDR3 sequences of SEQ ID NOs:128, 129 and 130, such the CDR1, CDR2 and CDR3 sequences of SEQ ID NOs:16, 17 and 18, respectively (**084**), optionally where the VH region is derived from an IgHV1-69-04 germline; and
c) a VH region comprising the CDR1, CDR2 and CDR3 sequences of SEQ ID NOs:137, 138 and 139, such the CDR1, CDR2 and CDR3 sequences of SEQ ID NOs:30, 163, and 31, respectively (**091**), optionally where the VH region is derived from an IgHV4-34-01 germline; and
d) a VH region comprising the CDR1, CDR2, and CDR3 sequences of SEQ ID NOs:140, 141 and 142, such as the CDR1, CDR2, and CDR3 sequences of SEQ ID NOs: 36, 37 and 38 (**129**), respectively, optionally where the VH region is derived from an IgHV3-30-01 germline.
e) a VH region comprising the CDR1, CDR2 and CDR3 sequences of SEQ ID NOs:146, 147 and 148, such as the CDR1, CDR2 and CDR3 sequences of SEQ ID NOS: 43, 44 and 45 (**127**); optionally where the VH region is derived from an IgHV5-51-01 germline;
f) a VH region comprising the CDR1, CDR2 and CDR3 sequences of SEQ ID NOs:149, 51 and 52, such as the CDR1, CDR2 and CDR3 sequences of SEQ ID NOs:50, 51 and 52, respectively (**159**), optionally where the VH region is derived from an IgHV5-51-01 germline;
g) a VH region comprising the CDR1, CDR2, and CDR3 sequences of SEQ ID NOs:143, 144 and 145, such as the CDR1, CDR2, and CDR3 sequences of SEQ ID NOs: 57, 58 and 59 (**098**), respectively, optionally where the VH region is derived from an IgHV3-23-01 germline;
h) a VH region comprising the CDR1, CDR2, and CDR3 sequences of SEQ ID NOs:71, 150 and 151, such as the CDR1, CDR2, and CDR3 sequences of SEQ ID NOs: 71, 72 and 73 (**132**), respectively, optionally where the VH region is derived from an IgHV1-18-01 germline;
i) a VH region comprising the CDR1, CDR2 and CDR3 sequences of SEQ ID NOs:221, 222 and 223, such as the CDR1, CDR2, and CDR3 sequences of SEQ ID NOs:187, 188 and 189, respectively (**060**), optionally where the VH region is derived from an IgHV5-51-1 germline;
j) A VH region comprising the CDR1, CDR2, and CDR3 sequences of SEQ ID NOs:194, 195 and 196, respectively (**106**), optionally where the VH region is derived from an IgHV5-51-1 germline;
k) a VH region comprising the CDR1, CDR2 and CDR3 sequences of SEQ ID NOs:224, 225 and 226, such the CDR1, CDR2 and CDR3 sequences of SEQ ID NOs:173, 174, and 175, respectively (**006**), optionally where the VH region is derived from an IgHV3-23-1 germline;
l) a VH region comprising the CDR1, CDR2, and CDR3 sequences of SEQ ID NOs:227, 228, and 229, such as the CDR1, CDR2, and CDR3 sequences of SEQ ID NOs: 180, 181 and 182 (**059**), respectively, optionally where the VH region is derived from an IgHV1-18-1 germline; and
m) a VH region comprising the CDR1, CDR2, and CDR3 sequences of SEQ ID NOs:230, 202 and 231, such as the CDR1, CDR2, and CDR3 sequences of SEQ ID NOs: 201, 202 and 203 (**111**), respectively, optionally where the VH region is derived from an IgHV1-69-4 germline.

The second antigen-binding region may be as described for the first antigen-binding region, but wherein the second antigen-binding region binds to a different epitope than the first antigen-binding region. The second antigen-binding region may be from trastuzumab or pertuzumab, comprising the VH and/or VL CDR1, 2 and 3 sequences or VH and/or VL sequences of trastuzumab or pertuzumab.

The bispecific antibody may comprise a first antigen-binding region and a second antigen-binding region, which first and second antigen-binding regions bind different epitopes on human epidermal growth factor receptor 2 (HER2), and wherein each of the first and second antigen-binding region block the binding to soluble HER2 of a reference antibody independently selected from the group consisting of:

a) an antibody comprising a VH region comprising the sequence of SEQ ID NO:63 and a VL region comprising the sequence of SEQ ID NO:67 (**153**),
b) an antibody comprising a variable heavy (VH) region comprising the sequence of SEQ ID NO:165 and a variable light (VL) region comprising the sequence of SEQ ID NO:169 (**005**),
c) an antibody comprising a VH region comprising the sequence of SEQ ID NO:1 and a VL region comprising the sequence of SEQ ID NO:5 (**169**), and
d) an antibody comprising a VH region comprising the sequence of SEQ ID NO:22 and a VL region comprising the sequence of SEQ ID NO:26 (**025**).

The bispecific antibody amy be one, wherein at least one of said first and second antigen-binding regions block the binding to soluble HER2 of an antibody of (a).

The bispecific antibody may be one, wherein at least one of said first and second antigen-binding regions block the binding to soluble HER2 of an antibody of (b).

The bispecific antibody may be one, wherein at least one of said first and second antigen-binding regions block the binding to soluble HER2 of an antibody of (c).

The bispecific antibody may be one, wherein at least one of said first and second antigen-binding regions block the binding to soluble HER2 of an antibody of (d).

The bispecific antibody may be one, wherein

(i) the first antigen-binding region blocks the binding to soluble HER2 of an antibody of (a) and the second antigen-binding region blocks the binding to soluble HER2 of an antibody of (b), or vice versa;
(ii) the first antigen-binding region blocks the binding to soluble HER2 of an antibody of (a) and the second antigen-binding region blocks the binding to soluble HER2 of an antibody of (c), or vice versa;
(iii) the first antigen-binding region blocks the binding to soluble HER2 of an antibody of (a) and the second antigen-binding region blocks the binding to soluble HER2 of an antibody of (d), or vice versa;
(iv) the first antigen-binding region blocks the binding to soluble HER2 of an antibody of (b) and the second antigen-binding region blocks the binding to soluble HER2 of an antibody of (c), or vice versa;
(v) the first antigen-binding region blocks the binding to soluble HER2 of an antibody of (b) and the second antigen-binding region blocks the binding to soluble HER2 of an antibody of (d), or vice versa;
(vi) the first antigen-binding region blocks the binding to soluble HER2 of an antibody of (c) and the second antigen-binding region blocks the binding to soluble HER2 of an antibody of (d), or vice versa.

The bispecific antibody may be one, wherein the first and second antigen-binding regions each comprises VH CDR1, CDR2, and CDR3 sequences independently selected from the group consisting of:

a) SEQ ID NOs:64, 65 and 66, respectively (**153**);
b) SEQ ID NOS: 43, 44 and 45, respectively (**127**);
c) SEQ ID NOs:50, 51 and 52, respectively (**159**);
d) SEQ ID NOs: 57, 58 and 59, respectively (**098**);
e) SEQ ID NOs: 71, 72 and 73, respectively (**132**)
f) SEQ ID NOs: 166, 167 and 168, respectively (**005**);
g) SEQ OD NOS: 173, 174, and 175, respectively (**006**);
h) SEQ ID NOS: 180, 181, and 182, respectively (**059**);
i) SEQ ID NOS:187, 188, and 189, respectively (**060**);
j) SEQ ID NOS:194, 195, and 196, respectively (**106**);
k) SEQ ID NOS:201, 202, and 203, respectively (**111**);
I) SEQ ID NOs: 2, 3 and 4, respectively (**169**);

m) SEQ ID NOS: 9, 10 and 11, respectively (**050**);
n) SEQ ID NOs: 16, 17 and 18, respectively (**084**);
o) SEQ ID NOS: 23, 24 and 25, respectively (**025**);
p) SEQ ID NOs:30, 163, and 31, respectively (**091**);
q) SEQ ID NOs: 36, 37 and 38, respectively (**129**);
r) the VH CDR1, CDR2 and CDR3 sequences of trastuzumab; and
s) the VH CDR1, CDR2 and CDR3 sequences of pertuzumab,

with the proviso that when the first antigen-binding region is from trastuzumab, the second antigen-binding region is not from pertuzumab, and vice versa.

For example, the first and second antigen-binding regions may each comprise a VH region and a VL region independently selected from the group consisting of

a) a VH region comprising the CDR1, CDR2 and CDR3 sequences of SEQ ID NOs:64, 65 and 66, respectively; and a VL region comprising the CDR1, CDR2 and CDR3 sequences of SEQ ID NOs:68, DAS, and SEQ ID NO:69, respectively (**153**);
b) a VH region comprising the CDR1, CDR2 and CDR3 sequences of SEQ ID NOs:43, 44 and 45, respectively; and a VL region comprising the CDR1, CDR2 and CDR3 sequences of SEQ ID NOs:47, AAS, and SEQ ID NO:48, respectively (**127**);
c) a VH region comprising the CDR1, CDR2 and CDR3 sequences of SEQ ID NOs:50, 51 and 52, respectively; and a VL region comprising the CDR1, CDR2 and CDR3 sequences of SEQ ID NOs:54, AAS, and SEQ ID NO:55, respectively (**159**);
d) a VH region comprising the CDR1, CDR2 and CDR3 sequences of SEQ ID NOs:57, 58 and 59, respectively; and a VL region comprising the CDR1, CDR2 and CDR3 sequences of SEQ ID NOs:60, AAS, and SEQ ID NO:61, respectively (**098**);
e) a VH region comprising the CDR1, CDR2 and CDR3 sequences of SEQ ID NOs:71, 72 and 73, respectively; and a VL region comprising the CDR1, CDR2 and CDR3 sequences of SEQ ID NOs:75, DAS, and SEQ ID NO:76, respectively (**132**);
f) a VH region comprising the CDR1, CDR2 and CDR3 sequences of SEQ ID NOs:166, 167 and 168, respectively; and a VL region comprising the CDR1, CDR2 and CDR3 sequences of SEQ ID NO: 170, GAS and SEQ ID NO:171, respectively (**005**);
g) a VH region comprising the CDR1, CDR2 and CDR3 sequences of SEQ ID NOs: 173, 174 and 175, respectively; and a VL region comprising the CDR1, CDR2 and CDR3 sequences of SEQ ID NOs:177, DAS, and SEQ ID NO:178, respectively (**006**);
h) a VH region comprising the CDR1, CDR2 and CDR3 sequences of SEQ ID NOs: 180, 181 and 182, respectively; and a VL region comprising the CDR1, CDR2 and CDR3 sequences of SEQ ID NOs: 184, GAS, and SEQ ID NO:185, respectively (**059**);
i) a VH region comprising the CDR1, CDR2 and CDR3 sequences of SEQ ID NOs:187, 188 and 189, respectively; and a VL region comprising the CDR1, CDR2 and CDR3 sequences of SEQ ID NOs: 191, GAS, and SEQ ID NO:192, respectively (**060**);
j) a VH region comprising the CDR1, CDR2 and CDR3 sequences of SEQ ID NOs: 194, 195 and 196, respectively; and a VL region comprising the CDR1, CDR2 and CDR3 sequences of SEQ ID NOs: 198, GAS, and SEQ ID NO:199, respectively (**106**);
k) a VH region comprising the CDR1, CDR2 and CDR3 sequences of SEQ ID NOs:201, 202 and 203, respectively; and a VL region comprising the CDR1, CDR2 and CDR3 sequences of SEQ ID NOs:205, GAS, and SEQ ID NO:206, respectively (**111**);
l) a VH region comprising the CDR1, CDR2 and CDR3 sequences of SEQ ID NOs:2, 3 and 4, respectively; and a VL region comprising the CDR1, CDR2 and CDR3 sequences of SEQ ID NOs:6, DAS, and SEQ ID NO:7, respectively (**169**);
m) a VH region comprising the CDR1, CDR2 and CDR3 sequences of SEQ ID NOs:9, 10 and 11, respectively; and a VL region comprising the CDR1, CDR2 and CDR3 sequences of SEQ ID NOs: 13, AAS, and SEQ ID NO:14, respectively (**050**);
n) a VH region comprising the CDR1, CDR2 and CDR3 sequences of SEQ ID NOs:16, 17 and 18, respectively; and a VL region comprising the CDR1, CDR2 and CDR3 sequences of SEQ ID NOs:20, VAS, and SEQ ID NO:21, respectively (**084**);
o) a VH region comprising the CDR1, CDR2 and CDR3 sequences of SEQ ID NOs:23, 24 and 25, respectively; and a VL region comprising the CDR1, CDR2 and CDR3 sequences of SEQ ID NOs:27, AAS, and SEQ ID NO:28, respectively (**025**);

p) a VH region comprising the CDR1, CDR2 and CDR3 sequences of SEQ ID NOs:30, 163 and 31, respectively; and a VL region comprising the CDR1, CDR2 and CDR3 sequences of SEQ ID NOs:33, AAS, and SEQ ID NO:34, respectively (**091**);

q) a VH region comprising the CDR1, CDR2 and CDR3 sequences of SEQ ID NOs:36, 37 and 38, respectively; and a VL region comprising the CDR1, CDR2 and CDR3 sequences of SEQ ID NOs:40, DAS, and SEQ ID NO:41, respectively (**129**);

t) a VH region comprising the VH CDR1, CDR2 and CDR3 sequences of trastuzumab and a VL region comprising the VL CDR1, CDR2 and CDR3 sequences of trastuzumab; and

u) a VH region comprising the VH CDR1, CDR2 and CDR3 sequences of pertuzumab and a VL region comprising the VL CDR1, CDR2 and CDR3 sequences of pertuzumab;

with the proviso that when the first antigen-binding region is from trastuzumab, the second antigen-binding region is not from pertuzumab, and vice versa.

Even more particularly, the first and the second antigen-binding regions may each comprise a VH region and a VL region independently selected from the group consisting of

a) a VH region comprising the CDR1, CDR2 and CDR3 sequences of SEQ ID NOs:64, 65 and 66, respectively; and a VL region comprising the CDR1, CDR2 and CDR3 sequences of SEQ ID NOs:68, DAS, and SEQ ID NO:69, respectively (**153**)

b) a VH region comprising the CDR1, CDR2 and CDR3 sequences of SEQ ID NOs: 166, 167 and 168, respectively; and a VL region comprising the CDR1, CDR2 and CDR3 sequences of SEQ ID NOs: 170, GAS and SEQ ID NO:171, respectively (**005**);

c) a VH region comprising the CDR1, CDR2 and CDR3 sequences of SEQ ID NOs:2, 3 and 4, respectively; and a VL region comprising the CDR1, CDR2 and CDR3 sequences of SEQ ID NOs:6, DAS, and SEQ ID NO:7, respectively (**169**); and

d) a VH region comprising the CDR1, CDR2 and CDR3 sequences of SEQ ID NOs:23, 24 and 25, respectively; and a VL region comprising the CDR1, CDR2 and CDR3 sequences of SEQ ID NOs:27, AAS, and SEQ ID NO:28, respectively (**025**).

The present disclosure relates to a bispecific antibody comprising a first antigen-binding region comprising the VH CDR3 sequence of SEQ ID NO:66 (**153**) and a second antigen-binding region comprising the VH CDR3 sequence of SEQ ID NO:168 (**005**), or vice versa.

The first antigen-binding region may further comprise the VL CDR3 sequence of SEQ ID NO:69 (**153**), and the second antigen-binding region may further comprise the VL CDR3 sequence of SEQ ID NO:171 (**005**).

The first antigen-binding region may further comprise the VH CDR1 sequence of SEQ ID NO:64 and the VH CDR2 sequence of SEQ ID NO:65 (**153**), and the second antigen-binding region may further comprise the VH CDR1 sequence of SEQ ID NO:166 and a VH CDR2 sequence of SEQ ID NO:167 (**005**).

The first antigen-binding region may further comprise the VL CDR1 sequence of SEQ ID NO: 68 and the VL CDR2 sequence of DAS (**153**), and the second antigen-binding region may further comprise the VL CDR1 sequence of SEQ ID NO:170 and a VL CDR2 sequence of GAS (**005**).

The bispecific antibody may comprise a first antigen-binding region comprising a VH region comprising SEQ ID NO:63 and a VL region comprising SEQ ID NO:67 (**153**), and a second antigen-binding region comprising a VH region comprising SEQ ID NO:165 and a VL region comprising SEQ ID NO:169 (**005**), or vice versa.

The present disclosure relates to a bispecific antibody comprising a first antigen-binding region comprising the VH CDR3 sequence of SEQ ID NO:66 (**153**) and a second antigen-binding region comprising the VH CDR3 sequence of SEQ ID NO:4 (**169**), or vice versa.

The first antigen-binding region may further comprise the VL CDR3 sequence of SEQ ID NO:69 (**153**), and the second antigen-binding region may further comprise the VL CDR3 sequence of SEQ ID NO:7 (**169**).

The first antigen-binding region may further comprise the VH CDR1 sequence of SEQ ID NO:64 and the VH CDR2 sequence of SEQ ID NO:65 (**153**), and the second antigen-binding region may further comprise the VH CDR1 sequence of SEQ ID NO:2 and a VH CDR2 sequence of SEQ ID NO:3 (**169**).

The first antigen-binding region may further comprise the VL CDR1 sequence of SEQ ID NO: 68 and the VL CDR2 sequence of DAS (**153**), and the second antigen-binding region further comprises the VL CDR1 sequence of SEQ ID NO: 6 and a VL CDR2 sequence of DAS (**169**).

The bispecific antibody may comprise a first antigen-binding region comprising a VH region comprising SEQ ID NO:63 and a VL region comprising SEQ ID NO:67 (**153**), and a second antigen-binding region comprising a VH region comprising SEQ ID NO:1 and a VL region comprising SEQ ID NO:5 (**169**), or vice versa.

The present disclosure relates to a bispecific antibody comprising a first antigen-binding region comprising the VH CDR3 sequence of SEQ ID NO:168 (**005**) and a second antigen-binding region comprising the VH CDR3 sequence of SEQ ID NO:4 (**169**), or vice versa. The first antigen-binding region may further comprise the VL CDR3 sequence of SEQ ID NO:171 (**005**), and the second antigen-binding region may further comprise the VL CDR3 sequence of SEQ ID NO:7 (**169**).

The first antigen-binding region may further comprise the VH CDR1 sequence of SEQ ID NO:166 and the VH CDR2 sequence of SEQ ID NO:167 (**005**), and the second antigen-binding region may further comprise the VH CDR1 sequence of SEQ ID NO:2 and a VH CDR2 sequence of SEQ ID NO:3 (**169**).

The first antigen-binding region may further comprise the VL CDR1 sequence of SEQ ID NO: 170 and the VL CDR2 sequence of GAS (**005**), and the second antigen-binding region may further comprise the VL CDR1 sequence of SEQ ID NO: 6 and a VL CDR2 sequence of DAS (**169**).

The bispecific antibody may comprise a first antigen-binding region comprising a VH region comprising SEQ ID NO:165 and a VL region comprising SEQ ID NO:169 (**005**), and a second antigen-binding region comprising a VH region comprising SEQ ID NO:1 and a VL region comprising SEQ ID NO:5 (**169**), or vice versa.

The present disclosure relates to a bispecific antibody comprising a first antigen-binding region comprising the VH CDR3 sequence of SEQ ID NO:25 (**025**) and a second antigen-binding region comprising the VH CDR3 sequence of SEQ ID NO:168 (**005**), or vice versa. The first antigen-binding region may further comprise the VL CDR3 sequence of SEQ ID NO:28 (**025**), and the second antigen-binding region may further comprise the VL CDR3 sequence of SEQ ID NO:171 (**005**).

The first antigen-binding region may further comprise the VH CDR1 sequence of SEQ ID NO:23 and the VH CDR2 sequence of SEQ ID NO:24 (**025**), and the second antigen-binding region further may comprise the VH CDR1 sequence of SEQ ID NO:166 and a VH CDR2 sequence of SEQ ID NO:167 (**005**).

The first antigen-binding region may further comprise the VL CDR1 sequence of SEQ ID NO: 27 and the VL CDR2 sequence of AAS (**025**), and the second antigen-binding region may further comprise the VL CDR1 sequence of SEQ ID NO: 170 and a VL CDR2 sequence of GAS (**005**).

The bispecific antibody may comprise a first antigen-binding region comprising a VH region comprising SEQ ID NO:22 and a VL region comprising SEQ ID NO:26 (**025**), and a second antigen-binding region comprising a VH region comprising SEQ ID NO:165 and a VL region comprising SEQ ID NO:169 (**005**), or vice versa.

The present disclosure relates to a bispecific antibody comprising a first antigen-binding region comprising the VH CDR3 sequence of SEQ ID NO:25 (**025**) and a second antigen-binding region comprising the VH CDR3 sequence of SEQ ID NO:66 (**153**), or vice versa. The first antigen-binding region may further comprise the VL CDR3 sequence of SEQ ID NO:28 (**025**), and the second antigen-binding region may further comprise the VL CDR3 sequence of SEQ ID NO:69 (**153**).

The first antigen-binding region may further comprise the VH CDR1 sequence of SEQ ID NO:23 and the VH CDR2 sequence of SEQ ID NO:24 (**025**), and the second antigen-binding region may further comprise the VH CDR1 sequence of SEQ ID NO:64 and a VH CDR2 sequence of SEQ ID NO:65 (**153**).

The first antigen-binding region may further comprise the VL CDR1 sequence of SEQ ID NO: 27 and the VL CDR2 sequence of AAS (**025**), and the second antigen-binding region may further comprise the VL CDR1 sequence of SEQ ID NO: 68 and a VL CDR2 sequence of DAS (**153**).

The bispecific antibody may comprise a first antigen-binding region comprising a VH region comprising SEQ ID NO:22 and a VL region comprising SEQ ID NO:26 (**025**), and a second antigen-binding region comprising a VH region comprising SEQ ID NO:63 and a VL region comprising SEQ ID NO:67 (**153**), or vice versa.

The present disclosure relates to a bispecific antibody comprising a first antigen-binding region comprising the VH CDR3 sequence of SEQ ID NO:25 (**025**) and a second antigen-binding region comprising the VH CDR3 sequence of SEQ ID NO:4 (**169**), or vice versa. The first antigen-binding region may further comprise the VL CDR3 sequence of SEQ ID NO:28 (**025**), and the second antigen-binding region may further comprise the VL CDR3 sequence of SEQ ID NO:7 (**169**).

The first antigen-binding region may further comprise the VH CDR1 sequence of SEQ ID NO:23 and the VH CDR2 sequence of SEQ ID NO:24 (**025**), and the second antigen-binding region may further comprise the VH CDR1 sequence of SEQ ID NO:2 and a VH CDR2 sequence of SEQ ID NO:3 (**169**).

The first antigen-binding region may further comprise the VL CDR1 sequence of SEQ ID NO: 27 and the VL CDR2 sequence of AAS (**025**), and the second antigen-binding region may further comprise the VL CDR1 sequence of SEQ ID NO: 6 and a VL CDR2 sequence of DAS (**169**).

The present disclosure relates to a bispecific antibody comprising a first antigen-binding region comprising a VH region comprising SEQ ID NO:22 and a VL region comprising SEQ ID NO:26 (**025**), and a second antigen-binding region comprising a VH region comprising SEQ ID NO:1 and a VL region comprising SEQ ID NO:5 (**169**), or vice versa.

The present disclosure also relates to a bispecific antibody comprising a first antigen-binding region which binds an epitope in HER2 Domain II and a second antigen-binding region which binds an epitope in HER2 Domain III or IV.

The second antigen-binding region may bind an epitope in HER2 Domain III.

The second antigen-binding region may bind an epitope in HER2 Domain IV.

The first antigen-binding region may block the binding to soluble HER2 of a reference antibody comprising a VH region comprising the sequence of SEQ ID NO:63 and a VL region comprising the sequence of SEQ ID NO:67 (**153**).

The first and/or second antigen-binding region may comprise a VH region and, optionally, a VL region, of any of the embodiments described above.

The present disclosure also relates to a bispecific antibody, wherein the first and second antigen-binding regions comprise human antibody VH sequences and, optionally, human antibody VL sequences.

The present disclosure relates to a bispecific antibody, wherein the first and second antigen-binding regions are from heavy-chain antibodies.

The present disclosure relates to a bispecific antibody, wherein the first and second antigen-binding regions comprise a first and second light chain.

The present invention disclosure to a bispecific antibody, wherein said first and second light chains are different.

The bispecific antibody may enhance HER2 downmodulation, in particular more than their monospecific counterparts, e.g. the antibody enhanced HER2 downmodulation by more 20%, such as more than 30% or more than 40% when determined as described in example 22, preferably wherein the antibody binds to the same epitopes as bispecific antibody selected from the group consisting of IgG1-005-ITL x IgG1-169-K409R, IgG1-025-ITL x IgG1-005-K409R, IgG1-025-ITL x IgG1-153-K409R, IgG1-025-ITL x IgG1-169-K409R, IgG1-153-ITL x IgG1-005-K409R; and IgG1-153-ITL x IgG1-169-K409R.

The bispecific antibody may specifically bind HER2-expressing AU565 cells and inhibits ligand-induced proliferation of the cells when determined as described in Example 24, and may bind the same epitopes as at least one bispecific antibody selected from the group consisting of: IgG1-005-ITL x IgG1-169-K409R, IgG1-025-ITL x IgG1-005-K409R, IgG1-025-ITL x IgG1-153-K409R, IgG1-025-ITL x IgG1-169-K409R, IgG1-153-ITL x IgG1-005-K409R; and IgG1-153-ITL x IgG1-169-K409R. In particular the bispecific antibody may inhibit proliferation of the AU565 cells more than their monospecific counterparts and is selected from the group consisting of IgG1-005-ITL x IgG1-169-K409R and IgG1-025-ITL x IgG1-005-K409R.

The bispecific antibody may induce PBMC-mediated cytotoxicity when determined as described in Example 31, and may bind the same epitopes as at least one bispecific antibody selected from the group consisting of: IgG1-153-ITL x IgG1-169-K409R and IgG1-005-ITL x IgG1-153-K409R. In particular the bispecific antibody induces higher levels of PBMC-mediated cytotoxicity than their monospecific counterparts, optionally more than the combination of their monospecific counterparts.

The bispecific antibody may reduce tumor growth and/or results in a better survival of mice in the NCI-N87 human gastric carcinoma xenograft model described in Example 32, and may bind the same epitopes as at least one bispecific antibody selected from the group consisting of: IgG1-153-ITL x IgG1-169-K409R and IgG1-005-ITL x IgG1-153-K409R. In particular the bispecific antibody may reduce tumor growth more than their monospecific counterparts, optionally more than the combination of their monospecific counterparts.

The present invention provides a bispecific antibody comprising a first antigen-binding region and a second antigen-binding region, which first and second antigen-binding regions bind different epitopes on human epidermal growth factor receptor 2 (HER2), and wherein the first antigen-binding region is selected from the group consisting of:

a) an antibody comprising a VH region comprising the sequence of SEQ ID NO:63 and a VL region comprising the sequence of SEQ ID NO:67 (**153**),
b) an antibody comprising a variable heavy (VH) region comprising the sequence of SEQ ID NO:165 and a variable light (VL) region comprising the sequence of SEQ ID NO: 169 (**005**), and
c) an antibody comprising a VH region comprising the sequence of SEQ ID NO:22 and a VL region comprising the sequence of SEQ ID NO:26 (**025**),

wherein the second antigen binding region comprises a VH region comprising SEQ ID NO: 1, and a VL region comprising SEQ ID NO:5 (**169**).

## Fc regions

The bispecific HER2xHER2 antibody may further comprise a first and a second Fc-region, which may be comprised in a first and a second Fab arm which respectively further comprise the first and second antigen-binding regions described above (or vice versa).Hence the bispecific antibody may comprise a first Fab-arm comprising a first antigen-binding region and a first Fc region, and a second Fab-arm comprising a second antigen-binding region and a second Fc region. Alternatively, the bispecific antibody may comprise a first Fab-arm comprising a first antigen-binding region and a second Fc region, and a second Fab-arm comprising a second antigen-binding region and a first Fc region.

The first and second Fc-regions of Fab-arms may be of any isotype, including, but not limited to, IgG1, IgG2, IgG3 and IgG4. Each of the first and second Fc regions may be of the IgG4 isotype or derived therefrom, optionally with one or more mutations or modifications. Each of the first and second Fc regions may be of the IgG1 isotype or derived therefrom, optionally with one or more mutations or modifications. One of the Fc regions may be of the IgG1 isotype and the other of the IgG4 isotype, or may be derived from such respective isotype, optionally with one or more mutations or modifications.

One or both Fc-regions may comprise an IgG1 wildtype sequence (SEQ ID NO:234).

One or both of the Fc regions may comprise a mutation removing the acceptor site for Asn-linked glycosylation or is otherwise manipulated to change the glycosylation properties. For example, in an IgG1 Fc-region, an N297Q mutation can be used to remove an Asn-linked glycosylation site. Accordingly, one or both Fc regions may comprise an IgG1 wildtype sequence with an N297Q mutation (SEQ ID NO: 235).

One or both of the Fc regions may be glyco-engineered to reduce fucose and thus enhance ADCC, *e.g.* by addition of compounds to the culture media during antibody production as described in US2009317869 or as described in van Berkel et al. (2010) Biotechnol. Bioeng. 105:350 or by using FUT8 knockout cells, *e.g.* as described in Yamane-Ohnuki et al (2004) Biotechnol. Bioeng 87:614. ADCC may alternatively be optimized using the method described by Umaña et al. (1999) Nature Biotech 17:176. One or both of the Fc-regions may have been engineered to enhance complement activation, *e.g.* as described in Natsume et al. (2009) Cancer Sci. 100:2411.

The first or second antigen-binding regions or a part thereof, *e.g.* one or more CDRs, may be of a species in the family Camelidae, see WO2010001251, or a species of cartilaginous fish, such as the nurse shark. In one embodiment, the first and second antigen-binding regions or heavy chains are from heavy-chain antibodies.

The first and/or second Fc-region may be conjugated to a drug, a prodrug or a toxin or contains an acceptor group for the same. Such acceptor group may *e.g.* be an unnatural amino acid.

The bispecific antibody of the invention may comprise a first Fc-region comprising a first CH3 region, and a second Fc-region comprising a second CH3 region, wherein the sequences of the first and second CH3 regions are different and are such that the heterodimeric interaction between said first and second CH3 regions is stronger than each of the homodimeric interactions of said first and second CH3 regions. More details on these interactions and how they can be achieved are provided in PCT/EP2011/056388, published as WO 11/131746.

As described further herein and in the Examples, a stable bispecific HER2xHER2 molecule can be obtained at high yield using a particular method on the basis of two homodimeric starting HER2 antibodies containing only a few, fairly conservative, asymmetrical mutations in the CH3 regions. Asymmetrical mutations mean that the sequences of said first and second CH3 regions contain amino acid substitutions at non-identical positions.

The first Fc-region may have an amino acid substitution at a position selected from the group consisting of: 366, 368, 370, 399, 405, 407 and 409, and the second Fc-region may have an amino acid substitution at a position selected from the group consisting of: 366, 368, 370, 399, 405, 407 and 409, and wherein the first and second Fc-regions are not substituted in the same positions.

The first Fc-region may have an amino acid substitution at position 366, and said second Fc-region may have an amino acid substitution at a position selected from the group consisting of: 368, 370, 399, 405, 407 and 409. The amino acid at position 366 may be selected from Ala, Asp, Glu, His, Asn, Val, or Gln.

The first Fc-region may have an amino acid substitution at position 368, and said second Fc-region may have an amino acid substitution at a position selected from the group consisting of: 366, 370, 399, 405, 407 and 409.

The first Fc-region may have an amino acid substitution at position 370, and said second HER2 antibody may have an amino acid substitution at a position selected from the group consisting of: 366, 368, 399, 405, 407 and 409.

The first Fc-region may have an amino acid substitution at position 399, and said second Fc-region my have an amino acid substitution at a position selected from the group consisting of: 366, 368, 370, 405, 407 and 409.

The first Fc-region may have an amino acid substitution at position 405, and said second Fc-region may have an amino acid substitution at a position selected from the group consisting of: 366, 368, 370, 399, 407 and 409.

The first Fc-region may have an amino acid substitution at position 407, and said second Fc-region may have an amino acid substitution at a position selected from the group consisting of: 366, 368, 370, 399, 405, and 409.

The first Fc-region may have an amino acid substitution at position 409, and said second Fc-region may have an amino acid substitution at a position selected from the group consisting of: 366, 368, 370, 399, 405, and 407.

Accordingly, the sequences of said first and second CH3 regions may contain asymmetrical mutations, *i.e.* mutations at different positions in the two CH3 regions, *e.g.* a mutation at position 405 in one of the CH3 regions and a mutation at position 409 in the other CH3 region.

The first Fc-region may have an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may have an amino-acid substitution at a position selected from the group consisting of: 366, 368, 370, 399, 405 and 407. Said first Fc-region may have an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may have an amino acid other than Phe at position 405, e.g. Lys, Leu, Met, Arg, His, Asp, Glu, Ser, Thr, Asn, Gln, Gly, Pro, Ala, Val, Ile, Tyr, Trp or Cys. In a further embodiment hereof, said first Fc-region may have an amino acid other than Lys, Leu or Met, e.g. Arg, His, Asp, Glu, Ser, Thr, Asn, Gln, Gly, Pro, Ala, Val, Ile, Phe, Tyr, Trp or Cys, at position 409 and said second Fc-region may have an amino acid other than Phe, Arg or Gly, e.g. e.g. Lys, Leu, Met, His, Asp, Glu, Ser, Thr, Asn, Gln, Pro, Ala, Val, Ile, Tyr, Trp or Cys, at position 405.

Said first Fc-region may comprise a Phe at position 405 and an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may comprise an amino acid other than Phe, e.g. Lys, Leu, Met, Arg, His, Asp, Glu, Ser, Thr, Asn, Gln, Gly, Pro, Ala, Val, Ile, Tyr, Trp or Cys, at position 405 and a Lys at position 409. Said first Fc-region may comprise a Phe at position 405 and an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may comprise an amino acid other than Phe, Arg or Gly at position 405, e.g. Lys, Leu, Met, His, Asp, Glu, Ser, Thr, Asn, Gln, Pro, Ala, Val, Ile, Tyr, Trp or Cys, and a Lys at position 409.

Said first Fc-region may comprise a Phe at position 405 and an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may comprise a Leu at position 405 and a Lys at position 409. Said first Fc-region comprises a Phe at position 405 and an Arg at position 409 and said second Fc-region may comprise an amino acid other than Phe, Arg or Gly, e.g. Lys, Leu, Met, His, Asp, Glu, Ser, Thr, Asn, Gln, Pro, Ala, Val, Ile, Tyr, Trp or Cys, at position 405 and a Lys at position 409. Said first Fc-region may comprise Phe at position 405 and an Arg at position 409 and said second Fc-region may comprise a Leu at position 405 and a Lys at position 409.

Said first Fc-region may comprise an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may comprise a Lys at position 409, a Thr at position 370 and a Leu at position 405. In a further embodiment, said first Fc-region comprises an Arg at position 409 and said second Fc-region comprises a Lys at position 409, a Thr at position 370 and a Leu at position 405.

Said first Fc-region may comprise a Lys at position 370, a Phe at position 405 and an Arg at position 409 and said second Fc-region comprises a Lys at position 409, a Thr at position 370 and a Leu at position 405.

Said first Fc-region may comprise an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may comprise a Lys at position 409 and: a) an Ile at position 350 and a Leu at position 405, or b) a Thr at position 370 and a Leu at position 405.

Said first Fc-region may comprise an Arg at position 409 and said second Fc region may comprise a Lys at position 409 and: a) an Ile at position 350 and a Leu at position 405, or b) a Thr at position 370 and a Leu at position 405.

Said first Fc-region may comprise a Thr at position 350, a Lys at position 370, a Phe at position 405 and an Arg at position 409 and said second HER2 antibody may comprise a Lys at position 409 and: a) an Ile at position 350 and a Leu at position 405, or b) a Thr at position 370 and a Leu at position 405.

Said first Fc-region may comprise a Thr at position 350, a Lys at position 370, a Phe at position 405 and an Arg at position 409 and said second Fc-region may comprise an Ile at position 350, a Thr at position 370, a Leu at position 405 and a Lys at position 409.

Said first Fc-region may have an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may have an amino acid other than Tyr, Asp, Glu, Phe, Lys, Gln, Arg, Ser or Thr at position 407, e.g. His, Asn, Gly, Pro, Ala, Val, Ile, Trp, Leu, Met or Cys. Said first Fc-region may have an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may have an Ala, Gly, His, Ile, Leu, Met, Asn, Val or Trp at position 407.

Said first Fc-region may have an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may have a Gly, Leu, Met, Asn or Trp at position 407.

Said first Fc-region may have a Tyr at position 407 and an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may have an amino acid other than Tyr, Asp, Glu, Phe, Lys, Gln, Arg, Ser or Thr at position 407, e.g. His, Asn, Gly, Pro, Ala, Val, Ile, Trp, Leu, Met or Cys, and a Lys at position 409.

Said first Fc-region may have a Tyr at position 407 and an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may have an Ala, Gly, His, Ile, Leu, Met, Asn, Val or Trp at position 407 and a Lys at position 409.

Said first Fc-region may have a Tyr at position 407 and an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and said second Fc-region may have a Gly, Leu, Met, Asn or Trp at position 407 and a Lys at position 409.

Said first Fc-region may have a Tyr at position 407 and an Arg at position 409 and said second Fc-region may have an amino acid other than Tyr, Asp, Glu, Phe, Lys, Gln, Arg, Ser or Thr at position 407, e.g. His, Asn, Gly, Pro, Ala, Val, Ile, Trp, Leu, Met or Cys and a Lys at position 409.

Said first Fc-region has a Tyr at position 407 and an Arg at position 409 and said second Fc-region may have an Ala, Gly, His, Ile, Leu, Met, Asn, Val or Trp at position 407 and a Lys at position 409.

Said first Fc-region may have a Tyr at position 407 and an Arg at position 409 and said second Fc-region may have a Gly, Leu, Met, Asn or Trp at position 407 and a Lys at position 409.

The first Fc-region may have an amino acid other than Lys, Leu or Met at position 409, e.g. Arg, His, Asp, Glu, Ser, Thr, Asn, Gln, Gly, Pro, Ala, Val, Ile, Phe, Tyr, Trp or Cys, and the second Fc-region may have

(i) an amino acid other than Phe, Leu and Met at position 368, e.g. Arg, His, Asp, Glu, Ser, Thr, Asn, Gln, Gly, Pro, Ala, Val, Ile, Lys, Tyr, Trp or Cys or
(ii) a Trp at position 370, or
(iii) an amino acid other than Asp, Cys, Pro, Glu or Gln at position 399, e.g. Arg, His, Ser, Thr, Asn, Gly, Ala, Val, Ile, Phe, Tyr, Trp, Lys, Leu, or Met, or
(iv) an amino acid other than Lys, Arg, Ser, Thr, or Trp at position 366, e.g. Leu, Met, His, Asp, Glu, Asn, Glu, Gly, Pro, Ala, Val, Ile, Phe, Tyr or Cys.

The first Fc-region may have an Arg, Ala, His or Gly at position 409, and the second FC-region may have

(i) a Lys, Gln, Ala, Asp, Glu, Gly, His, Ile, Asn, Arg, Ser, Thr, Val, or Trp at position 368, or
(ii) a Trp at position 370, or
(iii) an Ala, Gly, Ile, Leu, Met, Asn, Ser, Thr, Trp, Phe, His, Lys, Arg or Tyr at position 399 or
(iv) an Ala, Asp, Glu, His, Asn, Val, Gln, Phe, Gly, Ile, Leu, Met, or Tyr at position 366.

The first Fc-region may have an Arg at position 409, and the second Fc-region may have

(i) an Asp, Glu, Gly, Asn, Arg, Ser, Thr, Val, or Trp at position 368, or
(ii) a Trp at position 370, or
(iii) a Phe, His, Lys, Arg or Tyr at position 399, or
(iv) an Ala, Asp, Glu, His, Asn, Val, Gln at position 366.

In addition to the above-specified amino-acid substitutions, said first and second FC-regions may contain further amino-acid substitutions, deletion or insertions relative to wild-type Fc sequences.

Said first and second Fab-arms (or heavy-chain constant domains) comprising the first and second Fc regions may comprise, except for the specified mutations, a sequence independently selected from the following:

a) (IgG1m(a)):

GQPREPQVYTLPPSRDELTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO:236);

b) (IgG1m(f)):

GQPREPQVYTLPPSREEMTKNQVSLTCLVKGFYPSDIAVEWESNGQPENNYKTTPPVLDSDGSF
FLYSKLTVDKSRWQQGNVFSCSVMHEALHNHYTQKSLSLSPGK (SEQ ID NO:237); and

c) (IgG1m(ax)):

GQPREPQVYTLPPSRDELTKNQVSLTCLVKGFYPSDIAVEWESNGQPENNYKTTPPVLDSDGSF
FLYSKLTVDKSRWQQGNVFSCSVMHEGLHNHYTQKSLSLSPGK (SEQ ID NO:238)

Optionally, neither said first nor said second Fc-region comprises a Cys-Pro-Ser-Cys sequence in the (core) hinge region.

Both said first and said second Fc-region may comprise a Cys-Pro-Pro-Cys sequence in the (core) hinge region.

One or both Fab-arms may comprise a sequence separately selected from the following:

a) IgG1 wildtype sequence (SEQ ID NO:234):

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGNVFSCSVMH
EALHNHYTQKSLSLSPGK

b) IgG1 N297Q (SEQ ID NO:235)

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFLF
PPKPKDTLMISRTPEVTCVVVDVSHEDPEVKFNWYVDGVEVHNAKTKPREEQYQSTYRVVSV
LTVLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSKLTVDKSRWQQGNVFSCSVMH
EALHNHYTQKSLSLSPGK

c) IgG1- LFLEDANQPS mut (SEQ ID NO:239)

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEFEGGPSVFLF
PPKPKDTLMISRTPEVTCVVVAVSHEDPEVKFNWYVDGVEVHNAKTKPREEQYQSTYRVVSV

LTVLHQDWLNGKEYKCKVSNKALPASIEKTISKAKGQPREPQVYTLPPSREEMTKNQVSLTCL
VKGFYPSDIAVEWESNGQPENNYKTTPPVLDSDGSFFLYSKLTVDKSRWQQGNVFSCSVMH
EALHNHYTQKSLSLSPGK

d) IgG1- F405L N297Q (SEQ ID NO:240)

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFLF
PPKPKDTLMISRTPEVTCVVVDVSHEDPEVKFNWYVDGVEVHNAKTKPREEQYQSTYRVVSV
LTVLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFLLYSKLTVDKSRWQQGNVFSCSVMH
EALHNHYTQKSLSLSPGK

e) IgG1- K409R N297Q (SEQ ID NO:241)

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFLF
PPKPKDTLMISRTPEVTCVVVDVSHEDPEVKFNWYVDGVEVHNAKTKPREEQYQSTYRVVSV
LTVLHQDWLNGKEYKCKVSNKALPAPIEKTISKAKGQPREPQVYTLPPSREEMTKNQVSLTCL
VKGFYPSDIAVEWESNGQPENNYKTTPPVLDSDGSFFLYSRLTVDKSRWQQGNVFSCSVMH
EALHNHYTQKSLSLSPGK

f) IgG1- F405L LFLEDANQPS (SEQ ID NO:242)

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEFEGGPSVFLF
PPKPKDTLMISRTPEVTCVVVAVSHEDPEVKFNWYVDGVEVHNAKTKPREEQYQSTYRVVSV
LTVLHQDWLNGKEYKCKVSNKALPASIEKTISKAKGQPREPQVYTLPPSREEMTKNQVSLTCL
VKGFYPSDIAVEWESNGQPENNYKTTPPVLDSDGSFLLYSKLTVDKSRWQQGNVFSCSVMH
EALHNHYTQKSLSLSPGK, and

g) IgG1 - K409R LFLEDANQPS (SEQ ID NO:243)

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPEFEGGPSVFLF
PPKPKDTLMISRTPEVTCVVVAVSHEDPEVKFNWYVDGVEVHNAKTKPREEQYQSTYRVVSV
LTVLHQDWLNGKEYKCKVSNKALPASIEKTISKAKGQPREPQVYTLPPSREEMTKNQVSLTCL
VKGFYPSDIAVEWESNGQPENNYKTTPPVLDSDGSFFLYSRLTVDKSRWQQGNVFSCSVMH
EALHNHYTQKSLSLSPGK;

h) IgG1 Fc region - ITL (SEQ ID NO:244)

ASTKGPSVFPLAPSSKSTSGGTAALGCLVKDYFPEPVTVSWNSGALTSGVHTFPAVLQSSGLY
SLSSVVTVPSSSLGTQTYICNVNHKPSNTKVDKRVEPKSCDKTHTCPPCPAPELLGGPSVFLF
PPKPKDTLMISRTPEVTCVVVDVSHEDPEVKFNWYVDGVEVHNAKTKPREEQYNSTYRVVSV
LTVLHQDWLNGKEYKCKVSNKALPAPIEKTISKAKGQPREPQVYILPPSREEMTKNQVSLTCL

VTGFYPSDIAVEWESNGQPENNYKTTPPVLDSDGSFLLYSKLTVDKSRWQQGNVFSCSVMH
EALHNHYTQKSLSLSPGK, AND

i) IgG1 Fc region - K409R (SEQ ID NO:245)

ASTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKRVEPKSCDKTHTCPPCPAPELLGGPSVFLFPPKPK
DTLMISRTPEVTCVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSREEMTKNQVSLTCLVKGFYPSDIA
VEWESNGQPENNYKTTPPVLDSDGSFFLYSRLTVDKSRWQQGNVFSCSVMHEALHNHYTQKSL
SLSPGK.

The antibody may be a bispecific antibody, comprising (i) a first Fab-arm comprising an Fc region and VH and VL sequences, which Fab-arm comprises the VH and optionally VL region sequences of (005), (025), (153) or (169), and which Fab-arm comprises an IgG1 wildtype Fc region, wherein the CH3 region contains a Leu at position 405, and optionally Ile at position 350 and Thr at position 370, and (ii) a second Fab-arm having an Fc region and VH and VL sequences, which Fab-arm comprises the VH and VL region sequences of (005), (025), (153) or (169), and which Fab-arm comprises a IgG1 wildtype Fc region, wherein the CH3 region contains an Arg at position 409. Specific embodiments are disclosed in the Examples.

The VH and VL region sequences of (005) may be selected from the group consisting of:

a) the VH CDR3 sequence of SEQ ID NO:168 (005),
b) the VH CDR3 sequence of SEQ ID NO:168 and VL CDR3 sequence of SEQ ID NO:171 (005),
c) the VH CDR1 sequence of SEQ ID NO:166, VH CDR2 sequence of SEQ ID NO:167, and VH CDR3 sequence of SEQ ID NO:168 (005),
d) the VH CDR1 sequence of SEQ ID NO:64, VH CDR2 sequence of SEQ ID NO:65, VH CDR3 sequence of SEQ ID NO:168, VL CDR1 sequence of SEQ ID NO:170, VL CDR2 sequence of GAS, and VL CDR3 sequence of SEQ ID NO:171 (005), and
e) VH region comprising SEQ ID NO:165 and VL region comprising SEQ ID NO:169 (005).

The VH and VL region sequences of (025) may be selected from the group consisting of:

a) the VH CDR3 sequence of SEQ ID NO:25 (025),
b) the VH CDR3 sequence of SEQ ID NO:25 and VL CDR3 sequence of SEQ ID NO:28 (025),
c) the VH CDR1 sequence of SEQ ID NO:23, VH CDR2 sequence of SEQ ID NO:24 and VH CDR3 sequence of SEQ ID NO:25 (025),
d) the VH CDR1 sequence of SEQ ID NO:23, VH CDR2 sequence of SEQ ID NO:24, VH CDR3 sequence of SEQ ID NO:25, VL CDR1 sequence of SEQ ID NO: 27, VL CDR2 sequence of AAS, and VL CDR3 sequence of SEQ ID NO:28 (025), and
e) the VH region comprising SEQ ID NO:22 and VL region comprising SEQ ID NO:26 (025).

The VH and VL region sequences of (153) may be selected from the group consisting of:

a) the VH CDR3 sequence of SEQ ID NO:66 (153),
b) the VH CDR3 sequence of SEQ ID NO:66 and VL CDR3 sequence of SEQ ID NO:69 (153),
c) the VH CDR1 sequence of SEQ ID NO:64, VH CDR2 sequence of SEQ ID NO:65 and VH CDR3 sequence of SEQ ID NO:66 (153),
d) the VH CDR1 sequence of SEQ ID NO:64, VH CDR2 sequence of SEQ ID NO:65, VH CDR3 sequence of SEQ ID NO:66, VL CDR1 sequence of SEQ ID NO: 68, VL CDR2 sequence of DAS, and VL CDR3 sequence of SEQ ID NO:69 (153), and
e) the VH region comprising SEQ ID NO:63 and VL region comprising SEQ ID NO:67 (153).

The VH and VL region sequences of (169) may be selected from the group consisting of:

a) the VH CDR3 sequence of SEQ ID NO:4 (169),
b) the VH CDR3 sequence of SEQ ID NO:4, and VL CDR3 sequence of SEQ ID NO:7 (169),
c) the VH CDR1 sequence of SEQ ID NO:2, VH CDR2 sequence of SEQ ID NO:3 and VH CDR3 sequence of SEQ ID NO:4 (169),
d) the VH CDR1 sequence of SEQ ID NO:2, VH CDR2 sequence of SEQ ID NO:3, VH CDR3 sequence of SEQ ID NO:4, VL CDR1 sequence of SEQ ID NO: 6, VL CDR2 sequence of DAS, and VL CDR3 sequence of SEQ ID NO:7 (**169**), and

e) the VH region comprising SEQ ID NO:1 and VL region comprising SEQ ID NO:5 (**169**).

As shown in Example 34, the F405L mutation appears sufficient to engage human IgG1 in Fab-arm exchange under the indicated. Furthermore, as indicated in the Examples other combinations of mutations may also be suitable.

The antibody may be a bispecific antibody, comprising (i) a first Fab-arm having an Fc region and VH and VL sequences, wherein the VH region comprises the amino acid sequence of SEQ ID NO:165, and the VL region comprises the amino acid sequence of SEQ ID NO:169 (**005**), optionally wherein the first Fab-arm comprises an IgG1,κ Fc region, wherein the CH3 region contains a Leu at position 405, and optionally Ile at position 350 and a Thr at position 370; and (ii) a second Fab-arm having an Fc region and VH and VL sequences wherein the VH region comprises the amino acid sequence of SEQ ID NO:1 and the VL region comprises the amino acid sequence of SEQ ID NO:5 (**169**), optionally wherein the second Fab-arm comprises an IgG1,κ Fc region having an Arg at position 409.

The bispecific antibody may comprise (i) a first Fab-arm having an Fc region and VH and VL sequences, wherein the VH region comprises the amino acid sequence of SEQ ID NO:22, and the VL region comprises the amino acid sequence of SEQ ID NO:26 (**025**), optionally wherein the first Fab-arm comprises an IgG1,κ Fc region, wherein the CH3 region contains a Leu at position 405, and optionally Ile at position 350 and a Thr at position 370; and (ii) a second Fab-arm having an Fc region and VH and VL sequences wherein the VH region comprises the amino acid sequence of SEQ ID NO:165 and the VL region comprises the amino acid sequence of SEQ ID NO:169 (**005**), optionally wherein the second Fab-arm comprises an IgG1,κ Fc region having an Arg at position 409.

The bispecific antibody may comprise (i) a first Fab-arm having an Fc region and VH and VL sequences, wherein the VH region comprises the amino acid sequence of SEQ ID NO:22, and the VL region comprises the amino acid sequence of SEQ ID NO:26 (**025**), optionally wherein the first Fab-arm comprises an IgG1,κ Fc region, wherein the CH3 region contains a Leu at position 405, and optionally Ile at position 350 and a Thr at position 370; and (ii) a second Fab-arm having an Fc region and VH and VL sequences wherein the VH region comprises the amino acid sequence of SEQ ID NO:63 and the VL region comprises the amino acid sequence of SEQ ID NO:37 (**153**), optionally wherein the second Fab-arm comprises an IgG1,κ Fc region having an Arg at position 409.

The bispecific antibody may comprise (i) a first Fab-arm having an Fc region and VH and VL sequences, wherein the VH region comprises the amino acid sequence of SEQ ID NO:22, and the VL region comprises the amino acid sequence of SEQ ID NO:26 (**025**), optionally wherein the first Fab-arm comprises an IgG1,κ Fc region, wherein the CH3 region contains a Leu at position 405, and optionally Ile at position 350 and a Thr at position 370; and (ii) a second Fab-arm having an Fc region and VH and VL sequences wherein the VH region comprises the amino acid sequence of SEQ ID NO:1 and the VL region comprises the amino acid sequence of SEQ ID NO:5 (**169**), optionally wherein the second Fab-arm comprises an IgG1,κ Fc region having an Arg at position 409.

The bispecific antibody comprises (i) a first Fab-arm having an Fc region and VH and VL sequences, wherein the VH region comprises the amino acid sequence of SEQ ID NO:63, and the VL region comprises the amino acid sequence of SEQ ID NO:67 (**153**), optionally wherein the first Fab-arm comprises an IgG1,κ Fc region, wherein the CH3 region contains a Leu at position 405, and optionally Ile at position 350 and a Thr at position 370; and (ii) a second Fab-arm having an Fc region and VH and VL sequences wherein the VH region comprises the amino acid sequence of SEQ ID NO:165 and the VL region comprises the amino acid sequence of SEQ ID NO:169 (**005**), optionally wherein the second Fab-arm comprises an IgG1,κ Fc region having an Arg at position 409.

The bispecific antibody comprises (i) a first Fab-arm having an Fc region and VH and VL sequences, wherein the VH region comprises the amino acid sequence of SEQ ID NO:63, and the VL region comprises the amino acid sequence of SEQ ID NO:67 (**153**), optionally wherein the first Fab-arm comprises an IgG1,κ Fc region, wherein the CH3 region contains a Leu at position 405, and optionally Ile at position 350 and a Thr at position 370; and (ii) a second Fab-arm having an Fc region and VH and VL sequences, wherein the VH region comprises the amino acid sequence of SEQ ID NO:1 and the VL region comprises the amino acid sequence of SEQ ID NO:5 (**169**), optionally wherein the second Fab-arm comprises an IgG1,κ Fc region having an Arg at position 409.

The first and/or second Fab-arm may further comprise CH1 and/or CL sequences.

A bispecific antibody of the present disclosure may be selected from the group consisting of: IgG1-005-ITL x IgG1-169-K409R, IgG1-025-ITL x IgG1-005-K409R, IgG1-025-ITL x IgG1-153-K409R, IgG1-025-ITL x IgG1-169-K409R, IgG1-153-ITL x IgG1-005-K409R; and IgG1-153-ITL x IgG1-169-K409R, wherein IgG1-005-ITL means 005 IgG1,κ having Ile at position 350, Thr at position 370, and Leu at position 405, IgG1-005-K409R means 005 IgG1,κ having an Arg at position 409, IgG1-025-ITL means 025 IgG1,κ having Ile at position 350, Thr at position 370, and Leu at position 405, IgG1-153-ITL means 153 IgG1,κ having contains Ile at position 350, Thr at position 370, and Leu at position 405, IgG1-153-K409R means 153 IgG1,κ having an Arg at position 409, IgG1-169-K409R means 169 IgG1,κ having an Arg at position 409, and wherein the bold numbers refer to antibodies described herein with the VH and VL regions comprising the sequences described in Table 1.

Bispecific antibody formats

The present disclosure provides bispecific HER2xHER2 antibodies which efficiently bind to and optionally internalize into HER2-expressing tumor cells, typically without significantly promoting ligand-independent proliferation of the cells. Depending on the desired functional properties for a particular use, particular antigen-binding regions can be selected from the set of antibodies or antigen-binding regions provided by the present invention or from those antibodies or antigen-binding regions sharing, e.g., an epitope or cross-blocking region with the antibodies or antigen-binding regions provided by the present invention. Many different formats and uses of bispecific antibodies are known in the art, and were recently been reviewed by Chames and Baty (2009) Curr Opin Drug Disc Dev 12: 276.

Exemplary bispecific antibody molecules of the disclosure comprise (i) a single antibody that has two arms comprising different antigen-binding regions, each one with a specificity to a HER2 epitope, (ii) a single antibody that has one antigen-binding region or arm specific to a first HER2 epitope and a second chain or arm specific to a second HER2 epitope, (iii) a single chain antibody that has specificity to a first HER2 epitope and a second HER2 epitope, e.g., via two scFvs linked in tandem by an extra peptide linker; (iv) a dual-variable-domain antibody (DVD-Ig), where each light chain and heavy chain contains two variable domains in tandem through a short peptide linkage (Wu et al., Generation and Characterization of a Dual Variable Domain Immunoglobulin (DVD-Ig™) Molecule, In: Antibody Engineering, Springer Berlin Heidelberg (2010)); (v) a chemically-linked bispecific (Fab')$_2$ fragment; (vi) a Tandab, which is a fusion of two single chain diabodies resulting in a tetravalent bispecific antibody that has two binding sites for each of the target antigens; (vii) a flexibody, which is a combination of scFvs with a diabody resulting in a multivalent molecule; (viii) a so called "dock and lock" molecule, based on the "dimerization and docking domain" in Protein Kinase A, which, when applied to Fabs, can yield a trivalent bispecific binding protein consisting of two identical Fab fragments linked to a different Fab fragment; (ix) a so-called Scorpion molecule, comprising, e.g., two scFvs fused to both termini of a human Fab-arm; and (x) a diabody.

The bispecific antibody may be a diabody, a cross-body, or a bispecific antibody obtained via a controlled Fab arm exchange as those described in the present invention.

Examples of different classes of bispecific antibodies include but are not limited to

- IgG-like molecules with complementary CH3 domains to force heterodimerisation
- recombinant IgG-like dual targeting molecules, wherein the two sides of the molecule each contain the Fab fragment or part of the Fab fragment of at least two different antibodies;
- IgG fusion molecules, wherein full length IgG antibodies are fused to extra Fab fragment or parts of Fab fragment;
- Fc fusion molecules, wherein single chain Fv molecules or stabilized diabodies are fused to heavy-chain constant-domains, Fc-regions or parts thereof;
- Fab fusion molecules, wherein different Fab-fragments are fused together;
- ScFv-and diabody-based and heavy chain antibodies (e.g., domain antibodies, nanobodies) wherein different single chain Fv molecules or different diabodies or different heavy-chain antibodies (e.g. domain antibodies, nanobodies) are fused to each other or to another protein or carrier molecule.

Examples of IgG-like molecules with complementary CH3 domains molecules include but are not limited to the Triomab/Quadroma (Trion Pharma/Fresenius Biotech), the Knobsinto-Holes (Genentech), CrossMAbs (Roche) and the electrostatically-matched (Amgen), the LUZ-Y (Genentech), the Strand Exchange Engineered Domain body (SEEDbody)(EMD Serono), the Biclonic (Merus) and the DuoBody (Genmab A/S).

Examples of recombinant IgG-like dual targeting molecules include but are not limited to Dual Targeting (DT)-Ig (GSK/Domantis), Two-in-one Antibody (Genentech), Cross-linked Mabs (Karmanos Cancer Center), mAb$^2$ (F-Star) and CovX-body (CovX/Pfizer).

Examples of IgG fusion molecules include but are not limited to Dual Variable Domain (DVD)-Ig (Abbott), IgG-like Bispecific (ImClone/Eli Lilly), Ts2Ab (MedImmune/AZ) and BsAb (Zymogenetics), HERCULES (Biogen Idec) and TvAb (Roche).

Examples of Fc fusion molecules include but are not limited to ScFv/Fc Fusions (Academic Institution), SCORPION (Emergent BioSolutions/Trubion, Zymogenetics/BMS), Dual Affinity Retargeting Technology (Fc-DART) (MacroGenics) and Dual(ScFv)$_2$-Fab (National Research Center for Antibody Medicine - China).

Examples of Fab fusion bispecific antibodies include but are not limited to F(ab)$_2$ (Medarex/AMGEN), Dual-Action or Bis-Fab (Genentech), Dock-and-Lock (DNL) (ImmunoMedics), Bivalent Bispecific (Biotecnol) and Fab-Fv (UCB-Celltech). Examples of ScFv-, diabody-based and domain antibodies include but are not limited to Bispecific T Cell Engager (BiTE) (Micromet, Tandem Diabody (Tandab) (Affimed), Dual Affinity Retargeting Technology (DART) (MacroGenics), Single-chain Diabody (Academic), TCR-like Antibodies (AIT, ReceptorLogics), Human Serum Albumin ScFv Fusion (Merrimack) and COMBODY (Epigen Biotech), dual targeting nanobodies (Ablynx), dual targeting heavy chain only domain antibodies.

Methods for preparing bispecific antibodies

Methods of preparing bispecific antibodies of the present disclosure include those described in WO 2008119353 (Genmab), WO 2011131746 (Genmab) and reported by van der Neut-Kolfschoten et al. (Science. 2007 Sep 14;317(5844): 1554-7). Examples of other platforms useful for preparing bispecific antibodies include but are not limited to BiTE (Micromet), DART (MacroGenics), Fcab and Mab[2] (F-star) , Fc-engineered IgG1 (Xencor) or DuoBody (based on Fab arm exchange, Genmab, this application, described below and in, *e.g.,* Example 20).

Traditional methods such as the hybrid hybridoma and chemical conjugation methods (Marvin and Zhu (2005) Acta Pharmacol Sin 26:649) can also be used. Co-expression in a host cell of two antibodies, consisting of different heavy and light chains, leads to a mixture of possible antibody products in addition to the desired bispecific antibody, which can then be isolated by, *e.g.,* affinity chromatography or similar methods.

Strategies favoring the formation of a functional bispecific, product, upon co-expression of different antibody constructs can also be used, *e.g.,* the method described by Lindhofer *et al.* (1995 J Immunol 155:219). Fusion of rat and mouse hydridomas producing different antibodies leads to a limited number of heterodimeric proteins because of preferential species-restricted heavy/light chain pairing. Another strategy to promote formation of heterodimers over homodimers is a "knob-into-hole" strategy in which a protuberance is introduced on a first heavy-chain polypeptide and a corresponding cavity in a second heavy-chain polypeptide, such that the protuberance can be positioned in the cavity at the interface of these two heavy chains so as to promote heterodimer formation and hinder homodimer formation. "Protuberances" are constructed by replacing small amino-acid side-chains from the interface of the first polypeptide with larger side chains. Compensatory "cavities" of identical or similar size to the protuberances are created in the interface of the second polypeptide by replacing large amino-acid side-chains with smaller ones (US patent 5,731,168). EP1870459 (Chugai) and WO 2009089004 (Amgen) describe other strategies for favoring heterodimer formation upon co-expression of different antibody domains in a host cell. In these methods, one or more residues that make up the CH3-CH3 interface in both CH3 domains are replaced with a charged amino acid such that homodimer formation is electrostatically unfavorable and heterodimerization is electrostatically favorable. WO2007110205 (Merck) describe yet another strategy, wherein differences between IgA and IgG CH3 domains are exploited to promote heterodimerization.

Another *in vitro* method for producing bispecific antibodies has been described in WO 2008119353 (Genmab) and WO 2011131746 (Genmab), wherein a bispecific antibody is formed by "Fab-arm" or "half-molecule" exchange (swapping of a heavy chain and attached light chain) between two monospecific IgG4- or IgG4-like antibodies upon incubation under reducing conditions. The resulting product is a bispecific antibody having two Fab arms which may comprise different sequences.

A preferred method for preparing bispecific HER2xHER2 antibodies of the present disclosure includes the method described in WO 2011131746 (Genmab) comprising the following steps:

a) providing a first HER2 antibody comprising a first Fc region , said Fc region comprising a first CH3 region,
b) providing a second HER2 antibody comprising a second Fc region , said Fc region comprising a second CH3 region, wherein the sequences of said first and second CH3 regions are different and are such that the heterodimeric interaction between said first and second CH3 regions is stronger than each of the homodimeric interactions of said first and second CH3 regions,
c) incubating said first antibody together with said second antibody under reducing conditions, and
d) obtaining said bispecific HER2xHER2 antibody.

The first and/or secon Fc region may be of an immunoglobulin.

Without being limited to theory, in step c), the heavy-chain disulfide bonds in the hinge regions of the parent antibodies are reduced and the resulting cysteines are then able to form inter heavy-chain disulfide bond with cysteine residues of another parent parent antibody molecule (originally with a different specificity). The reducing conditions in step c) may comprise the addition of a reducing agent, *e.g.* a reducing agent selected from the group consisting of: 2-mercaptoethylamine (2-MEA), dithiothreitol (DTT), dithioerythritol (DTE), glutathione, tris(2-carboxyethyl)phosphine (TCEP), L-cysteine and beta-mercaptoethanol, preferably a reducing agent selected from the group consisting of: 2-mercaptoethylamine, dithiothreitol and tris(2-carboxyethyl)phosphine. Step c) may comprises restoring the conditions to become non-reducing or less reducing, for example by removal of a reducing agent, *e.g.* by desalting.

Typically, in this method, the first and second antibodies are a first and second HER2 antibody binding to different epitopes of HER2 and/or comprising different antigen-binding sequences. Said first and/or second homodimeric proteins may be full-length antibodies. For this method any of the first and second HER2 antibodies described above may used, including first and second HER2 antibodies comprising a first and/or second Fc regions. Examples of such first and second Fc regions, including combinations of such first and second Fc regions may include any of those described above. The first and second HER2 antibodies may be chosen so as to obtain a bispecific antibody as described herein.

Typically, in this method, the first and second antibodies are a first and second HER2 antibody binding to different epitopes of HER2 and/or comprising different antigen-binding sequences. Said first and/or second homodimeric proteins may be full-length antibodies.

The Fc regions of both said first and said second antibodies may be of the IgG1 isotype. One of the Fc regions of said antibodies is of the IgG1 isotype and the other of the IgG4 isotype. The resulting bispecific antibody may comprise an Fc region of an IgG1 and an Fc region of IgG4 and may thus have interesting intermediate properties with respect to activation of effector functions. A similar product can be obtained if said first and/or said second antibody comprises a mutation removing the acceptor site for Asn-linked glycosylation or is otherwise manipulated to change the glycosylation properties.

One or both of the antibodies may be glyco-engineered to reduce fucose and thus enhance ADCC, *e.g.* by addition of compounds to the culture media during antibody production as described in US2009317869 or as described in van Berkel et al. (2010) Biotechnol. Bioeng. 105:350 or by using FUT8 knockout cells, *e.g.* as described in Yamane-Ohnuki et al (2004) Biotechnol. Bioeng 87:614. ADCC may alternatively be optimized using the method described by Umaña et al. (1999) Nature Biotech 17:176. One or both of the antibodies may have been engineered to enhance complement activation, *e.g.* as described in Natsume et al. (2009) Cancer Sci. 100:2411.

One or both of the antibodies may have been engineered to reduce or increase the binding to the neonatal Fc receptor (FcRn) in order to manipulate the serum half-life of the heterodimeric protein. One of the antibody starting proteins may have been engineered to not bind Protein A, thus allowing to separate the heterodimeric protein from said homodimeric starting protein by passing the product over a protein A column.

The antibody or a part thereof, *e.g.* one or more CDRs, may be of a species in the family Camelidae, see WO2010001251, or a species of cartilaginous fish, such as the nurse shark, or may be a heavy-chain or domain antibody.

The first and/or second HER2 antibody may be conjugated to a drug, a prodrug or a toxin or contains an acceptor group for the same. Such acceptor group may *e.g.* be an unnatural amino acid.

As described above, the sequences of the first and second CH3 regions of the starting HER2 antibodies are different and are such that the heterodimeric interaction between said first and second CH3 regions is stronger than each of the homodimeric interactions of said first and second CH3 regions WO 2011131746 (Genmab). More details on these interactions and how they can be achieved are provided in PCT/EP2011/056388.

In particular, a stable bispecific HER2xHER2 molecule can be obtained at high yield using the above method of the invention on the basis of two homodimeric starting HER2 antibodies containing only a few, fairly conservative, asymmetrical mutations in the CH3 regions. Asymmetrical mutations mean that the sequences of said first and second CH3 regions contain amino acid substitutions at non-identical positions.

The first HER2 antibody may have an amino acid substitution at a position selected from the group consisting of: 366, 368, 370, 399, 405, 407 and 409, and the second HER2 antibody may have an amino acid substitution at a position selected from the group consisting of: 366, 368, 370, 399, 405, 407 and 409, and wherein the first and second HER2 antibodies are not substituted in the same positions.

The first HER2 antibody my have an amino acid substitution at position 366, and said second HER2 antibody may have an amino acid substitution at a position selected from the group consisting of: 368, 370, 399, 405, 407 and 409. The amino acid at position 366 may be selected from Ala, Asp, Glu, His, Asn, Val, or Gln.

The first HER2 antibody protein may have an amino acid substitution at position 368, and said second HER2 antibody may have an amino acid substitution at a position selected from the group consisting of: 366, 370, 399, 405, 407 and 409.

The first HER2 antibody may have an amino acid substitution at position 370, and said second HER2 antibody may have an amino acid substitution at a position selected from the group consisting of: 366, 368, 399, 405, 407 and 409.

The first HER2 antibody may have an amino acid substitution at position 399, and said second HER2 antibody may have an amino acid substitution at a position selected from the group consisting of: 366, 368, 370, 405, 407 and 409.

The first HER2 antibody may ahve an amino acid substitution at position 405, and said second HER2 antibody may have an amino acid substitution at a position selected from the group consisting of: 366, 368, 370, 399, 407 and 409.

The first HER2 antibody may have an amino acid substitution at position 407, and said second HER2 antibody may have an amino acid substitution at a position selected from the group consisting of: 366, 368, 370, 399, 405, and 409.

The first HER2 antibody may have an amino acid substitution at position 409, and said second HER2 antibody may have an amino acid substitution at a position selected from the group consisting of: 366, 368, 370, 399, 405, and 407.

Accordingly, the sequences of said first and second CH3 regions may contain asymmetrical mutations, *i.e.* mutations at different positions in the two CH3 regions, *e.g.* a mutation at position 405 in one of the CH3 regions and a mutation at position 409 in the other CH3 region.

The first HER2 antibody may have an amino acid other than Lys, Leu or Met at position 409, and said second HER2 antibody may have an amino-acid substitution at a position selected from the group consisting of: 366, 368, 370, 399, 405 and 407. In one such embodiment, said first HER2 antibody may have an amino acid other than Lys, Leu or Met at position 409, and said second HER2 antibody may have an amino acid other than Phe at position 405. In a further embodiment hereof, said first HER2 antibody may have an amino acid other than Lys, Leu or Met at position 409, and said second HER2 antibody may have an amino acid other than Phe, Arg or Gly at position 405.

Said first HER2 antibody may comprises a Phe at position 405 and an amino acid other than Lys, Leu or Met at position 409 and said second HER2 antibody may comprise an amino acid other than Phe at position 405 and a Lys at position 409. Said first HER2 antibody may comprise a Phe at position 405 and an amino acid other than Lys, Leu or Met at position 409 and said second HER2 antibody may comprise an amino acid other than Phe, Arg or Gly at position 405 and a Lys at position 409.

Said first HER2 antibody may comprise a Phe at position 405 and an amino acid other than Lys, Leu or Met at position 409 and said second HER2 antibody may comprise a Leu at position 405 and a Lys at position 409. Said first HER2 antibody may comprise a Phe at position 405 and an Arg at position 409 and said second HER2 antibody comprises an amino acid other than Phe, Arg or Gly at position 405 and a Lys at position 409. Said first HER2 antibody comprises Phe at position 405 and an Arg at position 409 and said second HER2 antibody may comprise a Leu at position 405 and a Lys at position 409.

Said first HER2 antibody may comprise an amino acid other than Lys, Leu or Met at position 409 and said second homodimeric protein comprises a Lys at position 409, a Thr at position 370 and a Leu at position 405. Said first homodimeric protein may comprise an Arg at position 409 and said second homodimeric protein comprises a Lys at position 409, a Thr at position 370 and a Leu at position 405.

Said first HER2 antibody may comprise a Lys at position 370, a Phe at position 405 and an Arg at position 409 and said second HER2 antibody may comprise a Lys at position 409, a Thr at position 370 and a Leu at position 405.

Said first HER2 antibody may comprise an amino acid other than Lys, Leu or Met at position 409 and said second HER2 antibody may comprise a Lys at position 409 and: a) an Ile at position 350 and a Leu at position 405, or b) a Thr at position 370 and a Leu at position 405.

Said first HER2 antibody may comprise an Arg at position 409 and said second HER2 antibody may comprise a Lys at position 409 and: a) an Ile at position 350 and a Leu at position 405, or b) a Thr at position 370 and a Leu at position 405.

Said first HER2 antibody may comprise a Thr at position 350, a Lys at position 370, a Phe at position 405 and an Arg at position 409 and said second HER2 antibody may comprise a Lys at position 409 and: a) an Ile at position 350 and a Leu at position 405, or b) a Thr at position 370 and a Leu at position 405.

Said first HER2 antibody may comprise a Thr at position 350, a Lys at position 370, a Phe at position 405 and an Arg at position 409 and said second may comprise an Ile at position 350, a Thr at position 370, a Leu at position 405 and a Lys at position 409.

Said first HER2 antibody may have an amino acid other than Lys, Leu or Met at position 409 and said second HER2 antibody may have an amino acid other than Tyr, Asp, Glu, Phe, Lys, Gln, Arg, Ser or Thr at position 407. Said first HER2 antibody may have an amino acid other than Lys, Leu or Met at position 409 and said second HER2 antibody may have an Ala, Gly, His, Ile, Leu, Met, Asn, Val or Trp at position 407.

Said first HER2 antibody may have an amino acid other than Lys, Leu or Met at position 409 and said second HER2 antibody may have a Gly, Leu, Met, Asn or Trp at position 407.

Said first HER2 antibody may have a Tyr at position 407 and an amino acid other than Lys, Leu or Met at position 409 and said second HER2 antibody may have an amino acid other than Tyr, Asp, Glu, Phe, Lys, Gln, Arg, Ser or Thr at position 407 and a Lys at position 409.

Said first HER2 antibody may have a Tyr at position 407 and an amino acid other than Lys, Leu or Met at position 409 and said second HER2 antibody may have an Ala, Gly, His, Ile, Leu, Met, Asn, Val or Trp at position 407 and a Lys at position 409.

Said first HER2 antibody may have a Tyr at position 407 and an amino acid other than Lys, Leu or Met at position 409 and said second HER2 antibody may have a Gly, Leu, Met, Asn or Trp at position 407 and a Lys at position 409.

Said first HER2 antibody may have a Tyr at position 407 and an Arg at position 409 and said second HER2 antibody may have an amino acid other than Tyr, Asp, Glu, Phe, Lys, Gln, Arg, Ser or Thr at position 407 and a Lys at position 409.

Said first HER2 antibody may havea Tyr at position 407 and an Arg at position 409 and said second HER2 antibody may have an Ala, Gly, His, Ile, Leu, Met, Asn, Val or Trp at position 407 and a Lys at position 409.

Said first HER2 antibody may have a Tyr at position 407 and an Arg at position 409 and said second HER2 antibody may have a Gly, Leu, Met, Asn or Trp at position 407 and a Lys at position 409.

The first HER2 antibody may have an amino acid other than Lys, Leu or Met at position 409, and the second HER2 antibody may have

(i) an amino acid other than Phe, Leu and Met at position 368, or
(ii) a Trp at position 370, or
(iii) an amino acid other than Asp, Cys, Pro, Glu or Gln at position 399, or

(iv) an amino acid other than Lys, Arg, Ser, Thr, or Trp at position 366, e.g. Leu, Met, His, Asp, Glu, Asn, Glu, Gly, Pro, Ala, Val, Ile, Phe, Tyr or Cys.

The first HER2 antibody may have an Arg, Ala, His or Gly at position 409, and the second homodimeric protein may have

(i) a Lys, Gln, Ala, Asp, Glu, Gly, His, Ile, Asn, Arg, Ser, Thr, Val, or Trp at position 368, or
(ii) a Trp at position 370, or
(iii) an Ala, Gly, Ile, Leu, Met, Asn, Ser, Thr, Trp, Phe, His, Lys, Arg or Tyr at position 399, or
(iv) an Ala, Asp, Glu, His, Asn, Val, Gln, Phe, Gly, Ile, Leu, Met, or Tyr at position 366.

The first HER2 antibody may have an Arg at position 409, and the second homodimeric protein may have

(i) an Asp, Glu, Gly, Asn, Arg, Ser, Thr, Val, or Trp at position 368, or
(ii) a Trp at position 370, or
(iii) a Phe, His, Lys, Arg or Tyr at position 399, or
(iv) an Ala, Asp, Glu, His, Asn, Val, Gln at position 366.

Other specific combinations include any of those described in the section relating to the Fc regions.

In addition to the above-specified amino-acid substitutions, said first and second homodimeric protein may contain further amino-acid substitutions, deletion or insertions relative to wild-type Fc sequences.

Said first and second CH3 regions may, except for the specified mutations, comprise the sequences of IgG1m(a) (SEQ ID NO:236), IgG1m(f) (SEQ ID NO:237), or IgG1m(ax) (SEQ ID NO:238)

Thus, neither said first nor said second HER2 antibody may be one that comprises a Cys-Pro-Ser-Cys sequence in the (core) hinge region.

Both said first and said second HER2 antibody may comprise a Cys-Pro-Pro-Cys sequence in the (core) hinge region.

The bispecific antibodies of the disclosure may also be obtained by co-expression of constructs encoding a first and second polypeptide in a single cell. Thus,the disclosure further relates to a method for producing a bispecific antibody, said method comprising the following steps:

a) providing a first nucleic-acid construct encoding a first polypeptide comprising a first Fc region of an immunoglobulin and a first antigen-binding region, said first Fc region comprising a first CH3 region,
b) providing a second nucleic-acid construct encoding a second polypeptide comprising a second Fc region and a second antigen-binding region, said second Fc region comprising a second CH3 region,

wherein the sequences of said first and second CH3 regions are different and are such that the heterodimeric interaction between said first and second CH3 regions is stronger than each of the homodimeric interactions of said first and second CH3 regions, and
wherein said first homodimeric protein has an amino acid other than Lys, Leu or Met at position 409 and said second homodimeric protein has an amino acid substitution at a position selected from the group consisting of: 366, 368, 370, 399, 405 and 407,
optionally wherein said first and second nucleic acid constructs encode light chain sequences of said first and second HER2 antibodies

c) co-expressing said first and second nucleic-acid constructs in a host cell, and
d) obtaining said heterodimeric protein from the cell culture.

The first antigen-binding region may be from a first HER2 antibody of the present invention.

The second antigen-binding region may be from a second HER2 antibody of the present invention.

Suitable expression vectors, including promoters, enhancers, etc., and suitable host cells for the production of antibodies are well-known in the art. Examples of host cells include yeast, bacterial and mammalian cells, such as CHO or HEK cells.

Said first CH3 region may have an amino acid other than Lys, Leu or Met at position 409 and said second CH3 region may have an amino acid other than Phe at position 405.

Said first CH3 region may have an amino acid other than Lys, Leu or Met at position 409 and said second CH3 region may have an amino acid other than Phe at position 405, such as other than Phe, Arg or Gly at position 405; or said first CH3 region may have an amino acid other than Lys, Leu or Met at position 409 and said second CH3 region may have an amino acid other than Tyr, Asp, Glu, Phe, Lys, Gln, Arg, Ser or Thr at position 407.

Said first and second polypeptides may be full-length heavy chains of two antibodies that bind different epitopes (*i.e.* said first and second nucleic-acid constructs encode full-length heavy chains of two antibodies that bind different epitopes), and thus the heterodimeric protein may be a bispecific antibody. This bispecific antibody can be a heavy-chain antibody, or said host cell may further express one or more nucleic-acid constructs encoding a light-chain. If only one light-chain construct is co-expressed with the heavy chain constructs, then a functional bispecific antibody is only formed if the light chain sequence is such that it can form a functional antigen-binding domain with each of the heavy chains. If two or more different light-chain constructs are co-expressed with the heavy chain, multiple products will be formed.

The co-expression method according to the disclosure may comprise any of the further features described under the *in vitro* method above.

The disclosure further relates to an expression vector comprising the first and second nucleic-acid constructs specified herein above. The expression vector may further comprise a nucleotide sequence encoding the constant region of a light chain, a heavy chain or both light and heavy chains of an antibody, *e.g.* a human antibody.

An expression vector in the context of the present disclosure may be any suitable vector, including chromosomal, non-chromosomal, and synthetic nucleic acid vectors (a nucleic acid sequence comprising a suitable set of expression control elements). Examples of such vectors include derivatives of SV40, bacterial plasmids, phage DNA, baculovirus, yeast plasmids, vectors derived from combinations of plasmids and phage DNA, and viral nucleic acid (RNA or DNA) vectors. A HER2 antibody-encoding nucleic acid may be comprised in a naked DNA or RNA vector, including, for example, a linear expression element (as described in for instance Sykes and Johnston, Nat Biotech 17, 355-59 (1997)), a compacted nucleic acid vector (as described in for instance US 6,077, 835 and/or WO 00/70087), a plasmid vector such as pBR322, pUC 19/18, or pUC 118/119, a "midge" minimally-sized nucleic acid vector (as described in for instance Schakowski et al., Mol Ther 3, 793-800 (2001)), or as a precipitated nucleic acid vector construct, such as a CaP04-precipitated construct (as described in for instance WO 00/46147, Benvenisty and Reshef, PNAS USA 83, 9551-55 (1986), Wigler et al., Cell 14, 725 (1978), and Coraro and Pearson, Somatic Cell Genetics 7, 603 (1981)). Such nucleic acid vectors and the usage thereof are well known in the art (see for instance US 5,589,466 and US 5,973,972).

Exemplary expression vectors for the antibodies of the disclosure are also described in Examples 2 and 3.

The vector may be one that is suitable for expression of the HER2 antibody in a bacterial cell. Examples of such vectors include expression vectors such as BlueScript (Stratagene), pIN vectors (Van Heeke & Schuster, J Biol Chem 264, 5503-5509 (1989), pET vectors (Novagen, Madison WI) and the like).

An expression vector may also or alternatively be a vector suitable for expression in a yeast system. Any vector suitable for expression in a yeast system may be employed. Suitable vectors include, for example, vectors comprising constitutive or inducible promoters such as alpha factor, alcohol oxidase and PGH (reviewed in: F. Ausubel et al., ed. Current Protocols in Molecular Biology, Greene Publishing and Wiley InterScience New York (1987), and Grant et al., Methods in Enzymol 153, 516-544 (1987)).

An expression vector may also or alternatively be a vector suitable for expression in mammalian cells, *e.g.* a vector comprising glutamine synthetase as a selectable marker, such as the vectors described in Bebbington (1992) Biotechnology (NY) 10:169-175.

A nucleic acid and/or vector may also comprises a nucleic acid sequence encoding a secretion/localization sequence, which can target a polypeptide, such as a nascent polypeptide chain, to the periplasmic space or into cell culture media. Such sequences are known in the art, and include secretion leader or signal peptides.

The expression vector may comprise or be associated with any suitable promoter, enhancer, and other expression-facilitating elements. Examples of such elements include strong expression promoters (e. g., human CMV IE promoter/enhancer as well as RSV, SV40, SL3-3, MMTV, and HIV LTR promoters), effective poly (A) termination sequences, an origin of replication for plasmid product in E. coli, an antibiotic resistance gene as selectable marker, and/or a convenient cloning site (*e.g.*, a polylinker). Nucleic acids may also comprise an inducible promoter as opposed to a constitutive promoter such as CMV IE.

The HER2 antibody-encoding expression vector may be positioned in and/or delivered to the host cell or host animal via a viral vector.

The disclosure further relates to a host cell comprising the first and second nucleic-acid constructs specified herein above.

Thus the present disclosure also relates to a recombinant eukaryotic or prokaryotic host cell which produces a bispecific antibody of the present invention, such as a transfectoma. Examples of host cells include yeast, bacterial, and mammalian cells, such as CHO or HEK cells. For example, the host cell may comprise a first and second nucleic acid construct stably integrated into the cellular genome. The present disclosure provides a cell comprising a non-integrated nucleic acid, such as a plasmid, cosmid, phagemid, or linear expression element, which comprises a first and second nucleic acid construct as specified above.

The disclosure further relates to a transgenic non-human animal or plant comprising nucleic acids encoding one or two sets of a human heavy chain and a human light chain, wherein the animal or plant produces an bispecific antibody of the invention of the invention.

The present disclosure also relates to a method for producing a bispecific antibody of the present disclosure, said method comprising the steps of

a) culturing a host cell of the present invention, and
b) purifying the bispecific antibody from the culture media.

Furthermore, the present disclosure also relates to a bispecific antibody obtainable by a method of the present invention.

Preparation of antibodies

Monoclonal antibodies, such as the first and second HER2 antibodies, for use according to the present disclosure, for example to provide an antigen-binding region sharing an epitope or cross-blocking region with an antibody of cross-block groups 1, 2, 3 or 4 may be produced, *e.g.,* by the hybridoma method first described by Kohler et al., Nature 256, 495 (1975), or may be produced by recombinant DNA methods. Monoclonal antibodies may also be isolated from phage antibody libraries using the techniques described in, for example, Clackson et al., Nature 352, 624-628 (1991) and Marks et al., J. Mol. Biol. 222, 581-597 (1991). Monoclonal antibodies may be obtained from any suitable source. Thus, for example, monoclonal antibodies may be obtained from hybridomas prepared from murine splenic B cells obtained from mice immunized with an antigen of interest, for instance in form of cells expressing the antigen on the surface, or a nucleic acid encoding an antigen of interest. Monoclonal antibodies may also be obtained from hybridomas derived from antibody-expressing cells of immunized humans or non-human mammals such as rats, dogs, primates, etc.

The antibody may be a human antibody. Human monoclonal antibodies directed against HER2 may be generated using transgenic or transchromosomal mice carrying parts of the human immune system rather than the mouse system. Such transgenic and transchromosomic mice include mice referred to herein as HuMAb® mice and KM mice, respectively, and are collectively referred to herein as "transgenic mice".

The HuMAb® mouse contains a human immunoglobulin gene miniloci that encodes unrearranged human heavy (μ and γ) and κ light chain immunoglobulin sequences, together with targeted mutations that inactivate the endogenous μ and κ chain loci (Lonberg, N. et al., Nature 368, 856-859 (1994)). Accordingly, the mice exhibit reduced expression of mouse IgM or κ and in response to immunization, the introduced human heavy and light chain transgenes, undergo class switching and somatic mutation to generate high affinity human IgG,κ monoclonal antibodies (Lonberg, N. *et al.* (1994), supra; reviewed in Lonberg, N. Handbook of Experimental Pharmacology 113, 49-101 (1994) , Lonberg, N. and Huszar, D., Intern. Rev. Immunol. Vol. 13 65-93 (1995) and Harding, F. and Lonberg, N. Ann. N.Y. Acad. Sci 764 536-546 (1995)). The preparation of HuMAb® mice is described in detail in Taylor, L. et al., Nucleic Acids Research 20, 6287-6295 (1992), Chen, J. et al., International Immunology 5, 647-656 (1993), Tuaillon et al., J. Immunol. 152, 2912-2920 (1994), Taylor, L. et al., International Immunology 6, 579-591 (1994), Fishwild, D. et al., Nature Biotechnology 14, 845-851 (1996). See also US 5,545,806, US 5,569,825, US 5,625,126, US 5,633,425, US 5,789,650, US 5,877,397, US 5,661,016, US 5,814,318, US 5,874,299, US 5,770,429, US 5,545,807, WO 98/24884, WO 94/25585, WO 93/1227, WO 92/22645, WO 92/03918 and WO 01/09187.

The HCo7, HCo12, HCo17 and HCo20 mice have a JKD disruption in their endogenous light chain (kappa) genes (as described in Chen et al., EMBO J. 12, 821-830 (1993)), a CMD disruption in their endogenous heavy chain genes (as described in Example 1 of WO 01/14424), and a KCo5 human kappa light chain transgene (as described in Fishwild et al., Nature Biotechnology 14, 845-851 (1996)). Additionally, the Hco7 mice have a HCo7 human heavy chain transgene (as described in US 5,770,429), the HCo12 mice have a HCo12 human heavy chain transgene (as described in Example 2 of WO 01/14424), the HCo17 mice have a HCo17 human heavy chain transgene (as described in Example 2 of WO 01/09187) and the HCo20 mice have a HCo20 human heavy chain transgene. The resulting mice express human immunoglobulin heavy and kappa light chain transgenes in a background homozygous for disruption of the endogenous mouse heavy and kappa light chain loci.

In the KM mouse strain, the endogenous mouse kappa light chain gene has been homozygously disrupted as described in Chen et al., EMBO J. 12, 811-820 (1993) and the endogenous mouse heavy chain gene has been homozygously disrupted as described in Example 1 of WO 01/09187. This mouse strain carries a human kappa light chain transgene, KCo5, as described in Fishwild et al., Nature Biotechnology 14, 845-851 (1996). This mouse strain also carries a human heavy chain transchromosome composed of chromosome 14 fragment hCF (SC20) as described in WO 02/43478. HCo12-Balb/C mice can be generated by crossing HCo12 to KCo5[J/K](Balb) as described in WO/2009/097006.

Splenocytes from these transgenic mice may be used to generate hybridomas that secrete human monoclonal antibodies according to well known techniques.

Further, HER2 antigen-binding regions may be obtained from human antibodies or antibodies from other species identified through display-type technologies, including, without limitation, phage display, retroviral display, ribosomal display, and other techniques, using techniques well known in the art and the resulting molecules may be subjected to additional maturation, such as affinity maturation, as such techniques are well known in the art (see for instance Hoogenboom et al., J. Mol. Biol. 227, 381 (1991) (phage display), Vaughan et al., Nature Biotech 14, 309 (1996) (phage display), Hanes and Plucthau, PNAS USA 94, 4937-4942 (1997) (ribosomal display), Parmley and Smith, Gene 73, 305-318 (1988) (phage display), Scott TIBS 17, 241-245 (1992), Cwirla et al., PNAS USA 87, 6378-6382 (1990), Russel et al., Nucl. Acids Research 21, 1081-1085 (1993), Hogenboom et al., Immunol. Reviews 130, 43-68 (1992), Chiswell and McCafferty TIBTECH 10, 80-84 (1992), and US 5,733,743). If display technologies are utilized to produce antibodies that are not human, such antibodies may be humanized.

The bispecific antibody can be of any isotype. The choice of isotype typically will be guided by the desired effector functions, such as ADCC induction. Exemplary isotypes are IgG1, IgG2, IgG3, and IgG4. Either of the human light chain constant regions, kappa or lambda, may be used. The effector function of the antibodies of the present invention may be changed by isotype switching to, *e.g.,* an IgG1, IgG2, IgG3, IgG4, IgD, IgA, IgE, or IgM antibody for various therapeutic uses. Both Fc-regions of an antibody of the present invention may be of the IgG1 isotype, for instance an IgG1,κ. The two Fc-regions of a bispecific antibody may be of the IgG1 and IgG4 isotypes, respectively. Optionally, the Fc-region may be modified in the hinge and/or CH3 region as described elsewhere herein.

The bispecific antibody may be a full-length antibody, preferably an IgG1 antibody, in particular an IgG1,κ antibody or a variant thereof. The bispecific antibody may comprise an antibody fragment or a single-chain antibody. Antibody fragments may *e.g.* be obtained by fragmentation using conventional techniques, and the fragments screened for utility in the same manner as described herein for whole antibodies. For example, F(ab')$_2$ fragments may be generated by treating an antibody with pepsin. The resulting F(ab')$_2$ fragment may be treated to reduce disulfide bridges with a reducing agent, such as dithiothreitol, to produce Fab' fragments. Fab fragments may be obtained by treating an antibody with papain. A F(ab')$_2$ fragment may also be produced by binding Fab' fragments via a thioether bond or a disulfide bond. Antibody fragments may also be generated by expression of nucleic acids encoding such fragments in recombinant cells (see for instance Evans et al., J. Immunol. Meth. 184, 123-38 (1995)). For example, a chimeric gene encoding a portion of an F(ab')$_2$ fragment could include DNA sequences encoding the $C_H1$ domain and hinge region of the H chain, followed by a translational stop codon to yield such a truncated antibody fragment molecule.

Bispecific HER2xHER2 antibodies may also be prepared from single chain antibodies. Single chain antibodies are peptides in which the heavy and light chain Fv regions are connected. The bispecific antibody may comprise a single-chain Fv (scFv) wherein the heavy and light chains in the Fv of a HER2 antibody of the present invention are joined with a flexible peptide linker (typically of about 10, 12, 15 or more amino acid residues) in a single peptide chain. Methods of producing such antibodies are described in for instance US 4,946,778, Pluckthun in The Pharmacology of Monoclonal Antibodies, vol. 113, Rosenburg and Moore eds. Springer-Verlag, New York, pp. 269-315 (1994), Bird et al., Science 242,423-426 (1988), Huston et al., PNAS USA 85, 5879-5883 (1988) and McCafferty et al., Nature 348, 552-554 (1990). A bispecific antibody can then be formed from two $V_H$ and $V_L$ from different single-chain HER2 antibodies, or a polyvalent antibody formed from more than two $V_H$ and $V_L$ chains.

One or both Fc-regions of the bispecific HER2xHER2 antibody of the disclosure may be effector-function-deficient. The effector-function-deficient HER2 antibody may be a human stabilized IgG4 antibody, which has been modified to prevent Fab-arm exchange (van der Neut Kolfschoten et al. (2007) Science 317(5844):1554-7). Examples of suitable human stabilized IgG4 antibodies are antibodies, wherein arginine at position 409 in a heavy chain constant region of human IgG4, which is indicated in the EU index as in Kabat *et al.,* is substituted with lysine, threonine, methionine, or leucine, preferably lysine (described in WO2006033386 (Kirin)) and/or wherein the hinge region has been modified to comprise a Cys-Pro-Pro-Cys sequence.

The stabilized IgG4 HER2 antibody may be an IgG4 antibody comprising a heavy chain and a light chain, wherein said heavy chain comprises a human IgG4 constant region having a residue selected from the group consisting of: Lys, Ala, Thr, Met and Leu at the position corresponding to 409 and/or a residue selected from the group consisting of: Ala, Val, Gly, Ile and Leu at the position corresponding to 405, and wherein said antibody optionally comprises one or more further substitutions, deletions and/or insertions, but does not comprise a Cys-Pro-Pro-Cys sequence in the hinge region. Preferably, said antibody comprises a Lys or Ala residue at the position corresponding to 409 or the CH3 region of the antibody has been replaced by the CH3 region of human IgG1, of human IgG2 or of human IgG3. See also WO2008145142 (Genmab) and WO 2011131746 (Genmab).

Even further, the stabilized IgG4 HER2 antibody may be an IgG4 antibody comprising a heavy chain and a light chain, wherein said heavy chain comprises a human IgG4 constant region having a residue selected from the group consisting of: Lys, Ala, Thr, Met and Leu at the position corresponding to 409 and/or a residue selected from the group consisting of: Ala, Val, Gly, Ile and Leu at the position corresponding to 405, and wherein said antibody optionally comprises one or more further substitutions, deletions and/or insertions and wherein said antibody comprises a Cys-Pro-Pro-Cys sequence in the hinge region. Preferably, said antibody comprises a Lys or Ala residue at the position corresponding to 409 or the CH3 region of the antibody has been replaced by the CH3 region of human IgG1, of human IgG2 or of human IgG3.

The effector-function-deficient HER2 antibody may be an antibody of a non-IgG4 type, e.g. IgG1, IgG2 or IgG3 which has been mutated such that the ability to mediate effector functions, such as ADCC, has been reduced or even eliminated. Such mutations have *e.g.* been described in Dall'Acqua WF et al., J Immunol. 177(2):1129-1138 (2006) and Hezareh M, J Virol. ;75(24):12161-12168 (2001).

Conjugates

The present disclosure further provides a bispecific HER2xHER2 antibody linked or conjugated to one or more therapeutic moieties, such as a cytotoxin, a chemotherapeutic drug, a cytokine, an immunosuppressant, and/or a radioisotope. Such conjugates are referred to herein as "immunoconjugates" or "drug conjugates". Immunoconjugates which include one or more cytotoxins are referred to as "immunotoxins".

A cytotoxin or cytotoxic agent includes any agent that is detrimental to (*e.g.*, kills) cells. Suitable therapeutic agents for forming immunoconjugates of the present invention include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, maytansine or an analog or derivative thereof, enediyene antitumor antibiotics including neocarzinostatin, calicheamycins, esperamicins, dynemicins, lidamycin, kedarcidin or analogs or derivatives thereof, anthracyclins, mitoxantrone, mithramycin, actinomycin D, 1-dehydrotestosterone, glucocorticoids, procaine, tetracaine, lidocaine, propranolol, and puromycin, antimetabolites (such as methotrexate, 6-mercaptopurine, 6-thioguanine, cytarabine, fludarabin, 5-fluorouracil, decarbazine, hydroxyurea, asparaginase, gemcitabine, cladribine), alkylating agents (such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin; as well as duocarmycin A, duocarmycin SA, CC-1065 (a.k.a. rachelmycin), or analogs or derivatives of CC-1065), dolastatin, pyrrolo[2,1-c][1,4] benzodiazepins (PDBs) or analogues thereof, antibiotics (such as dactinomycin (formerly actinomycin), bleomycin, daunorubicin (formerly daunomycin), doxorubicin, idarubicin, mithramycin, mitomycin, mitoxantrone, plicamycin, anthramycin (AMC)), anti-mitotic agents (*e.g.*, tubulin-inhibitors) such as monomethyl auristatin E, monomethyl auristatin F, or other analogs or derivatives of dolastatin 10; Histone deacetylase inhibitors such as the hydroxamic acids trichostatin A, vorinostat (SAHA), belinostat, LAQ824, and panobinostat as well as the benzamides, entinostat, CI994, mocetinostat and aliphatic acid compounds such as phenylbutyrate and valproic acid, proteasome inhibitors such as Danoprevir, bortezomib, amatoxins such as $\alpha$-amantin, diphtheria toxin and related molecules (such as diphtheria A chain and active fragments thereof and hybrid molecules); ricin toxin (such as ricin A or a deglycosylated ricin A chain toxin), cholera toxin, a Shiga-like toxin (SLT-I, SLT-II, SLT-IIV), LT toxin, C3 toxin, Shiga toxin, pertussis toxin, tetanus toxin, soybean Bowman-Birk protease inhibitor, Pseudomonas exotoxin, alorin, saporin, modeccin, gelanin, abrin A chain, modeccin A chain, alpha-sarcin, Aleurites fordii proteins, dianthin proteins, Phytolacca americana proteins (PAPI, PAPII, and PAP-S), momordica charantia inhibitor, curcin, crotin, sapaonaria officinalis inhibitor, gelonin, mitogellin, restrictocin, phenomycin, and enomycin toxins. Other suitable conjugated molecules include antimicrobial/lytic peptides such as CLIP, Magainin 2, mellitin, Cecropin, and P18; ribonuclease (RNase), DNase I, Staphylococcal enterotoxin-A, pokeweed antiviral protein, diphtherin toxin, and Pseudomonas endotoxin. See, for example, Pastan et al., Cell 47, 641 (1986) and Goldenberg, Calif. A Cancer Journal for Clinicians 44, 43 (1994). Therapeutic agents that may be administered in combination with a HER2 antibody of the present invention as described elsewhere herein, such as, *e.g.,* anti-cancer cytokines or chemokines, are also candidates for therapeutic moieties useful for conjugation to an antibody of the present invention.

The drug conjugates of the present disclosure comprise a bispecific antibody as disclosed herein conjugated to auristatins or auristatin peptide analogs and derivates (US5635483; US5780588). Auristatins have been shown to interfere with microtubule dynamics, GTP hydrolysis and nuclear and cellular division (Woyke et al (2001) Antimicrob. Agents and Chemother. 45(12): 3580-3584) and have anti-cancer (US5663149) and antifungal activity (Pettit et al., (1998) Antimicrob. Agents and Chemother. 42:2961-2965. The auristatin drug moiety may be attached to the antibody via a linker, through the N (amino) terminus or the C (terminus) of the peptidic drug moiety.

Exemplary auristatin embodiments include the N-terminus-linked monomethyl auristatin drug moieties DE and DF, disclosed in Senter et al., Proceedings of the American Association for Cancer Research. Volume 45, abstract number 623, presented March 28, 2004 and described in US 2005/0238649).

An exemplary auristatin embodiment is MMAE (monomethyl auristatin E). Another exemplary auristatin embodiment is MMAF (monomethyl auristatin F).

A bispecific antibody of the disclosure may comprise a conjugated nucleic acid or nucleic acid-associated molecule. The conjugated nucleic acid may be a cytotoxic ribonuclease, an antisense nucleic acid, an inhibitory RNA molecule (*e.g.*, a siRNA molecule) or an immunostimulatory nucleic acid (*e.g.*, an immunostimulatory CpG motif-containing DNA molecule). A HER2xHER2 antibody of the disclosure may be conjugated to an aptamer or a ribozyme.

Bispecific antibodies comprising one or more radiolabeled amino acids are provided. A radiolabeled bispecific antibody may be used for both diagnostic and therapeutic purposes (conjugation to radiolabeled molecules is another possible feature). Non-limiting examples of labels for polypeptides include 3H, 14C, 15N, 35S, 90Y, 99Tc, and 125I, 131I, and 186Re. Methods for preparing radiolabeled amino acids and related peptide derivatives are known in the art, (see, for instance Junghans *et al.,* in Cancer Chemotherapy and Biotherapy 655-686 (2nd Ed., Chafner and Longo, eds., Lippincott Raven (1996)) and U.S. 4,681,581, U.S. 4,735,210, U.S. 5,101,827, U.S. 5,102,990 (US RE35,500), U.S. 5,648,471 and U.S. 5,697,902. For example, a radioisotope may be conjugated by the chloramine-T method.

The bispecific antibody may be conjugated to a radioisotope or to a radioisotope-containing chelate. For example, the bispecific antibody can be conjugated to a chelator linker, e.g. DOTA, DTPA or tiuxetan, which allows for the bispecific antibody to be complexed with a radioisotope. The bispecific antibody may also or alternatively comprise or be conjugated to one or more radiolabeled amino acids or other radiolabeled molecule. A radiolabeled HER2xHER2 antibody may be used for both diagnostic and therapeutic purposes. In one embodiment the bispecific antibody of the present invention is conjugated to an alpha-emitter. Non-limiting examples of radioisotopes include $^3$H, $^{14}$C, $^{15}$N, $^{35}$S, $^{90}$Y, $^{99}$Tc, $^{125}$I, $^{111}$In, $^{131}$I, $^{186}$Re, $^{213}$Bs, $^{225}$Ac and $^{227}$Th.

The bispecific antibody of the present disclosure may be conjugated to a cytokine selected from the group consisting of IL-2, IL-4, IL-6, IL-7, IL-10, IL-12, IL-13, IL-15, IL-18, IL-23, IL-24, IL-27, IL-28a, IL-28b, IL-29, KGF, IFNo, IFNβ, IFNγ, GM-CSF, CD40L, Flt3 ligand, stem cell factor, ancestim, and TNFo.

Bispecific antibodies may also be chemically modified by covalent conjugation to a polymer to for instance increase their circulating half-life. Exemplary polymers, and methods to attach them to peptides, are illustrated in for instance US 4,766,106, US 4,179,337, US 4,495,285 and US 4,609,546. Additional polymers include polyoxyethylated polyols and polyethylene glycol (PEG) (*e.g.*, a PEG with a molecular weight of between about 1,000 and about 40,000, such as between about 2,000 and about 20,000).

Any method known in the art for conjugating the bispecific antibody to the conjugated molecule(s), such as those described above, may be employed, including the methods described by Hunter et al., Nature 144, 945 (1962), David et al., Biochemistry 13, 1014 (1974), Pain et al., J. Immunol. Meth. 40, 219 (1981) and Nygren, J. Histochem. and Cytochem. 30, 407 (1982). Such bispecific antibodies may be produced by chemically conjugating the other moiety to the N-terminal side or C-terminal side of the bispecific antibody or fragment thereof (*e.g.*, a HER2 bispecific antibody H or L chain) (see, *e.g.,* Antibody Engineering Handbook, edited by Osamu Kanemitsu, published by Chijin Shokan (1994)). Such conjugated bispecific antibody derivatives may also be generated by conjugation at internal residues or sugars, where appropriate.

The agents may be coupled either directly or indirectly to a bispecific antibody of the present invention. One example of indirect coupling of a second agent is coupling via a spacer or linker moiety to cysteine or lysine residues in the bispecific antibody. A HER2xHER2 antibody may be conjugated to a prodrug molecule that can be activated *in vivo* to a therapeutic drug via a spacer or linker. The linker may be cleavable under intracellular conditions, such that the cleavage of the linker releases the drug unit from the bispecific antibody in the intracellular environment. The linker may be cleavable by a cleavable agent that is present in the intracellular environment (e. g. within a lysosome or endosome or caveola). For example, the spacers or linkers may be cleaveable by tumor-cell associated enzymes or other tumor-specific conditions, by which the active drug is formed. Examples of such prodrug techologies and linkers are described in WO02083180, WO2004043493, WO2007018431, WO2007089149, WO2009017394 and WO201062171 by Syntarga BV, *et al.* Suitable antibody-prodrug technology and duocarmycin analogs can also be found in U.S. Patent No. 6,989,452 (Medarex). The linker can also or alternatively be, *e.g.* a peptidyl linker that is cleaved by an intracellular peptidase or protease enzyme, including but not limited to, a lysosomal or endosomal protease. The peptidyl linker may be at least two amino acids long or at least three amino acids long. Cleaving agents can include cathepsins B and D and plasmin, all of which are known to hydrolyze dipeptide drug derivatives resulting in the release of active drug inside the target cells (see e. g. Dubowchik and Walker, 1999, Pharm. Therapeutics 83:67-123). The peptidyl linker may be cleavable by an intracellular protease is a Val-Cit (valine-citrulline) linker or a Phe-Lys (phenylalanine-lysine) linker (see *e.g.* US6214345, which describes the synthesis of doxorubicin with the Val-Cit linker and different examples of Phe-Lys linkers). Examples of the structures of a Val-Cit and a Phe-Lys linker include but are not limited to MC-vc-PAB described below, MC-vc-GABA, MC-Phe-Lys-PAB or MC-Phe-Lys-GABA, wherein MC is an abbreviation for maleimido caproyl, vc is an abbreviation for Val-Cit, PAB is an abbreviation for *p*-aminobenzylcarbamate and GABA is an abbreviation for γ-aminobutyric acid. An advantage of using intracellular proteolytic release of the therapeutic agent is that the agent is typically attenuated when conjugated and the serum stabilities of the conjugates are typically high.

The linker unit may be one that is not cleavable and the drug may be released by antibody degradation (see US 2005/0238649). Typically, such a linker is not substantially sensitive to the extracellular environment. As used herein, "not substantially sensitive to the extracellular environment" in the context of a linker means that no more than 20%, typically no more than about 15%, more typically no more than about 10%, and even more typically no more than about 5%, no more than about 3%, or no more than about 1% of the linkers, in a sample of antibody drug conjugate compound, are cleaved when the antibody drug conjugate compound presents in an extracellular environment (*e.g.* plasma). Whether a linker is not substantially sensitive to the extracellular environment can be determined for example by incubating the antibody drug conjugate compound with plasma for a predetermined time period (*e.g.* 2, 4, 8, 16 or 24 hours) and then quantitating the amount of free drug present in the plasma. Exemplary embodiments comprising MMAE or MMAF and various linker components have the following structures (wherein Ab means antibody and p, representing the drug-loading (or average number of cytostatic or cytotoxic drugs per antibody molecule), is 1 to about 8, *e.g.* p may be from 4-6, such as from 3-5, or p may be 1, 2, 3, 4, 5, 6, 7 or 8).

Examples where a cleavable linker is combined with an auristatin include MC-vc-PAB-MMAF (also designated as vcMMAF) and MC-vc-PAB-MMAF (also designated as vcMMAE), wherein MC is an abbreviation for maleimido caproyl, vc is an abbreviation for the Val-Cit (valine-citruline) based linker, and PAB is an abbreviation for *p*-aminobenzylcarbamate.

Other examples include auristatins combined with a non-cleavable linker, such as mcMMAF (mc (MC is the same as mc in this context) is an abbreviation of maleimido caproyl).

The drug linker moiety may be vcMMAE. The vcMMAE drug linker moiety and conjugation methods are disclosed in WO2004010957, US7659241, US7829531, US7851437 and US 11/833,028 (Seattle Genetics, Inc.) , and the vcMMAE drug linker moiety is bound to the anti-HER2 bispecific antibodies at the cysteines using a method similar to those disclosed in therein.

The drug linker moiety may be mcMMAF. The mcMMAF drug linker moiety and conjugation methods are disclosed in US7498298, US 11/833,954, and WO2005081711 (Seattle Genetics, Inc.) , and the mcMMAF drug linker moiety is bound to the anti-HER2 bispecific antibodies at the cysteines using a method similar to those disclosed in therein.

The bispecific antibody of the present disclosure may be attached to a chelator linker, *e.g.* tiuxetan, which allows for the bispecific antibody to be conjugated to a radioisotope.

Each arm (or Fab-arm) of the bispecific antibody may be coupled directly or indirectly to the same one or more therapeutic moieties.

Optionally, only one arm of the bispecific antibody is coupled directly or indirectly to one or more therapeutic moieties.

Each arm of the bispecific antibody may be coupled directly or indirectly to different therapeutic moieties. For example, where the bispecific antibody is prepared by controlled Fab-arm exchange of two different monospecific HER2 antibodies, e.g. a first and second HER2 antibody, as described herein, such bispecific antibodies can be obtained by using monospecific antibodies which are conjugated or associated with different therapeutic moieties. Accordingly, the present disclosure provides for a method of preparing bispecific HER2xHER2 antibodies comprising the following steps:

a) providing a first HER2 antibody comprising an Fc region of an immunoglobulin and a first therapeutic moiety, said Fc region comprising a first CH3 region,
b) providing a second HER2 antibody comprising an Fc region of an immunoglobulin and a second therapeutic moiety, said Fc region comprising a second CH3 region,
wherein the sequences of said first and second CH3 regions are different and are such that the heterodimeric interaction between said first and second CH3 regions is stronger than each of the homodimeric interactions of said first and second CH3 regions,
c) incubating said first HER2 antibody together with said second HER2 antibody under reducing conditions, and
d) obtaining said bispecific HER2xHER2 antibody.

The first and second therapeutic moieties may be the same. The first and second therapeutic moieties may be different.

Compositions

In a further main aspect, the invention relates to a pharmaceutical composition comprising the bispecific antibody according to the inventoin and
a pharmaceutically-acceptable carrier.

The pharmaceutical composition of the present invention may contain one bispecific antibody of the present invention or a combination of different bispecific antibodies of the present invention.

The pharmaceutical compositions may be formulated in accordance with conventional techniques such as those disclosed in Remington: The Science and Practice of Pharmacy, 19th Edition, Gennaro, Ed., Mack Publishing Co., Easton, PA, 1995. A pharmaceutical composition of the present invention may *e.g.* include diluents, fillers, salts, buffers, detergents (e. g., a nonionic detergent, such as Tween-20 or Tween-80), stabilizers (e. g., sugars or protein-free amino acids), preservatives, tissue fixatives, solubilizers, and/or other materials suitable for inclusion in a pharmaceutical composition.

Pharmaceutically acceptable carriers include any and all suitable solvents, dispersion media, coatings, antibacterial and antifungal agents, isotonicity agents, antioxidants and absorption delaying agents, and the like that are physiologically compatible with a bispecific antibody of the present invention. Examples of suitable aqueous and nonaqueous carriers which may be employed in the pharmaceutical compositions of the present invention include water, saline, phosphate buffered saline, ethanol, dextrose, polyols (such as glycerol, propylene glycol, polyethylene glycol, and the like), and suitable mixtures thereof, vegetable oils, carboxymethyl cellulose colloidal solutions, tragacanth gum and injectable organic esters, such as ethyl oleate, and/or various buffers. Pharmaceutically acceptable carriers include sterile aqueous solutions or dispersions and sterile powders for the extemporaneous preparation of sterile injectable solutions or dispersion. Proper fluidity may be maintained, for example, by the use of coating materials, such as lecithin, by the maintenance of the required particle size in the case of dispersions, and by the use of surfactants.

Pharmaceutical compositions of the present invention may also comprise pharmaceutically acceptable antioxidants for instance (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole, butylated hydroxytoluene, lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Pharmaceutical compositions of the present invention may also comprise isotonicity agents, such as sugars, polyalcohols, such as mannitol, sorbitol, glycerol or sodium chloride in the compositions.

The pharmaceutical compositions of the present invention may also contain one or more adjuvants appropriate for the chosen route of administration such as preservatives, wetting agents, emulsifying agents, dispersing agents, preservatives or buffers, which may enhance the shelf life or effectiveness of the pharmaceutical composition. The bispecific antibodies of the present invention may be prepared with carriers that will protect the bispecific antibody against rapid release, such as a controlled release formulation, including implants, transdermal patches, and microencapsulated delivery systems. Such carriers may include gelatin, glyceryl monostearate, glyceryl distearate, biodegradable, biocompatible polymers such as ethylene vinyl acetate, polyanhydrides, polyglycolic acid, collagen, polyorthoesters, and polylactic acid alone or with a wax, or other materials well known in the art. Methods for the preparation of such formulations are generally known to those skilled in the art.

Sterile injectable solutions may be prepared by incorporating the active compound in the required amount in an appropriate solvent with one or a combination of ingredients e.g. as enumerated above, as required, followed by sterilization microfiltration. Generally, dispersions are prepared by incorporating the active compound into a sterile vehicle that contains a basic dispersion medium and the required other ingredients *e.g.* from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof.

The actual dosage levels of the active ingredients in the pharmaceutical compositions may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The pharmaceutical composition may be administered by any suitable route and mode. In one embodiment, a pharmaceutical composition of the present invention is administered parenterally. "Administered parenterally" as used herein means modes of administration other than enteral and topical administration, usually by injection, and include epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion.

The pharmaceutical composition may be administered by intravenous or subcutaneous injection or infusion.

Uses

In a further main aspect, the invention relates to a bispecific HER2xHER2 antibody of the invention for use as a medicament.

The bispecific antibodies of the invention may be used for a number of purposes. In particular, the antibodies of the invention may be used for the treatment of various forms of cancer, including metastatic cancer and refractory cancer.

The bispecific antibodies of the invention may be used for the treatment of breast cancer, including primary, metastatic, and refractory breast cancer.

The bispecific antibodies of the invention may be used for the treatment of a form of cancer selected from the group consisting of prostate cancer, non-small cell lung cancer, bladder cancer, ovarian cancer, gastric cancer, colorectal cancer, esophageal cancer, squamous cell carcinoma of the head & neck, cervical cancer, pancreatic cancer, testis cancer, malignant melanoma and a soft-tissue cancer (*e.g.* synovial sarcoma).

Similarly, the disclosure relates to a method for killing a tumor cell expressing HER2, comprising administration, to an individual in need thereof, of an effective amount of an antibody of the invention, such as an antibody drug-conjugate (ADC).

The present disclosure also relates to a method for inhibiting growth and/or proliferation of one or more tumor cells expressing HER2, comprising administration, to an individual in need thereof, of a bispecific antibody according to the present invention.

The present disclosure also relates to a method for treating cancer, comprising

a) selecting a subject suffering from a cancer comprising tumor cells expressing HER2, and
b) administering to the subject the bispecific antibody of the present invention or a pharmaceutical composition of the present invention

Said tumor cell may be involved in a form of cancer selected from the group consisting of: breast cancer, prostate cancer, non-small cell lung cancer, bladder cancer, ovarian cancer, gastric cancer, colorectal cancer, esophageal cancer and squamous cell carcinoma of the head & neck, cervical cancer, pancreatic cancer, testis cancer, malignant melanoma, and a soft-tissue cancer (*e.g.*, synovial sarcoma).

The tumor cell may be one that co-expresses HER2 and at least one other member of the EGFR family, preferably EGFR, HER3, or both of EGFR and HER3, and is a tumor cell involved in breast cancer, colorectal cancer, endometrial/cervical cancer, lung cancer, malignant melanoma, ovarian cancer, pancreatic cancer, prostate cancer, testis cancer, a soft-tissue tumor (*e.g.*, synovial sarcoma), or bladder cancer.

The disclosure also relates to a method for treating cancer in a subject, comprising selecting a subject suffering from a cancer comprising tumor cells co-expressing HER2 and EGFR and/or HER3, and administering to the subject a bispecific antibody of the invention, optionally in the form of a bispecific antibody conjugated to a cytotoxic agent or drug. The subject suffers from a cancer selected from the group consisting of breast cancer, colorectal cancer, endometrial/cervical cancer, lung cancer, malignant melanoma, ovarian cancer, pancreatic cancer, prostate cancer, testis cancer, a soft-tissue tumor (*e.g.*, synovial sarcoma), or bladder cancer.

Also, the disclosure relates to the use of a bispecific antibody that binds to human HER2 for the preparation of a medicament for the treatment of cancer, such as one of the specific cancer indications mentioned above.

The invention further relates to a bispecific antibody for use in the treatment of cancer, such as one of the cancer indications mentioned above.

In the methods of treatment of the present disclosure, the efficacy of the treatment may be being monitored during the therapy, *e.g.* at predefined points in time, by determining tumor burden or HER2 expression levels on the relevant tumor cells.

Dosage regimens in the above methods of treatment and uses are adjusted to provide the optimum desired response (*e.g.*, a therapeutic response). For example, a single bolus may be administered, several divided doses may be administered over time or the dose may be proportionally reduced or increased as indicated by the exigencies of the therapeutic situation. Parenteral compositions may be formulated in dosage unit form for ease of administration and uniformity of dosage.

The efficient dosages and the dosage regimens for the bispecific antibodies depend on the disease or condition to be treated and may be determined by the persons skilled in the art. An exemplary, non-limiting range for a therapeutically effective amount of a compound of the present invention is about 0.1-100 mg/kg, such as about 0.1-50 mg/kg, for example about 0.1-20 mg/kg, such as about 0.1-10 mg/kg, for instance about 0.5, about such as 0.3, about 1, about 3, about 5, or about 8 mg/kg.

A physician or veterinarian having ordinary skill in the art may readily determine and prescribe the effective amount of the pharmaceutical composition required. For example, the physician or veterinarian could start doses of the bispecific antibody employed in the pharmaceutical composition at levels lower than that required in order to achieve the desired therapeutic effect and gradually increase the dosage until the desired effect is achieved. In general, a suitable daily dose of a composition of the present invention will be that amount of the bispecific antibody which is the lowest dose effective to produce a therapeutic effect. Administration may *e.g.* be parenteral, such as intravenous, intramuscular or subcutaneous. In one embodiment, the bispecific antibodies may be administered by infusion in a weekly dosage of from 10 to 500 mg/m$^2$, such as of from 200 to 400 mg/m$^2$. Such administration may be repeated, *e.g.,* 1 to 8 times, such as 3 to 5 times. The administration may be performed by continuous infusion over a period of from 2 to 24 hours, such as of from 2 to 12 hours. In one embodiment, the bispecific antibodies may be administered by slow continuous infusion over a long period, such as more than 24 hours, in order to reduce toxic side effects.

The bispecific antibodies may be administered in a weekly dosage of from 250 mg to 2000 mg, such as for example 300 mg, 500 mg, 700 mg, 1000 mg, 1500 mg or 2000 mg, for up to 8 times, such as from 4 to 6 times when given once a week. Such regimen may be repeated one or more times as necessary, for example, after 6 months or 12 months. The dosage may be determined or adjusted by measuring the amount of bispecific antibody of the present invention in the blood upon administration by for instance taking out a biological sample and using anti-idiotypic antibodies which target the antigen binding region of the HER2 bispecific antibodies of the present invention.

The efficient dosages and the dosage regimens for the bispecific antibodies depend on the disease or condition to be treated and may be determined by the persons skilled in the art. An exemplary, non-limiting range for a therapeutically effective amount of a bispecific antibody of the present invention is about 0.1-100 mg/kg, such as about 0.1-50 mg/kg, for example about 0.1-20 mg/kg, such as about 0.1-10 mg/kg, for instance about 0.5, about such as 0.3, about 1, about 3, about 5, or about 8 mg/kg.

The bispecific antibodies may be administered by maintenance therapy, such as, *e.g.,* once a week for a period of 6 months or more.

A bispecific antibody may also be administered prophylactically in order to reduce the risk of developing cancer, delay the onset of the occurrence of an event in cancer progression, and/or reduce the risk of recurrence when a cancer is in remission.

The bispecific antibodies of the invention may also be administered in combination therapy, *i.e.,* combined with other therapeutic agents relevant for the disease or condition to be treated. Accordingly, the bispecific antibody-containing medicament may be for combination with one or more further therapeutic agent, such as a cytotoxic, chemotherapeutic or anti-angiogenic agent.

Such combined administration may be simultaneous, separate or sequential. For simultaneous administration the agents may be administered as one composition or as separate compositions, as appropriate. The present disclosure thus also provides methods for treating a disorder involving cells expressing HER2 as described above, which methods comprise administration of a bispecific antibody of the present invention combined with one or more additional therapeutic agents as described below.

The present disclosure provides a method for treating a disorder involving cells expressing HER2 in a subject, which method comprises administration of a therapeutically effective amount of a bispecific antibody of the present invention, and optionally at least one additional therapeutic agent, or an antibody binding to a different epitope than said HER2 antibody, to a subject in need thereof.

The present disclosure provides a method for treating or preventing cancer, which method may comprise administration of a therapeutically effective amount of a bispecific antibody of the present invention and at least one additional therapeutic agent to a subject in need thereof.

Such an additional therapeutic agent may be selected from an antimetabolite, such as methotrexate, 6-mercaptopurine, 6-thioguanine, cytarabine, fludarabine, 5-fluorouracil, decarbazine, hydroxyurea, asparaginase, gemcitabine or cladribine.

Such an additional therapeutic agent may be selected from an alkylating agent, such as mechlorethamine, thioepa, chlorambucil, melphalan, carmustine (BSNU), lomustine (CCNU), cyclophosphamide, busulfan, dibromomannitol, streptozotocin, dacarbazine (DTIC), procarbazine, mitomycin C, cisplatin and other platinum derivatives, such as carboplatin.

Such an additional therapeutic agent may be selected from an anti-mitotic agent, such as taxanes, for instance docetaxel, and paclitaxel, and vinca alkaloids, for instance vindesine, vincristine, vinblastine, and vinorelbine.

Such an additional therapeutic agent may be selected from a topoisomerase inhibitor, such as topotecan or irinotecan, or a cytostatic drug, such as etoposide and teniposide.

Such an additional therapeutic agent may be selected from a growth factor inhibitor, such as an inhibitor of ErbB1 (EGFR) (such as an EGFR antibody, *e.g.* zalutumumab, cetuximab, panitumumab or nimotuzumab or other EGFR inhibitors, such as gefitinib or erlotinib), another inhibitor of ErbB2 (HER2/neu) (such as a HER2 antibody, *e.g.* trastuzumab, trastuzumab-DM1 or pertuzumab) or an inhibitor of both EGFR and HER2, such as lapatinib).

Such an additional therapeutic agent may be selected from a tyrosine kinase inhibitor, such as imatinib (Glivec, Gleevec STI571) or lapatinib, PTK787/ZK222584.

The present disclosure provides a method for treating a disorder involving cells expressing HER2 in a subject, which method may comprise administration of a therapeutically effective amount of a bispecific antibody of the present disclosure and at least one inhibitor of angiogenesis, neovascularization, and/or other vascularization to a subject in need thereof

Examples of such angiogenesis inhibitors are urokinase inhibitors, matrix metalloprotease inhibitors (such as marimastat, neovastat, BAY 12-9566, AG 3340, BMS-275291 and similar agents), inhibitors of endothelial cell migration and proliferation (such as TNP-470, squalamine, 2-methoxyestradiol, combretastatins, endostatin, angiostatin, penicillamine, SCH66336 (Schering-Plough Corp, Madison, NJ), R115777 (Janssen Pharmaceutica, Inc, Titusville, NJ) and similar agents), antagonists of angiogenic growth factors (such as such as ZD6474, SU6668, antibodies against angiogenic agents and/or their receptors (such as VEGF (*e.g.* bevacizumab), bFGF, and angiopoietin-1), thalidomide, thalidomide analogs (such as CC-5013), Sugen 5416, SU5402, antiangiogenic ribozyme (such as angiozyme), interferon α (such as interferon a2a), suramin and similar agents), VEGF-R kinase inhibitors and other anti-angiogenic tyrosine kinase inhibitors (such as SU011248), inhibitors of endothelial-specific integrin/survival signaling (such as vitaxin and similar agents), copper antagonists/chelators (such as tetrathiomolybdate, captopril and similar agents), carboxyamido-triazole (CAI), ABT-627, CM101, interleukin-12 (IL-12), IM862, PNU145156E as well as nucleotide molecules inhibiting angiogenesis (such as antisense-VEGF-cDNA, cDNA coding for angiostatin, cDNA coding for p53 and cDNA coding for deficient VEGF receptor-2).

Other examples of such inhibitors of angiogenesis, neovascularization, and/or other vascularization are anti-angiogenic heparin derivatives (e.g., heperinase III), temozolomide, NK4, macrophage migration inhibitory factor, cyclooxygenase-2 inhibitors, inhibitors of hypoxia-inducible factor 1, anti-angiogenic soy isoflavones, oltipraz, fumagillin and analogs thereof, somatostatin analogues, pentosan polysulfate, tecogalan sodium, dalteparin, tumstatin, thrombospondin, NM-3, combrestatin, canstatin, avastatin, antibodies against other targets, such as anti-alpha-v/beta-3 integrin and anti-kininostatin antibodies.

A therapeutic agent for use in combination with a HER2 bispecific antibody for treating the disorders as described above may be an anti-cancer immunogen, such as a cancer antigen/tumor-associated antigen (e.g., epithelial cell adhesion molecule (EpCAM/TACSTD1), mucin 1 (MUC1), carcinoembryonic antigen (CEA), tumor-associated glycoprotein 72 (TAG-72), gp100, Melan-A, MART-1, KDR, RCAS1, MDA7, cancer-associated viral vaccines (e.g., human papillomavirus vaccines) or tumor-derived heat shock proteins,

A therapeutic agent for use in combination with a HER2 bispecific antibody for treating the disorders as described above may be an anti-cancer cytokine, chemokine, or combination thereof. Examples of suitable cytokines and growth factors include IFNγ, IL-2, IL-4, IL-6, IL-7, IL-10, IL-12, IL-13, IL-15, IL-18, IL-23, IL-24, IL-27, IL-28a, IL-28b, IL-29, KGF, IFNα (e.g., INFa2b), IFNβ, GM-CSF, CD40L, Flt3 ligand, stem cell factor, ancestim, and TNFα. Suitable chemokines may include Glu-Leu-Arg (ELR)-negative chemokines such as IP-10, MCP-3, MIG, and SDF-1a from the human CXC and C-C chemokine families. Suitable cytokines include cytokine derivatives, cytokine variants, cytokine fragments, and cytokine fusion proteins.

A therapeutic agent for use in combination with a bispecific antibody for treating the disorders as described above may be a cell cycle control/apoptosis regulator (or "regulating agent"). A cell cycle control/apoptosis regulator may include molecules that target and modulate cell cycle control/apoptosis regulators such as (i) cdc-25 (such as NSC 663284), (ii) cyclin-dependent kinases that overstimulate the cell cycle (such as flavopiridol (L868275, HMR1275), 7-hydroxystaurosporine (UCN-01, KW-2401), and roscovitine (R-roscovitine, CYC202)), and (iii) telomerase modulators (such as BIBR1532, SOT-095, GRN163 and compositions described in for instance US 6,440,735 and US 6,713,055). Non-limiting examples of molecules that interfere with apoptotic pathways include TNF-related apoptosis-inducing ligand (TRAIL)/apoptosis-2 ligand (Apo-2L), antibodies that activate TRAIL receptors, IFNs,L and anti-sense Bcl-2.

A therapeutic agent for use in combination with a bispecific antibody for treating the disorders as described above may be a hormonal regulating agent, such as agents useful for anti-androgen and anti-estrogen therapy. Examples of such hormonal regulating agents are tamoxifen, idoxifene, fulvestrant, droloxifene, toremifene, raloxifene, diethylstilbestrol, ethinyl estradiol/estinyl, an antiandrogene (such as flutaminde/eulexin), a progestin (such as such as hydroxyprogesterone caproate, medroxyprogesterone/provera, megestrol acepate/megace), an adrenocorticosteroid (such as hydrocortisone, prednisone), luteinizing hormone-releasing hormone (and analogs thereof and other LHRH agonists such as buserelin and goserelin), an aromatase inhibitor (such as anastrazole/arimidex, aminoglutethimide/cytraden, exemestane) or a hormone inhibitor (such as octreotide/- sandostatin).

A therapeutic agent for use in combination with a bispecific antibody for treating the disorders as described above may be an anti-anergic agent, such as molecules that block the activity of CTLA-4, e.g. ipilimumab.

A therapeutic agent for use in combination with a bispecific antibody for treating the disorders as described above may be an anti-cancer nucleic acid or an anti-cancer inhibitory RNA molecule.

Examples of other anti-cancer agents, which may be relevant as therapeutic agents for use in combination with a bispecific antibody according to the invention for treating the disorders as described above are differentiation inducing agents, retinoic acid analogues (such as all trans retinoic acid, 13-cis retinoic acid and similar agents), vitamin D analogues (such as seocalcitol and similar agents), inhibitors of ErbB3, ErbB4, IGF-IR, insulin receptor, PDGFRa, PDGFRbeta, Flk2, Flt4, FGFR1, FGFR2, FGFR3, FGFR4, TRKA, TRKC, RON (such as an anti-RON antibody), Sea, Tie, Tie2, Eph, Ret, Ros, Alk, LTK, PTK7 and similar agents.

Examples of other anti-cancer agents, which may be relevant as therapeutic agents for use in combination with a bispecific antibody according to the disclosure for treating the disorders as described above are estramustine and epirubicin.

Examples of other anti-cancer agents, which may be relevant as therapeutic agents for use in combination with a bispecific antibody according to the disclosure for treating the disorders as described above are a HSP90 inhibitor like 17-allyl amino geld-anamycin, antibodies directed against a tumor antigen such as PSA, CA125, KSA, integrins, e.g. integrin β1, or inhibitors of VCAM. Examples of other anti-cancer agents, which may be relevant as therapeutic agents for use in combination with a bispecific antibody for treating the disorders as described above are calcineurin-inhibitors (such as valspodar, PSC 833 and other MDR-1 or p-glycoprotein inhibitors), TOR-inhibitors (such as sirolimus, everolimus and rapamcyin). and inhibitors of "lymphocyte homing" mechanisms (such as FTY720), and agents with effects on cell signaling such as adhesion molecule inhibitors (for instance anti-LFA).

The bispecific antibody of the disclosure may be for use in combination with one or more other therapeutic antibodies, such as ofatumumab, zanolimumab, daratumumab, ranibizumab, nimotuzumab, panitumumab, hu806, daclizumab (Zenapax), basiliximab (Simulect), infliximab (Remicade), adalimumab (Humira), natalizumab (Tysabri), omalizumab (Xolair), efalizumab (Raptiva), ipilimumab and/or rituximab.

Two or more different antibodies of the present disclosure or therapeutic conjugates thereof, as described herein may be used in combination for the treatment of disease. Particularly interesting combinations include two or more non-blocking antibodies. Such combination therapy may lead to binding of an increased number of antibody molecules per cell, which may give increase efficacy, e.g. via activation of complement-mediated lysis.

In addition to the above, other embodiments of combination therapies of the disclosure include the following: For the treatment of breast cancer, a bispecific antibody of the present disclosure or a therapeutic conjugate thereof, in combination with methotrexate, paclitaxel, doxorubicin, carboplatin, cyclophosphamide, daunorubicin, epirubicin, 5-fluorouracil, gemcitabine, ixabepilone, mutamycin, mitoxantrone, vinorelbine, docetaxel, thiotepa, vincristine, capecitabine, an EGFR antibody (e.g. zalutumumab, cetuximab, panitumumab or nimotuzumab) or other EGFR inhibitor (such as gefitinib or erlotinib), another HER2 antibody or -conjugate (such as, e.g., trastuzumab, trastuzumab-DM1 or pertuzumab), an inhibitor of both EGFR and HER2 (such as lapatinib), and/or in combination with a HER3 inhibitor.

For the treatment of non-small-cell lung cancer, a bispecific antibody of the present disclosure or a therapeutic conjugate thereof in combination with EGFR inhibitors, such as an EGFR antibody, e.g. zalutumumab, cetuximab, panitumumab or nimotuzumab or other EGFR inhibitors (such as gefitinib or erlotinib), or in combination with an another HER2 agent (such as a HER2 antibody, e.g. trastuzumab, trastuzumab-DM1 or pertuzumab) or in combination with an inhibitor of both EGFR and HER2, such as lapatinib, or in combination with a HER3 inhibitor.

For the treatment of colorectal cancer, a bispecific antibody of the present disclosure or a therapeutic conjugate thereof, in combination with one or more compounds selected from: gemcitabine, bevacizumab, FOLFOX, FOLFIRI, XELOX, IFL, oxaliplatin, irinotecan, 5-FU/LV, Capecitabine, UFT, EGFR targeting agents, such as cetuximab, panitumumab, zalutumumab; VEGF inhibitors, or tyrosine kinase inhibitors such as sunitinib.

For the treatment of prostate cancer, a bispecific antibody of the present disclosure or a therapeutic conjugate thereof, in combination with one or more compounds selected from: hormonal/antihormonal therapies; such as antiandrogens, Luteinizing hormone releasing hormone (LHRH) agonists, and chemotherapeutics such as taxanes, mitoxantrone, estramustine, 5FU, vinblastine, and ixabepilone.

Radiotherapy - surgery

The present disclosure provides a method for treating a disorder involving cells expressing HER2 in a subject, which method comprises administration of a therapeutically effective amount of a bispecific antibody, such as a HER2xHER2 antibody of the present disclosure, and radiotherapy to a subject in need thereof.

The present disclosure provides a method for treating or preventing cancer, which method comprises administration of a therapeutically effective amount of a bispecific antibody, such as a HER2xHER2 antibody of the present disclosure, and radiotherapy to a subject in need thereof.

The present disclosure provides the use of a bispecific antibody of the present disclosure, for the preparation of a pharmaceutical composition for treating cancer to be administered in combination with radiotherapy.

Radiotherapy may comprise radiation or associated administration of radiopharmaceuticals to a patient is provided. The source of radiation may be either external or internal to the patient being treated (radiation treatment may, for example, be in the form of external beam radiation therapy (EBRT) or brachytherapy (BT)). Radioactive elements that may be used in practicing such methods include, e.g., radium, cesium-137, iridium-192, americium-241, gold-198, cobalt-57, copper-67, technetium-99, iodide-123, iodide-131, and indium-111.

The present disclosure provides a method for treating or preventing cancer, which method comprises administration to a subject in need thereof of a therapeutically effective amount of a bispecific antibody of the present disclosure, in combination with surgery.

Diagnostic uses

The bispecific antibodies of the disclosure may also be used for diagnostic purposes. Thus, the disclosure relates to a diagnostic composition comprising a bispecific HER2xHER2 antibody as defined herein.

The bispecific antibodies of the present disclosure may be used *in vivo* or *in vitro* for diagnosing diseases wherein activated cells expressing HER2 play an active role in the pathogenesis, by detecting levels of HER2, or levels of cells which contain HER2 on their membrane surface. This may be achieved, for example, by contacting a sample to be tested, optionally along with a control sample, with the bispecific antibody under conditions that allow for formation of a complex between the bispecific antibody and HER2.

Thus, the disclosure relates to a method for detecting the presence of HER2 antigen, or a cell expressing HER2, in a sample comprising:

- contacting the sample with a bispecific HER2xHER2 antibody of the disclosure under conditions that allow for formation of a complex between the bispecific antibody and HER2; and
- analyzing whether a complex has been formed.

The method may be performed *in vitro.*

More specifically, the present disclosure provides methods for the identification of, and diagnosis of invasive cells and tissues, and other cells targeted by bispecific antibodies of the present invention, and for the monitoring of the progress of therapeutic treatments, status after treatment, risk of developing cancer, cancer progression, and the like.

Suitable labels for the bispecific antibody and/or secondary antibodies used in such techniques are well-known in the art.

Further, the disclosure relates to a kit for detecting the presence of HER2 antigen, or a cell expressing HER2, in a sample comprising

- a bispecific HER2xHER2 antibody of the disclosure or a bispecific molecule of the disclosure; and
- instructions for use of the kit.

The present disclosure provides a kit for diagnosis of cancer comprising a container comprising a bispecific HER2xHER2 antibody, and one or more reagents for detecting binding of the bispecific antibody to HER2. Reagents may include, for example, fluorescent tags, enzymatic tags, or other detectable tags. The reagents may also include secondary or tertiary antibodies or reagents for enzymatic reactions, wherein the enzymatic reactions produce a product that may be visualized.

The present invention is further illustrated by the following examples, which should not be construed as limiting the scope of the invention, which is limited only by the scope of the claims.

## EXAMPLES

### Example 1 - Expression constructs for HER2 and HER2 variants

Fully codon-optimized constructs for expression of full length HER2 (1255 aa, Swissprot P04626), the extracellular domain (ECD) of HER2 (HER2-ECDHis, aa 1-653 with a C-terminal His6 tag), the naturally occurring HER2 splice variant (HER2-delex16, resulting from exon 16 deletion and lacking aa 633-648) and a truncated form of the HER2 receptor (HER2-stumpy, aa 648-1256), were generated. The construct contained suitable restriction sites for cloning and an optimal Kozak sequence (Kozak, M., Gene 1999;234(2):187-208.). The constructs were cloned in the mammalian expression vector pEE13.4 (Lonza Biologics; Bebbington, C.R., etal., Biotechnology (N Y) 1992;10(2):169-75) and fully sequenced to confirm the correctness of the construct.

### Example 2 - Expression constructs for Pertuzumab, trastuzumab, C1 and F5

Fully codon-optimized constructs for expression of the heavy chain (HC) and the light chain (LC) of the IgG1 antibodies pertuzumab, C1 and F5 in HEK cells, were generated. The variable regions encoded by these constructs are identical to those described in U.S. Patent No. 6,949,245 for pertuzumab heavy chain and light chain and U.S. Patent No. 7,244,826 for C1 and F5 heavy and light chain. For C1 and F5, the mammalian expression vectors p33G1f and p33K or p33L (pcDNA3.3 (Invitrogen)) containing the fully codon optimized constant region for the human IgG1 heavy chain (allotype f), the human kappa light chain or the human lambda light chain, respectively, were used. For pertuzumab, the mammalian expression vectors pG1f (pEE12.4 (Lonza Biologics) and pKappa (pEE6.4 (Lonza Biologics), containing the fully codon-optimized constant region for the human IgG1 heavy chain (allotype f) and the human kappa light chain, respectively, were used.

Trastuzumab (Herceptin®) can be produced in the same manner, using the heavy and light chain sequences described in, *e.g.,* U.S. Patent No. 7,632,924.

**Example 3** - **Transient expression of HER2 and HER2 variants in HEK-293 or CHO cells**

Freestyle™ 293-F (a HEK-293 subclone adapted to suspension growth and chemically defined Freestyle medium, (HEK-293F)) cells were obtained from Invitrogen and transfected with the appropriate plasmid DNA, using 293fectin (Invitrogen) according to the manufacturer's instructions. In the case of antibody expression, the appropriate heavy chain and light chain expression vectors were co-expressed.

pEE13.4HER2, pEE13.4HER2-delex16 and pEE13.4HER2-stumpy were transiently transfected in the Freestyle™ CHO-S (Invitrogen) cell line using Freestyle MAX transfection reagent (Invitrogen). Expression of HER2 and HER2-delex16 was tested by means of FACS analysis as described below.

**Example 4** - **Stable polyclonal pool expression of HER2 and HER2 variants in NS0**

pEE13.4HER2, pEE13.4HER2-delex16 and pEE13.4HER2-stumpy were stably transfected in NS0 cells by nucleofection (Amaxa). A pool of stably transfected cells was established after selection on glutamine dependent growth, based on the integrated glutamine synthetase selection marker (Barnes, L.M., et al., Cytotechnology 2000;32(2):109-123).

**Example 5** - **Purification of His-tagged HER2**

HER2ECDHis was expressed in HEK-293F cells. The His-tag in HER2ECDHis enabled purification with immobilized metal affinity chromatography, since the His-tagged protein binds strongly to the resin beads, while other proteins present in the culture supernatant do not bind strongly.

In this process, a chelator fixed onto the chromatographic resin was charged with $Co^{2+}$ cations. HER2ECDHis containing supernatant was incubated with the resin in batch mode (i.e. solution). After incubation, the beads were retrieved from the supernatant and packed into a column. The column was washed in order to remove weakly bound proteins. The strongly bound HER2ECDHis proteins were then eluted with a buffer containing imidazole, which competes with the binding of His to $Co^{2+}$. The eluent was removed from the protein by buffer exchange on a desalting column.

**Example 6** - **Immunization procedure of transgenic HuMAb® mice**

Antibodies 001, 019, 021, 025, 027, 032, 033, 035, 036, 049, 050, 051, 054, 055, 084, 091, 094, 098, 100, 105, 123 and 124 were derived from the following immunization: three female HCo12 mice, one male and two female HCo12-Balb/C mice, one male HCo17 mouse and one male HCo20 mouse (Medarex, San José, CA, USA) were immunized alternating with $5 \times 10^6$ NS0 cells stably transfected with HER2ECD intraperitoneal (IP) and 20 μg HER2ECDHis protein coupled to the hapten Keyhole Limpet Hemocyanin (KLH) subcutaneous (SC) at the tail base, with an interval of fourteen days. A maximum of eight immunizations was performed per mouse (four IP and four SC immunizations). The first immunization with cells was done in complete Freunds' adjuvant (CFA; Difco Laboratories, Detroit, MI, USA). For all other immunizations, cells were injected IP in PBS and KLH coupled HER2ECD was injected SC using incomplete Freunds' adjuvant (IFA; Difco Laboratories, Detroit, MI, USA).

Antibodies 125, 127, 129, 132, 152, 153 and 159 were derived from the following immunization: one male and two female HCo12-Balb/C mice, one female HCo20 mouse, and one female HCo12 mouse (Medarex) were immunized alternating with $5 \times 10^6$ NS0 cells stably transfected with HER2delex16 IP and 20 μg HER2ECDHis protein coupled to the hapten Keyhole Limpet Hemocyanin (KLH) SC at the tail base, with an interval of fourteen days. A maximum of eight immunizations was performed per mouse (four IP and four SC immunizations). The first immunization with cells was done in complete Freunds' adjuvant (CFA; Difco Laboratories, Detroit, MI, USA). For all other immunizations, cells were injected IP in PBS and KLH coupled HER2ECD was injected SC using incomplete Freunds' adjuvant (IFA; Difco Laboratories, Detroit, MI, USA).

Antibody 143, 160, 161, 162, 166 and 169 were derived from the following immunization: one female and one male Hco12 mouse, one female Hco12-Balb/C mouse, one male HCo17 mouse and one male HCo20 mouse (Medarex) were immunized alternating with 20 μg HER2ECDHis protein coupled to the hapten Keyhole Limpet Hemocyanin (KLH), alternating IP and SC at the tail base with an interval of fourteen days. A maximum of eight immunizations was performed per mouse (four IP and four SC immunizations). The first immunization was done IP in complete Freunds' adjuvant (CFA; Difco Laboratories, Detroit, MI, USA). The other immunizations were injected using incomplete Freunds' adjuvant (IFA; Difco Laboratories, Detroit, MI, USA).

Antibodies 005, 006, 041, 044, 059, 060, 067, 072, 093, 106 and 111 were derived from the following immunization procedure: two female HCo12 mice, one female and one male HCo12-Balb/C mouse, one female and one male HCo17 mouse, and two male HCo20 mice (Medarex, San José, CA, USA) were immunized every fortnight, alternating between $5 \times 10^6$ NS0 cells stably transfected with HER2ECD intraperitoneal (IP) and 20 μg HER2ECDHis protein coupled

to the hapten Keyhole Limpet Hemocyanin (KLH) subcutaneous (SC) at the tail base. A maximum of eight immunizations was performed per mouse (four IP and four SC immunizations). The first immunization with cells was done in complete Freunds' adjuvant (CFA; Difco Laboratories, Detroit, MI, USA). For all other immunizations, cells were injected IP in PBS and KLH coupled HER2ECD was injected SC using incomplete Freunds' adjuvant (IFA; Difco Laboratories, Detroit, MI, USA).

Antibody 150 was derived from immunization of one female HCo17 mouse (Medarex) alternating with $5 \times 10^6$ NS0 cells stably transfected with HER2delex16 IP and 20 $\mu$g HER2ECDHis protein coupled to the hapten Keyhole Limpet Hemocyanin (KLH) SC at the tail base, with an interval of fourteen days. A maximum of eight immunizations was performed (four IP and four SC immunizations). The first immunization with cells was done in complete Freunds' adjuvant (CFA; Difco Laboratories, Detroit, MI, USA). For all other immunizations, cells were injected IP in PBS and KLH coupled HER2ECD was injected SC using incomplete Freunds' adjuvant (IFA; Difco Laboratories, Detroit, MI, USA).

Antibody 163 was derived from immunization of one male HCo20 mouse (Medarex) with 20 $\mu$g HER2ECDHis protein coupled to the hapten Keyhole Limpet Hemocyanin (KLH), alternating IP and SC at the tailbase with an interval of fourteen days. A maximum of eight immunizations was performed (four IP and four SC immunizations). The first immunization was done IP in complete Freunds' adjuvant (CFA; Difco Laboratories, Detroit, MI, USA). The other immunizations were injected using incomplete Freunds' adjuvant (IFA; Difco Laboratories, Detroit, MI, USA).

Mice with at least two sequential titers against TC1014-HER2, TC1014-HER2delex16 or TC1014-HER2stumpy in the antigen specific FMAT screening assay (as described in Example 7), were considered positive and fused.

**Example 7 - Homogeneous screening assay for the detection of antigen-specific HER2 antibodies**

The presence of HER2 antibodies in sera of immunized mice (Example 6) or HuMab (human monoclonal antibody) hybridoma (Example 8) or transfectoma (Example 10) culture supernatant was determined by homogeneous antigen specific screening assays (four quadrant) using Fluorometric Micro volume Assay Technology (FMAT; Applied Biosystems, Foster City, CA, USA). For this, a combination of 4 cell based assays was used. Binding to TC1014-HER2 (CHO-S cells transiently expressing the HER2 receptor; produced as described above), TC1014-HER2delex16 (CHO-S cells transiently expressing the extracellular domain of HER2-delex (a 16 amino acid deletion mutant of the HER2 receptor; produced as described above) and TC1014-HER2stumpy (CHO-S cells transiently expressing the extracellular stumpy domain of the HER2 receptor; produced as described above) as well as CHO-S wild type cells (negative control cells which do not express HER2) was determined. Samples were added to the cells to allow binding to HER2. Subsequently, binding of HuMab was detected using a fluorescent conjugate (Goat anti-Human IgG-Cy5; Jackson ImmunoResearch). TH1014-Pertuzumab (produced in HEK-293F cells) was used as a positive control and HuMAb®-mouse pooled serum and HuMab-KLH were used as negative controls. The samples were scanned using an Applied Biosystems 8200 Cellular Detection System (8200 CDS) and 'counts x fluorescence' was used as read-out. Samples were stated positive when counts were higher than 50 and counts x fluorescence were at least three times higher than the negative control.

**Example 8 - HuMab hybridoma generation**

HuMAb® mice with sufficient antigen-specific titer development (defined as above) were sacrificed and the spleen and lymph nodes flanking the abdominal aorta and vena cava were collected. Fusion of splenocytes and lymph node cells to a mouse myeloma cell line was done by electrofusion using a CEEF 50 Electrofusion System (Cyto Pulse Sciences, Glen Burnie, MD, USA), essentially according to the manufacturer's instructions. Next, the primary wells were subcloned using the ClonePix system (Genetix, Hampshire, UK). To this end specific primary well hybridoma's were seeded in semisolid medium made from 40% CloneMedia (Genetix, Hampshire, UK) and 60% HyQ 2x complete media (Hyclone, Waltham, USA). The subclones were retested in the antigen-specific binding assay as described in Example 7 and IgG levels were measured using an Octet (Fortebio, Menlo Park, USA) in order to select the most specific and best producing clone per primary well for further expansion. Further expansion and culturing of the resulting HuMab hybridomas were done based upon standard protocols (e.g. as described in Coligan J.E., Bierer, B.E., Margulies, D.H., Shevach, E.M. and Strober, W., eds. Current Protocols in Immunology, John Wiley & Sons, Inc., 2006). Clones derived by this process were designated PC1014.

**Example 9 - Mass Spectrometry of antibodies**

Small aliquots of 0.8 mL antibody containing supernatant from 6-well or Hyperflask stage were purified using PhyTip columns containing Protein G resin (PhyNexus Inc., San Jose, USA) on a Sciclone ALH 3000 workstation (Caliper Lifesciences, Hopkinton, USA). The PhyTip columns were used according to manufacturer's instructions, although buffers were replaced by: Binding Buffer PBS (B.Braun, Medical B.V., Oss, Netherlands) and Elution Buffer 0.1M Glycine-HCl pH 2.7 (Fluka Riedel-de Haën, Buchs, Germany). After purification, samples were neutralized with 2M Tris-HCl, pH 9.0 (Sigma-Aldrich, Zwijndrecht, Netherlands). Alternatively, in some cases larger volumes of culture supernatant were purified using MabSelect SuRe columns (GE Heath Care).

After purification, the samples were placed in a 384-well plate (Waters, 100 μl square well plate, part# 186002631). Samples were deglycosylated overnight at 37°C with N-glycosidase F (Roche cat no 11365177001. DTT (15 mg/mL) was added (1 μL/well) and incubated for 1 h at 37°C. Samples (5 or 6 μL) were desalted on an Acquity UPLC™ (Waters, Milford, USA) with a BEH300 C18, 1.7μm, 2.1x 50 mm column at 60°C. MQ water and LC-MS grade acetonitrile (Biosolve, cat no 01204101, Valkenswaard, The Netherlands) with both 0.1% formic acid (Fluka, cat no 56302, Buchs, Germany), were used as Eluens A and B, respectively. Time-of-flight electrospray ionization mass spectra were recorded on-line on a micrOTOF™ mass spectrometer (Bruker, Bremen, Germany) operating in the positive ion mode. Prior to analysis, a 900-3000 m/z scale was calibrated with ES tuning mix (Agilent Technologies, Santa Clara, USA). Mass spectra were deconvoluted with DataAnalysis™ software v. 3.4 (Bruker) using the Maximal Entropy algorithm searching for molecular weights between 5 and 80 kDa.

After deconvolution, the resulting heavy and light chain masses for all samples were compared in order to find duplicate antibodies. This was sometimes due to the presence of an extra light chain, but in the comparison of the heavy chains, the possible presence of C-terminal lysine variants was also taken into account. This resulted in a list of unique antibodies, *i.e.,* a unique combination of specific heavy and light chains. In case duplicate antibodies were found, one unique antibody was selected based on results from other tests.

**Example 10** - **Sequence analysis of the HER2 antibody variable domains and cloning in expression vectors**

Total RNA of the HER2 HuMabs was prepared from $5 \times 10^6$ hybridoma cells and 5'-RACE-Complementary DNA (cDNA) was prepared from 100 ng total RNA, using the SMART RACE cDNA Amplification kit (Clontech), according to the manufacturer's instructions. VH and VL coding regions were amplified by PCR and cloned directly, in frame, in the pGlf and pKappa expression vectors, by ligation independent cloning (Aslanidis, C. and P.J. de Jong, Nucleic Acids Res 1990;18(20): 6069-74). The appropriate heavy chain and light chain vectors were transiently co-expressed in Freestyle™ 293-F cells using 293fectin. Clones derived by this process were designated TH1014. For each antibody, 16 VL clones and 8 VH clones were sequenced. Clones with predicted heavy and light chain mass in agreement with the mass of the hybridoma derived material of the same antibody (as determined by mass spectrometry) were selected for further study and expression.

The resulting sequences are shown in Figures 1 and 2 and in the Sequence Listing. Selected sequences are also described in more detail below. CDR sequences were defined according to IMGT (Lefranc MP. et al., Nucleic Acids Research, 27, 209-212, 1999 and Brochet X. Nucl. Acids Res. 36, W503-508 (2008)). Table 1, Table 2 and Table 3 give an overview of antibody sequence information or germline sequences, and Table 4 shows consensus sequences.

**Table 1A and 1B:** Heavy chain variable region (VH), light chain variable region (VL) and CDR sequences of HuMabs 169, 050, 084, 025, 091, 129, 127, 159, 098, 153, and 132 (Table 1A) and HuMabs 005, 006, 059, 060, 106, and 111 (Table 1B).

| 1A: | | |
|---|---|---|
| SEQ ID No:1 | VH 169 | QVQLVQSGAEVKKPGASVKVSCKASGYTFTNYGISW VRQAPGQGLEWMGWLSAYSGNTIYAQKLQGRVTMT TDTSTTTAYMELRSLRSDDTAVYYCARDRIVVRPDYF DYWGQGTLVTVSS |
| SEQ ID No:2 | VH 169, CDR1 | GYTFTNYG |
| SEQ ID No:3 | VH 169, CDR2 | LSAYSGNT |
| SEQ ID No:4 | VH 169, CDR3 | ARDRIVVRPDYFDY |
| SEQ ID No:5 | VL 169 | EIVLTQSPATLSLSPGERATLSCRASQSVSSYLAWYQ QKPGQAPRLLIYDASNRATGIPARFSGSGSGTDFTLTI SSLEPEDFAVYYCQQRSNWPRTFGQGTKVEIK |
| SEQ ID No:6 | VL 169, CDR1 | QSVSSY |
| | VL 169, CDR2 | DAS |
| SEQ ID No:7 | VL 169, CDR3 | QQRSNWPRT |

| 1A: | | |
|---|---|---|
| SEQ ID No:8 | VH 050 | EVQLLESGGGLVQPGGSLRLSCAASGFTFSSYAMNW VRQAPGKGLEWVSAISGRGGTTYYADSVKGRFTISR DNSKNTLYLQMSSLRAEDTAVYYCAKARANWDYFDY WGQGTLVTVSS |
| SEQ ID No:9 | VH 050, CDR1 | GFTFSSYA |
| SEQ ID No:10 | VH 050, CDR2 | ISGRGGTT |
| SEQ ID No:11 | VH 050, CDR3 | AKARANWDYFDY |
| SEQ ID No:12 | VL 050 | DIQMTQSPSSVSASVGDRVTITCRASQGISSWLAWY QHKPGKAPKLLIYAASILQSGVPSRFSGSGSGTDFTL TISSLQPEDFATYYCQQANSFPITFGQGTRLEIK |
| SEQ ID No:13 | VL 050, CDR1 | QGISSW |
| | VL 050, CDR2 | AAS |
| SEQ ID No:14 | VL 050, CDR3 | QQANSFPIT |
| SEQ ID No:15 | VH 084 | QVQLVQSGAEVKKPGSSVKVSCKASGGTFRTYAINW VRQAPGQGLEWMGRINTVLGIVNHAQKFQGRVTITA DKSTNTAYMELNSLRSEDTAVYYCAREKGVDYYYGIE VWGQGTTVTVSS |
| SEQ ID No:16 | VH 084, CDR1 | GGTFRTYA |
| SEQ ID No:17 | VH 084, CDR2 | INTVLGIV |
| SEQ ID No:18 | VH 084, CDR3 | AREKGVDYYYGIEV |
| SEQ ID No:19 | VL 084 | DIQMTQSPSSVSASVGDRVTITCRASQGISSWLAWY QHKPGKAPKLLIYVASTLQSGVPSRFSGSGSGTDFTL TISSLQPEDFATYYCQQANSFPLTFGGGTKVEIK |
| SEQ ID No:20 | VL 084, CDR1 | QGISSW |
| | VL 084, CDR2 | VAS |
| SEQ ID No:21 | VL 084, CDR3 | QQANSFPLT |
| SEQ ID No:22 | VH 025 | QVQLQQWGAGLLKPSETLSLTCAVYGGSFSDYYWN WIRQPPGKGLEWIGEIHHSGSTNYNPSLKSRVTISVD TSKNQFSLKLSSVTAADTAVYYCARGYYDSGVYYFDY WAQGTLVTVSS |
| SEQ ID No:23 | VH 025, CDR1 | GGSFSDYY |
| SEQ ID No:24 | VH 025, CDR2 | IHHSGST |
| SEQ ID No:25 | VH 025, CDR3 | ARGYYDSGVYYFDY |
| SEQ ID No:26 | VL 025 | DIQMTQSPSSLSASVGDRVTITCRASQGISRWLAWY QQKPEKAPKSLIYAASSLRSGVPSRFSGSGSGTDFTL TISSLQPEDFATYYCQQYNSYPITFGQGTRLEIK |
| SEQ ID No:27 | VL 025, CDR1 | QGISRW |
| | VL 025, CDR2 | AAS |
| SEQ ID No:28 | VL 025, CDR3 | QQYNSYPIT |

(continued)

| 1A: | | |
|---|---|---|
| SEQ ID No:29 | VH 091 | QVQLQQWGAGLLKPSETLSLTCAVSGGSFSGYYWT WIRQPPGKGLEWIGEIYHSGDTNYNPSLKSRVTISVD TSKNQFSLKLYSVTAADTAVYYCARLYFGSGIYYLDY WGQGTLVTVSS |
| SEQ ID No:30 | VH 091, CDR1 | GGSFSGYY |
| SEQ ID No:163 | VH 091, CDR2 | IYHSGDT |
| SEQ ID No:31 | VH 091, CDR3 | ARLYFGSGIYYLDY |
| SEQ ID No:32 | VL 091 | DIQMTQSPSSLSASVGDRVTITCRASQGISSWLVWY QQKPEKAPKSLIYAASSLQSGVPSRFSGSGSGTDFTL TISSLQPEDFATYYCQQYNSFPPTFGQGTKVEIK |
| SEQ ID No:33 | VL 091, CDR1 | QGISSW |
| | VL 091, CDR2 | AAS |
| SEQ ID No:34 | VL 091, CDR3 | QQYNSFPPT |
| SEQ ID No:35 | VH 129 | QVQLVESGGGVVQPGRSLRLSCAASGFTFSTFAIHW VRQAPGKGLEWVAVISYDGGHKFYADSVKGRFTISR DNSKNTLYLQMNSLRAEDTAMYYCARGLGVWGAFD YWGQGTLVTVSS |
| SEQ ID No:36 | VH 129, CDR1 | GFTFSTFA |
| SEQ ID No:37 | VH 129, CDR2 | ISYDGGHK |
| SEQ ID No:38 | VH 129, CDR3 | ARGLGVWGAFDY |
| SEQ ID No:39 | VL 129 | EIVLTQSPATLSLSPGERATLSCRASQSVSSYLAWYQ QKPGQAPRLLIYDASNRATGIPARFSGSGSGTDFTLTI SSLEPEDFAVYYCQQRSNWWTFGQGTKVEIK |
| SEQ ID No:40 | VL 129, CDR1 | QSVSSY |
| | VL 129, CDR2 | DAS |
| SEQ ID No:41 | VL 129, CDR3 | QQRSNWWT |
| SEQ ID No:42 | VH 127 | EVQLVQSGAEVKKPGESLTISCKGSGYSFSIYWIGW VRQMPGKGLEWMGIIFPGDSDIRYSPSFQGQVTISA DKSISTAYLQWSSLKASDTAMYYCARQPGDWSPRH WYFDLWGRGTLVTVSS |
| SEQ ID No:43 | VH 127, CDR1 | GYSFSIYW |
| SEQ ID No:44 | VH 127, CDR2 | IFPGDSDI |
| SEQ ID No:45 | VH 127, CDR3 | ARQPGDWSPRHWYFDL |
| SEQ ID No:46 | VL 127 | VIWMTQSPSLLSASTGDRVTISCRMSQGISSYLAWY QQKPGKAPELLIYAASTLQSGVPSRFSGSGSGTDFTL TISYLQSEDFATYYCQQYYSFPLTFGGGTKVEIK |
| SEQ ID No:47 | VL 127, CDR1 | QGISSY |
| | VL 127, CDR2 | AAS |
| SEQ ID No:48 | VL 127, CDR3 | QQYYSFPLT |

(continued)

| 1A: | | |
|---|---|---|
| SEQ ID No:49 | VH 159 | EVQLVQSGAEVKKPGESLKISCKGSGYNFTSYWIGW VRQMPGKGLEWMGIIYPGDSDTRYSPSFQGQVTISA DKSISTAYLQWSSLKASDTAMYYCARWGTYYDILTG YFNWFDPWGQGTLVTVSS |
| SEQ ID No:50 | VH 159, CDR1 | GYNFTSYW |
| SEQ ID No:51 | VH 159, CDR2 | IYPGDSDT |
| SEQ ID No:52 | VH 159, CDR3 | ARWGTYYDILTGYFN |
| SEQ ID No:53 | VL 159 | DIQMTQSPSSLSASVGDRVTITCRASQGISSWLAWY QQKPEKAPKSLIYAASSLQSGVPSRFSGSGSGTDFTL TISSLQPEDFATYYCQQYYIYPWTFGQGTKVEIK |
| SEQ ID No:54 | VL 159, CDR1 | QGISSW |
| | VL 159, CDR2 | AAS |
| SEQ ID No:55 | VL 159, CDR3 | QQYYIYPWT |
| SEQ ID No:56 | VH 098 | EVQLLESGGGLVQPGGSLRLSCAASGFTFSNYGMSW VRQAPGKGLEWVSAISGSAYSTYYADSVKGRFTISR DNSKNTLWLQMNSLRAEDTAVYYCAKAHYHGSGSYY TLFDYWGQGTLVTVSS |
| SEQ ID No:57 | VH 098, CDR1 | GFTFSNYG |
| SEQ ID No:58 | VH 098, CDR2 | ISGSAYST |
| SEQ ID No:59 | VH 098, CDR3 | AKAHYHGSGSYYTLFDY |
| SEQ ID No:60 | VL 098 | DIQMTQSPSSLSASVGDRVTITCRASQGISSWLAWY QQKPEKAPKSLIYAASSLQSGVPSRFSGSGSGTDFTL TISSLQPEDFATYYCQQYNSYPYTFGQGTKLEIK |
| SEQ ID No:61 | VL 098, CDR1 | QGISSW |
| | VL 098, CDR2 | AAS |
| SEQ ID No:62 | VL 098, CDR3 | QQYNSYPYT |
| SEQ ID No:63 | VH 153 | QVQLVESGGGVVQPGRSLRLSCAASGFTFSDYVIHW VRQAPGKGLEWVTVISYDGSNKYYADSVKGRFTISR DNSKNTLYLQMNSLSAEDTAMYYCARGGITGTTGVF DYWGQGTLVTVSS |
| SEQ ID No:64 | VH 153, CDR1 | GFTFSDYV |
| SEQ ID No:65 | VH 153, CDR2 | ISYDGSNK |
| SEQ ID No:66 | VH 153, CDR3 | ARGGITGTTGVFDY |
| SEQ ID No:67 | VL 153 | DIQMTQSPSSLSASVGDRVTITCRASQGISSWLAWY QQKPEKAPKSLIYDASSLQSGVPSRFSGSGYGTDFSL TISSLQPEDFAIYYCQQYKSYPITFGQGTRLEIK |
| SEQ ID No:68 | VL 153, CDR1 | QGISSW |
| | VL 153, CDR2 | DAS |
| SEQ ID No:69 | VL 153, CDR3 | QQYKSYPIT |

(continued)

| 1A: | | |
|---|---|---|
| SEQ ID No:70 | VH 132 | QVQLVQSGAEVKKPGASVKVSCKASGYTFTSYGISW VRQAPGQGLEWMGWISAYNGNSNYVQKFQGRVTM TTDTTTSTAYMELRSLTSDDTAVYYCAREYSYDSGTY FYYGMDVWGQGTTVTVSS |
| SEQ ID No:71 | VH 132, CDR1 | GYTFTSYG |
| SEQ ID No:72 | VH 132, CDR2 | ISAYNGNS |
| SEQ ID No:73 | VH 132, CDR3 | AREYSYDSGTYFYYGMDV |
| SEQ ID No:74 | VL 132 | EIVLTQSPATLSLSPGERATLSCRASQSVSSYLAWYQ QKPGQAPRLLIYDASNRATGIPARFSGSGSGTDFTLTI SSLEPEDFAVYYCQQRSNWPMYTFGQGTKLEIK |
| SEQ ID No:75 | VL 132, CDR1 | QSVSSY |
| | VL 132, CDR2 | DAS |
| SEQ ID No:76 | VL 132, CDR3 | QQRSNWPMYT |
| 1B) | | |
| SEQ ID No: 165 | VH 005 | EVQLVQSGAEVKKPGESLKISCKASGYSFHFYWIGW VRQMPGKGLEWMGSIYPGDSDTRYRPSFQGQVTISA DKSISTAYLQWTSLKASDTAIYYCARQRGDYYYFYGM DVWGQGTTVTVSS |
| SEQ ID No: 166 | VH 005, CDR1 | GYSFHFYW |
| SEQ ID No: 167 | VH 005, CDR2 | IYPGDSDT |
| SEQ ID No: 168 | VH 005, CDR3 | ARQRGDYYYFYGMDV |
| SEQ ID No: 169 | VL 005 | EIVLTQSPGTLSLSPGERATLSCRASQSVSSSYLAWY QQKPGQVPRLLIYGASSRATGIPDRFSGSGSGTDFTL TISRLEPEDFAVYYCQQYGSSLTFGGGTKVEIK |
| SEQ ID No: 170 | VL 005, CDR1 | QSVSSSY |
| | VL 005, CDR2 | GAS |
| SEQ ID No: 171 | VL 005, CDR3 | QQYGSSLT |
| SEQ ID No: 172 | VH 006 | EVQLLESGGGLVQPGGSLRLSCAASGFTFSNYALIWV RQAPGKGLEWVSIIRGGAGSTYYADSVKGRFTISRD NSKNTLYLQMNSLRAEDTAVYYCAKARIWGPLFDYW GQGTLVTVSS |
| SEQ ID No: 173 | VH 006, CDR1 | GFTFSNYA |
| SEQ ID No: 174 | VH 006, CDR2 | IRGGAGST |
| SEQ ID No: 175 | VH 006, CDR3 | AKARIWGPLFDY |
| SEQ ID No: 176 | VL 006 | EIVLTQSPATLSLSPGERATLSCRASQSVSSYLAWYQ QKPGQAPRLLIYDASNRATGIPARFSGSGSGTDFTLTI SSLEPEDFAVYYCQQRSNWPPLTFGGGTKVEIK |
| SEQ ID No: 177 | VL 006, CDR1 | QSVSSY |
| | VL 006, CDR2 | DAS |
| SEQ ID No: 178 | VL 006, CDR3 | QQRSNWPPLT |

(continued)

| 1B) | | |
|---|---|---|
| SEQ ID No: 179 | VH 059 | QVQLVQSGAEVKKPGASVRVPCKASGYTFTRYGISW VRQAPGQGLEWMGWISAYNGKTYYAQKLQGRVTMT TDTSTSTAYMELRSLRSDDTAVYYCARSPLLWFEELY FDYWGQGTLVTVSS |
| SEQ ID No: 180 | VH 059, CDR1 | GYTFTRYG |
| SEQ ID No: 181 | VH 059, CDR2 | ISAYNGKT |
| SEQ ID No: 182 | VH 059, CDR3 | ARSPLLWFEELYFDY |
| SEQ ID No: 183 | VL 059 | EIVLTQSPGTLSLSPGERATLSCRASQSVSSTYLAWY QQKPGQAPRLLIYGASSRATGIPDRFSGSGSGTDFTL TISRLEPEDFAVYYCQQYGTSLFTFGPGTKVDIK |
| SEQ ID No: 184 | VL 059, CDR1 | QSVSSTY |
| | VL 059, CDR2 | GAS |
| SEQ ID No: 185 | VL 059, CDR3 | QQYGTSLFT |
| SEQ ID No: 186 | VH 060 | EVQLVQSGAEVKKPGESLKISCKGSGYRFTSYWIGW VRQMPGKGLEWMGSIYPGDSYTRNSPSFQGQVTISA DKSIATAYLQWNSLKASDTAMYYCARHAGDFYYFDG LDVWGQGTTVTVSS |
| SEQ ID No: 187 | VH 060, CDR1 | GYRFTTSYW |
| SEQ ID No: 188 | VH 060, CDR2 | IYPGDSYT |
| SEQ ID No: 189 | VH 060, CDR3 | ARHAGDFYYFDGLDV |
| SEQ ID No: 190 | VL 060 | EIVLTQSPGTLSLSPGERATLSCRASQSVSSSYLAWY QQKPGQAPRLLIYGASSRATGIPDRFSGSGSGTDFTL TISRLEPEDFAVYYCQQYGSSPPITFGQGTRLEIK |
| SEQ ID No: 191 | VL 060, CDR1 | QSVSSSY |
| | VL 060, CDR2 | GAS |
| SEQ ID No: 192 | VL 060, CDR3 | QQYGSSPPIT |
| SEQ ID No: 193 | VH 106 | EVQLVQSGAEVKKPGESLKISCKGSGYSFTRYWIGW VRQMPGKGLEWMGIIYPGDSDTRYSPSFQGQVTISA DKSISTAYLQWSSLKASDTAMYYCARLTGDRGFDYY SGMDVWGQGTTVTVSS |
| SEQ ID No: 194 | VH 106, CDR1 | GYSFTRYW |
| SEQ ID No: 195 | VH 106, CDR2 | IYPGDSDT |
| SEQ ID No: 196 | VH 106, CDR3 | ARLTGDRGFDYYSGMDV |
| SEQ ID No: 197 | VL 106 | EIVLTQSPGTLSLSPGERATLSCRASQSVSSSYLAWY QQKPGQAPRLLIYGASSRATGIPDRFSGSGSGTDFTL TISRLEPEDFAVYYCQQYGSSFTFGPGTKVDIK |
| SEQ ID No: 198 | VL 106, CDR1 | QSVSSSY |
| | VL 106, CDR2 | GAS |
| SEQ ID No: 199 | VL 106, CDR3 | QQYGSSFT |

(continued)

| 1B) | | |
|---|---|---|
| SEQ ID No: 200 | VH 111 | QVQLVQSGAEVKKPGSSVKVSCKASGGTFSSYGISW VRQAPGPGLEWMGRIIPILGIANYAQKFQGRVTITAD KSTNTAYMELSSLRSEDTAVYYCARDQEYSSNWYYW GQGTLVTVSS |
| SEQ ID No: 201 | VH 111, CDR1 | GGTFSSYG |
| SEQ ID No: 202 | VH 111, CDR2 | IIPILGIA |
| SEQ ID No: 203 | VH 111, CDR3 | ARDQEYSSNWYY |
| SEQ ID No: 204 | VL 111 | EIVLTQSPGTLSLSPGERATLSCRASQSVRSSYLAWY QQKPGQAPRLLIYGASSRATGIPDRFSGSGSGTDFTL TISRLEPEDFAVYYCQLYGSSPTFGPGTKVDIK |
| SEQ ID No: 205 | VL 111, CDR1 | QSVRSSY |
| | VL 111, CDR2 | GAS |
| SEQ ID No: 206 | VL 111, CDR3 | QLYGSSPT |

**Table 2:** Mouse origin and heavy and light chain sequence homologies of selected HuMabs.

| HuMab: | Mouse: | Strain: | Germline VH: | Germline VL: |
|---|---|---|---|---|
| 169 | 361494 | HCo20 | IgHV1-18-01 | IgKV3-11-01 |
| 050 | 350633 | HCo12 | IgHV3-23-01 | IgKV1-12-01 |
| 084 | 350615 | HCo12-BalbC | IgHV1-69-04 | IgKV1-12-01 |
| 025 | 350631 | HCo12 | IgHV4-34-01 | IgKV1D-16-01 |
| 091 | 350630 | HCo12 | IgHV4-34-01 | IgKV1D-16-01 |
| 129 | 359783 | HCo12-BalbC | IgHV3-30-3-01 | IgKV3-11-01 |
| 127 | 359783 | HCo12-BalbC | IgHV5-51-01 | IgKV1D-8-01 |
| 159 | 363503 | HCo12 | IgHV5-51-01 | IgKV1D-16-01 |
| 098 | 350659 | HCo17 | IgHV3-23-01 | IgKV1D-16-01 |
| 153 | 359785 | HCo12-BalbC | IgHV3-30-3-01 | IgKV1D-16-01 |
| 132 | 361487 | HCo20 | IgHV1-18-01 | IgKV3-11-01 |
| 005 | 350611 | HCo12-BalbC | IgHV5-51-1 | IgKV3-20-01 |
| 006 | 350611 | HCo12-BalbC | IgHV3-23-1 | IgKV3-11-01 |
| 059 | 350654 | HCo17 | IgHV1-18-1 | IgKV3-20-01 |
| 060 | 350654 | HCo17 | IgHV5-51-1 | IgKV3-20-01 |
| 106 | 350660 | HCo17 | IgHV5-51-1 | IgKV3-20-01 |
| 111 | 350660 | HCo17 | IgHV1-69-4 | IgKV3-20-01 |

**Table 3A and 3B:** Heavy chain variable region (VH), light chain variable region (VL) sequences of HuMabs 049, 051, 055, 123, 161, 124, 001, 143, 019, 021, 027, 032, 035, 036, 054, 094 (3A) and HuMabs 041, 150, 067, 072, 163, 093, and 044 (3B). The respective CDRs correspond to those underlined in Figures 1 and 2, for VH and VL sequences, respectively.

| 3A: | | |
|---|---|---|
| SEQ ID No: 77 | VH 049 | EVQLLESGGDLVQPGGSLRLSCAASGFTFSSYAMSWVRQAPG KGLEWVSAISGRGGTTYYADSVKGRFTISRDNSKSTLCLQMNS LRAEDTAVYYCAKARANWDYFDYWGQGTLVTVSS |
| SEQ ID No: 78 | VL 049 | DIQMTQSPSSVSASVGDRVTITCRASQGISSWLAWYQHKPGK APKLLIYAASILQSGVPSRFSGSGSGTDFTLTISSLRPEDFATYY CQQANSFPITFGQGTRLEIK |
| SEQ ID No: 79 | VH 051 | EVQLLESGGDLVQPGGSLRLSCAASGFTFSSYAMSWVRQAPG KGLEWVSAISGRGGTTYYADSVKGRFTISRDNSKSTLCLQMNS LRAEDTAVYYCAKARANWDYFDYWGQGTLVTVSS |
| SEQ ID No: 80 | VL 051 | DIQMTQSPSSVSASVGDRVTITCRASQGISSWLAWYQHKPGK APKLLIYAASILQSGVPSRFSGSGSGTDFTLTISSLRPEDFATYY CQQANSFPITFGQGTRLEIK |
| SEQ ID No: 81 | VH 055 | EVQLLESGGGLVQPGGSLRLSCAASGFTFSSYAMNWVRQAPG KGLEWVSAISGRGGTTYYADSVKGRFTISRDNSKSTLCLQMNS LRAEDTAVYYCAKARANWDYFDYWGQGTLVTVSS |
| SEQ ID No: 82 | VL 055 | DIQMTQSPSSVSASVGDRVTITCRASQGISSWLAWYQHKPGK APKLLIYAASILQSGVPSRFSGSGSGTDFTLTISSLRPEDFATYY CQQANSFPITFGQGTRLEIK |
| SEQ ID No: 83 | VH 123 | QVQLVQSGAEVKKPGASVKVSCKAAGYTFTNYGISWVRQAPG QALEWMGWITTYSSNTIYAQKLGRVTMTTDTSTSTAYMELRS LRSDDTAVYYCARDRVVVRPDYFDYWGQGTLVTVSS |
| SEQ ID No: 84 | VL 123 | EIVLTQSPATLSLSPGERATLSCRASQSVSSYLAWYQQKPGQAP RLLIYDTSNRATGIPARFSGSGSGTDFTLTISSLEPEDFAVYYCQ QRSHWPRTFGQGTKVEIK |
| SEQ ID No: 85 | VH 161 | QVQLVQSGAEVKKPGASVKVSCKASGYTFTNYGISWVRQAPG QGLEWMGWLSAYSGNTIYAQKLGRVTMTTDTSTTTAYMELR SLRSDDTAVYYCARDRIVVRPDYFDYWGQGTLVTVSS |
| SEQ ID No: 86 | VL 161 | EIVLTQSPATLSLSPGERATLSCRASQSVSSYLAWYQQKPGQAP RLLIYDASNRATGIPARFSGSGSGTDFTLTISSLEPEDFAVYYCQ QRSNWPRTFGQGTKVEIK |
| SEQ ID No: 87 | VH 124 | QVQLVQSGAEVKKPGASVKVSCKAAGYTFTNYGISWVRQAPG QGLEWMGWIITYNGNTIYAQRFQDRVTMTTDTSTSTAYMELRS LRSDDTAVYYCARDRIIVRPDYFDYWGQGTLVTVSS |
| SEQ ID No: 88 | VL 124 | EIVLTQSPATLSLSPGERATLSCRASQSVSSYLAWYQQKPGQAP RLLIYDASNRATGIPARFSGSGSGTDFTLTISSLEPEDFAVYYCQ QRSNWPRTFGQGTKVEIK |
| SEQ ID No: 89 | VH 001 | QVQLQQWGAGLLKPSETLSLTCAVYGGSFSGYYWNWIRQPPG KGLEWIGEINHSGSTNYNPSLKSRVTISVDTSKNQFSLKLSSVT AADTAVYYCARGNYGSGYYYFDLWGRGTQVTVSS |

(continued)

| 3A: | | |
|---|---|---|
| SEQ ID No: 90 | VL 001 | DIQMTQSPSSLSASVGDRVTITCRASQGISSWLAWYQQKPEK APKSLIFAASSLQSGVPSRFSGSGSGTDFTLTISSLQPEDFATYY CQQYISFPITFGQGTRLEIK |
| SEQ ID No: 91 | VH 143 | QVQLQQWGAGLLKPSETLSLTCAVYGGSFSGYYWNWIRQPPG KGLEWIGEIHHSGSANYNPSLMSRVTISVDTSKNQFSLQLSSV TAADTAVYYCARGYYGSGYYYFDYWGQGTLVTVSS |
| SEQ ID No: 92 | VL 143 | DIQMTQSPSSLSASVGDRVTITCRASQGISSWLAWYQQKPEK APKSLIYAASRLQSGVPSRFSGSGSGTDFTLTISSLQPEDFATYY CQQYNSYPITFGQGTRLEIK |
| SEQ ID No: 93 | VH 019 | QVQLQQWGAGLLKPSETLSLTCAVYGGSFSDYYWNWIRQPPG KGLEWIGEIHHVGSTNYNPSLKSRVTISVDTSKSQFSLKLSSVT AADTAVYYCARGYYDSGVYYFDYWAQGTLVTVSS |
| SEQ ID No: 94 | VL 019 | DIQMTQSPSSLSASVGDRVTITCRASQGISRWLAWYQQKPEK APKSLIYAASSLRSGVPSRFSGSGSGTDFTLTISSLQPEDFATYY CQQYNSYPITFGQGTRLEIK |
| SEQ ID No: 95 | VH 021 | QVQLQQWGAGLLKPSETLSLTCAVYGGSFSDYYWNWIRQPPG KGLEWIGEIHHSGSTNYNPSLKSRVTISVDTSKNQFSLKLSSVT AADTAVYYCARGYYASGVYYFDYWGQGTLVTVSS |
| SEQ ID No: 96 | VL 021 | DIQMTQSPSSLSASVGDRVTITCRASQGISSWLAWYQQKPEK APKSLIYAASSLQSGVPSRFSGSGSGTDFTLTISSLQPEDFATYY CQQYNSYPITFGQGTRLEIK |
| SEQ ID No: 97 | VH 027 | QVQLQQWGAGLLKPSETLSLTCAVYGGSFSDYFWNWIRQPPG KGLEWIGEIHHSGSTNYNPSLKSRVTISVDTSKNQFSLNLSSVT AADTAVYYCARGLIGSGYYYFDYWDQGTLVTVSS |
| SEQ ID No: 98 | VL 027 | DIQMTQSPSSLSASVGDRVTITCRASQGISSWLAWYQQKPEK APKSLIYAASSLQSGVPSRFSGSGSGTDFTLTISSLQPEDFATYY CQQYNSYPITFGQGTRLEIK |
| SEQ ID No: 99 | VH 032 | QVQLQQWGAGLLKPSETLSLTCAVYGGSFSGYYWSWIRQPPG KGLEWIGEINHSGDTNYNPSLTSRVTISVDTSKNQFSLKLSSVT AADTAVYYCARLFYGSGIYYFDYWGQGTLVTVSS |
| SEQ ID No: 100 | VL 032 | DIQMTQSPSSLSASVGDRVTITCRASQGISSWLAWYQQKPEK APKSLIYATFRLQSGVPSRFSGSGSGTDFTLTISSLQPEDFATYY CQQYNSFPPTFGQGTKVEIK |
| SEQ ID No: 101 | VH 035 | QVQLQQWGAGLLKPSETLSLTCAIYGGSFSGYYWSWIRQPPG KGLEWIGEINHSGDTNYNPSLTSRVTISVDTSKNQFSLKLSSVT AADTAVYYCARLFYGSGIYYFDYWGQGTLVTVSS |
| SEQ ID No: 102 | VL 035 | DIQMTQSPSSLSASVGDRVTITCRASQGISSWLAWYQQKPEK APKSLIYATFRLQSGVPSRFSGSGSGTDFTLTISSLQPEDFATYY CQQYNSFPPTFGQGTKVEIK |
| SEQ ID No: 103 | VH 036 | QVQLQQWGAGLLKPSETLSLTCAVYGGSFSDYYWSWIRQPPG KGLEWIGEINHSGSTNYNPSLKSRVTISVDTSKNQFSLKLSSVT AADTAVYYCARLYYGSGTYYFDYWGQGTLVTVSS |

(continued)

| 3A: | | |
|---|---|---|
| SEQ ID No: 104 | VL 036 | DIQMTQSPSSLSASVGDRVTITCRASQGISSWLTWYQQKPEKA PKSLIYAASRLQSGVPSRFSGSGSGTDFTLTISSLQPEDFATYYC QQYNSFPPTFGQGTKVEIK |
| SEQ ID No: 105 | VH 054 | QVQLQQWGAGLLKPSETLSLTCAVYGGSFSGYYWSWIRQPPG KGLEWIGEIHHSGSTNYNPSLKSRVTISVDTSKNQFSLKLSSVT AADTAVYYCARLWYGSGSYYFDYWGQGTLVTVSS |
| SEQ ID No: 106 | VL 054 | DIQMTQSPSSLSASVGDRVTITCRASQGISSWLAWYQQKPEK APKSLIYAASSLQSGVPSRFSGSGSGTDFTLTISSLQPEDFATYY CQQYNSFPPTFGGGTKVEIK |
| SEQ ID No: 107 | VH 094 | QVQLQQWGAGLLKPSETLSLTCAVSGGSFSGYYWTWIRQPPG KGLEWIGEIYHSGDTNYNPSLKSRVTISVDTSKNQFSLKLYSVT AADTAVYYCARLYFGSGIYYLDYWGQGTLVTVSS |
| SEQ ID No: 108 | VL 094 | DIQMTQSPSSLSASVGDRVTITCRASQGISSWLVWYQQKPEK APKSLIYAASSLQSGVPSRFSGSGSGTDFTLTISSLQPEDFATYY CQQYNSFPPTFGQGTKVEIK |
| SEQ ID No: 109 | VH 105 | EVQLLESGGGLVQPGGSLRLSCAASGFTFSNYGMSWVRQAPG KGLEWVSAISGSAYSTYYADSVKGRFTISRDNSKNTLWLQMNS LRAEDTAVYYCAKAHYHGSGSYYTLFDYWGQGTLVTVSS |
| SEQ ID No: 110 | VL 105 | DIQMTQSPSSLSASVGDRVTITCRASQGISSWLAWYQQKPEK APKSLIYAASSLQSGVPSRFSGSGSGTDFTLTISSLQPEDFATYY CQQYNSYPYTFGQGTKLEIK |
| SEQ ID No: 111 | VH 100 | EVQLLESGGGLVQPGGSLRLSCAASGFTFNNYGMNWVRQAPG KGLEWVSAISGTGYSTYYADSVKGRFTISRDNSKNTLYLQMNS LRAEDTAVYYCAKAHYFGSGSYYTLFDYWGQGTLVTVSS |
| SEQ ID No: 112 | VL 100 | DIQMTQSPSSLSASVGDRVTITCRASQGISSWLAWYQQKPEK APKSLIYAASSLQSGVPSRFSGSGSGTDFTLTISSLQPEDFATYY CQQYNSYPYTFGQGTKLEIK |
| SEQ ID No: 113 | VH 125 | EVQLLESGGGLVQPGGSLRLSCAASGFTFTDYAMNWVRQAPG KGLEWVSTISGSGYATYYADSVKGRFTISRDNSKTTLYLQMNS LRAEDTAVYYCAKGHTLGSGSYYTLFDYWGQGTLVTVSS |
| SEQ ID No: 114 | VL 125 | DIQMTQSPSSLSASVGDRVTITCRASQGINSWLAWYQQKPEK APKSLIYAASSLQSGVPSRFSGSGSGTDFTLTISSLQPEDFATYY CQQYNSYPYTFGQGTKLEIK |
| SEQ ID No: 115 | VH 162 | EVQLWESGGGSVQPGGSLRLSCAASGFTFSSYGMSWVRQAP GKGLEWVSGISGSGYSTYYADSVKGRFTISRDNSKNTLYLQMN SLRAEDTAVYYCAKGYYHGSGSYYTSFDYWGQGTLVTVSS |
| SEQ ID No: 116 | VL 162 | DIQMTQSPSSLSASVGDRVTITCRASQGISSWLAWYQQKPEK APKSLIYAASSLQSGVPSRFSGSGSGTDFTLTISSLQPEDFATYY CQQYNSYPLTFGGGTKVEIK |
| SEQ ID No: 117 | VH 033 | QVQLVESGGGVVQTGRSLRLSCAASGFTFSSHAMHWVRQAPG KGLEWVAAISYDGSNKYYADSVKGRFTISRDNSKNTLYLQMNS LRAEDTAVYYCARGDYISSSGVFDYWGQGTLVTVSS |

(continued)

| 3A: | | |
|---|---|---|
| SEQ ID No: 118 | VL 033 | DIQMTQSPSSLSASVGDRVTITCRASQGISSWLAWYQQKPEK APKSLIYAASSLQSGVPSRFSGSGSGTDFTLTISSLQPEDFATYY CQQYNSYPITFGQGTRLEIK |
| SEQ ID No: 119 | VH 160 | QVQLVESGGGVVQPGRSLRLSCAASGFTFSSHAMHWVRQAPG KGLEWVAAISYDGSNKYYADSVKGRFTISRDNSKNTMYLQMN SLRAEDTAMCYCARGSITGSTGVFDYWGQGTLVTVSS |
| SEQ ID No: 120 | VL 160 | DIQMTQSPSSLSASVGDRVTITCRASQDISSWLAWYQQKPEK APKSLIYAASSLQSGVPSRFSGSGSGTDFTLTISSLQPEDFATYY CQQYNSYPITFGQGTRLEIK |
| SEQ ID No: 121 | VH 166 | QVQLVESGGGVVQPGRSLRLSCAASGFTFSSYAMHWVRQAPG KGLEWVAVISYDGSNEYYADSVKGRFTISRDNSKNTLYLQMNS LRAEDTAVYYCARGSIIGSTGVFDYWGQGTLVTVSS |
| SEQ ID No: 122 | VL 166 | DIQMTQSPSSLSASVGDRVTITCRASQGISNWLAWYQQKPEK APKSLIYDASSLQSGVPSRFSGSGSGTDFTLTISSLQPEDFATYY CQQYNSYPITFGQGTRLEIK |
| SEQ ID No: 123 | VH 152 | QVQVVESGGGVVQPGRSLRLSCAASGFTFSSYAMHWVRQAPG KGLEWVAVISYDGSYKYYADSVKGRFTISRDNSKNTLYLQMNS LRAEDTAVYYCARGSITGSTGVFDYWGQGTLVTVSS |
| SEQ ID No: 124 | VL 152 | DIQMTQSPSSLSASVGDRVTITCRASQGINSWLAWYQQKPEK APKSLIYDASSLQSGVPSRFSGSGSGTDFTLTISSLQPENFATYY CQQYNSYPITFGQGTRLEIK |
| SEQ ID No: 125 | VH 167 | QVQLVESGGGVVQPGRSLRLSCAASGFTFSSYAIHWVRQAPG KGLEWVAVISYDGSNKYYADSVKGRFTISRDNSKNTLYLQMNS LRAEDTAVYYCARGSITGSTGVFDYWGQGTLVTVSS |
| SEQ ID No: 126 | VL 167 | DIQMTQSPSSLSASVGDRVTITCRASQGISNWLAWYQQKPEK APKSLIYDASSLQSGVPSRFSGSGSGTDFTLTISSLQPEDFATYY CQQYNSYPITFGQGTRLEIK |
| 3B: | | |
| SEQ ID No: 207 | VH 041 | EVQLVQSGAEVKKPGESLKISCKGSGYSFTSYWIGWVRQMPG KGLEWMGSIYPGDSHTRYRPSFQGQVTISADKSISTAYLQWSS LKASDTAMYYCARQKGDFYYFFGLDVWGQGTAITVSS |
| SEQ ID No: 208 | VL 041 | EIVLTQSPGTLSLSPGERATLSCRASQSVSSSYLAWYQQKPGQ APRLLIYGASSRATGIPDRFSGSGSGTDFTLTISRLEPEDFAVYY CQQYGSSLTFGGGTKVEIK |
| SEQ ID No: 209 | VH 150 | EVQLVQSGAEVKKPGESLKISCKGSGYSFTSYWIGWVRQMPG KGLEWMGSIYPGDSHTRYRPSFQGQVTISADKSISTAYLQWSS LKASDTAMYYCARQAGDYYYYNGMDVWGQGTTVTVSS |
| SEQ ID No: 201 | VL 150 | EIVLTQSPGTLSLSPGERATLSCRASQSVSSSYLTWYQQKPGQ APRLLIYGASSRATGIPDRFSGSGSGTDFTLTISRLEPEDFAVYY CQQYGSSLTFGGGTKVEIK |

(continued)

| 3B: | | |
|---|---|---|
| SEQ ID No: 211 | VH 067 | EVQLVQSGAEVKKPGESLKISCKGSGYSFTSYWIGWVRQMPG KGLEWMGIIYPGDSDTRYSPSFQGQVTISVDKSISTAYLQWSS LKASDTAMYYCARQKGDYYYHYGLDVWGQGTTVTVSS |
| SEQ ID No: 212 | VL 067 | EIVLTQSPGTLSLSPGERATLSCRASQSVSSSYLAWYQQKPGQ APRLLIYGASSRATGIPDRFSGSGSGTDFTLTISRLEPEDFAVYY CQQYGSSPRLTFGGGTKVEIK |
| SEQ ID No: 213 | VH 072 | EVQLVQSGAEVKKPGESLKISCKGSGYSFTSYWIGWVRQMPG KGLEWMGIIYPGDSDTRYSPSFQGQVTISADKSISTAYLQWSS LKASDTAMYYCARQKGDYYYFNGLDVWGQGTTVTVSS |
| SEQ ID No: 214 | VL 072 | EIVLTQSPGTLSLSPGERATLSCRASQSVSSSYLAWYQQKPGQ APRLLIYGASSRATGIPDRFSGSGSGTDFTLTISRLEPEDFAVYY CQQYGSSPRLTFGGGTKVEIK |
| SEQ ID No: 215 | VH 163 | EVQLVQSGAEVKKPGESLKISCQGSGYRFISYWIGWVRQMPG KGLEWMGRIYPGDSDTRYSPSFQGQVTISVDKSISTAYLQWSS LKASDTAMYYCARQRGDYYYFNGLDVWGQGTTVTVSS |
| SEQ ID No: 216 | VL 163 | EIVLTQSPGTLSLSPGERATLSCRASQSVSSSYLAWYQQKPGQ APRLLIYGASSRATGIPDRFSGSGSGTDFTLTISRLEPEDFAVYY CQQYGSSLTFGGGTKVEIK |
| SEQ ID No: 217 | VH 093 | EVQLVQSGAEVKKPGESLKISCKGSGYSFTSYWIGWVRQMPG KGLEWMGRIYPGDSDTRYSPSFQGQVTISADKSITTAYLQWSS LRASDTAMYYCARQRGDYYYFFGLDIWGQGTTVTVSL |
| SEQ ID No: 218 | VL 093 | EIVLTQSPGTLSLSPGERATLSCRASQSVSSSYLAWYQQKPGQ APRLLIYGASSRATGIPDRFSGSGSGTDFTLTISRLEPEDFAVYY CQQYGSSLTFGGGTKVEIK |
| SEQ ID No: 219 | VH 044 | EVQLVQSGAEVKKPGESLKISCKGSGYRFSSYWIGWVRQMPG KGLEWMGSIFPGDSDTRYSPSFQGQVTISADKSITTAYLQWSS LKASDTAMYYCARQAGDYYYYNGMDVWGQGTTVTVSS |
| SEQ ID No: 220 | VL 044 | EIVLTQSPGTLSLSPGERATLSCRASQSVSSSYLAWYQQKPGQ APRLLIYGASSRATGIPDRFSGSGSGTDFTLTISRLEPEDFAVYY CQQYGSSLTFGGGTKVEIK |

**Table 4:** Consensus CDRs based on sequence alignments shown in Figures 1 and 2.

| SEQ ID No: 9 050-049-051-055 | IgHV3-23-1 | VH CDR1 | GFTFSSYA | |
|---|---|---|---|---|
| SEQ ID No: 127 050-049-051-055 | IgHV3-23-1 | VH CDR2 | ISGX1GGX2T | Wherein X1=R or S, and X2=T or S; preferably, wherein X1=R and X2=T |
| SEQ ID No: 11 050-049-051-055 | IgHV3-23-1 | VH CDR3 | AKARANWDYFDY | |
| SEQ ID No: 128 084 | IgHV1-69-04 | VH CDR1 | GGTFX1X2YA | Wherein X1=R or S, and X2=T or S; preferably, wherein X1=R and X2=T |
| SEQ ID No: 129 084 | Ig HV 1-69-04 | VH CDR2 | IX1X2X3LGIX4 | Wherein X1=N or I, X2=T or P, X3=V or I, and X4=V or A, preferably, wherein X1=N, X2=T, X3=V, and X4=V |
| SEQ ID No: 130 084 | IgHV1-69-04 | VH CDR3 | AREKGVDYYYGX1X2 | Wherein X1=I or M, X2=E or D; preferably, wherein X1=I, X2=E |
| SEQ ID No: 131 169-123-161-124 | IgHV1-18-1 | VH CDR1 | GYTFTXYG | Wherein X=N or S, preferably N |
| SEQ ID No: 132 169-123-161-124 | IgHV1-18-1 | VH CDR2 | X1X2X3YX4 X5NT | Wherein X1=L or I, X2 = S, T, or I; X3=A or T; X4=S or N; X5 = G or S; preferably, wherein X2=S, X3=A, and X4=S |
| SEQ ID No: 133 169-123-161-124 | IgHV1-18-1 | VH CDR3 | ARDRX1X2VRP DYFDY | Wherein X1=I or V, X2=V or I; preferably, wherein X1=I and X2=V |
| SEQ ID No: 134 025-001-143-019-021-027 | IgHV4-34-01 | VH CDR1 | GGSFSX1YX2 | Wherein X1=D or G and X2=Y or F; preferably, wherein X1=D and X2=Y |
| SEQ ID No: 135 025-001-143-019-021-027 | IgHV4-34-01 | VH CDR2 | IX1HX2GSX3 | Wherein X1=H or N, X2=S or V, and X3=T or A; preferably, wherein X1=H, X2=S, and X3=T |
| SEQ ID No: 136 025-001-143-019-021-027 | IgHV4-34-01 | VH CDR3 | ARGX1X2X3SG X4YYFDX5 | Wherein X1=Y, N or L; X2=Y or I, X3=D, G or A; X4=V or Y; and X5=Y or L; preferably, wherein X1=Y, X2=Y, X3=D, X4=V, and X5=Y |
| SEQ ID No: 137 091-032-035-036-054-094 | IgHV4-34-01 | VH CDR1 | GGSFSX1YY | Wherein X1=G or D, preferably G |
| SEQ ID No: 138 091-032-035-036-054-094 | IgHV4-34-01 | VH CDR2 | IX1HSGX2T | Wherein X1=Y, N or H; and X2=D or S; preferably, wherein X1=Y and X2=D |

| SEQ ID No: 139 091-032-035-036-054-094 | IgHV4-34-01 | VH CDR3 | ARLX1X2GSGX3YYX4DY | Wherein X1=Y, For W; X2=F or Y; X3=I, T or S; and X4=L or F; preferably, wherein X1=Y, X2=F, X3=I, and X4=L |
|---|---|---|---|---|
| SEQ ID No: 140 129 | IgHV3-30-01 | VH CDR1 | GFTFSX1X2A | Wherein X1=T or F, X2=F or Y; preferably, wherein X1=T and X2=F |
| SEQ ID No: 141 129 | IgHV3-30-01 | VH CDR2 | ISYDGX1X2K | Wherein X1=G or S, X2=H or N; preferably, wherein X1=G and X2=H |
| SEQ ID No: 142 129 | IgHV3-30-01 | VH CDR3 | ARGLGVWGX1FDY | Wherein X1=A or Y, preferably A |
| SEQ ID No: 143 098-105-100-125-162 | IgHV3-23-01 | VH CDR1 | GFTFX1X2YX3 | Wherein X1=S, N or T; X2=N, D or S; and X3=G or A; preferably, wherein X1=S, X2=N and X3=G |
| SEQ ID No: 144 098-105-100-125-162 | IgHV3-23-01 | VH CDR2 | ISGX1X2X3X4T | Wherein X1=S or T, X2=A or G, X3=Y or G, X4=S or A; preferably, wherein X1=S, X2=A, X3=Y, X4=S |
| SEQ ID No: 145 098-105-100-125-162 | IgHV3-23-01 | VH CDR3 | AKX1X2X3X4GSGSYYTX5FDY | Wherein X1=A or G; X2=H or Y; X3=Y or T; X4=H, For L; X5=L or S; preferably, wherein X1=A; X2=H; X3=Y; X4=H; X5=L |
| SEQ ID No: 146 127 | IgHV5-51-01 | VH CDR1 | GYSFX1X2YW | Wherein X1=S or T, X2=I or S; preferably, wherein X1=S, X2=I |
| SEQ ID No: 147 127 | IgHV5-51-01 | VH CDR2 | IX1PGDSDX2 | Wherein X1=F or Y, X2=I or T; preferably, wherein X1=F, X2=I |
| SEQ ID No: 148 127 | IgHV5-51-01 | VH CDR3 | ARQPGDWSPRHWYFDL | |
| SEQ ID No: 149 159 | IgHV5-51-01 | VH CDR1 | GYXFTSYW | Wherein X=N or S, preferably N |
| SEQ ID No:51 159 | IgHV5-51-01 | VH CDR2 | IYPGDSDT | |
| SEQ ID No:52 159 | IgHV5-51-01 | VH CDR3 | ARWGTYYDILTGYFN | |
| SEQ ID No:71 132 | IgHV1-18-01 | VH CDR1 | GYTFTSYG | |

EP 2 699 260 B1

| | | | | |
|---|---|---|---|---|
| SEQ ID No: 150 132 | IgHV1-18-01 | VH CDR2 | ISAYNGNX | Wherein X=S or T, preferably S |
| SEQ ID No: 151 132 | IgHV1-18-01 | VH CDR3 | AREYSYDSGTY FYYGMDV | |
| SEQ ID No: 152 153-033-160-166-152-167 | IgHV3-30-03-01 | VH CDR1 | GFTFSX1X2X3 | Wherein X1=D or S, X2=Y or H, X3=V or A; preferably, wherein X1=D, X2=Y, X3=V |
| SEQ ID No: 153 153-033-160-166-152-167 | IgHV3-30-03-01 | VH CDR2 | ISYDGSX1X2 | Wherein X1=N or Y, X2=K or E, preferably wherein X1=N and X2=K |
| SEQ ID No: 154 153-033-160-166-152-167 | IgHV3-30-03-01 | VH CDR3 | ARGX1X2X3X4 X5X6GX7FDY | Wherein X1=G, D or S; X2=I or Y; X3=T or I; X4=G or S; X5=T or S; X6=T or S; X7=Y or V; preferably, wherein X1=G; X2=I; X3=T; X4=G; X5=T; X6=T; and X7=V |
| SEQ ID No: 13 050-084-049-051-055 | IgKV1-12-01 | VL CDR1 | QGISSW | |
| 050-084-049-051-055 | IgKV1-12-01 | VL CDR2 | XAS | Wherein X=A or V |
| SEQ ID No:155 050-084-049-051-055 | IgKV1-12-01 | VL CDR3 | QQANSFPXT | Wherein X=I or L |
| SEQ ID No:6 169-124-161-123 | IgKV3-11-01 | VL CDR1 | QSVSSY | |
| 169-124-161-123 | IgKV3-11-01 | VL CDR2 | DXS | Wherein X=A or T, preferably A |
| SEQ ID No:156 169-124-161-123 | IgKV3-11-01 | VL CDR3 | QQRSXWPRT | Wherein X=N or H, preferably N |
| SEQ ID No:157 025-001-019-143-021-027 | IgKV1D-16-01 | VL CDR1 | QGISXW | Wherein X=R or S, preferably R |
| 025-001-019-143-021-027 | IgKV1D-16-01 | VL CDR2 | AAS | |
| SEQ ID No:164 025-001-019-143-021-027 | IgKV1D-16-01 | VL CDR3 | QQYNSXPIT | Wherein X=Y or F, preferably Y |
| SEQ ID No:33 091-032-035-036-054-094 | IgKV1D-16-01 | VL CDR1 | QGISSW | |

(continued)

| | | | | |
|---|---|---|---|---|
| 091-032-035-036-054-094 | IgKV1D-16-01 | VL CDR2 | AX1X2 | Wherein X1=A or T, and X2=S or F; preferably, wherein X1=A and X2=S |
| SEQ ID No:158 091-032-035-036-054-094 | IgKV1D-16-01 | VL CDR3 | QQYNSFPPT | |
| SEQ ID No:159 098-100-105-125-162 | IgKV1D-16-01 | VL CDR1 | QGIXSW | Wherein X=S or N, preferably S |
| 098-100-105-125-162 | IgKV1D-16-01 | VL CDR2 | AAS | |
| SEQ ID No:160 098-100-105-125-162 | IgKV1D-16-01 | VL CDR3 | QQYNSYPXT | Wherein X=Y or L, preferably Y |
| SEQ ID No:161 153-152-166-167-160-033 | IgKV1D-16-01 | VL CDR1 | QGIX1X2W | Wherein X1=S or N; X2=S or N; preferably, wherein X1=X2=S |
| 153-152-166-167-160-033 | IgKV1D-16-01 | VL CDR2 | XAS | Wherein X=D or A, preferably D |
| SEQ ID No:162 153-152-166-167-160-033 | IgKV1D-16-01 | VL CDR3 | QQYXSYPIT | Wherein X=K or N, preferably K |
| SEQ ID No: 221 005-060-106-041-150-067-072-163-093-044 | IgHV5-51-1 | VH CDR1 | GYX1FX2X3YW | wherein X1=S or R; X2=S, T, H, or I; and X3=S, R, or F; preferably, wherein X2=H or T |
| SEQ ID No: 222 005-060-106-041-150-067-072-163-093-044 | IgHV5-51-1 | VH CDR2 | IX1PGDSX2T | wherein X1=Y or F; X2=D, Y, or H preferably, wherein X2=D or Y |
| SEQ ID No: 223 005-060-106-041-150-067-072-163-093-044 | IgHV5-51-1 | VH CDR3 | ARX1X2X3X4X5X6X7X8YX9X10GX11DX12 | wherein X1=Q, H, or L; X2= R, A, T, or K; X3=G; X4=D; X5=R or none; X6=G or none; X7=Y or F; X8=Y or D; X9=Y, F, or H; X10=Y, D, S, F, or N; X11=M or L; and X12=V or I; preferably, wherein X1=Q, X2= R or A; X5=X6=none; X7=Y or F; X8=Y; X9=F; X10=Y; and X12=V |
| SEQ ID No: 224 006 | IgHV3-23-1 | VH CDR1 | GFTFSXYA | wherein X=N or S, preferably N |
| SEQ ID No: 225 006 | IgHV3-23-1 | VH CDR2 | IX1GX2X3GST | wherein X1=R or S; X2=G or S; and X3=A or G, preferably wherein X1=R; X2=G; and X3=A |

| SEQ ID No: 226 006 | IgHV3-23-1 | VH CDR3 | AKRIWGPXFDY | wherein X=L or Y, preferably L |
|---|---|---|---|---|
| SEQ ID No: 227 059 | IgHV1-18-1 | VH CDR1 | GYTFTXYG | wherein X=R or S, preferably R |
| SEQ ID No: 228 059 | IgHV1-18-1 | VH CDR2 | ISAYNGXT | wherein X=K or N, preferably K |
| SEQ ID No: 229 059 | IgHV1-18-1 | VH CDR3 | ARSPLLWFEELYFDY | |
| SEQ ID No:230 111 | IgHV1-69-4 | VH CDR1 | GGTFSSYX | wherein X=G or A, preferably G |
| SEQ ID No: 202 111 | Ig HV 1-69-4 | VH CDR2 | IIPILGIA | |
| SEQ ID No: 231 111 | Ig HV 1-69-4 | VH CDR3 | ARDQEYSSX1X2X3 | wherein X1=N or Y; X2=W or F; and X3=Y or D, preferably wherein X1=N; X2=W; and X3=Y |
| SEQ ID No: 232 005-059-060-106-111-041-150-067-072-163-093-044 | IgKV3-20-01 | VL CDR1 | QSVX1SX2Y | wherein X1=S or R and X2=S or T |
| 005-059-060-106-111-041-150-067-072-163-093-044 | IgKV3-20-01 | VL CDR2 | GAS | |
| SEQ ID No: 233 005-059-060-106-111-041-150-067-072-163-093-044 | IgKV3-20-01 | VL CDR3 | QX1YGX2SX3X4X5T | wherein X1=Q or L; X2=S or T; X3=P or none; X4=P, L, R, or none; and X5=L, F, I, or none; preferably, wherein X4=P, L, or none |
| SEQ ID No: 177 006 | IgKV3-11-01 | VL CDR1 | QSVSSY | |
| 006 | IgKV3-11-01 | VL CDR2 | DAS | |
| SEQ ID No: 178 006 | IgKV3-11-01 | VL CDR3 | QQRSNWPPLT | |

**Example 11** - **Purification of antibodies**

Culture supernatant was filtered over 0.2 μm dead-end filters, loaded on 5 mL MabSelect SuRe columns (GE Health Care) and eluted with 0.1 M sodium citrate-NaOH, pH 3. The eluate was immediately neutralized with 2M Tris-HCl, pH 9 and dialyzed overnight to 12.6 mM NaH2PO4, 140 mM NaCl, pH 7.4 (B.Braun). Alternatively, subsequent to purification, the eluate was loaded on a HiPrep Desalting column and the antibody was exchanged into 12.6 mM NaH2PO4, 140 mM NaCl, pH 7.4 (B.Braun) buffer. After dialysis or exchange of buffer, samples were sterile filtered over 0.2 μm dead-end filters. Purity was determined by SDS-PAGE and concentration was measured by nephelometry and absorbance at 280 nm. Purified antibodies were stored at 4°C. Mass spectrometry was performed to identify the molecular mass of the antibody heavy and light chains expressed by the hybridomas as described in Example 9.

**Example 12** - **Binding of HER2 antibody clones to tumor cells expressing membrane-bound HER2 measured by means of FACS analysis**

The binding of HER2 antibodies to AU565 cells (purchased at ATCC, CRL-2351) and A431 cells (purchased at ATCC, CRL-1555), was tested using flow cytometry (FACS Canto II, BD Biosciences). Qifi analysis (Dako, Glostrup, Denmark) revealed that AU565 cells expressed on average 1,000,000 copies of HER2 protein per cell, whereas A431 cells expressed on average 15,000 copies per cell. Binding of HER2 antibodies was detected using a Phycoerythrin (PE)-conjugated goat-anti-human IgG antibody (Jackson). Trastuzumab (clinical-grade Herceptin®) was used as positive control antibody, and an isotype control antibody was used as negative control antibody. $EC_{50}$ values were determined by means of non-linear regression (sigmoidal dose-response with variable slope) using GraphPad Prism V4.03 software (GraphPad Software, San Diego, CA, USA).

All tested HER2 antibodies bound to HER2 expressed on both AU565 and A431 cells in a dose-dependent manner. For antibodies of cross-block groups 1, 2 and 3, the $EC_{50}$ values for binding varied between 0.336-2.290 μg/mL for AU565 cells and 0.068-1.135 μg/mL for A431 cells (Figure 3A-D). For antibodies of cross-block group 4, the $EC_{50}$ values for binding varied between 0.304-2.678 μg/mL for AU565 cells and 0.106-1.982 μg/mL for A431 cells (Figure 3E and F). Especially on A431 cells, large differences in $EC_{50}$ values were observed between the tested antibodies. However, antibody 098 had the best (i.e., lowest) $EC_{50}$ value on both types of cells. Also some differences in maximum binding levels were observed between different antibodies, on both AU565 and A431 cells. Of the tested cross-block groups 1-3 antibodies, antibody 098 also had the highest maximum binding level on AU565 cells, whereas antibody 025 had the highest maximum binding level on A431 cells. For antibodies of cross-block group 4, antibodies 005 and 006 demonstrated higher maximum binding levels on A431 as compared to other HER2 antibodies.

**Example 13** - **Binding of HER2 antibodies to membrane-bound HER2 expressed on Rhesus epithelial cells measured by means of FACS analysis**

To determine cross-reactivity with Rhesus HER2, the binding of HER2 antibodies to HER2-positive Rhesus epithelial cells (4MBr-5 purchased at ATCC) was tested using flow cytometry (FACS Canto II, BD Biosciences). A Phycoerythrin-conjugated goat-anti-human IgG antibody (Jackson) was used as a secondary conjugate. An isotype control antibody was used as negative control antibody.

All tested HER2 antibodies were cross-reactive with Rhesus monkey HER2 (Figure 4A and B). At both tested concentrations (1 μg/mL and 10 μg/mL), the HER2 antibodies were able to bind specifically to Rhesus monkey HER2. Antibody 127 demonstrated poor binding at 1 μg/mL concentration, but showed good binding at 10 μg/mL concentration. Antibody 098 had the highest binding level at both antibody concentrations. No binding was observed with the isotype control antibody.

**Example 14** - **Competition of HER2 antibodies for binding to soluble HER2ECDHis measured in sandwich-ELISA**

The optimal coating concentrations of the tested HER2 antibodies and optimal HER2ECDHis concentration were determined in the following manner: ELISA wells were coated overnight at 4°C with HER2 HuMabs serially diluted in PBS (0.125-8 μg/mL in 2-fold dilutions). Next, the ELISA wells were washed with PBST (PBS supplemented with 0.05% Tween-20 [Sigma-Aldrich, Zwijndrecht, The Netherlands]) and blocked for one hour at room temperature (RT) with PBSTC (PBST supplemented 2% [v/v] chicken serum [Gibco, Paisley, Scotland]). The ELISA wells were then washed with PBST and incubated for one hour at RT with HER2ECDHis serially diluted in PBSTC (0.25-2 μg/mL in 2-fold dilutions). Unbound HER2ECDHis was washed away with PBST, and bound HER2ECDHis was incubated for one hour at RT with 0.25 μg/mL biotinylated rabbit-anti-6xhis-biot (Abcam, Cambridge, UK). The plate was thereafter washed with PBST and incubated for one hour with 0.1 μg/mL Streptavidin-poly-HRP (Sanquin, Amsterdam, The Netherlands) diluted in PBST. After washing, the reaction was visualized through a 15 minutes incubation with 2,2'-azino-bis (3-ethylbenzothiazoline-6-sulfonic acid) (ABTS: one ABTS tablet diluted in 50 mL ABTS buffer (Roche Diagnostics, Almere, The Netherlands)) at RT protected from light. The colorization was stopped by adding an equal volume of oxalic acid (Sigma-Aldrich, Zwijndrecht, The Netherlands). Fluorescence at 405 nm was measured on a microtiter plate reader (Biotek Instruments, Winooski, USA). The antibody concentrations that resulted in sub-optimal binding of each antibody were determined and used for the following cross-block experiments.

Each HER2 antibody was coated to the ELISA wells at the sub-optimal dose that was determined as described above. After blocking of the ELISA wells, the wells were incubated with the predetermined concentration of 1 $\mu$g/mL biotinylated HER2ECDHis in the presence or absence of an excess of a second (competitor) HER2 antibody. The ELISA was then performed as described above. Residual binding of HER2ECDHis to the coated antibody was expressed as a percentage relative to the binding observed in the absence of competitor antibody. Percentage competition was then determined as 100 minus the percentage of inhibition. 75% competition was considered as complete cross-block, whereas 25-74% competition was considered as partial cross-block, and 0-24% competition was considered non-blocking.

*Cross-block groups 1, 2 and 3:*

As shown in Table 5A, all HER2 antibodies of these groups were found to be able to block binding to HER2ECDHis, at least partially, for themselves. After dividing the antibodies into 3 major cross-block groups, all antibodies were tested for competition with at least one representative antibody from each group.

The first group comprised trastuzumab and antibodies 169, 050 and 084, which blocked each other for binding to HER2ECDHis, but did not cross-block antibodies from other groups.

The second group comprised pertuzumab and antibodies 025, 091 and 129, which blocked each other for binding to HER2ECDHis, except for antibodies 129 and 091 which both cross-blocked pertuzumab and 025, but not each other. None of the antibodies of group 2 blocked antibodies from other groups.

A third group comprised antibodies C1, F5, 127, 098, 132, 153 and 159, which did not cross-block any antibody from the other groups. Within this group 3, some variation was observed. Antibody 127 was the only antibody that was able to cross-block all other antibodies in this group for binding to HER2ECDHis; antibody 159 cross-blocked all other antibodies within this group, except 132; clone 098 cross-blocked all antibodies of group 3, except 132 and 153; antibody 153 cross-blocked 127, 132 and 159 for binding to HER2ECDHis, but not 098, C1 or F5; clone 132 cross-blocked 127, 132 and 153. When added as competitor antibodies, F5 and C1 only demonstrated cross-blocking of each other. However, the reverse reaction also revealed competition with antibodies 127, 098 and 159, but not 153 and 132. Possibly, these differences may have resulted from lower affinities of antibodies C1 and F5 for HER2ECDHis.

Values higher than 100% can be explained by avidity effects and the formation of antibody-HER2ECDHis complexes containing two non-competing antibodies.

*Cross-block group 4:*

As shown in Table 5B, all HER2 antibodies of this group competed for binding to HER2ECDHis, at least partially, with themselves. Trastuzumab (clinical grade Herceptin®) and pertuzumab (TH1014-pert, transiently produced in HEK-293 cells) could only compete with themselves, and not with any of the other listed HER2 antibodies of cross-block group 4. C1 and F5 (both transiently produced in HEK-293 cells) competed with each other for binding to HER2ECDHis, but did not compete with other HER2 antibodies of cross-block group 4.

Antibodies 005, 006, 059, 060, 106 and 111 all competed with each other for binding to HER2ECDHis, but did not cross-block with trastuzumab, pertuzumab, C1 or F5. Clones 005, 059, 060 and 106 only blocked 006 when 006 was the competitor antibody. In the reverse reaction where 006 was immobilized, no blocking was found with 005, 059, 060 or 106. This was possibly a result of the higher apparent affinity of clone 006 compared to 005, 059, 060, 106 and 111. Values higher than 100% can be explained by avidity effects and the formation of antibody-HER2ECDHis complexes containing two non-blocking antibodies.

**Table 5:** Competition and cross-blocking of HER2 antibodies for binding to HER2ECDHis

**5A:**

| Immobilized mAb ↓ | Competing mAb → | | | | | | | | | | | | | | |
|---|---|---|---|---|---|---|---|---|---|---|---|---|---|---|---|
| | tras | 169 | 050 | 084 | pert | 025 | 091 | 129 | C1 | F5 | 127 | 159 | 098 | 153 | 132 |
| Trastuzumab | 6 | 15 | 6 | 51 | 100 | 107 | 100 | 85 | 103 | 99 | 115 | 90 | 101 | 101 | 101 |
| TH1014-169 | 19 | 45 | 21 | 73 | 101 | 98 | 105 | 106 | ND | ND | ND | ND | 105 | 102 | ND |
| TH1014-050 | 13 | 30 | 12 | 74 | 95 | 104 | 98 | 110 | ND | ND | ND | ND | 102 | 104 | ND |
| TH1014-084 | 74 | 73 | 76 | 20 | 101 | 106 | 104 | 104 | ND | ND | ND | ND | 109 | 98 | ND |
| TH1014-pert | 104 | 100 | 94 | 95 | 9 | 20 | 19 | 39 | 106 | 125 | 116 | 81 | 103 | 100 | 109 |
| TH1014-025 | 98 | 98 | 100 | 104 | 8 | 18 | 21 | 15 | ND | ND | ND | ND | 102 | 99 | ND |

| | | | | | | | | | | | | | | | |
|---|---|---|---|---|---|---|---|---|---|---|---|---|---|---|---|
| TH1014-091 | 99 | 99 | 95 | 100 | 5 | 13 | 15 | 78 | ND | ND | ND | ND | 98 | 98 | ND |
| TH1014-129 | 93 | 99 | 97 | 92 | 22 | 55 | 76 | 12 | ND | ND | ND | ND | 106 | 98 | ND |
| TH1014-C1 | 89 | ND | ND | ND | ND | ND | ND | ND | 65 | 58 | 73 | 53 | 58 | 77 | 90 |
| TH1014-F5 | 197 | ND | ND | ND | ND | ND | ND | ND | 70 | 21 | 62 | 15 | 16 | 80 | 125 |
| TH1014-127 | 102 | ND | ND | ND | ND | ND | ND | ND | 112 | 88 | 11 | 8 | 58 | 21 | 44 |
| TH1014-159 | 111 | ND | ND | ND | 112 | ND | ND | ND | 96 | 86 | 15 | 6 | 11 | 40 | 79 |
| TH1014-098 | 107 | 102 | 100 | 103 | 104 | 108 | 104 | 107 | 125 | 96 | 21 | 9 | 17 | 110 | 142 |
| TH1014-153 | 134 | 111 | 103 | 107 | 121 | 97 | 102 | 106 | 257 | 96 | 27 | 23 | 115 | 28 | 33 |
| TH1014-132 | 353 | ND | ND | ND | 288 | ND | ND | ND | 422 | 379 | 30 | 131 | 309 | 41 | 32 |
| Cross-block group | 1 | 1 | 1 | 1 | 2 | 2 | 2 | 2b | 3a | 3a | 3a | 3a | 3a | 3b | 3b |

**5B:**

| | Competing mAb: → | | | | | | | | | |
|---|---|---|---|---|---|---|---|---|---|---|
| Immobilized mAb ↓ | Tras | Pert | C1 | F5 | 106 | 111 | 005 | 006 | 059 | 060 |
| **Trastuzumab** | 6 | 100 | 103 | 99 | 114 | 166 | 137 | 110 | 120 | 119 |
| **TH1014-pert** | 104 | 9 | 106 | 125 | 115 | 145 | 151 | 125 | 132 | 118 |
| **TH1014-C1** | 89 | 85 | 65 | 58 | 84 | 86 | 98 | 99 | 89 | 93 |
| **TH1014-F5** | 197 | 178 | 70 | 21 | 129 | 183 | 178 | 192 | 165 | 185 |
| **PC1014-106** | 323 | 275 | 471 | 495 | 26 | 21 | 25 | 25 | 25 | 23 |
| **PC1014-111** | 110 | 102 | 122 | 119 | 75 | 14 | 51 | 10 | 65 | 36 |
| **PC1014-005** | 126 | 115 | 157 | 227 | 54 | 32 | 18 | 15 | 22 | 12 |
| **PC1014-006** | 163 | 136 | 136 | 153 | 127 | 47 | 148 | 20 | 129 | 125 |
| **PC1014-059** | 117 | 107 | 78 | 128 | 23 | 12 | 13 | 11 | 12 | 11 |
| **PC1014-060** | 106 | 99 | 108 | 126 | 37 | 35 | 30 | 6 | 14 | 19 |
| **Cross-block group** | 1 | 2 | 3 | 3 | 4 | 4 | 4 | 4 | 4 | 4 |

75 – >100% competition

25 – 74% competition

0 – 24% competition

Depicted values are mean percentages of binding relative to the binding observed in the absence of competitor antibody, of two independent experiments. Competition experiments with HEK produced TH1014-C1 and TH1014-F5 were performed once. Trastuzumab (clinical grade Herceptin®) and HEK-produced pertuzumab (TH1014-pert) were also tested.

**Example 15 - Antibody-dependent cell-mediated cytotoxicity (ADCC) of HER2 antibodies**

SK-BR-3 cells (purchased at ATCC, HTB-30) were harvested ($5 \times 10^6$ cells), washed (twice in PBS, 1500 rpm, 5 min) and collected in 1 mL RPMI 1640 medium supplemented with 10% cosmic calf serum (CCS) (HyClone, Logan, UT, USA), to which 200 μCi $^{51}$Cr (Chromium-51; Amersham Biosciences Europe GmbH, Roosendaal, The Netherlands) was added. The mixture was incubated in a shaking water bath for 1.5 hours at 37°C. After washing of the cells (twice in PBS, 1500 rpm, 5 min), the cells were resuspended in RPMI 1640 medium supplemented with 10% CCS, counted by trypan blue exclusion and diluted to a concentration of $1 \times 10^5$ cells/mL.

Meanwhile, peripheral blood mononuclear cells (PBMCs) were isolated from fresh buffy coats (Sanquin, Amsterdam, The Netherlands) using standard Ficoll density centrifugation according to the manufacturer's instructions (lymphocyte separation medium; Lonza, Verviers, France). After resuspension of cells in RPMI 1640 medium supplemented with 10% CCS, cells were counted by trypan blue exclusion and concentrated to $1\times10^7$ cells/mL.

Trastuzumab was produced in CHO cells resulting in an (increased) non-core fucosylation grade of 12.4%, whereas the other HER2 antibodies were produced in HEK cells, resulting on average in 4% non-core fucosylation.

For the ADCC experiment, 50 $\mu$L $^{51}$Cr-labeled SK-BR-3 cells (5.000 cells) were pre-incubated with 15 $\mu$g/mL HER2 antibody (IgG1,$\kappa$) in a total volume of 100 $\mu$L RPMI medium supplemented with 10% CCS in a 96-well microtiter plate. After 15 min at RT, 50 $\mu$L PBMCs (500,000 cells) were added, resulting in an effector to target ratio of 100:1. The maximum amount of cell lysis was determined by incubating 50 $\mu$L $^{51}$Cr-labeled SK-BR-3 cells (5.000 cells) with 100 $\mu$L 5% Triton-X100. The amount of spontaneous lysis was determined by incubating 5.000 $^{51}$Cr-labeled SK-BR-3 cells in 150 $\mu$L medium, without any antibody or effector cells. The level of antibody-independent cell lysis was determined by incubating 5.000 SK-BR-3 cells with 500,000 PBMCs without antibody. Subsequently, the cells were incubated 4 hr at 37°C, 5% $CO_2$. To determine the amount of cell lysis, the cells were centrifuged (1.200 rpm, 3 min) and 75 $\mu$L of supernatant was transferred to micronic tubes, after which the released $^{51}$Cr was counted using a gamma counter. The measured counts per minute (cpm) were used to calculate the percentage of antibody-mediated lysis as follows:

$$(cpm\ sample - cpm\ Ab\text{-}independent\ lysis)/(cpm\ max.\ lysis - cpm\ spontaneous\ lysis)\times 100\%$$

HER2 antibodies from cross-block groups 1 and 2 induced efficient lysis of SK-BR-3 cells through ADCC (Figure 5A). From group 3, antibody 153 was the only antibody that induced efficient ADCC, antibody 132 induced about 10% ADCC, and clones 098, 159 and 127 did not induce ADCC. All HER2 antibodies from cross-block group 4 induced efficient lysis of SK-BR-3 cells through ADCC (Figure 5B). The average percentage lysis by the different antibodies of cross-block group 4 varied between 15% and 28%, in contrast to trastuzumab (Herceptin®), which showed on average 41% lysis. Without being bound by theory, the higher percentage lysis by trastuzumab possibly resulted from an increased non-core fucosylation grade (12.4%) due to its CHO production, compared to ~4% non-core fucosylation on the other HEK-produced HER2 antibodies, or by recognizing an epitope that induces less internalization of the HER2 receptor-antibody complexes.

**Example 16** - **Inhibition of ligand-independent proliferation of AU565 cells by HER2 antibodies**

HER2 antibodies were tested for their ability to inhibit proliferation of AU565 cells *in vitro*. Due to the high HER2 expression levels on AU565 cells (~1,000,000 copies per cell as described in Example 12), HER2 is constitutively active in these cells and thus not dependent on ligand-induced heterodimerization.

In a 96-well tissue culture plate (Greiner bio-one, Frickenhausen, Germany), 9.000 AU565 cells were seeded per well in the presence of 10 $\mu$g/mL HER2 antibody in serum-free cell culture medium. As a control, cells were seeded in serum-free medium without antibody. After 3 days, the amount of viable cells was quantified with Alamarblue (BioSource International, San Francisco, US) according to the manufacturer's instructions. Fluorescence was monitored using the EnVision 2101 Multilabel reader (PerkinElmer, Turku, Finland) with standard Alamarblue settings. The Alamarblue signal of antibody-treated cells was plotted as a percentage relative to untreated cells. Dunnett's test was applied for statistical analysis.

The percentage proliferation of AU565 cells after HER2 antibody treatment was compared to untreated cells, which was set to 100%. Of the tested Group 1 antibodies, trastuzumab, 050 and 169 demonstrated significant inhibition of AU565 cell proliferation ($P<0.05$), whereas 084 had no effect. None of the tested antibodies from group 2 (Pertuzumab, 025, 092 and 129) was able to inhibit AU565 cell proliferation. The tested antibodies from group 3 (098 and 153) did not inhibit AU565 proliferation. In contrast, both antibodies induced enhanced proliferation of AU565 cells compared to untreated cells (098 more than 153). See Figure 6. For trastuzumab and pertuzumab, this was in accordance with the results described by Juntilla et al. (Cancer Cell 2009;15(5):353-355).

From cross-block group 4, TH1014-F5 significantly enhanced proliferation of AU565 cells indicating that this is an agonistic antibody, whereas none of the other antibodies of cross-block group 4 tested (005, 060 and pertuzumab) had a substantial effect on AU565 proliferation (data not shown). Enhancing proliferation can be an advantage in some therapeutic applications of ADC-conjugates, e.g., where the cytotoxic action of the drug relies on, or is enhanced by, cell proliferation.

**Example 17** - **Inhibition of ligand-induced proliferation of MCF-7 cells by HER2 antibodies**

Since HER2 is an orphan receptor, its signaling is mainly dependent on activation of other ErbB-family members such as EGFR and Her3. Upon ligand binding, these two receptors can bind to and activate the HER2 receptor, resulting in e.g. proliferation. Various publications describe that pertuzumab efficiently inhibits Heregulin-(31-induced proliferation (Franklin MC. Cancer Cell 2004 / Landgraf R. BCR 2007). For trastuzumab, it has been described that it has little effect on Heregulin-(31-induced HER2/HER3 heterodimerization and proliferation (Larsen SS., et al., Breast Cancer Res Treat 2000;58:41-56; Agus DB., et al., Cancer Cell 2002;2:127-137; Wehrman *et al.* (2006), *supra*).

To investigate the ability of the present human HER2 antibodies to interfere with Heregulin-$\beta$1-induced HER2/HER3 heterodimers, a Heregulin-$\beta$1-induced proliferation assay was performed. Therefore, MCF7 cells (purchased at ATCC, HTB-22) expressing ~20.000 HER2 molecules per cell, were seeded in a 96-wells tissue culture plate (Greiner bio-one) (2.500 cells/well) in complete cell culture medium. After 4 hours, the cell culture medium was replaced with starvation medium containing 1% Cosmic Calf Serum (CCS) and 10 $\mu$g/mL HER2 antibody. Next, Heregulin-$\beta$1 (PeproTech, Princeton Business Park, US) diluted in 1% CCS containing starvation medium was added to the wells to a final concentration of 1.5 ng/mL. After 4 days incubation, the amount of viable cells was quantified with Alamarblue (BioSource International) according to the manufacturer's instructions. Fluorescence was monitored using the EnVision 2101 Multilabel reader (PerkinElmer) with standard Alamarblue settings. The Alamarblue signal of HER2 antibody-treated ligand-induced cells was plotted as a percentage signal compared to ligand-induced cells incubated without HER2 antibody. Dunnett's test was applied for statistical analysis.

The percentage of viable MCF7 cells stimulated with Heregulin-$\beta$1 and treated with the indicated HER2 antibody, relative to the viable cells after stimulation with Heregulin-$\beta$1 in the absence of HER2 antibody, was calculated. There was no MCF-7 proliferation in absence of both Heregulin-$\beta$1 and antibody. As shown in Figure 7, antibodies 025, 091, 129, 153 and pertuzumab (TH1014-pert) demonstrated significant inhibition of Heregulin-$\beta$1-induced MCF-7 proliferation ($P<0.05$). Also trastuzumab showed some inhibition of Heregulin-$\beta$1-induced proliferation of MCF-7 cells, although not as efficient as the other tested HER2 antibodies. It has been reported that domain IV of HER2 is involved in the stabilization of EGFR/HER2 heterodimers, but without details on its contribution to HER2/HER3 heterodimers (Wehrman *et al., supra)*. Antibodies 050, 084, 169 and 098 had no statistically significant effect on Heregulin-$\beta$1-induced proliferation of MCF-7 cells. Without being limited to theory, this suggests that these antibodies do not inhibit ligand-induced HER2/HER3 heterodimerization.

### Example 18 - HER2 antibodies tested in an anti-kappa-ETA' assay

To investigate the suitability of HER2 antibodies for an antibody-drug conjugate approach, a generic *in vitro* cell-based killing assay using kappa-directed pseudomonas-exotoxin A (anti-kappa-ETA') was developed. The assay makes use of a high affinity anti-kappa domain antibody conjugated to a truncated form of the pseudomonas-exotoxin A. Upon internalization, the anti-kappa-ETA' domain antibody undergoes proteolysis and disulfide-bond reduction, separating the catalytic from the binding domain. The catalytic domain is transported from the Golgi to the endoplasmic reticulum via the KDEL retention motif, and subsequently translocated to the cytosol where it inhibits protein synthesis and induces apoptosis (ref. Kreitman RJ. BioDrugs 2009;23(1):1-13). In this assay, to identify HER2 antibodies that enable internalization and killing through the toxin, HER2 antibodies are preconjugated with the anti-kappa-ETA' before incubation with HER2-positive cells.

First, the optimal concentration of anti-kappa-ETA' was determined for each cell line, *i.e.* the maximally tolerated dose that does not lead to induction of non-specific cell death. AU565 cells (7.500 cells/well) and A431 cells (2500 cells/well) were seeded in normal cell culture medium in 96-wells tissue culture plate (Greiner bio-one) and allowed to adhere for at least 4 hours. Next, cells were incubated with 100, 10, 1, 0.1, 0.01, 0.001 and 0 $\mu$g/mL anti-kappa-ETA' dilutions in normal cell culture medium. After 3 days, the amount of viable cells was quantified with Alamarblue (BioSource International, San Francisco, US) according to the manufacturer's instruction. Fluorescence was monitored using the EnVision 2101 Multilabel reader (PerkinElmer, Turku, Finland) with standard Alamarblue settings. The highest concentration anti-kappa-ETA' that did not kill the cells by itself was used for following experiments (0.5 $\mu$g/mL for AU565 and 1 $\mu$g/mL for A431).

Next, antibody-mediated internalization and killing by the toxin was tested for different HER2 antibodies. Cells were seeded as described above. Dilution-series of HER2 antibodies were pre-incubated for 30 minutes with the predetermined concentration anti-kappa-ETA' before adding them to the cells. After 3 days of incubation, the amount of viable cells was quantified as described above. The Alamarblue signal of cells treated with anti-kappa-ETA' conjugated antibodies was plotted compared to cells treated with antibody alone. 23.4 $\mu$g/mL Staurosporin was used as positive control for cell killing. An isotype control antibody was used as negative control.

*Cross-block groups 1, 2 and 3:*

As shown in Figure 8A,B and Table 6A, all anti-kappa-ETA'-conjugated HER2 antibodies were able to kill AU565 cells in a dose-dependent manner. All tested anti-kappa-ETA'-conjugated HER2 antibodies demonstrated better killing of AU565 cells (70.3 - 49.9 %) compared to both anti-kappa-ETA'-conjugated trastuzumab (31.9 %) and anti-kappa-ETA'-conjugated pertuzumab (TH1014-pert) (47.51 %) and the $EC_{50}$ values were increased (12.12 - 46.49 ng/mL compared to 78.49 ng/mL for anti-kappa-ETA'-conjugated trastuzumab and 117.8 ng/mL for anti-kappa-ETA'-conjugated pertuzumab). Antibody 159 had the highest percentage of cell-kill, and 098 the lowest $EC_{50}$.

As shown in Figure 8C,D and Table 7A, antibodies 025, 091, 098, 129 and 153 were able to induce effective killing of A431 cells ($\geq$75%). The highest percentage of cell-kill, and lowest $EC_{50}$ was shown by antibody 098. When conjugated to anti-kappa-ETA', trastuzumab and isotype control antibody did not induce killing of A431 cells. Antibodies 169, 084 and pertuzumab induced percentages of cell kill of no more than about 50%. No cell kill was observed with non-conjugated HER2 antibodies.

*Cross-block group 4:*

As shown in Table 6B, all anti-kappa-ETA'-conjugated HER2 antibodies of cross-block group 4 were able to kill AU565 cells in a dose-dependent manner (50-72% cell killing). Antibodies 005 and 111 demonstrated more than three times improved $EC_{50}$ values (resp. 15.13 and 24.20 ng/mL) compared to trastuzumab (78.49 ng/mL). Non-conjugated HER2 antibodies of cross-block group 4 did not induce killing of AU565 cells at the concentrations tested.

As shown in Table 7B, antibodies 005 and 060 were able to induce effective killing of A431 cells ($\geq$85%) when conjugated to anti-kappa-ETA'. Antibodies 005 and 111 demonstrated killing of A431 cells already at low antibody concentrations (10 ng/mL) with $EC_{50}$ values of ~10 ng/mL. No cell kill was observed with non-conjugated HER2 antibodies of cross-block group 4.

**Table 6:** Data shown are $EC_{50}$ values and maximal percentage cell kill of AU565 cells treated with anti-kappa-ETA'-conjugated HER2 antibodies (A, cross-block groups 1, 2 and 3; B, cross-block group 4), measured in one representative experiment. Cell-kill induced by Staurosporin was set as 100% and MFI of untreated cells was set as 0%. Ndet = not detected.

| A: | | |
|---|---|---|
| antibody | % cells killed | EC50 ng/mL |
| PC1014-159 | 70.3 | 34.93 |
| PC1014-127 | 69.0 | 34.46 |
| PC1014-132 | 61.6 | 39.35 |
| PC1014-129 | 60.8 | 30.85 |
| PC1014-153 | 60.3 | 32.26 |
| PC1014-025 | 60.0 | 16.71 |
| PC1014-098 | 58.7 | 12.12 |
| PC1014-084 | 58.1 | 26.97 |
| PC1014-050 | 52.4 | 12.71 |
| PC1014-091 | 50.6 | 46.49 |
| PC1014-169 | 49.9 | 35.62 |
| TH1014-pert | 47.5 | 117.8 |
| trastuzumab | 31.9 | 78.49 |
| isotype control | Ndet | Ndet |
| B: | | |
| antibody | % cells killed | EC50 ng/mL |
| PC1014-111 | 72.0 | 24.2 |
| PC1014-005 | 69.7 | 15.13 |
| PC1014-059 | 67.0 | 67.65 |
| PC1014-060 | 64.3 | 79.38 |

(continued)

| B: | | |
|---|---|---|
| antibody | % cells killed | EC50 ng/mL |
| PC1014-106 | 59.1 | 107.9 |
| PC1014-006 | 50.4 | 45.14 |
| Trastuzumab | 31.9 | 78.49 |
| isotype control | Ndet | Ndet |

**Table 7:** Data shown are $EC_{50}$ values and maximal percentage cell kill of A431 cells treated with anti-kappa-ETA'-conjugated HER2 antibodies (A, cross-block groups 1, 2 and 3; B, cross-block group 4), measured in one representative experiment. Cell kill induced by Staurosporin was set as 100% and MFI of untreated cells was set as 0%. "NDet" means not detected.

| A: | | |
|---|---|---|
| antibody | % cells killed | EC50 ng/mL |
| PC1014-025 | 86.7 | ~9.77 |
| PC1014-084 | 50.5 | ND |
| PC 1014-091 | 83.3 | ~9.86 |
| PC1014-098 | 87.2 | 1.65 |
| PC1014-129 | 75.9 | ~10.60 |
| PC1014-153 | 82.4 | ~10.11 |
| PC1014-169 | 34.0 | ND |
| TH1014-pert | 37.0 | 61.58 |
| trastuzumab | Ndet | Ndet |
| isotype control | NDet | NDet |
| B: | | |
| antibody | % cells killed | $EC_{50}$ ng/mL |
| PC1014-005 | 88.5 | ~ 10.07 |
| PC1014-060 | 85.0 | ~ 10.03 |
| Trastuzumab | NDet | NDet |
| isotype control | NDet | NDet |

## Example 19 - Internalization of HER2 antibodies measured with an FMAT-based fab-CypHer5E assay

To investigate whether the enhanced killing of AU565 cells by the described HER2 antibodies compared trastuzumab (Herceptin) and pertuzumab in the kappa-toxin-ETA' assay described in the previous Example correlated with enhanced internalization of HER2 antibodies, a fab-CypHer5E-based internalization assay was performed. CypHer5E is a pH-sensitive dye which is non-fluorescent at basic pH (extracellular: culture medium) and fluorescent at acidic pH (intracellular: lysosomes), with an acid dissociation constant (pKa) of 7.3.

AU565 cells were seeded in 384-well tissue culture plates (Greiner bio-one), at a density of 3.000 cells/well in normal cell culture medium supplemented with 240 ng/mL fab-CypHer5E (conjugation of Goat-fab-anti-Human IgG [Jackson] with CypHer5E [GE Healthcare, Eindhoven, The Netherlands] was made according to manufacturer's instructions). Next, HER2 antibodies were serially diluted in normal cell culture medium, added to the cells and left at room temperature for 9 hours. Mean fluorescent intensities (MFI) of intracellular CypHer5E were measured using the 8200 FMAT (Applied Biosystems, Nieuwerkerk A/D IJssel, The Netherlands) and 'counts x fluorescence' was used as read-out. An isotype control antibody was used as negative control antibody. $EC_{50}$ values and maximal MFI were determined by means of non-linear regression (sigmoidal dose-response with variable slope) using GraphPad Prism V4.03 software (GraphPad Software, San Diego, CA, USA).

*Cross-block groups 1, 2 and 3:*

The results are shown in Table 8A, depicting the $EC_{50}$ and maximal MFI values for all tested HER2 antibodies of cross-block groups 1, 2 and 3 in the CypHer5E internalization assay with AU565 cells. The maximal MFI values indicate how many HER2 receptors are internalized upon antibody binding. All HER2 antibodies showed higher maximal MFI values (137,904 - 38,801) compared to trastuzumab (35,000) and pertuzumab (TH1014-pert) (32,366), indicating that the tested HER2 antibodies induced enhanced receptor internalization. Notably, antibodies that did not compete for HER2 binding with trastuzumab or TH1014-pert induced more receptor internalization compared to antibodies that did compete with trastuzumab and TH1014-pert, with the highest MFI achieved by antibodies 098 and 127. Without being limited to theory, this might be inherent to an inability to inhibit HER2 heterodimerization.

*Cross-block group 4:*

The results are shown in Table 8B, depicting the $EC_{50}$ values and maximal MFI for all tested HER2 antibodies of cross-block group 4 in the CypHer5E internalization assay with AU565 cells. The maximal MFI values reflect how many HER2 antibodies were internalized upon binding. All tested human HER2 antibodies of cross-block group 4 showed higher maximal MFI values (130.529-57.428) than trastuzumab (35.000) and TH1014-pert (35.323), indicating that these antibodies induced enhanced receptor internalization. The enhanced internalization of TH1014-F5 may be a result from its agonistic activity and the induction of HER2-HER2 dimerization (see Example 16).

**Table 8:** Cypher-5-based internalization assay of HER2 antibodies. Data shown are MFI and $EC_{50}$ values of one representative experiment of two experiments with AU565 cells treated with fab-CypHer5E-labeled HER2 antibodies. Some $EC_{50}$ values could not be calculated (ND).

**A:**

| Cypher 5 | | |
|---|---|---|
| **Antibody** | **$EC_{50}$ ng/mL** | **Maximal MFI** |
| PC1014-025 | 30.05 | 63428 |
| PC1014-091 | 32.99 | 50711 |
| PC1014-129 | 7.15 | 60302 |
| TH1014-pert | 530 | 32366 |

| PC1014-169 | ND | 38801 |
| PC1014-084 | 30.51 | 71059 |
| trastuzumab | 21.70 | 35000 |
| PC1014-098 | 13.77 | 134575 |
| PC1014-127 | ~9.68 | 137904 |
| PC1014-159 | ND | 92427 |
| TH1014-F5 | 22.65 | 113116 |
| PC1014-132 | 11.42 | 112270 |
| PC1014-153 | ~14.91 | 87531 |

mAbs that compete with Herceptin

mAbs that compete with TH1014-pert

mAbs that compete with TH1014-F5

Non-competing mAbs

**B:**

| Cypher 5 | | |
|---|---|---|
| **Antibody** | **$EC_{50}$ ng/mL** | **Maximal MFI** |
| PC1014-006 | 23.08 | 130829 |
| PC1014-005 | 21.37 | 95117 |
| PC1014-111 | 35.22 | 81680 |
| PC1014-059 | 14.77 | 77123 |
| PC1014-060 | 36.16 | 68184 |
| PC1014-106 | 68.60 | 57428 |
| TH1014-F5 | 22.65 | 113116 |
| TH1014-pert | ~1041 | 35323 |
| Trastuzumab | 21.70 | 35000 |

### Example 20: Generation of bispecific HER2 x HER2 antibodies by 2-MEA-induced Fab-arm exchange

Bispecific HER2 x HER2 antibodies (used in Examples 21-24, 30-33) were derived from IgG1,κ antibodies being modified in their Fc regions (either K409R or T350I/K370T/F405L, which is further referred to as ITL) to allow heterodimerization in the process of bispecific antibody generation as described further in this example. The following Fc-modified IgG1,κ antibodies were used: IgG1-HER2-005-ITL, IgG1-HER2-025-ITL, IgG1-HER2-153-ITL, IgG1-HER2-005-K409R, IgG1-HER2-153-K409R, IgG1-HER2-169-K409R. Also IgG1-HER2-153-N297Q-K409R was used. The N297Q mutation makes the Fc-domain of the antibodies inert. An inert Fc-domain prevents the antibody to interact with Fc-receptors present on *e.g.* monocytes.

Heavy and light chain variable region sequences for the HER2 antibodies 005, 025, 153 and 169 are described in Example 10.

The following heavy chain constant domain sequences were used for the different Fc-variants (Antibody sequences were defined according to IMGT (Lefranc MP. et al., Nucleic Acids Research, 27, 209-212, 1999 and Brochet X. Nucl. Acids Res. 36, W503-508 (2008))):

IgG1 Fc region - WT (SEQ ID NO:234)

>ASTKGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKRVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAVEWESNGQPENNYKT
TPPVLDSDGSFFLYSKLTVDKSRWQQGNVFSCSVMHEALHNHYTQKSLSLSPGK

IgG1 Fc region - ITL (SEQ D NO:244)

>ASTKGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKRVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEYKCKV
SNKALPAPIEKTISKAKGQPREPQVYILPPSREEMTKNQVSLTCLVTGFYPSDIAVEWESNGQPENNYKT
TPPVLDSDGSFLLYSKLTVDKSRWQQGNVFSCSVMHEALHNHYTQKSLSLSPGK

IgG1 Fc region - K409R (SEQ ID NO:245)

>ASTKGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKRVEPKSCDKTHTCPPCPAPELLGGPSVFLFPPKPKDTLMISR
TPEVTCVVVDVSHEDPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAVEWESNGQPENNYKT
TPPVLDSDGSFFLYSRLTVDKSRWQQGNVFSCSVMHEALHNHYTQKSLSLSPGK

IgG1 Fc region - K409R N297Q (SEQ ID NO:241)

>ASTKGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKRVEPKSCDKTHTCPPCPAPELLGGPSVFLFPPKPKDTLMISR
TPEVTCVVVDVSHEDPEVKFNWYVDGVEVHNAKTKPREEQYQSTYRVVSVLTVLHQDWLNGKEYKCKV
SNKALPAPIEKTISKAKGQPREPQVYTLPPSREEMTKNQVSLTCLVKGFYPSDIAVEWESNGQPENNYKT
TPPVLDSDGSFFLYSRLTVDKSRWQQGNVFSCSVMHEALHNHYTQKSLSLSPGK

The following heavy and light chain variable region sequences for the HIV gp120-specific negative control antibody b12, were used (sequence as described by Barbas, CF. J Mol Biol. 1993 Apr 5;230(3):812-23.)

VH b12 (SEQ ID NO:246)

>QVQLVQSGAEVKKPGASVKVSCQASGYRFSNFVIHWVRQAPGQRFEWMGWINPYNGNKEFSAKFQ
DRVTFTADTSANTAYMELRSLRSADTAVYYCARVGPYSWDDSPQDNYYMDVWGKGTTVIVSS

VL b12 (SEQ ID NO:247)

>EIVLTQSPGTLSLSPGERATFSCRSSHSIRSRRVAWYQHKPGQAPRLVIHGVSNRASGISDRFSGSGS
GTDFTLTITRVEPEDFALYYCQVYGASSYTFGQGTKLERK

The following heavy and light chain variable region sequences for the negative control antibody IgG1-KLH, were used.

VH KLH (SEQ ID NO:248)

>QVQLVESGGGVVQPGRSLRLSCAASGFTFSSYGMHWVRQAPGKGLEWVAIGRFDGSNKYYADSVK
GRFTISRDNSKNTLYLQMNSLRAEDTAVYYCARGPHRIAAAGNFDYWGQGTLVTVSSAS

VL KLH (SEQ ID NO:249)

>EIVLTQSPATLSLSPGERATLSCRASQSVSSYLAWYQQKPGQAPRLLIYDASHRATGIPARFSGSGSGT
DFTLTISSLEPEDFAVYYCQQRSNWPPWTFGQGTKVEIK

The following sequences for the CD16-specific negative control antibody IgG1-3G8-QITL, were used.

VH 3G8 (SEQ ID NO:250)

>QVTLKESGPGILQPSQTLSLTCSFSGFSLRTSGMGVGWIRQPSGKGLEWLAHIWWDDDKRYNPALK
SRLTISKDTSSNQVFLKIASVDTADTATYYCAQINPAWFAYWGQGTLVTVSA

VL 3G8 (SEQ ID NO:251)

>DIVLTQSPASLAVSLGQRATISCKASQSVDFDGDSFMNWYQQKPGQPPKLLIYTTSNLESGIPARFSA
SGSGTDFTLNIHPVEEEDTATYYCQQSNEDPYTFGGGTKLEL K

IgG1 Fc region - QITL (SEQ ID NO:252)

>ASTKGPSVFPLAPSSKSTSGGTAALGCLVKDYFPEPVTVSWNSGALTSGVHTFPAVLQSSGLYSLSSV
VTVPSSSLGTQTYICNVNHKPSNTKVDKRVEPKSCDKTHTCPPCPAPELLGGPSVFLFPPKPKDTLMISR
TPEVTCVVVDVSHEDPEVKFNWYVDGVEVHNAKTKPREEQY<u>Q</u>STYRVVSVLTVLHQDWLNGKEYKCKV
SNKALPAPIEKTISKAKGQPREPQVY<u>I</u>LPPSREEMTKNQVSLTCLV<u>T</u>GFYPSDIAVEWESNGQPENNYKT
TPPVLDSDGSF<u>L</u>LYSKLTVDKSRWQQGNVFSCSVMHEALHNHYTQKSLSLSPGK

The following sequences for the control antibody trastuzumab (Herceptin) were used (sequence as described in, *e.g.,* U.S. Patent No. 7,632,924)

VH Herceptin (SEQ ID NO:253)

>MELGLSWVFLVAILEGVQCEVQLVESGGGLVQPGGSLRLSCAASGFNIKDTYIHWVRQAPGKGLEWV
ARIYPTNGYTRYADSVKGRFTISADTSKNTAYLQMNSLRAEDTAVYYCSRWGGDGFYAMDYWGQGTLV
TVSS

VL Herceptin (SEQ ID NO:254)

>MDMRVPAQLLGLLLLWLRGARCDIQMTQSPSSLSASVGDRVTITCRASQDVNTAVAWYQQKPGKAP
KLLIYSASFLYSGVPSRFSGSRSGTDFTLTISSLQPEDFATYYCQQHYTTPPTFGQGTKVEIK

Monospecific parental antibodies were produced, under serum-free conditions, by performing a transient cotransfection of the relevant heavy and light chain expression vectors in HEK-293F cells (Invitrogen), using 293fectin (Invitrogen), according to the manufacturer's instructions. IgG1 antibodies were purified by protein A affinity chromatography. The cell culture supernatants were filtered over a 0.20 $\mu$M dead-end filter, followed by loading on a 5 mL Protein A column (rProtein A FF, GE Healthcare, Uppsala, Sweden) and elution of the IgG with 0.1 M citric acid-NaOH, pH 3. The eluate was immediately neutralized with 2 M Tris-HCI, pH 9 and dialyzed overnight to 12.6 mM sodium phosphate, 140 mM NaCl, pH 7.4 (B. Braun, Oss, The Netherlands). After dialysis, samples were sterile filtered over a 0.20 $\mu$M dead-end filter. Concentration of the purified IgGs was determined by nephelometry and absorbance at 280 nm. Purified proteins were analyzed by SDS-PAGE, IEF, mass spectrometry and glycoanalysis.

Stable bispecific IgG1 antibodies were generated *in vitro* using a method that is based on the natural process of IgG4 Fab-arm exchange as described in WO 2008119353 (Genmab) and by van der Neut-Kolfschoten et al. (Science. 2007 Sep 14;317(5844): 1554-7). The basis for this novel method to generate bispecific IgG1 antibodies is the use of complimentary CH3 domains, which promote the formation of heterodimers under specific assay conditions (WO 2011131746). Complimentary CH3 domains were obtained by introducing T350I-K370T-F405L or, alternatively F405L, in the first, and K409R in the second of the two "parental" monospecific IgG1 molecules that are combined for the production of bispecific antibodies, according to the following procedure. In a first step, a 1:1 mixture of the two parental antibodies was incubated under mild reducing conditions. Therefore, the antibody mixture was incubated for 90 min at 37°C in 100 $\mu$L 25 mM 2-mercaptoethylamine-HCl (2-MEA) in PBS (0.5 mg/mL final concentration for each parental antibody). Due to the specific design of the homodimers, the reduced products naturally recombine to bispecific heterodimers during this reduction step. Next, the reduction reaction was stopped by removing the reducing agent 2-MEA by using Zeba desalting spin plates (7K, Thermo Fisher Scientific) according to the manufacturer's protocol. Concentrations of the bispecific samples were determined by measuring absorbance at 280 nm using a Nanodrop ND-1000 spectrophotometer (Isogen Life Science, Maarssen, The Netherlands).

**Example 21** - **HER2 x HER2 bispecific antibodies tested in an *in vitro* kappa-directed ETA' killing assay**

This example shows that HER2 x HER2 bispecific antibodies can deliver a cytotoxic agent into tumor cells after internalization in a generic *in vitro* cell-based killing assay as described in Example 18 using kappa-directed pseudomonas-exotoxin A (anti-kappa-ETA'). This assay makes use of a high affinity anti-kappa domain antibody conjugated to a truncated form of the pseudomonas-exotoxin A. Similar fusion proteins of antibody binding proteins (IgG-binding motif from Streptococcal protein A or protein G) and diphtheria toxin or Pseudomonas exotoxin A have previously been used (Mazor Y. et al., J. Immunol. Methods 2007; 321:41-59); Kuo SR. et al., 2009 Bioconjugate Chem. 2009; 20:1975-1982). These molecules in contrast to anti-kappa-ETA' bound the Fc part of complete antibodies. Upon internalization and endocytic sorting the anti-kappa-ETA' domain antibody undergoes proteolysis and disulfide-bond reduction, separating the catalytic from the binding domain. The catalytic domain is then transported from the Golgi to the endoplasmic reticulum via a KDEL retention motif, and subsequently translocated to the cytosol where it inhibits protein synthesis and induces apoptosis (Kreitman RJ. et. al., BioDrugs 2009; 23:1-13).

The bispecific antibodies were produced according to the procedure described in Example 20. The HER2 x HER2 bispecific antibodies were pre-incubated with the anti-kappa-ETA' before incubation with A431 cells. A431 cells express ~15,000 HER2 molecules per cell (determined via Qifi analysis) and are not sensitive to treatment with 'naked' HER2-antibodies.

The assay was performed as described in Example 18.

First, the optimal concentration of anti-kappa-ETA' was determined for each cell line, *i.e.* the maximally tolerated dose that does not lead to induction of non-specific cell death. A431 cells (2500 cells/well) were seeded in normal cell culture medium in a 96-wells tissue culture plate (Greiner bio-one) and allowed to adhere for at least 4 hours. These cells were incubated with an anti-kappa-ETA' dilution series, 100, 10, 1, 0.1, 0.01, 0.001 and 0 $\mu$g/mL in normal cell culture medium. After 3 days, the amount of viable cells was quantified with Alamarblue (BioSource International, San Francisco, US) according to the manufacturer's instruction. Fluorescence was monitored using the EnVision 2101 Multilabel reader (PerkinElmer, Turku, Finland) with standard Alamarblue settings. The highest concentration anti-kappa-ETA' that did not kill the cells by itself (1 $\mu$g/mL for A431 cells) was used for following experiments.

Next, the effect of HER2 x HER2 bispecific antibodies and HER2 monospecific antibodies pre-incubated with anti-kappa-ETA' was tested for their ability to induce cell kill. A431 cells were seeded as described above. A dilution series of the HER2 specific antibodies (monospecific and bispecific antibodies) was made and pre-incubated for 30 min with the predetermined concentration of anti-kappa-ETA' before adding them to the cells. After 3 days incubation at 37°C, the amount of viable cells was quantified as described above. The Alamarblue signal of cells treated with anti-kappa-ETA' pre-incubated with the antibodies was plotted compared to cells treated without antibody treatment. $EC_{50}$ values and maximal cell death were calculated using GraphPad Prism 5 software. Staurosporin (23.4 $\mu$g/mL) was used as positive control for cell killing. An isotype control antibody (IgG1/kappa; IgG1-3G8-QITL) was used as negative control.

Figure 9 and Table 9 shows that all anti-kappa-ETA' pre-incubated HER2 bispecific antibodies were able to kill A431 cells in a dose-dependent manner. These results demonstrate that the HER2 bispecific antibodies tested were comparably effective as the most effective one of the parental monospecific antibodies present in the combination in this anti-kappa-ETA' assay. In addition, the efficacy of bispecific antibody 005X169, 025X169 and 153X169 showed that the efficacy of a monospecific antibody which lacks activity in this *in vitro* kappa-directed ETA' killing, HER2 specific antibody **(169),** can be increased through bispecific combination with another HER2 specific antibody.

**Table 9:** $EC_{50}$ values and maximal percentage cell kill of AU565 cells treated with anti-kappa-ETA'-conjugated HER2 x HER2 bispecific antibodies. "Ndet" means not detected.

| antibody | percentage kill | EC50 [ng/mL] |
|---|---|---|
| Herceptin | 2.79 | Ndet |
| IgG1-005-ITL | 79.34 | 2.57 |
| IgG1-005-K409R | 79.83 | 2.87 |
| IgG1-025-ITL | 69.81 | 3.76 |
| IgG1-153-ITL | 70.66 | 12.45 |
| IgG1-153-K409R | 72.84 | 15.47 |
| IgG1-169-K409R | 16.45 | 3.45 |
| IgG1-005-ITL x IgG1-169-K409R | 59.94 | 4.28 |
| IgG1-025-ITL x IgG1-005-K409R | 63.45 | 4.27 |
| IgG1-025-ITL x IgG1-153-K409R | 80.82 | 7.66 |
| IgG1-025-ITL x IgG1-169-K409R | 45.88 | 7.97 |
| IgG1-153-ITL x IgG1-005-K409R | 80.05 | 4.51 |
| IgG1-153-ITL x IgG1-169-K409R | 84.68 | 29.14 |

**Example 22** - **HER2 receptor downmodulation by incubation with bispecific antibodies targeting different HER2 epitopes**

HER2 x HER2 bispecific antibodies may bind two different epitopes on two spatially different HER2 receptors. This may allow other HER2 x HER2 bispecific antibodies to bind to the remaining epitopes on these receptors. This could result in multivalent receptor crosslinking (compared to dimerization induced by monospecific antibodies) and consequently enhance receptor downmodulation. To investigate whether HER2 x HER2 bispecific antibodies induce enhanced downmodulation of HER2, AU565 cells were incubated with antibodies and bispecific antibodies for three days. Total levels of HER2 and levels of antibody bound HER2 were determined.

AU565 cells were seeded in a 24-well tissue culture plate (100.000 cells / well) in normal cell culture medium and cultured for three days at 37°C in the presence of 10 μg/mL HER2 antibody with either the ITL or the K409R mutation or HER2 x HER2 bispecific antibodies. As a control, the combination of two monospecific HER2 antibodies, with unmodified IgG1 backbones, was also tested (1:1), at a final concentration of 10 μg/mL. After washing with PBS, cells were lysed by incubating them for 30 min at room temperature with 25 μL Surefire Lysis buffer (Perkin Elmer, Turku, Finland). Total protein levels were quantified using bicinchoninic acid (BCA) protein assay reagent (Pierce) following manufacturer's protocol. HER2 protein levels in the lysates were analyzed using a HER2-specific sandwich ELISA. Rabbit-anti-human HER2 intracellular domain antibody (Cell Signaling) was used to capture HER2 and biotinylated goat-anti-human HER2 polyclonal antibody R&D systems, Minneapolis, USA), followed by streptavidin-poly-HRP, were used to detect bound HER2. The reaction was visualized using 2,2'-azino-bis 3-ethylbenzothiazoline-6-sulfonic acid (one ABTS tablet diluted in 50 mL ABTS buffer [Roche Diagnostics, Almere, The Netherlands]) and stopped with oxalic acid (Sigma-Aldrich, Zwijndrecht, The Netherlands). Fluorescence at 405 nm was measured on a microtiter plate reader (Biotek Instruments, Winooski, USA) and the amount of HER2 was expressed as a percentage relative to untreated cells.

The results shown in Figure 10 and Table 10 demonstrate that all the tested HER2 x HER2 bispecific antibodies induced HER2 downmodulation. Interestingly, all HER2 x HER2 bispecific antibodies demonstrated increased HER2 downmodulation compared to both of their monospecific counterparts.

**Table 10:** HER2 x HER2 bispecific induced downmodulation of HER2 depicted as percentage HER2 compared to untreated cells

| antibody | % HER2 compared to untreated cells |
|---|---|
| Herceptin | 71 |
| IgG1-005-ITL | 54 |
| IgG1-005-K409R | 50 |
| IgG1-025-ITL | 64 |
| IgG1-153-ITL | 43 |
| IgG1-153-K409R | 40 |
| IgG1-169-K409R | 64 |
| IgG1-005-ITL x IgG1-169-K409R | 29 |
| IgG1-025-ITL x IgG1-005-K409R | 38 |
| IgG1-025-ITL x IgG1-153-K409R | 29 |
| IgG1-025-ITL x IgG1-169-K409R | 34 |
| IgG1-153-ITL x IgG1-005-K409R | 23 |
| IgG1-153-ITL x IgG1-169-K409R | 28 |
| IgG1-005 + IgG1-169 | 28 |
| IgG1-025 + IgG1-005 | 28 |
| IgG1-025 + IgG1-153 | 23 |
| TgG1-025 + IgG1-169 | 25 |
| IgG1-153 + IgG1-005 | 23 |
| IgG1-153 + IgG1-169 | 23 |
| isotype control | 108 |

**Example 23** - **Colocalization of HER2 x HER2 bispecific antibodies with lysosomal marker LAMP1 analyzed by confocal microscopy**

The HER2 downmodulation as described in Example 22 indicated that HER2 x HER2 bispecific antibodies were able to increase lysosomal degradation of HER2. To confirm these findings, confocal microscopy technology was applied. AU565 cells were grown on glass coverslips (thickness 1.5 micron, Thermo Fisher Scientific, Braunschweig, Germany) in standard tissue culture medium at 37°C for 3 days. Cells were pre-incubated for 1 hour with 50 $\mu$g/mL leupeptin (Sigma) to block lysosomal activity after which 10 $\mu$g/mL HER2 monospecific antibodies or HER2 x HER2 bispecific antibodies were added. Also the combination of two monospecific IgG1 antibodies (1:1) was tested at a final concentration of 10 $\mu$g/mL. The cells were incubated for an additional 3 or 18 hours at 37°C. Hereafter the cells were washed with PBS and incubated for 30 min. at room temperature with 4% formaldehyde (Klinipath). Slides were washed with blocking buffer (PBS supplemented with 0.1% saponin [Roche] and 2% BSA [Roche]) and incubated for 20 min with blocking buffer containing 20 mM $NH_4Cl$ to quench formaldehyde. Slides were washed again with blocking buffer and incubated for 45 min at room temperature with mouse-anti-human CD107a (LAMP1) (BD Pharmingen) to stain/identify lysosomes. Following washing with blocking buffer, the slides were incubated 30 min at room temperature with a cocktail of secondary antibodies; goat-anti-mouse IgG-Cy5 (Jackson) and goat-anti-human IgG-FITC (Jackson). Slides were washed again with blocking buffer and mounted overnight on microscope slides using 20 $\mu$L mounting medium (6 gram Glycerol [Sigma] and 2.4 gram Mowiol 4-88 [Omnilabo] was dissolved in 6 mL distilled water to which 12 mL 0.2M Tris [Sigma] pH8.5 was added followed by incubation for 10 min at 50-60°C. Mounting medium was aliquoted and stored at -20°C.). Slides were imaged with a Leica SPE-II confocal microscope (Leica Microsystems) equipped with a 63x 1.32-0.6 oil immersion objective lens and LAS-AF software. To allow for quantification of overlapping pixel intensities, saturation of pixels should be avoided. Therefore the FITC laser intensity was decreased to 10%, smart gain was set at 830 V and smart offset was set at -9.48 %. By using these settings, the bispecific antibodies were clearly visualized without pixel saturation, but the monospecific antibodies were sometimes difficult to detect. To compare lysosomal colocalization between monospecific and bispecific antibodies, these settings were kept the same for all analyzed confocal slides.

12-bit grayscale TIFF images were analyzed for colocalisation using MetaMorph® software (version Meta Series 6.1, Molecular Devices Inc, Sunnyvale California, USA). FITC and Cy5 images were imported as stacks and background was subtracted. Identical thresholds settings were used (manually set) for all FITC images and all Cy5 images. Colocalisation was depicted as the pixel intensity of FITC in the region of overlap (ROI), were the ROI is composed of all Cy5 positive regions. To compare different slides stained with several HER2 antibodies, HER2 x HER2 bispecific antibodies or the combination of two different monospecific antibodies the images were normalized using the pixel intensity of Cy5. Goat-anti-mouse IgG-Cy5 was used to stain the lysosomal marker LAMP1 (CD107a). The pixel intensity of LAMP1 should not differ between various HER2 antibodies or the HER2 x HER2 bispecific antibodies tested (one cell had a pixel intensity of Cy5 of roughly 200.000).

Normalized values for colocalization of FITC and Cy5 =

$$[(\text{TPI-FITC} \times \text{percentage FITC-Cy5 colocalization})/100] \times [200.000/\text{TPI-Cy5}]$$

In this formula, TPI stands for Total Pixel Intensity.

Figure 11 and Table 11 present colocalization, as measured by the FITC pixel intensity overlapping with Cy5 for various monospecific HER2 antibodies and HER2 x HER2 bispecific antibodies. For each antibody or bispecific molecule depicted, three different images were analyzed from one slide containing ~ 1, 3 or >5 cells. Significant variation was observed between the different images within each slide. However, it was evident that all HER2 x HER2 bispecific antibodies demonstrate increased colocalisation with the lysosomal marker LAMP1, when compared with their monospecific counterparts. These results indicate that once internalized, HER2 x HER2 bispecific antibodies are efficiently sorted towards lysosomal compartments, making them suitable for a bispecific antibody drug conjugate approach.

**Table 11:** Mean FITC pixel intensities overlapping with Cy5 depicted as arbitrary units

| antibody | FITC pixel intensity in lysosomes [arbitrary units] |
|---|---|
| Herceptin | 0.218 |
| IgG1-005-ITL | 0.070 |
| IgG1-025-ITL | 0.268 |
| IgG1-153-ITL | 0.102 |
| IgG1-169-K409R | 0.220 |
| IgG1-005-ITL x IgG1-169- K409R | 0.531 |
| IgG1-025-ITL x IgG1-005-K409R | 0.347 |
| IgG1-025-ITL x IgG1-153-K409R | 0.582 |
| IgG1-025-ITL x IgG1-169- K409R | 0.439 |
| IgG1-153-ITL x IgG1-005-K409R | 0.494 |
| IgG1-153-ITL x IgG1-169-K409R | 0.604 |
| IgG1-025 + IgG1-169 | 0.576 |
| IgG1-153 + IgG1-005 | 0.636 |
| IgG1-153 + IgG1-169 | 0.626 |

**Example 24** - **Inhibition of proliferation of AU565 cells upon incubation with HER2 monospecific or HER2 X HER2 bispecific antibodies**

The HER2 x HER2 bispecific antibodies were tested for their ability to inhibit proliferation of AU565 cells *in vitro*. Due to the high HER2 expression levels on AU565 cells (~1.000.000 copies per cell as determined with Qifi-kit), HER2 is constitutively active in these cells and thus not dependent on ligand-induced heterodimerization. In a 96-wells tissue culture plate (Greiner bio-one, Frickenhausen, Germany), 9.000 AU565 cells were seeded per well in the presence of 10 μg/mL HER2 antibody or HER2 x HER2 bispecific antibodies in serum-free cell culture medium. As a control, cells were seeded in serum-free medium without antibody or bispecific antibodies. After three days, the amount of viable cells was quantified with Alamarblue (BioSource International, San Francisco, US) according to the manufacturer's instructions. Fluorescence was monitored using the EnVision 2101 Multilabel reader (PerkinElmer, Turku, Finland) with standard Alamarblue settings. The Alamarblue signal of antibody-treated cells was plotted as a percentage relative to untreated cells.

Figure 12 and Table 12 depicts the fluorescent intensity of Alamarblue of AU565 cells after incubation with HER2 antibodies and HER2XHER2 bispecific antibodies. Herceptin® (trastuzumab) was included as positive control and demonstrated inhibition of proliferation as described by Juntilla TT. et al., Cancer Cell 2009; 15: 429-440. All HER2 x HER2 bispecific antibodies were able to inhibit proliferation of AU565 cells. Bispecific antibodies: IgG1-005-ITL x IgG1-169-K409R and IgG1-025-ITL x IgG1-005-K409R were more effective compared to their monospecific antibody counterparts in this assay.

Table 12: Percentage viable AU565 cells after treatment with HER2 x HER2 bispecific antibodies.

| antibody | percentage viable cells |
|---|---|
| Herceptin | 62 |
| IgG1-005-ITL | 91 |
| IgG1-005-K409R | 96 |
| IgG1-025-ITL | 79 |
| IgG1-153-ITL | 98 |
| IgG1-153-K409R | 97 |
| IgG1-169-K409R | 63 |
| IgG1-005-ITL x IgG1-169-K409R | 49 |
| IgG1-025-ITL x IgG1-005-K409R | 61 |
| IgG1-025-ITL x IgG1-153-K409R | 74 |
| IgG1-025-ITL x IgG1-169-K409R | 76 |
| IgG1-153-ITL x IgG1-005-K409R | 71 |
| IgG1-153-ITL x IgG1-169-K409R | 77 |
| isotype control | 95 |

**Example 25** - **HER2 downmodulation**

To investigate if enhanced HER2 internalization induced by Group 3 antibodies 098 and 153 and Group 4 antibody 005 also results in enhanced receptor downmodulation, AU565 cells were incubated with HER2 antibodies for 3 days, and analyzed for presence of HER2. AU565 cells were seeded in a 24-wells tissue culture plate (100.000 cells/well) in normal cell culture medium and cultured for 3 days at 37°C in the presence of 10 $\mu$g/mL HER2 antibody. After washing with PBS, cells were lysed by incubating 30 min at room temperature with 25 $\mu$L Surefire Lysis buffer (Perkin Elmer, Turku, Finland). Total protein levels were quantified using bicinchoninic acid (BCA) protein assay reagent (Pierce) according to the manufacturer's protocol. HER2 protein levels in the lysates were analyzed using a HER2-specific sandwich ELISA. Rabbit-anti-human HER2 intracellular domain antibody (Cell Signaling) was used to capture HER2 and biotinylated goat-anti-human HER2 polyclonal antibody (R&D), followed by streptavidin-poly-HRP, were used to detect bound HER2. The reaction was visualized using 2,2'-azino-bis 3-ethylbenzothiazoline-6-sulfonic acid (ABTS: dilute one ABTS tablet in 50 mL ABTS buffer [Roche Diagnostics, Almere, The Netherlands]) and stopped with oxalic acid (Sigma-Aldrich, Zwijndrecht, The Netherlands). Fluorescence at 405 nm was measured on a microtiter plate reader (Biotek Instruments, Winooski, USA) and the amount of HER2 was expressed as a percentage relative to untreated cells.

The results shown in Figure 13 and Table 13 demonstrate that both tested Group 3 antibodies (098 and 153) induced more than 50% HER2 downmodulation. In contrast, antibodies 025, 169 and Herceptin barely induced downmodulation (approximately 20% of untreated cells) while antibody 005 induced moderate downmodulation (approximately 30% of untreated cells). This was in line with enhanced internalization observed by antibodies 098, 153 and 005.

Table 13: Antibody induced downmodulation of HER2 depicted as percentage HER2 compared to untreated cells.

| antibody | % HER2 compared to untreated cells |
|---|---|
| Herceptin | 80 |
| IgG1-1014-169 | 82 |
| IgG1-1014-025 | 85 |
| IgG1-1014-098 | 44 |
| IgG1-1014-153 | 50 |
| IgG1-1014-005 | 70 |
| isotype control | 108 |

**Example 26** - **Colocalization of HER2 antibodies with lysosomal marker LAMP1 analyzed by confocal microscopy.**

The HER2 downmodulation assay as described in Example 25 and the CypHer-5E based internalization assay as described in Example 19 indicated that HER2 antibodies from groups 3 and 4 were more efficiently internalized and targeted towards lysosomes compared to antibodies from Groups 1 and 2. To confirm the enhanced lysosomal transport of antibodies from groups 3 and 4, AU565 cells were cultured on glass coverslips and treated for 18 hours with the indicated antibodies. Cells were fixed, permeabilized and stained with FITC-conjugated goat anti-human IgG1 to visualize antibody and mouse anti-human CD107a (LAMP1) followed by goat anti-mouse IgG-Cy5 to identify lysosomes.

The results are depicted in Figure 14 and Table 14, and show that the FITC pixel intensity overlapping with Cy5 for various monospecific HER2 antibodies. From each slide three different images were analyzed containing ~ 1, 3 or >5 cells. Significant variation was observed between the different images within each slide. Still, it was evident that antibodies 005, 098 and 153 were more efficiently targeted towards lysosomal compartments, compared to 025, pertuzumab, 169 and Herceptin. This correlated well with the enhanced internalization and receptor degradation induced by these antibodies.

Table 14: Mean FITC pixel intensities overlapping with Cy5 depicted as arbitrary units

| antibody | FITC pixel intensity in lysosomes [arbitrary units] |
|---|---|
| TH1014-005 | 0.619 |
| TH1014-098 | 0.522 |
| TH1014-153 | 0.409 |
| TH1014-025 | 0.248 |
| TH1014-pert | 0.214 |
| TH1014-169 | 0.255 |
| Herceptin | 0.236 |

**Example 27** - **HER2 extracellular domain shuffle human-to-chicken**

To further define the HER2 binding regions recognized by antibodies from the four different cross-competition groups described in Example 14, a HER2 extracellular domain shuffle experiment was performed. To this end, a small gene-synthesis library with five constructs was generated, swapping the sequences of domain I, II, III or IV of the extracellular domain of human HER2 to the corresponding sequence of chicken HER2 (Gallus gallus isoform B NCBI: NP_001038126.1): 1) fully human HER2 (Uniprot P04626) hereafter named hu-HER2, 2) hu-HER2 with chicken domain I (replacing amino acids (aa) 1-203 of the human HER2 with the corresponding chicken HER2 region) hereafter named hu-HER2-ch(I), 3) hu-HER2 with chicken domain II (replacing amino acids (aa) 204-330 of the human HER2 with the corresponding chicken HER2 region) hereafter named hu-HER2-ch(II), 4) hu-HER2 with chicken domain III (replacing aa 331-507 of the human HER2 with the corresponding chicken HER2 region) hereafter named hu-HER2-ch(III) and 5) hu-HER2 with chicken domain IV (replacing aa 508-651 of the human HER2 with the corresponding chicken HER2 region) hereafter named hu-HER2-ch(IV). The human and chicken HER2 orthologs show 67% homology in their extracellular domain with 62% homology in domain I, 72% homology in domain II, 63% homology in domain III and 68% homology in domain IV. The constructs were transiently transfected in the Freestyle™ CHO-S (Invitrogen) cell line using Freestyle MAX transfection reagent (Invitrogen) according to the instructions of the manufacturer, and transfected cells were cultured for 20 hours. HER2 antibody binding to the transfected cells was analyzed by means of flow cytometry: The transfected CHO-S cells were harvested, washed with FACS buffer and incubated with 10 $\mu$g/mL HER2 antibody (30 minutes on ice). Binding of HER2 antibodies was detected using a Phycoerythrin (PE)-conjugated goat-anti-human IgG antibody (Jackson). To check if expression between different batches was the same, cells were fixed and permeabilized using Cytofix/Cytoperm solution (BD) according manufacturer's instruction and stained with a rabbit-anti-human intracellular HER2 antibody (DAKO) in combination with a secondary PE-conjugated goat-anti-rabbit antibody (Jackson). An isotype control antibody was used as negative control. Fluorescence was measured on a FACSCanto-II (BD) and binding curves were made by means of non-linear regression (sigmoidal dose-response with variable slope) using GraphPad Prism V4.03 software (GraphPad Software, San Diego, CA, USA). Loss of binding was used as read out to identify which HER2 domains were recognized by the different antibodies.

Exemplary binding curves for antibody 153 are shown in Figure 15. All binding results are shown in Table 15. Group 1 HER2 antibodies 050, 084, 169 and Herceptin showed loss of binding to Hu-HER2-ch(IV), but not to the proteins with one of the remaining domains shuffled, demonstrating that the epitopes of Group 1 mAbs reside in HER2 domain IV. Group 2 antibodies 025, 091, 129 and pertuzumab showed only loss of binding to Hu-HER2-ch(II), indicating that the epitope resides in HER2 domain II. Antibodies 098 and 153 were both defined to Group 3 in cross-competition assays (not shown) but showed some variation in the shuffle experiment. Antibody 098 clearly showed loss of binding to Hu-HER2-ch(I) and a minor decrease in binding to Hu-HER2-ch(II), while 153 showed only loss of binding to Hu-HER2-ch(II). These data suggest that Group 3 mAbs 098 and 153 can also bind, at least partially, to the HER2 domain II, with epitopes that possibly extend into HER2 domain I, as is the case for 098. Antibodies 005, 006, 060 and 111 showed loss of binding upon substitution of HER2 domain III, which demonstrated that the epitope resides in HER2 domain III. Interestingly, antibodies 059 and 106 demonstrated loss of binding to both hu-HER2-ch(III) and hu-HER2-ch(I), implying that antibodies 059 and 106 recognize a conformational epitope within these two domains.

**Table 15**: Summary of HER2 antibody binding to different HER2ECD receptor constructs. FL; hu-HER2, I; hu-HER2-ch(I), II; hu-HER2-ch(II), III; hu-HER2-ch(III), IV; hu-HER2-ch(IV). +++ indicates normal binding, ++ indicates reduced EC$_{50}$ but the similar maximal binding compared to binding observed to hu-HER2, + indicates reduced EC$_{50}$ and reduced maximal binding detected compared to binding observed to hu-HER2, - indicates no binding.

| | | HER2-domain shuffled | | | | |
|---|---|---|---|---|---|---|
| Antibody | Group | FL | I | II | III | IV |
| Herceptin | 1 | +++ | +++ | +++ | +++ | - |
| 050 | 1 | +++ | +++ | +++ | +++ | - |
| 084 | 1 | +++ | +++ | +++ | +++ | - |
| 169 | 1 | +++ | +++ | +++ | +++ | + |
| Pertuzumab | 2 | +++ | +++ | + | +++ | +++ |
| 025 | 2 | +++ | +++ | - | +++ | +++ |
| 091 | 2 | +++ | +++ | - | +++ | +++ |
| 129 | 2 | +++ | +++ | - | +++ | +++ |
| 153 | 3 | +++ | +++ | - | +++ | +++ |
| 098 | 3 | +++ | - | ++ | +++ | +++ |
| 005 | 4 | +++ | +++ | +++ | - | +++ |
| 006 | 4 | +++ | +++ | +++ | - | +++ |
| 059 | 4 | +++ | - | +++ | - | +++ |
| 060 | 4 | +++ | +++ | +++ | - | +++ |
| 106 | 4 | +++ | - | +++ | - | +++ |
| 111 | 4 | +++ | +++ | +++ | - | +++ |

**Example 28** - *In vivo* **efficacy of HER2 HuMabs 005, 091, 084 and 169 in NCI-N87 human gastric carcinoma xenografts in SCID mice**

The *in vivo* effect of HER2-HuMabs 091 (cross-competition Group 2), 084 and 169 (both cross-competition Group 1), and 005 (cross-block Group 4) on tumor growth and survival in a NCI-N87 human gastric carcinoma xenograft model in female CB.17 severe combined immunodeficiency (SCID) mice was determined. $10 \times 10^6$ NCI-N87 tumor cells in 50% matrigel were injected s.c. in female SCID mice, 10 mice per group. Eight days after tumor inoculation, intravenous treatment with HER2-HuMabs 005, 091, 084, and 169 or control antibody HuMab-HepC was started. In Figure 16 (A) and (C), this is indicated as day 1, day of treatment initiation. The first dose was at 40 mg/kg, followed by 10 mg/kg on days 4, 8, 11, 15, 18, 22, and 25 after treatment initiation. Tumor volume was determined at least 2 times per week. Volumes ($mm^3$) were calculated from caliper (PLEXX) measurements as (width$^2$ x length)/2.

The results are depicted in Figure 16A, 16B, 16C and 16D, which show that the mice administered with HuMab 005, 084, 169 and 091 demonstrated slower tumor growth (A) and better survival (B) than the mice that received negative control antibody HuMab-HepC. All treatments were well-tolerated.

**Example 29** - **Therapeutic treatment of BT-474 breast tumor xenografts in Balb/C nude mice**

The effect of therapeutic treatment of five different HER2 HuMabs on human subcutaneous BT-474 breast tumor xenografts in Balb/C nude mice was determined. BT-474 tumor cells were injected 24 to 72 hours after a whole body irradiation with a γ-source (1.8 Gy, Co60, BioMep, France). $2 \times 10^7$ BT-474 cells in 200 μl of RPMI 1640 containing matrigel (50:50, v:v; BD Biosciences) were injected subcutaneously into the right flank of female Balb/C nude mice. Body weight and tumor volume of the mice was recorded twice a week. Tumor volumes ($mm^3$) were calculated from caliper (PLEXX) measurements as: (width$^2$ × length)/2.

Treatment with HER2 HuMabs was started when the tumors reached a mean volume of 100-200 mm3. Tumor bearing mice were randomized into groups of 8 mice. One group received twice weekly intravenous (i.v.) injections of the control mAb HuMab-HepC. Four other groups received twice weekly i.v. injections of HER2 HuMab 025, 129, 153 and 091, with a first dose of 20 mg/kg and following 9 doses of 5 mg/kg.

The results are depicted in Figure 17A and 17B and show that BT-474 tumor growth was partially inhibited with HuMab 129 and HuMab 153 treatment (about 30 and 50% of inhibition compared to HuMab-HepC control treatment). HuMab-025 and HuMab-091 strongly inhibited the BT-474 tumor growth and the time to reach a tumor volume of 800 mm$^3$ was significantly delayed by these antibodies. Survival was also improved in the HER2 HuMab receiving mice.

**Example 30** - **Downmodulation of HER2 surface expression by incubation with bispecific antibodies targeting different HER2 epitopes**

Multivalent receptor crosslinking by bispecific HER2 × HER2 antibodies can result in enhanced receptor downmodulation. In this Example, the effect of such bispecific antibodies on the expression of the receptor on the cell surface of SKOV3 cells was analyzed. SKOV3 (ATCC) is an ovarian carcinoma cell line that has ~2 × 10e5 HER2 copy numbers per cell.

SKOV3 cells were seeded in 96-wells non-binding plates (100,000 cells/well) in serum-free culture medium and incubated for 30 min at 37°C. Next, cells were incubated for 3 hours at 37°C with 10 μg/mL antibody, with or without 100 μM monensin (Dako), which blocks receptor recycling. The combination of two monospecific IgG1 antibodies (1:1) was also tested (10 μg/mL final total antibody concentration). Quantification of cell surface expressed HER2 molecules was done by indirect immunofluorescence staining and flow cytometry using QIFIKIT® (Dako) according to the manufacturer's instructions. Briefly, cells were incubated with a non-competing mouse anti-HER2 antibody (R&D, cat: IBD0207061, 1:50) for 30 min at 4°C. Next, detection was done using goat anti-mouse IgG-FITC (Dako, cat: F0479, 1:50) for 30 min at 4°C. Mean Fluorescence Intensity (MFI) of FITC was measured using flow cytometry on a FACS-Canto-II (BD Pharmingen). For calibration, the goat anti-mouse IgG-FITC detection antibody was applied to a series of bead populations with a defined number of mouse IgG molecules per bead. The measured MFI of the individual bead populations were plotted against the known number of antibody molecules on the beads to obtain a calibration curve that was used to interpolate the number of HER2 molecules per cell from the measured MFI.

Figure 18 and Table 16 shows that monospecific HER2 antibodies have no significant effect on the HER2 surface expression in SKOV3 cells on their own. However, treatment with a combination of two monospecific HER2 antibodies or HER2 × HER2 bispecific antibodies clearly reduced the amount of surface-expressed HER2. The addition of monensin resulted for all samples in only a minor decrease in surface-expressed HER2 levels compared to those without monensin. This suggests that the majority of internalized HER2 molecules is intracellularly degraded, rather than recycled.

Table 16:

| | - monensin | + monensin |
|---|---|---|
| untreated | 169923 | 135108 |
| IgG1-b12 | 153217 | 132255 |
| IgG1-005 | 150486 | 122154 |
| IgG1-025 | 136833 | 121230 |
| IgG1-153 | 161516 | 102301 |
| IgG1-005 + IgG1-025 | 87435 | 67858 |
| IgG1-005 + IgG1-153 | 96817 | 66815 |
| IgG1-025 + IgG1-153 | 100981 | 77325 |
| IgG1-025-ITL × IgG1-005-K409R | 114429 | 103408 |
| IgG1-025-ITL × IgG1-153-K409R | 95912 | 63770 |
| IgG1-153-ITL × IgG1-005-K409R | 83028 | 62407 |

**Example 31: Induction of PBMC-mediated cytotoxicity by bispecific HER2×HER2 antibodies**

HER2xHER2 antibodies were tested in an *in vitro* cytotoxicity assay using AU565 cells with Peripheral blood mononuclear cells (PBMC) as effector cells, and compared to their parental monospecific HER2 antibodies and the combination thereof. AU565 cells were cultured to near confluency. Cells were washed twice with PBS, and trypsinized for 5 minutes at 37°C. 12 mL culture medium was added to inactivate trypsin and cells were spun down for 5 min, 800 rpm. Cells were resuspended in 10 mL culture medium and a single cell suspension was made by passing the cells through a cell strainer. 100 μL of a 5×105 cells/mL suspension was added to each well of a 96-wells culture plate, and cells were incubated for 3 hrs at 37°C, 5% CO2 to allow adherence to the plate. PBMCs were isolated from a buffy coat from healthy volunteers using Leucosep 30 mL tubes, according to the manufacturer's protocol (Greiner Bio-one). Isolated cells were resuspended in culture medium to a final concentration op 10×10$^6$ cells/mL. Culture medium was removed from the adhered AU565 cells, and replaced with 50 μL/well 2x concentrated antibody-dilution and 50 μL/well of the $10{\times}10^6$/mL PBMC suspension. Plates were incubated for 3 days at 37°C, 5% CO2. Supernatants were removed and plates were washed twice with PBS. To each well, 150 μL culture medium and 15 μL alamarBlue solution was added. Plates were incubated for 4 hours at 37°C, 5% CO2, and absorbance was measured (Envision, Perkin Elmer).

Figure 19 shows that the ability of the monospecific HER2 antibodies to induce PBMC-mediated cytotoxicity was retained in bispecific HER2 × HER2 antibodies. The efficacy of the HER2 × HER2 bispecific antibodies was comparable (025-ITLx169-K409R; 005-ITLx169-K409R; 025-ITLx005-K409R) or tended to be better (153-ITLx005-K409R and 153-ITLx169-K409R) than for the monospecific parental antibodies or the combination thereof. Moreover, it was shown that it was required that both parental antibodies that are used to generate a HER2 × HER2 antibody need to contain activating Fc-domains, *i.e.* proper interaction with Fc-receptors, to be able to induce PBMC-mediated cytotoxicity in the HER2 × HER2 antibody. Antibody Fc glycosylation is known to be critical for IgG-Fcγ receptor interactions and thus antibody-dependent cellular cytotoxicity (ADCC). Deglycosylation by introduction of the N297Q mutation in IgG1-153-K409R resulted in loss of PBMC-mediated cytotoxicity of both IgG1-153-K409R-N297Q and IgG1-153-ITLxIgG1-153-K409R-N297Q.

**Example 32: *In vivo* efficacy of HER2 × HER2 bispecific antibodies in NCI-N87 human gastric carcinoma xenografts in SCID mice**

The *in vivo* anti-tumor efficacy of HER2 × HER2 bispecific antibodies was compared to that of the corresponding parental monospecific HER2 antibodies and combinations thereof in a human gastric carcinoma NCI-N87 xenograft tumor model in SCID mice. Six to eleven weeks old female SCID (C.B-17/IcrPrkdc-scid/CRL) mice were used. At day 0, $5{\times}10^6$ NCI-N87 cells were inoculated subcutaneously in 200 μL in the right flank of each mouse. Seven days after tumor inoculation, the animals were sorted into eight groups (n=7) with comparable average tumor size and treatment was started. Saturating doses of antibodies (HER2 monospecific antibody, HER2 × HER2 bispecific antibody or a combination of two HER2 monospecific antibodies) were injected intra peritoneal (i.p.). IgG1-b12 was used as an isotype control antibody. Mice were treated on days 7, 14 and 21 after tumor inoculation with the doses indicated in Table 17.

**Table 17**

|  | 1st dose | 2nd dose | 3th dose |
|---|---|---|---|
| IgG1-005 | 800 μg | 400 μg | 200 μg |
| IgG1-153 | 1000 μg | 500 μg | 250 μg |
| IgG1-169 | 600 μg | 300 μg | 150 μg |
| IgG1-169 + IgG1-153 | 300 μg (169) + 500 μg (153) | 150 μg (169) + 250 μg (153) | 75 μg (169) + 125 μg (153) |
| IgG1-005 + IgG1-153 | 400 μg (005) + 500 μg (153) | 200 μg (005) + 250 μg (153) | 100 μg (005) + 125 μg (153) |
| IgG1-153-ITL × IgG1-169-K409R | 800 μg | 400 μg | 200 μg |
| IgG1-005-ITL × IgG1-153-K409R | 800 μg | 400 μg | 200 μg |
| IgG1-b12 | 800 μg | 400 μg | 200 μg |

Tumors were measured twice per week using calipers until an endpoint tumor volume of 1500 mm3 or until the end of the study (day 63). Figure 20 shows that on day 41 of the experiment, none of the monospecific HER2 antibodies significantly inhibited tumor growth compared to negative control antibody b12, with IgG1-005 and IgG1-153 even showing a trend towards being agonistic in this model. Both tested bispecific HER2 × HER2 antibodies IgG1-153-ITL × IgG1-169-K409R and IgG1-005-ITL × IgG1-153-K409R showed significant inhibition of tumor growth compared to their monospecific counterparts. Moreover, both bispecific HER2 × HER2 antibodies showed on day 41 a lower mean tumor volume than the combination of the monospecific counterparts, which was for IgG1-153-ITL × IgG1-169-K409R statistically significant.

**Example 33: *In vivo* efficacy of Her2 × Her2 bispecific antibodies in NCI-N87 human gastric carcinoma xenografts in SCID mice.**

The *in vivo* anti-tumor efficacy of the Her2 × Her2 bispecific antibody IgG1-153-ITL × IgG1-169-K409R was tested in a human gastric carcinoma NCI-N87 xenograft tumor model in SCID mice as described in Example 32. At day 0, $5 \times 10^6$ NCI-N87 cells were inoculated s.c. in 200 μL in the right flank of each mouse. Seven days after tumor inoculation, the animals were sorted into groups (n=9) with comparable average tumor size. Mice were treated by intra peritoneal injection of saturating antibody doses on days 7 and 14 after tumor inoculation. Treatment groups are shown in Table 18.

Table 18: Treatment groups and dosing

|  | 1st dose | 2nd dose |
|---|---|---|
| IgG1-153-K409R | 800 μg (40 mg/kg) | 400 μg (20 mg/kg) |
| IgG1-169 | 800 μg (40 mg/kg)) | 400 μg (20 mg/kg) |
| IgG1-169 + IgG1-153 | 320 μg 153 (16 mg/kg) + 400 μg 169 (20 mg/kg) | 130 μg 153 (8 mg/kg) + 200 μg 169 (10 mg/kg) |
| IgG1-153-ITL × IgG1-169-K409R | 800 μg (40 mg/kg) | 400 μg (20 mg/kg) |
| Herceptin | 800 μg (40 mg/kg) | 400 μg (20 mg/kg) |
| IgG1-b12 | 800 μg (40 mg/kg) | 400 μg (20 mg/kg) |

Tumors were measured twice per week using calipers until an endpoint tumor volume of 1500 mm$^3$ or until the end of the study. Figure 21A shows that none of the monospecific HER2 antibodies inhibited tumor growth significantly compared to negative control antibody b12. A significant inhibition of tumor growth was found for the bispecific HER2 × HER2 antibody IgG1-153-ITL × IgG1-169-K409R compared to the isotype control antibody b12. Figure 21B shows a Kaplan-Meier plot displaying the percentage of mice with tumors <400 mm$^3$. The group treated with the HER2xHER2 bispecific IgG1-153-ITL × IgG1-169-K409R antibody shows signifcant tumor inhibition compared to the control and all other groups.

**Example 34: Unraveling the requirement of the T350I, K370T and F405L substitutions for Fab-arm exchange engagement of human IgG1**

To further identify the determinants in the IgG1 CH3 domain that are required for IgG1 to be engaged in Fab-arm exchange, IgG1 containing the triple mutation T350I-K370T-F405L (ITL) was compared to the double mutants T350I-K370T (IT), T350I-F405L (IL) and K370T-F405L (TL) were studied using antibodies 2F8 and 7D8, respectively described in WO 02/100348 and WO 04/035607. Also the single mutant F405L (L) was tested. 2-MEA was used as a reductant to induce *in vitro* Fab-arm exchange (50 μg of each antibody in 100 μL PBS/25 mM 2-MEA for 90 min at 37°C). For the single mutant F405L antibody, unpurified antibody from supernatant of a transient transfection was used after buffer-exchange to PBS using Amicon Ultra centrifugal devices (30k, Millipore, cat. no. UFC803096). To stop the reduction reaction, the reducing agent 2-MEA was removed by desalting the samples using spin columns. The generation of bispecific antibodies was determined by bispecific binding measured in an ELISA.

The triple (ITL), double mutations (IT, IL and TL) and single mutation (L) were introduced in IgG1-2F8. These mutants were combined with IgG4-7D8, containing a CPSC hinge (wild type) or a stabilized hinge (IgG4-7D8-CPPC), for Fab-arm exchange using 25 mM 2-MEA for 90 min at 37°C. Figures 22A-B show that the IgG1-2F8-IL and -TL mutants showed Fab-arm exchange to the same level as the triple mutant ITL, irrespective of the combined IgG4-7D8 (CPSC or CPPC hinge). In contrast, no bispecific binding was found for the combination with the IgG1-2F8-IT mutant. Figure 22C shows that also the IgG1-2F8-F405L mutant showed Fab-arm exchange, irrespective of the combined IgG4-7D8 (CPSC or CPPC hinge). These data indicate that the F405L mutation is sufficient to engage human IgG1 for Fab-arm exchange under the conditions mentioned above.

**Example 35: Determinants at the IgG1 409 position for engagement in 2-MEA-induced Fab-arm exchange in combination with IgG1-ITL**

2-MEA can induce Fab-arm exchange between human IgG1-ITL and IgG4-CPPC. The CH3 interface residues of human IgG1 and IgG4 differ at position 409 only: lysine (K) in IgG1 and arginine (R) in IgG4. Therefore, it was tested whether substitution of lysine at position 409 by arginine or any other amino acid (K409X) could enable IgG1 to engage in 2-MEA-induced Fab-arm exchange with IgG1-ITL. Combinations of 10 μg human IgG1-2F8-ITL and 10 μg IgG1-7D8-K409X in 20 μl PBS/25 mM 2-MEA (final concentration of 0.5 mg/mL for each antibody) were incubated for 90 min at 37°C. Unpurified antibodies from supernatants of transient transfections were used after buffer-exchange to PBS using Amicon Ultra centrifugal devices (30k, Millipore, cat. no. UFC803096). After the Fab-arm exchange reaction, 20 μL PBS was added to each sample and the reducing agent was removed by desalting the samples using spin desalting plate. Dilution series of the antibody samples (total antibody concentration 0-20 μg/mL in 3-fold dilutions) were used in an ELISA to measure bispecific binding.

Figure 23A shows the results of bispecific binding upon 2-MEA induced Fab-arm exchange between IgG1-2F8-ITL × IgG1-7D8-K409X. In Figure 23B, the exchange is presented as bispecific binding relative to a purified batch of bispecific antibody derived from a 2-MEA-induced Fab-arm-exchange between IgG1-2F8-ITL and IgG4-7D8-CPPC, which was set to 100%. These data were also scored as (-) no Fab-arm exchange, (+/-) low, (+) intermediate or (++) high Fab-arm exchange, as presented in Table 19. No Fab-arm exchange (-) was found when the 409 position in IgG1-7D8 was K (=wild type IgG1), L or M. Fab-arm exchange was found to be intermediate (+) when the 409 position in IgG1-7D8 was F, I, N or Y and high (++) when the 409 position in IgG1-7D8 was A, D, E, G, H, Q, R, S, T, V or W.

**Table 19:** 2-MEA-induced Fab-arm exchange between IgG1-2F8-ITL and IgG1-7D8-K409X mutants. The generation of bispecific antibodies after 2-MEA-induced *in vitro* Fab-arm exchange between IgG1-2F8-ITL and IgG1-7D8-K409X mutants was determined by a sandwich ELISA. (-) no, (+/-) low, (+) intermediate, (++) high Fab-arm exchange.

|  | Fab-arm exchange |
| --- | --- |
| IgG1-7D8-K409X | x IgG1-2F8-ITL |
| A | ++ |
| D | ++ |
| E | ++ |
| F | + |
| G | ++ |
| H | ++ |
| I | + |
| K | - |
| L | - |
| M | - |
| N | + |
| Q | ++ |
| R | ++ |
| S | ++ |
| T | ++ |
| V | ++ |
| W | ++ |
| Y | + |

**Example 36: Determinants at the IgG1 405 position for engagement in 2-MEA-induced Fab-arm-exchange in combination with IgG1-K409R**

In Example 34 it is described that the F405L mutation is sufficient to enable human IgG1 to engage in Fab-arm-exchange when combined with IgG4-7D8. To further test the determinants at the IgG1 405 position for engagement in 2-MEA-induced Fab-arm-exchange in combination with human IgG1-K409R, all possible IgG1-2F8-F405X mutants (with the exception of C and P) were combined with IgG1-7D8-K409R. The procedure was performed with purified antibodies as described in Example 35.

Figure 24 shows the results of bispecific binding upon 2-MEA-induced Fab-arm-exchange between IgG1-2F8-F405X × IgG1-7D8-K409R. These data were also scored as (-) no Fab-arm exchange, (+/-) low, (+) intermediate or (++) high Fab-arm exchange, as presented in Table 20. No Fab-arm exchange (-) was found when the 405 position in IgG1-2F8 was F (= wild type IgG1). Fab-arm exchange was found to be low (+/-) when the 405 position in IgG1-2F8 was G or R. Fab-arm exchange was found to be high (++) when the 405 position in IgG1-2F8 was A, D, E, H, I, K, L, M, N, Q, S, T, V, W or Y. These data indicate that particular mutations at the IgG1 405 position allow IgG1 to engage in 2-MEA-induced Fab-arm-exchange when combined with IgG1-K409R.

**Table 20:** 2-MEA-induced Fab-arm-exchange between IgG1-2F8-F405X mutants and IgG1-7D8-K409R. The generation of bispecific antibodies after 2-MEA-induced *in vitro* Fab-arm-exchange between IgG1-2F8-F405X mutants and IgG1-7D8-K409R was determined by a sandwich ELISA. (-) no, (+/-) low, (+) intermediate, (++) high Fab-arm-exchange.

| IgG1-2F8-F405X | Fab-arm-exchange x IgG1-7D8-K409R |
|:---:|:---:|
| A | ++ |
| D | ++ |
| E | ++ |
| F | - |
| G | +/- |
| H | ++ |
| I | ++ |
| K | ++ |
| L | ++ |
| M | ++ |
| N | ++ |
| Q | ++ |
| R | +/- |
| S | ++ |
| T | ++ |
| V | ++ |
| W | ++ |
| Y | ++ |

**Example 37: Determinants at the IgG1 407 position for engagement in 2-MEA-induced Fab-arm-exchange in combination with IgG1-K409R**

In the previous Example, it is described that certain single mutations at position F405 are sufficient to enable human IgG1 to engage in Fab-arm-exchange when combined with IgG1-K409R. To test whether other determinants implicated in the Fc:Fc interface positions in the CH3 domain could also mediate the Fab-arm-exchange mechanism, mutagenesis of the IgG1 407 position was performed and the mutants were tested for engagement in 2-MEA-induced Fab-arm-exchange in combination with human IgG1-K409R. All possible IgG1-2F8-Y407X mutants (with the exception of C and P) were combined with IgG1-7D8-K409R. The procedure was performed with purified antibodies.

Figure 25 shows the results of bispecific binding upon 2-MEA-induced Fab-arm-exchange between IgG1-2F8-Y407X × IgG1-7D8-K409R. These data were also scored as (-) no Fab-arm exchange, (+/-) low, (+) intermediate or (++) high Fab-arm exchange, as presented in Table 21. No Fab-arm exchange (-) was found when the 407 position in IgG1-2F8 was Y (= wild type IgG1), E, K, Q, or R. Fab-arm exchange was found to be low (+/-) when the 407 position in IgG1-2F8 was D, F, I, S or T and intermediate (+) when the 407 position in IgG1-2F8 was A, H, N or V, and high (++) when the 407 position in IgG1-2F8 was G, L, M or W. These data indicate that particular single mutations at the IgG1 407 position allow IgG1 to engage in 2-MEA-induced Fab-arm-exchange when combined with IgG1-K409R.

**Table 21:** 2-MEA-induced Fab-arm-exchange between IgG1-2F8-Y407X mutants and IgG1-7D8-K409R. The generation of bispecific antibodies after 2-MEA-induced *in vitro* Fab-arm exchange between IgG1-2F8-Y407X mutants and IgG1-7D8-K409R was determined by a sandwich ELISA. (-) no, (+/-) low, (+) intermediate, (++) high Fab-arm-exchange.

| IgG1-2F8-Y407X | Fab-arm-exchange x IgG1-7D8-K409R |
|---|---|
| A | + |
| D | +/- |
| E | - |
| F | +/- |
| G | ++ |
| H | + |
| I | +/- |
| K | - |
| L | ++ |
| M | ++ |
| N | + |
| Q | - |
| R | - |
| S | +/- |
| T | +/- |
| V | + |
| W | ++ |
| Y | - |

**Example 38: Determinants at the IgG1 368 position for engagement in 2-MEA-induced Fab-arm exchange in combination with IgG1-K409R**

Examples 34 and 37 show that certain single mutations at position F405 and Y407 are sufficient to enable human IgG1 to engage in Fab-arm exchange when combined with IgG1-K409R. As illustrated in this example further determinants implicated in the Fc:Fc interface positions in the CH3 domain may also mediate the Fab-arm exchange mechanism. To this effect mutagenesis of the IgG1 368 position was performed and the mutants were tested for engagement in 2-MEA-induced Fab-arm-exchange in combination with human IgG1-K409R. All possible IgG1-2F8-L368X mutants (with the exception of C and P) were combined with IgG1-7D8-K409R. The procedure was performed with purified antibodies.

Figure 26 shows the results of bispecific binding upon 2-MEA-induced Fab-arm exchange between IgG1-2F8-L368X × IgG1-7D8-K409R. These data were also scored as (-) no Fab-arm exchange, (+/-) low, (+) intermediate or (++) high Fab-arm exchange, as presented in Table 22. No Fab-arm exchange (-) was found when the 368 position in IgG1-2F8 was L (= wild type IgG1), For M. Fab-arm exchange was found to be low (+/-) when the 368 position in IgG1-2F8 was Y. Fab-arm exchange was found to be intermediate (+) when the 368 position in IgG1-2F8 was K and high (++) when the 368 position in IgG1-2F8 was A, D, E, G, H, I, N, Q, R, S, T, V, or W. These data indicate that particular mutations at the IgG1 368 position allow IgG1 to engage in 2-MEA-induced Fab-arm exchange when combined with IgG1-K409R.

**Table 22:** 2-MEA-induced Fab-arm exchange between IgG1-2F8-L368X mutants and IgG1-7D8-K409R. The generation of bispecific antibodies after 2-MEA-induced *in vitro* Fab-arm exchange between IgG1-2F8-L368X mutants and IgG1-7D8-K409R was determined by a sandwich ELISA. (-) no, (+/-) low, (+) intermediate or (++) high Fab-arm exchange.

| | Fab-arm exchange |
|---|---|
| IgG1-2F8-L368X | x IgG1-7D8-K409R |
| A | ++ |
| D | ++ |
| E | ++ |
| F | - |
| G | ++ |
| H | ++ |
| I | ++ |
| K | + |
| L | - |
| M | - |
| N | ++ |
| Q | ++ |
| R | ++ |
| S | ++ |
| T | ++ |
| V | ++ |
| W | ++ |

**Example 39: Determinants at the IgG1 370 position for engagement in 2-MEA-induced Fab-arm exchange in combination with IgG1-K409R**

Examples 34, 37 and 38 show that certain single mutations at positions F405, Y407 or L368 are sufficient to enable human IgG1 to engage in Fab-arm exchange when combined with IgG1-K409R. As illustrated in this example further determinants implicated in the Fc:Fc interface positions in the CH3 domain may also mediate the Fab-arm exchange mechanism. To this effect mutagenesis of the IgG1 370 position was performed and the mutants were tested for engagement in 2-MEA-induced Fab-arm-exchange in combination with human IgG1-K409R. All possible IgG1-2F8-K370X mutants (with the exception of C and P) were combined with IgG1-7D8-K409R. The procedure was performed with purified antibodies.

Figure 27 shows the results of bispecific binding upon 2-MEA-induced Fab-arm exchange between IgG1-2F8-K370X × IgG1-7D8-K409R. These data were also scored as (-) no Fab-arm exchange, (+/-) low, (+) intermediate or (++) high Fab-arm exchange, as presented in Table 23. No Fab-arm exchange (-) was found when the 370 position in IgG1-2F8 was K (= wild type IgG1), A, D, E, F, G, H, I, L, M, N, Q, R, S, T, V or Y. Only substitution of K370 with W resulted in intermediate Fab-arm exchange (+). These data indicate that only one mutation at the IgG1 370 position (K370W) allows IgG1 to engage in 2-MEA-induced Fab-arm exchange when combined with IgG1-K409R.

**Table 23:** 2-MEA-induced Fab-arm exchange between IgG1-2F8-K370X mutants and IgG1-7D8-K409R. The generation of bispecific antibodies after 2-MEA-induced *in vitro* Fab-arm exchange between IgG1-2F8-K370X mutants and IgG1-7D8-K409R was determined by a sandwich ELISA. (-) no, (+/-) low, (+) intermediate or (++) high Fab-arm exchange.

|  | Fab-arm exchange |
|---|---|
| IgG1-2F8-K370X | x IgG1-7D8-K409R |
| A | - |
| D | - |
| E | - |
| F | - |
| G | - |
| H | - |
| I | - |
| K | - |
| L | - |
| M | - |
| N | - |
| Q | - |
| R | - |
| S | - |

| T | - |
|---|---|
| V | - |
| W | + |
| Y | - |

### Example 40: Determinants at the IgG1 399 position for engagement in 2-MEA-induced Fab-arm exchange in combination with IgG1-K409R

The preceding Examples show that certain single mutations at positions F405, Y407, L368 or K370 are sufficient to enable human IgG1 to engage in Fab-arm exchange when combined with IgG1-K409R. As illustrated in this example further determinants implicated in the Fc:Fc interface positions in the CH3 domain may also mediate the Fab-arm exchange mechanism. To this effect mutagenesis of the IgG1 399 position was performed and the mutants were tested for engagement in 2-MEA-induced Fab-arm-exchange in combination with human IgG1-K409R. All possible IgG1-2F8-D399X mutants (with the exception of C and P) were combined with IgG1-7D8-K409R. The procedure was performed with purified antibodies as described in Example 35.

Figure 28 shows the results of bispecific binding upon 2-MEA-induced Fab-arm exchange between IgG1-2F8-D399X × IgG1-7D8-K409R. These data were also scored as (-) no, (+/-) low, (+) intermediate or (++) high Fab-arm exchange, as presented in Table 24. No Fab-arm exchange (-) was found when the 399 position in IgG1-2F8 was D (= wild type IgG1), E and Q. Fab-arm exchange was found to be low (+/-) when the 399 position in IgG1-2F8 was V, intermediate (+) when the 399 position in IgG1-2F8 was G, I, L, M, N, S, T or W. Fab-arm exchange was found to be high (++) when the 399 position in IgG1-2F8 was A, F, H, K, R or Y. These data indicate that particular mutations at the IgG1 399 position allow IgG1 to engage in 2-MEA-induced Fab-arm exchange when combined with IgG1-K409R.

**Table 24**: 2-MEA-induced Fab-arm exchange between IgG1-2F8-D399X mutants and IgG1-7D8-K409R. The generation of bispecific antibodies after 2-MEA-induced *in vitro* Fab-arm exchange between IgG1-2F8-D399X mutants and IgG1-7D8-K409R was determined by a sandwich ELISA. (-) no, (+/-) low, (+) intermediate or (++) high Fab-arm exchange.

| IgG1-2F8-D399X | Fab-arm exchange x IgG1-7D8-K409R |
|---|---|
| A | ++ |
| D | - |
| E | - |
| F | ++ |

| | |
|---|---|
| G | + |
| H | ++ |
| I | + |
| K | ++ |
| L | + |
| M | + |
| N | + |
| Q | - |
| R | ++ |
| S | + |
| T | + |
| V | +/- |
| W | + |
| Y | ++ |

**Example 41: Determinants at the IgG1 366 position for engagement in 2-MEA-induced Fab-arm exchange in combination with IgG1-K409R**

Examples 34 to 40 show that certain single mutations at positions F405, Y407, L368, K370 or D399 are sufficient to enable human IgG1 to engage in Fab-arm exchange when combined with IgG1-K409R. As illustrated in this example further determinants implicated in the Fc:Fc interface positions in the CH3 domain may also mediate the Fab-arm exchange mechanism. To this effect mutagenesis of the IgG1 366 position was performed and the mutants were tested for engagement in 2-MEA-induced Fab-arm-exchange in combination with human IgG1-K409R. All possible IgG1-2F8-T366X mutants (with the exception of C and P) were combined with IgG1-7D8-K409R. The procedure was performed with purified antibodies as described in Example 35.

Figure 29 shows the results of bispecific binding upon 2-MEA-induced Fab-arm exchange between IgG1-2F8-T366X × IgG1-7D8-K409R. These data were also scored as (-) no, (+/-) low, (+) intermediate or (++) high Fab-arm exchange, as presented in Table 25. No Fab-arm exchange (-) was found when the 366 position in IgG1-2F8 was T (= wild type IgG1), K, R, S or W. Fab-arm exchange was found to be low (+/-) when the 366 position in IgG1-2F8 was F, G, I, L, M or Y, intermediate (+) when the 366 position in IgG1-2F8 was A, D, E, H, N, V or Q. These data indicate that particular mutations at the IgG1 366 position allow IgG1 to engage in 2-MEA-induced Fab-arm exchange when combined with IgG1-K409R.

**Table 25: 2-MEA-induced Fab-arm exchange between IgG1-2F8-T366X mutants and IgG1-7D8-K409R**

The generation of bispecific antibodies after 2-MEA-induced *in vitro* Fab-arm exchange between IgG1-2F8-T366X mutants and IgG1-7D8-K409R was determined by a sandwich ELISA. (-) no, (+/-) low, (+) intermediate or (++) high Fab-arm exchange.

| IgG1-2F8-T366X | Fab-arm exchange x IgG1-7D8-K409R |
|---|---|
| A | + |
| D | + |
| E | + |
| F | +/- |
| G | +/- |
| H | + |
| I | +/- |
| K | - |
| L | +/- |
| M | +/- |
| N | + |
| Q | + |
| R | - |
| S | - |
| T | - |
| V | + |
| W | - |
| Y | +/- |

**Claims**

1. A bispecific antibody comprising a first antigen-binding region and a second antigen-binding region, which first and second antigen-binding regions bind different epitopes on human epidermal growth factor receptor 2 (HER2), and wherein the first antigen-binding region is selected from the group consisting of:

   a) an antibody comprising a VH region comprising the sequence of SEQ ID NO:63 and a VL region comprising the sequence of SEQ ID NO:67 (**153**),
   b) an antibody comprising a variable heavy (VH) region comprising the sequence of SEQ ID NO: 165 and a variable light (VL) region comprising the sequence of SEQ ID NO: 169 (**005**), and
   c) an antibody comprising a VH region comprising the sequence of SEQ ID NO:22 and a VL region comprising the sequence of SEQ ID NO:26 (**025**),

   wherein the second antigen binding region comprises a VH region comprising SEQ ID NO:1, and a VL region comprising SEQ ID NO:5 (**169**).

2. The bispecific antibody of claim 1, wherein said bispecific antibody further comprises a first Fc region and a second Fc region.

3. The bispecific antibody of any one of the preceding claims comprising a first Fab-arm comprising the first antigen-binding region and a first Fc-region, and a second Fab-arm comprising the second antigen-binding region and a second Fc-region.

4. The bispecific antibody of any one of claims 1 to 2 comprising a first Fab-arm comprising the second antigen-binding region and a first Fc-region, and a second Fab-arm comprising the first antigen-binding region and a second Fc-region.

5. The bispecific antibody of any one of claims 2 to 4, wherein the isotypes of the first and second Fab-arms are independently selected from IgG1, IgG2, IgG3, and IgG4.

6. The bispecific antibody of claim 5, wherein the isotypes of the first and second Fc-regions are independently selected from IgG1 and IgG4.

7. The bispecific antibody of claim 6, wherein one of the first and second Fc-regions is of an IgG1 isotype and one is of an IgG4 isotype.

8. The bispecific antibody of claim 6, wherein the isotypes of the first and second Fc-region are of IgG1 isotype.

9. The bispecific antibody of any one of claims 2 to 8, wherein the first Fc-region has an amino acid substitution at a position selected from the group consisting of 409, 366, 368, 370, 399, 405 and 409, and said second Fc-region has an amino acid substitution at a position selected from the group consisting of 405, 366, 368, 370, 399, 407 and 409, and wherein said first Fc-region and said second Fc-region are not substituted in the same positions, and wherein said amino acid positions are according to the Eu-index as described in Kabat et al.

10. The bispecific antibody of any one of claims 2 to 9, wherein

a) the first Fc-region has an amino acid other than Lys, Leu or Met at position 409 and the second Fc region has an amino acid other than Phe at position 405;
b) the first Fc-region has an amino acid other than Lys, Leu or Met at position 409 and the second Fc-region has an amino acid other than Phe, Arg or Gly at position 405;
c) the first Fc-region comprises a Phe at position 405 and an amino acid other than Lys, Leu or Met at position 409 and said second Fc-region comprises an amino acid other than Phe at position 405 and a Lys at position 409;
d) the first Fc-region comprises a Phe at position 405 and an amino acid other than Lys, Leu or Met at position 409 and the second Fc-region comprises an amino acid other than Phe, Arg or Gly at position 405 and a Lys at position 409;
e) the first Fc-region comprises a Phe at position 405 and an amino acid other than Lys, Leu or Met at position 409 and the second Fc-region comprises a Leu at position 405 and a Lys at position 409;
f) the first Fc-region comprises a Phe at position 405 and an Arg at position 409 and said second Fc-region comprises an amino acid other than Phe, Arg or Gly at position 405 and a Lys at position 409;
g) the first Fc-region comprises Phe at position 405 and an Arg at position 409 and the second Fc-region comprises a Leu at position 405 and a Lys at position 409;
h) the first Fc-region comprises an amino acid other than Lys, Leu or Met at position 409 and the second Fc-region comprises a Lys at position 409, a Thr at position 370 and a Leu at position 405;
i) the first Fc-region comprises an Arg at position 409 and the second Fc-region comprises a Lys at position 409, a Thr at position 370 and a Leu at position 405; or
j) the first Fc-region comprises a Lys at position 370, a Phe at position 405 and an Arg at position 409 and the second Fc-region comprises a Lys at position 409, a Thr at position 370 and a Leu at position 405.

11. The bispecific antibody of any one of claims 2 to 9, wherein said first and second Fc regions, except for the specified mutations, comprise the sequence of SEQ ID NO:236 (IgG1m(a)).

12. The bispecific antibody of any one of claims 2 to 9, wherein neither said first nor said second Fc-region comprises a Cys-Pro-Ser-Cys sequence in the hinge region; or wherein both of said first and said second Fc-region comprise a Cys-Pro-Pro-Cys sequence in the hinge region.

13. The bispecific antibody of any one of claims 2 to 12, wherein the first and second Fc-regions are human antibody Fc-regions.

14. The bispecific antibody of any one of claims 3 to 13, wherein said first and second Fab arm, except for the specified mutations, comprise a sequence selected from the group consisting of SEQ ID NOS: 234, 235, 236, 237, 238, 239, 240, 241, 242, 243, 244 and 245.

15. The bispecific antibody of any one of the preceding claims, wherein the first and second antigen-binding regions comprise human antibody VH sequences and, optionally, human antibody VL sequences.

**16.** The bispecific antibody of any one of claims 2 to 15, wherein the first and/or the second Fc-region comprise a mutation removing the acceptor site for Asn-linked glycosylation.

**17.** The bispecific antibody of any one of the preceding claims, which is conjugated to one or more other moieties, such as a drug, radioisotope, cytokine or cytotoxic moiety, or contains one or more acceptor group for the same.

**18.** The bispecific antibody of claim 17, which is conjugated to

a) at least one cytotoxic moiety selected from the group consisting of maytansine, calicheamicin, duocarmycin, rachelmycin (CC-1065), monomethyl auristatin E, monomethyl auristatin For an analog, derivative, or prodrug of any thereof;
b) a cytokine selected from the group consisting of IL-2, IL-4, IL-6, IL-7, IL-10, IL-12, IL-13, IL-15, IL-18, IL-23, IL-24, IL-27, IL-28a, IL-28b, IL-29, KGF, IFNα, IFNβ, IFNγ, GM-CSF, CD40L, Flt3 ligand, stem cell factor, ancestim, and TNFa; or
c) a radioisotope, such as an alpha emitter.

**19.** A recombinant eukaryotic or prokaryotic host cell which produces a bispecific antibody as defined in any one of claims 1 to 17.

**20.** A pharmaceutical composition comprising a bispecific antibody as defined in any one of claims 1 to 18 and a pharmaceutically acceptable carrier.

**21.** The bispecific antibody of any one of claims 1 to 18 for use as a medicament.

**22.** The bispecific antibody of any one of claims 1 to 18 for use in the treatment of cancer.

**23.** The bispecific antibody for the use of claim 22, wherein the cancer is selected from the group consisting of breast cancer, prostate cancer, non-small cell lung cancer, bladder cancer, ovarian cancer, gastric cancer, colorectal cancer, esophageal cancer, squamous cell carcinoma of the head and neck, cervical cancer, pancreatic cancer, testis cancer, malignant melanoma and soft-tissue cancer.

**24.** The bispecific antibody for the use of any one of claims 21 to 23, wherein the bispecific antibody is for the treatment of cancer in combination with one or more further therapeutic agent, such as a chemotherapeutic agent.

**25.** A method for producing a bispecific antibody of any one of claims 1 to 17, said method comprising the steps of

a) culturing a host cell of claim 19, and
b) purifying the bispecific antibody from the culture media.

**Patentansprüche**

**1.** Bispezifischer Antikörper, umfassend eine erste antigenbindende Region und eine zweite antigenbindende Region, wobei die erste und die zweite antigenbindende Region unterschiedliche Epitope auf dem humanen epidermalen Wachstumsfaktorrezeptor 2 (HER2) binden, und wobei die erste antigenbindende Region ausgewählt ist aus der Gruppe bestehend aus:

a) einem Antikörper, der eine VH-Region, die die Sequenz von SEQ ID NO:63 umfasst, und eine VL-Region, die die Sequenz von SEQ ID NO:67 (**153**) umfasst, umfasst,
b) einem Antikörper, der eine variable schwere Region (VH), die die Sequenz von SEQ ID NO:165 umfasst, und eine variable leichte Region (VL), die die Sequenz von SEQ ID NO:169 (**005**) umfasst, umfasst, und
c) einem Antikörper, der eine VH-Region, die die Sequenz von SEQ ID NO:22 umfasst, und eine VL-Region, die die Sequenz von SEQ ID NO:26 (**025**) umfasst, umfasst,

wobei die zweite antigenbindende Region eine VH-Region, umfassend SEQ ID NO:1, und eine VL-Region, umfassend SEQ ID NO:5 (**169**), umfasst.

**2.** Bispezifischer Antikörper nach Anspruch 1, wobei der bispezifische Antikörper ferner eine erste Fc-Region und eine zweite Fc-Region umfasst.

3. Bispezifischer Antikörper nach einem der vorhergehenden Ansprüche, umfassend einen ersten Fab-Arm, der die erste antigenbindende Region und eine erste Fc-Region umfasst, und einen zweiten Fab-Arm, der die zweite antigenbindende Region und eine zweite Fc-Region umfasst.

4. Bispezifischer Antikörper nach einem der Ansprüche 1 bis 2, umfassend einen ersten Fab-Arm, der die zweite antigenbindende Region und eine erste Fc-Region umfasst, und einen zweiten Fab-Arm, der die erste antigenbindende Region und eine zweite Fc-Region umfasst.

5. Bispezifischer Antikörper nach einem der Ansprüche 2 bis 4, wobei die Isotypen des ersten und zweiten Fab-Arms unabhängig voneinander aus IgGl, IgG2, IgG3 und IgG4 ausgewählt sind.

6. Bispezifischer Antikörper nach Anspruch 5, wobei die Isotypen der ersten und zweiten Fc-Region unabhängig voneinander aus IgGl und IgG4 ausgewählt sind.

7. Bispezifischer Antikörper nach Anspruch 6, wobei eine der ersten und zweiten Fc-Regionen von einem IgGl-Isotyp und eine von einem IgG4-Isotyp ist.

8. Bispezifischer Antikörper nach Anspruch 6, wobei die Isotypen der ersten und zweiten Fc-Region vom IgGl-Isotyp sind.

9. Bispezifischer Antikörper nach einem der Ansprüche 2 bis 8, wobei die erste Fc-Region eine Aminosäuresubstitution an einer Position aufweist, die aus der Gruppe bestehend aus 409, 366, 368, 370, 399, 405 und 409 ausgewählt ist, und die zweite Fc-Region eine Aminosäuresubstitution an einer Position aufweist, die aus der Gruppe bestehend aus 405, 366, 368, 370, 399, 407 und 409 ausgewählt ist, und wobei die erste Fc-Region und die zweite Fc-Region nicht an denselben Positionen substituiert sind, und wobei die Aminosäurepositionen dem Eu-Index entsprechen, wie in Kabat et al. beschrieben.

10. Bispezifischer Antikörper nach einem der Ansprüche 2 bis 9, wobei

a) die erste Fc-Region eine andere Aminosäure als Lys, Leu oder Met an Position 409 aufweist und die zweite Fc-Region eine andere Aminosäure als Phe an Position 405 aufweist;
b) die erste Fc-Region eine andere Aminosäure als Lys, Leu oder Met an Position 409 aufweist und die zweite Fc-Region eine andere Aminosäure als Phe, Arg oder Gly an Position 405 aufweist;
c) die erste Fc-Region ein Phe an Position 405 und eine andere Aminosäure als Lys, Leu oder Met an Position 409 umfasst und die zweite Fc-Region eine andere Aminosäure als Phe an Position 405 und ein Lys an Position 409 umfasst;
d) die erste Fc-Region ein Phe an Position 405 und eine andere Aminosäure als Lys, Leu oder Met an Position 409 umfasst und die zweite Fc-Region eine andere Aminosäure als Phe, Arg oder Gly an Position 405 und ein Lys an Position 409 umfasst;
e) die erste Fc-Region ein Phe an Position 405 und eine andere Aminosäure als Lys, Leu oder Met an Position 409 umfasst und die zweite Fc-Region ein Leu an Position 405 und ein Lys an Position 409 umfasst;
f) die erste Fc-Region ein Phe an Position 405 und ein Arg an Position 409 umfasst und die zweite Fc-Region eine andere Aminosäure als Phe, Arg oder Gly an Position 405 und ein Lys an Position 409 umfasst;
g) die erste Fc-Region Phe an Position 405 und ein Arg an Position 409 umfasst und die zweite Fc-Region ein Leu an Position 405 und ein Lys an Position 409 umfasst;
h) die erste Fc-Region eine andere Aminosäure als Lys, Leu oder Met an Position 409 umfasst und die zweite Fc-Region ein Lys an Position 409, ein Thr an Position 370 und ein Leu an Position 405 umfasst;
i) die erste Fc-Region ein Arg an Position 409 umfasst und die zweite Fc-Region ein Lys an Position 409, einen Thr an Position 370 und ein Leu an Position 405 umfasst; oder
j) die erste Fc-Region ein Lys an Position 370, ein Phe an Position 405 und ein Arg an Position 409 umfasst und die zweite Fc-Region ein Lys an Position 409, ein Thr an Position 370 und ein Leu an Position 405 umfasst.

11. Bispezifischer Antikörper nach einem der Ansprüche 2 bis 9, wobei die erste und die zweite Fc-Region mit Ausnahme der spezifizierten Mutationen die Sequenz von SEQ ID NO:236 (IgGlm(a)) umfassen.

12. Bispezifischer Antikörper nach einem der Ansprüche 2 bis 9, wobei weder die erste noch die zweite Fc-Region eine Cys-Pro-Ser-Cys-Sequenz in der Gelenkregion umfasst; oder wobei sowohl die erste als auch die zweite Fc-Region eine Cys-Pro-Pro-Cys-Sequenz in der Gelenkregion umfasst.

13. Bispezifischer Antikörper nach einem der Ansprüche 2 bis 12, wobei die erste und die zweite Fc-Region menschliche Antikörper-Fc-Regionen sind.

14. Bispezifischer Antikörper nach einem der Ansprüche 3 bis 13, wobei der erste und der zweite Fab-Arm, mit Ausnahme der spezifizierten Mutationen, eine Sequenz umfassen, die ausgewählt ist aus der Gruppe bestehend aus SEQ ID NOS: 234, 235, 236, 237, 238, 239, 240, 241, 242, 243, 244 und 245.

15. Bispezifischer Antikörper nach einem der vorhergehenden Ansprüche, wobei die ersten und zweiten antigenbindenden Regionen menschliche Antikörper-VH-Sequenzen und gegebenenfalls menschliche Antikörper-VL-Sequenzen umfassen.

16. Bispezifischer Antikörper nach einem der Ansprüche 2 bis 15, wobei die erste und/oder die zweite Fc-Region eine Mutation umfasst, die die Akzeptorstelle für Asn-verknüpfte Glykosylierung entfernt.

17. Bispezifischer Antikörper nach einem der vorhergehenden Ansprüche, der mit einer oder mehreren anderen Einheiten, wie einem Arzneimittel, Radioisotop, Zytokin oder einer zytotoxischen Einheit, konjugiert ist oder eine oder mehrere Akzeptorgruppen für dieselben enthält.

18. Bispezifischer Antikörper nach Anspruch 17, der konjugiert ist mit

   a) mindestens einer zytotoxischen Einheit, ausgewählt aus der Gruppe bestehend aus Maytansin, Calicheamicin, Duocarmycin, Rachelmycin (CC-1065), Monomethylauristatin E, Monomethylauristatin F oder einem Analogon, Derivat oder Prodrug eines dieser Stoffe;
   b) einem Zytokin, ausgewählt aus der Gruppe bestehend aus IL-2, IL-4, IL-6, IL-7, IL-10, IL-12, IL-13, IL-15, IL-18, IL-23, IL-24, IL-27, IL-28a, IL-28b, IL-29, KGF, IFN$\alpha$, IFN$\beta$, IFN$\gamma$, GM-CSF, CD40L, Flt3-Ligand, Stammzellfaktor, Ancestim und TNFa; oder
   c) einem Radioisotop, wie einem Alphastrahler.

19. Rekombinante eukaryotische oder prokaryotische Wirtszelle, die einen bispezifischen Antikörper, wie in einem der Ansprüche 1 bis 17 definiert, produziert.

20. Pharmazeutische Zusammensetzung, die einen bispezifischen Antikörper, wie in einem der Ansprüche 1 bis 18 definiert, und einen pharmazeutisch akzeptablen Träger umfasst.

21. Bispezifischer Antikörper nach einem der Ansprüche 1 bis 18 zur Verwendung als Arzneimittel.

22. Bispezifischer Antikörper nach einem der Ansprüche 1 bis 18 zur Verwendung bei der Behandlung von Krebs.

23. Bispezifischer Antikörper zur Verwendung nach Anspruch 22, wobei der Krebs ausgewählt ist aus der Gruppe bestehend aus Brustkrebs, Prostatakrebs, nicht-kleinzelligem Lungenkrebs, Blasenkrebs, Eierstockkrebs, Magenkrebs, kolorektalem Krebs, Speiseröhrenkrebs, Plattenepithelkarzinom von Kopf und Hals, Gebärmutterhalskrebs, Bauchspeicheldrüsenkrebs, Hodenkrebs, malignem Melanom und Weichteilgewebekrebs.

24. Bispezifischer Antikörper zur Verwendung nach einem der Ansprüche 21 bis 23, wobei der bispezifische Antikörper für die Behandlung von Krebs in Kombination mit einem oder mehreren weiteren therapeutischen Mitteln, wie einem Chemotherapeutikum, bestimmt ist.

25. Verfahren zur Herstellung eines bispezifischen Antikörpers nach einem der Ansprüche 1 bis 17, wobei das Verfahren die folgenden Schritte umfasst:

   a) Kultivierung einer Wirtszelle nach Anspruch 19 und
   b) Reinigen des bispezifischen Antikörpers aus dem Kulturmedium.

**Revendications**

1. Anticorps bispécifique comprenant une première région de liaison à l'antigène et une deuxième région de liaison à l'antigène, lesquelles première et deuxième régions de liaison à l'antigène se lient au niveau d'épitopes différents sur le récepteur HER2 (récepteur 2 du facteur de croissance épidermique humain), et dans lequel la première région de liaison à l'antigène est choisie dans l'ensemble formé par :

> a) un anticorps comprenant une région VH comportant la séquence identifiée Séquence N° 63 et une région VL comportant la séquence identifiée Séquence N° 67 (**153**),
> b) un anticorps comprenant une région VH (région variable de chaîne lourde) comportant la séquence identifiée Séquence N° 165 et une région VL (région variable de chaîne légère) comportant la séquence identifiée Séquence N° 169 (**005**),
> c) et un anticorps comprenant une région VH comportant la séquence identifiée Séquence N° 22 et une région VL comportant la séquence identifiée Séquence N° 26 (**025**),

> et dans lequel la deuxième région de liaison à l'antigène comprend une région VH comportant la séquence identifiée Séquence N° 1 et une région VL comportant la séquence identifiée Séquence N° 5 (**169**).

2. Anticorps bispécifique conforme à la revendication 1, lequel anticorps bispécifique comprend en outre une première région Fc et une deuxième région Fc.

3. Anticorps bispécifique conforme à l'une des revendications précédentes, comprenant un premier bras Fab comportant la première région de liaison à l'antigène et une première région Fc, et un deuxième bras Fab comportant la deuxième région de liaison à l'antigène et une deuxième région Fc.

4. Anticorps bispécifique conforme à l'une des revendications 1 à 2, comprenant un premier bras Fab comportant la deuxième région de liaison à l'antigène et une première région Fc, et un deuxième bras Fab comportant la première région de liaison à l'antigène et une deuxième région Fc.

5. Anticorps bispécifique conforme à l'une des revendications 2 à 4, dans lequel les isotypes des premier et deuxième bras Fab sont choisis indépendamment parmi IgG1, IgG2, IgG3 et IgG4.

6. Anticorps bispécifique conforme à la revendication 5, dans lequel les isotypes des première et deuxième régions Fc sont choisis indépendamment parmi IgG1 et IgG4.

7. Anticorps bispécifique conforme à la revendication 6, dans lequel une des première et deuxième régions Fc est d'un isotype IgG1 et une est d'un isotype IgG4.

8. Anticorps bispécifique conforme à la revendication 6, dans lequel les isotypes des première et deuxième régions Fc sont un isotype IgG1.

9. Anticorps bispécifique conforme à l'une des revendications 2 à 8, dans lequel la première région Fc présente une substitution d'acide aminé en une position choisie dans l'ensemble formé par les positions 409, 366, 368, 370, 399, 405 et 409 et la deuxième région Fc présente une substitution d'acide aminé en une position choisie dans l'ensemble formé par les positions 405, 366, 368, 370, 399, 407 et 409, et dans lequel ladite première région Fc et ladite deuxième région Fc ne présentent pas de substitution en les mêmes positions, étant entendu que lesdites positions d'acide aminé sont indiquées selon l'index Eu décrit chez Kabat et coll.

10. Anticorps bispécifique conforme à l'une des revendications 2 à 9, dans lequel

> a) la première région Fc comporte un acide aminé autre que Lys, Leu ou Met en position 409, et la deuxième région Fc comporte un acide aminé autre que Phe en position 405,
> b) la première région Fc comporte un acide aminé autre que Lys, Leu ou Met en position 409, et la deuxième région Fc comporte un acide aminé autre que Phe, Arg ou Gly en position 405,
> c) la première région Fc comporte un Phe en position 405 et un acide aminé autre que Lys, Leu ou Met en position 409, et la deuxième région Fc comporte un acide aminé autre que Phe en position 405 et un Lys en position 409,
> d) la première région Fc comporte un Phe en position 405 et un acide aminé autre que Lys, Leu ou Met en position 409, et la deuxième région Fc comporte un acide aminé autre que Phe, Arg ou Gly en position 405 et un Lys en position 409,

e) la première région Fc comporte un Phe en position 405 et un acide aminé autre que Lys, Leu ou Met en position 409, et la deuxième région Fc comporte un Leu en position 405 et un Lys en position 409,

f) la première région Fc comporte un Phe en position 405 et un Arg en position 409, et la deuxième région Fc comporte un acide aminé autre que Phe, Arg ou Gly en position 405 et un Lys en position 409,

g) la première région Fc comporte un Phe en position 405 et un Arg en position 409, et la deuxième région Fc comporte un Leu en position 405 et un Lys en position 409,

h) la première région Fc comporte un acide aminé autre que Lys, Leu ou Met en position 409, et la deuxième région Fc comporte un Lys en position 409, un Thr en position 370 et un Leu en position 405,

i) la première région Fc comporte un Arg en position 409, et la deuxième région Fc comporte un Lys en position 409, un Thr en position 370 et un Leu en position 405,

j) ou la première région Fc comporte un Lys en position 370, un Phe en position 405 et un Arg en position 409, et la deuxième région Fc comporte un Lys en position 409, un Thr en position 370 et un Leu en position 405.

11. Anticorps bispécifique conforme à l'une des revendications 2 à 9, dans lequel lesdites première et deuxième régions Fc comprennent la séquence identifiée Séquence N° 236 (IgG1m(a)), excepté les mutations spécifiées.

12. Anticorps bispécifique conforme à l'une des revendications 2 à 9, dans lequel ni ladite première région Fc, ni ladite deuxième, ne comporte une séquence Cys-Pro-Ser-Cys dans la région charnière, ou bien dans lequel les deux régions Fc, ladite première et ladite deuxième, comportent une séquence Cys-Pro-Pro-Cys dans la région charnière.

13. Anticorps bispécifique conforme à l'une des revendications 2 à 12, dans lequel les première et deuxième régions Fc sont des régions Fc d'anticorps humain.

14. Anticorps bispécifique conforme à l'une des revendications 3 à 13, dans lequel lesdits premier et deuxième bras Fab comprennent une séquence choisie dans l'ensemble des séquences identifiées Séquences N° 234, 235, 236, 237, 238, 239, 240, 241, 242, 243, 244 et 245, excepté les mutations spécifiées.

15. Anticorps bispécifique conforme à l'une des revendications précédentes, dans lequel les première et deuxième régions de liaison à l'antigène comportent des séquences de région VH d'anticorps humain, et, en option, des séquences de région VL d'anticorps humain.

16. Anticorps bispécifique conforme à l'une des revendications 2 à 15, dans lequel la première et/ou la deuxième région(s) Fc comporte(nt) une mutation qui élimine le site accepteur pour la glycosylation avec liaison à Asn.

17. Anticorps bispécifique conforme à l'une des revendications précédentes, qui est conjugué à une ou plusieurs autres entités, telles qu'un médicament, un isotope radioactif, une cytokine ou une entité cytotoxique, ou qui contient un ou plusieurs groupe(s) accepteur(s) d'une ou de telle(s) entité(s).

18. Anticorps bispécifique conforme à la revendication 17, qui est conjugué

a) à au moins une entité cytotoxique, choisie dans l'ensemble formé par les maytansine, calichéamicine, duocarmycine, rachelmycine (CC-1065), monométhyl-auristatine E et monométhyl-auristatine F et les analogues, dérivés ou précurseurs de n'importe laquelle d'entre celles-ci,
b) à une cytokine choisie dans l'ensemble formé par les IL-2, IL-4, IL-6, IL-7, IL-10, IL-12, IL-13, IL-15, IL-18, IL-23, IL-24, IL-27, IL-28a, IL-28b, IL-29, KGF, IFN$\alpha$, IFN$\beta$, IFN$\gamma$, GM-CSF, CD40L, ligand de Flt3, facteur de cellule souche, ancestime et TNF$\alpha$,
c) ou à un isotope radioactif, comme un émetteur de rayons alpha.

19. Cellule hôte eucaryote ou procaryote recombinante, qui produit un anticorps bispécifique tel que défini dans l'une des revendications 1 à 17.

20. Composition pharmaceutique comprenant un anticorps bispécifique tel que défini dans l'une des revendications 1 à 18 et un véhicule pharmacologiquement admissible.

21. Anticorps bispécifique conforme à l'une des revendications 1 à 18 pour utilisation en tant que médicament.

**22.** Anticorps bispécifique conforme à l'une des revendications 1 à 18 pour utilisation dans le traitement d'un cancer.

**23.** Anticorps bispécifique pour utilisation conforme à la revendication 22, pour lequel le cancer est choisi dans l'ensemble constitué par les cancer du sein, cancer de la prostate, cancer du poumon non à petites cellules, cancer de la vessie, cancer des ovaires, cancer gastrique, cancer colorectal, cancer de l'oesophage, carcinome épidermoïde de la tête et du cou, cancer du col de l'utérus, cancer du pancréas, cancer du testicule, mélanome malin et cancer des tissus mous.

**24.** Anticorps bispécifique pour utilisation conforme à l'une des revendications 21 à 23, lequel anticorps bispécifique est conçu pour le traitement d'un cancer en association avec un ou plusieurs agent(s) thérapeutique(s) supplémentaire(s), comme un agent de chimiothérapie.

**25.** Procédé de production d'un anticorps bispécifique conforme à l'une des revendications 1 à 17, lequel procédé comporte les étapes suivantes :

a) cultiver une cellule hôte conforme à la revendication 19,
b) et purifier l'anticorps bispécifique à partir du milieu de culture.

**Figure 1A**

IgHV3-23-01 / IGHJ4-02 – VH alignment (Group 1)

```
IgHV1-23-01   EVQLLESGGGLVQPGGSLRLSCAASGFTFSSYAMSWVRQAPGKGLEWVSAISGSGGSTYYADSVKG
TH1014-050    EVQLLESGGGLVQPGGSLRLSCAASGFTFSSYAMNWVRQAPGKGLEWVSAISGRGGTYYADSVKG
VH1014-049    EVQLLESGGDLVQPGGSLRLSCAASGFTFSSYAMSWVRQAPGKGLEWVSAISGRGGTYYADSVKG
VH1014-051    EVQLLESGGDLVQPGGSLRLSCAASGFTFSSYAMSWVRQAPGKGLEWVSAISGRGGTYYADSVKG
VH1014-055    EVQLLESGGGLVQPGGSLRLSCAASGFTFSSYAMNWVRQAPGKGLEWVSAISGRGGTYYADSVKG
Consensus     EVQLLESGGXLVQPGGSLRLSCAASGFTFSSYAMXWVRQAPGKGLEWVSAISGXGGXTYYADSVKG

IgHV1-23-01   RFTISRDNSKNTLYLQMNSLRAEDTAVYYCAK------YFDYWGQGTLVTVSS
TH1014-050    RFTISRDNSKNTLYLQMSSLRAEDTAVYYCAKARANWDYFDYWGQGTLVTVSS
VH1014-049    RFTISRDNSKSTLCLQMNSLRAEDTAVYYCAKARANWDYFDYWGQGTLVTVSS
VH1014-051    RFTISRDNSKSTLCLQMNSLRAEDTAVYYCAKARANWDYFDYWGQGTLVTVSS
VH1014-055    RFTISRDNSKSTLCLQMNSLRAEDTAVYYCAKARANWDYFDYWGQGTLVTVSS
Consensus     RFTISRDNSKXTLXLQMXSLRAEDTAVYYCAKARANWDYFDYWGQGTLVTVSS
```

**Figure 1B**

IgHV1-69-04 / IGHJ6-02 – VH alignment (Group 1)

```
IgHV1-69-04   QVQLVQSGAEVKKPGSSVKVSCKASGGTFSSYAISWVRQAPGQGLEWMGRIIPILGIANYAQKFQG
TH1014-084    QVQLVQSGAEVKKPGSSVKVSCKASGGTFRTYAINWVRQAPGQGLEWMGRINTVLGIVNHAQKFQG
Consensus     QVQLVQSGAEVKKPGSSVKVSCKASGGTFXXYAISWVRQAPGQGLEWMGRIXXXLGIXNXAQKFQG

IgHV1-69-04   RVTITADKSTSTAYMELSSLRSEDTAVYYCAR--------GMDVWGQGTTVTVSS
TH1014-084    RVTITADKSTNTAYMELNSLRSEDTAVYYCAREKGVDYYYGIEVWGQGTTVTVSS
Consensus     RVTITADKSTXTAYMELXSLRSEDTAVYYCAREKGVDYYYGXXVWGQGTTVTVSS
```

**Figure 1C**

<u>IgHV1-18-01 / IGHJ4-02 – VH alignment (Group 1)</u>

```
IgHV1-18-01  QVQLVQSGAEVKKPGASVKVSCKASGYTFTSYGISWVRQAPGQGLEWMGWISAYNGNTYAQKLQG
TH1014-169   QVQLVQSGAEVKKPGASVKVSCKASGYTFTNYGISWVRQAPGQGLEWMGWLSAYSGNTIYAQKLQG
VH1014-123   QVQLVQSGAEVKKPGASVKVSCKAAGYTFTNYGISWVRQAPGQALEWMGWITTYSSNTIYAQKLQG
VH1014-161   QVQLVQSGAEVKKPGASVKVSCKASGYTFTNYGISWVRQAPGQGLEWMGWLSAYSGNTIYAQKLQG
VH1014-124   QVQLVQSGAEVKKPGASVKVSCKAAGYTFTNYGISWVRQAPGQGLEWMGWIITYNGNTIYAQRFQD
Consensus    QVQLVQSGAEVKKPGASVKVSCKASGYTFTXYGISWVRQAPGQXLEWMGWXXXYXXNTXYAQXXQG

IgHV1-18-01  RVTMTTDTSTSTAYMELRSLRSDDTAVYYCAR--------YFDYWGQGTLVTVSS
TH1014-169   RVTMTTDTSTTTAYMELRSLRSDDTAVYYCARDRIVVRPDYFDYWGQGTLVTVSS
VH1014-123   RVTMTTDTSTSTAYMELRSLRSDDTAVYYCARDRVVVRPDYFDYWGQGTLVTVSS
VH1014-161   RVTMTTDTSTTTAYMELRSLRSDDTAVYYCARDRIVVRPDYFDYWGQGTLVTVSS
VH1014-124   RVTMTTDTSTSTAYMELRSLRSDDTAVYYCARDRIIVRPDYFDYWGQGTLVTVSS
Consensus    RVTMTTDTSTXTAYMELRSLRSDDTAVYYCARDRXXVRPDYFDYWGQGTLVTVSS
```

**Figure 1D**

<u>IgHV4-34-01 / IGHJ4-02 – VH alignment (Group 2, No. 1)</u>

```
IgHV4-34-01  QVQLQQWGAGLLKPSETLSLTCAVYGGSFSGYYWSWIRQPPGKGLEWIGEINHSGSTNYNPSLKSR
TH1014-025   QVQLQQWGAGLLKPSETLSLTCAVYGGSFSDYYWNWIRQPPGKGLEWIGEIHHSGSTNYNPSLKSR
VH1014-001   QVQLQQWGAGLLKPSETLSLTCAVYGGSFSGYYWNWIRQPPGKGLEWIGEINHSGSTNYNPSLKSR
VH1014-143   QVQLQQWGAGLLKPSETLSLTCAVYGGSFSGYYWNWIRQPPGKGLEWIGEIHHSGSANYNPSLMSR
VH1014-019   QVQLQQWGAGLLKPSETLSLTCAVYGGSFSDYYWNWIRQPPGKGLEWIGEIHHVGSTNYNPSLKSR
VH1014-021   QVQLQQWGAGLLKPSETLSLTCAVYGGSFSDYYWNWIRQPPGKGLEWIGEIHHSGSTNYNPSLKSR
VH1014-027   QVQLQQWGAGLLKPSETLSLTCAVYGGSFSDYFWNWIRQPPGKGLEWIGEIHHSGSTNYNPSLKSR
Consensus    QVQLQQWGAGLLKPSETLSLTCAVYGGSFSXYXWXWIRQPPGKGLEWIGEIXHXGSXNYNPSLXSR

IgHV4-34-01  VTISVDTSKNQFSLKLSSVTAADTAVYYCAR--------YFDYWGQGTLVTVSS
TH1014-025   VTISVDTSKNQFSLKLSSVTAADTAVYYCARGYYDSGVYYFDYWAQGTLVTVSS
VH1014-001   VTISVDTSKNQFSLKLSSVTAADTAVYYCARGNYGSGYYYFDLWGRGTQVTVSS
VH1014-143   VTISVDTSKNQFSLQLSSVTAADTAVYYCARGYYGSGYYYFDYWGQGTLVTVSS
VH1014-019   VTISVDTSKSQFSLKLSSVTAADTAVYYCARGYYDSGVYYFDYWAQGTLVTVSS
VH1014-021   VTISVDTSKNQFSLKLSSVTAADTAVYYCARGYYASGVYYFDYWGQGTLVTVSS
VH1014-027   VTISVDTSKNQFSLNLSSVTAADTAVYYCARGLIGSGYYYFDYWDQGTLVTVSS
Consensus    VTISVDTSKXQFSLXLSSVTAADTAVYYCARGXXXSGXYYFDXWXXGTXVTVSS
```

**Figure 1E**

<u>IgHV4-34-01 / IGHJ4-02 – VH alignment (Group 2, No. 2)</u>

```
IgHV4-34-01   QVQLQQWGAGLLKPSETLSLTCAVYGGSFSGYYWSWIRQPPGKGLEWIGEINHSGSTNYNPSLKSR
TH1014-091    QVQLQQWGAGLLKPSETLSLTCAVSGGSFSGYYWTWIRQPPGKGLEWIGEIYHSGDTNYNPSLKSR
VH1014-032    QVQLQQWGAGLLKPSETLSLTCAVYGGSFSGYYWSWIRQPPGKGLEWIGEINHSGDTNYNPSLTSR
VH1014-035    QVQLQQWGAGLLKPSETLSLTCALYGGSFSGYYWSWIRQPPGKGLEWIGEINHSGDTNYNPSLTSR
VH1014-036    QVQLQQWGAGLLKPSETLSLTCAVYGGSFSDYYWSWIRQPPGKGLEWIGEINHSGSTNYNPSLKSR
VH1014-054    QVQLQQWGAGLLKPSETLSLTCAVYGGSFSGYYWSWIRQPPGKGLEWIGEIHHSGSTNYNPSLKSR
VH1014-094    QVQLQQWGAGLLKPSETLSLTCAVSGGSFSGYYWTWIRQPPGKGLEWIGEIYHSGDTNYNPSLKSR
Consensus     QVQLQQWGAGLLKPSETLSLTCAXXGGSFSXYYWXWIRQPPGKGLEWIGEIXHSGXTNYNPSLXSR

IgHV4-34-01   VTISVDTSKNQFSLKLSSVTAADTAVYYCAR-------YFDYWGQGTLVTVSS
TH1014-091    VTISVDTSKNQFSLKLYSVTAADTAVYYCARLYFGSGIYYLDYWGQGTLVTVSS
VH1014-032    VTISVDTSKNQFSLKLSSVTAADTAVYYCARLFYGSGIYYFDYWGQGTLVTVSS
VH1014-035    VTISVDTSKNQFSLKLSSVTAADTAVYYCARLFYGSGIYYFDYWGQGTLVTVSS
VH1014-036    VTISVDTSKNQFSLKLSSVTAADTAVYYCARLYYGSGTYYFDYWGQGTLVTVSS
VH1014-054    VTISVDTSKNQFSLKLSSVTAADTAVYYCARLWYGSGSYYFDYWGQGTLVTVSS
VH1014-094    VTISVDTSKNQFSLKLYSVTAADTAVYYCARLYFGSGIYYLDYWGQGTLVTVSS
Consensus     VTISVDTSKNQFSLKLXSVTAADTAVYYCARLXXGSGXYYXDYWGQGTLVTVSS
```

**Figure 1F**

<u>IgHV3-30-3-01 / IGHJ4-02 – VH alignment (Group 2)</u>

```
IgHV1-30-…    QVQLVESGGGVVQPGRSLRLSCAASGFTFSSYAMHWVRQAPGKGLEWVAVISYDGSNKYYADSVKGR
TH1014-129    QVQLVESGGGVVQPGRSLRLSCAASGFTFSTFAIHWVRQAPGKGLEWVAVISYDGGHKFYADSVKGR
Consensus     QVQLVESGGGVVQPGRSLRLSCAASGFTFSXXAXHWVRQAPGKGLEWVAVISYDGXXKXYADSVKGR

IgHV3-30-…    FTISRDNSKNTLYLQMNSLRAEDTAVYYCAR------YFDYWGQGTLVTVSS
TH1014-129    FTISRDNSKNTLYLQMNSLRAEDTAMYYCARGLGVWGAFDYWGQGTLVTVSS
Consensus     FTISRDNSKNTLYLQMNSLRAEDTAXYYCARGLGVWGXFDYWGQGTLVTVSS
```

**Figure 1G**

IgHV3-23-1 / IGHJ4-02 – VH alignment (Group 3a)

```
IgHV3-23-1   EVQLLESGGGLVQPGGSLRLSCAASGFTFSSYAMSWVRQAPGKGLEWVSAISGSGGSTYYADSVKG
TH1014-098   EVQLLESGGGLVQPGGSLRLSCAASGFTFSNYGMSWVRQAPGKGLEWVSAISGSAYSTYYADSVKG
VH1014-105   EVQLLESGGGLVQPGGSLRLSCAASGFTFSNYGMSWVRQAPGKGLEWVSAISGSAYSTYYADSVKG
VH1014-100   EVQLLESGGGLVQPGGSLRLSCAASGFTFNNYGMNWVRQAPGKGLEWVSAISGTGYSTYYADSVKG
VH1014-125   EVQLLESGGGLVQPGGSLRLSCAASGFTFTDYAMNWVRQAPGKGLEWVSTISGSGYATYYADSVKG
VH1014-162   EVQLWESGGGSVQPGGSLRLSCAASGFTFSSYGMSWVRQAPGKGLEWVSGISGSGYSTYYADSVKG
Consensus    EVQLXESGGGXVQPGGSLRLSCAASGFTFXXYXMXWVRQAPGKGLEWVSXISGXXXXTYYADSVKG

IgHV3-23-1   RFTISRDNSKNTLYLQMNSLRAEDTAVYYCAK-----------YFDYWGQGTLVTVSS
TH1014-098   RFTISRDNSKNTLWLQMNSLRAEDTAVYYCAKAHYHGSGSYYTLFDYWGQGTLVTVSS
VH1014-105   RFTISRDNSKNTLWLQMNSLRAEDTAVYYCAKAHYHGSGSYYTLFDYWGQGTLVTVSS
VH1014-100   RFTISRDNSKNTLYLQMNSLRAEDTAVYYCAKAHYFGSGSYYTLFDYWGQGTLVTVSS
VH1014-125   RFTISRDNSKTTLYLQMNSLRAEDTAVYYCAKGHTLGSGSYYTLFDYWGQGTLVTVSS
VH1014-162   RFTISRDNSKNTLYLQMNSLRAEDTAVYYCAKGYYHGSGSYYTSFDYWGQGTLVTVSS
Consensus    RFTISRDNSKXTLXLQMNSLRAEDTAVYYCAKXXXXGSGSYYTXFDYWGQGTLVTVSS
```

**Figure 1H**

IgHV5-51-01 / IGHJ2-01 – VH alignment (Group 3a, No. 1)

```
IgHV5-51-01  EVQLVQSGAEVKKPGESLKISCKGSGYSFTSYWIGWVRQMPGKGLEWMGIIYPGDSDTRYSPSFQG
TH1014-127   EVQLVQSGAEVKKPGESLTISCKGSGYSFSIYWIGWVRQMPGKGLEWMGIIFPGDSDIRYSPSFQG
Consensus    EVQLVQSGAEVKKPGESLXISCKGSGYSFXXYWIGWVRQMPGKGLEWMGIIXPGDSDXRYSPSFQG

IgHV5-51-01  QVTISADKSISTAYLQWSSLKASDTAMYYCAR----------YFDLWGRGTLVTVSS
TH1014-127   QVTISADKSISTAYLQWSSLKASDTAMYYCARQPGDWSPRHWYFDLWGRGTLVTVSS
Consensus    QVTISADKSISTAYLQWSSLKASDTAMYYCARQPGDWSPRHWYFDLWGRGTLVTVSS
```

**Figure 1I**

IgHV5-51-01-01 / IGHJ5-02 – VH alignment (Group 3a, No. 2)

```
IgHV5-51-01  EVQLVQSGAEVKKPGESLKISCKGSGYSFTSYWIGWVRQMPGKGLEWMGIIYPGDSDTRYSPSFQG
TH1014-159   EVQLVQSGAEVKKPGESLKISCKGSGYNFTSYWIGWVRQMPGKGLEWMGIIYPGDSDTRYSPSFQG
Consensus    EVQLVQSGAEVKKPGESLKISCKGSGYXFTSYWIGWVRQMPGKGLEWMGIIYPGDSDTRYSPSFQG

IgHV5-51-01  QVTISADKSISTAYLQWSSLKASDTAMYYCAR------------NWFDPWGQGTLVTVSS
TH1014-159   QVTISADKSISTAYLQWSSLKASDTAMYYCARWGTYYDILTGYFNWFDPWGQGTLVTVSS
Consensus    QVTISADKSISTAYLQWSSLKASDTAMYYCARWGTYYDILTGYFNWFDPWGQGTLVTVSS
```

**Figure 1J**

<u>IgHV1-18-01 / IGHJ6-02  – VH alignment (Group 3b)</u>

```
IgHV1-18-01   QVQLVQSGAEVKKPGASVKVSCKASGYTFTSYGISWVRQAPGQGLEWMGWISAYNGNTNYAQKLQG
TH1014-132    QVQLVQSGAEVKKPGASVKVSCKASGYTFTSYGISWVRQAPGQGLEWMGWISAYNGNSNYVQKFQG
Consensus     QVQLVQSGAEVKKPGASVKVSCKASGYTFTSYGISWVRQAPGQGLEWMGWISAYNGNXNYXQKXQG

IgHV1-18-01   RVTMTTDTSTSTAYMELRSLRSDDTAVYYCAR------------GMDVWGQGTTVTVSS
TH1014-132    RVTMTTDTTTSTAYMELRSLTSDDTAVYYCAREYSYDSGTYFYYGMDVWGQGTTVTVSS
Consensus     RVTMTTDTSTSTAYMELRSLRSDDTAVYYCAREYSYDSGTYFYYGMDVWGQGTTVTVSS
```

**Figure 1K**

<u>IgHV3-30-3-01 / IGHJ4-02  – VH alignment (Group 3b)</u>

```
IgHV3-30...   QVQLVESGGGVVQPGRSLRLSCAASGFTFSSYAMHWVRQAPGKGLEWVAVISYDGSNKYYADSVKG
TH1014-153    QVQLVESGGGVVQPGRSLRLSCAASGFTFSDYVIHWVRQAPGKGLEWVTVISYDGSNKYYADSVKG
VH1014-033    QVQLVESGGGVVQTGRSLRLSCAASGFTFSSHAMHWVRQAPGKGLEWVAAISYDGSNKYYADSVKG
VH1014-160    QVQLVESGGGVVQPGRSLRLSCAASGFTFSSHAMHWVRQAPGKGLEWVAAISYDGSNKYYADSVKG
VH1014-166    QVQLVESGGGVVQPGRSLRLSCAASGFTFSSYAMHWVRQAPGKGLEWVAVISYDGSNEYYADSVKG
VH1014-152    QVQVVESGGGVVQPGRSLRLSCAASGFTFSSYAMHWVRQAPGKGLEWVAVISYDGSYKYYADSVKG
VH1014-167    QVQLVESGGGVVQPGRSLRLSCAASGFTFSSYAIHWVRQAPGKGLEWVAVISYDGSNKYYADSVKG
Consensus     QVQLVESGGGVVQXGRSLRLSCAASGFTFSXXXXHWVRQAPGKGLEWVXXISYDGSXXYYADSVKG

IgHV3-30...   RFTISRDNSKNTLYLQMNSLRAEDTAVYYCAR--------YFDYWGQGTLVTVSS
TH1014-153    RFTISRDNSKNTLYLQMNSLSAEDTAMYYCARGGITGTTGVFDYWGQGTLVTVSS
VH1014-033    RFTISRDNSKNTLYLQMNSLRAEDTAVYYCARGDYISSSGVFDYWGQGTLVTVSS
VH1014-160    RFTISRDNSKNTMYLQMNSLRAEDTAMCYCARGSITGSTGVFDYWGQGTLVTVSS
VH1014-166    RFTISRDNSKNTLYLQMNSLRAEDTAVYYCARGSIIGSTGVFDYWGQGTLVTVSS
VH1014-152    RFTISRDNSKNTLYLQMNSLRAEDTAVYYCARGSITGSTGVFDYWGQGTLVTVSS
VH1014-167    RFTISRDNSKNTLYLQMNSLRAEDTAVYYCARGSITGSTGVFDYWGQGTLVTVSS
Consensus     RFTISRDNSKNTXYLQMNSLXAEDTAXXYCARGXXXXXGXFDYWGQGTLVTVSS
```

**Figure 1L**

<u>IgHV5-51-1 / IGHJ6-02 – VH alignment</u>

```
IgHV5-51-1   EVQLVQSGAEVKKPGESLKISCKGSGYSFTSYWIGWVRQMPGKGLEWMGIIYPGDSDTRYSPSFQ
TH1014-005   EVQLVQSGAEVKKPGESLKISCKASGYSFHFYWIGWVRQMPGKGLEWMGSIYPGDSDTRYRPSFQ
TH1014-060   EVQLVQSGAEVKKPGESLKISCKGSGYRFTSYWIGWVRQMPGKGLEWMGSIYPGDSYTRNSPSFQ
TH1014-106   EVQLVQSGAEVKKPGESLKISCKGSGYSFTRYWIGWVRQMPGKGLEWMGIIYPGDSDTRYSPSFQ
VH1014-041   EVQLVQSGAEVKKPGESLKISCKGSGYSFTSYWIGWVRQMPGKGLEWMGSIYPGDSHTRYRPSFQ
VH1014-150   EVQLVQSGAEVKKPGESLKISCKGSGYSFTSYWIGWVRQMPGKGLEWMGSIYPGDSHTRYRPSFQ
VH1014-067   EVQLVQSGAEVKKPGESLKISCKGSGYSFTSYWIGWVRQMPGKGLEWMGIIYPGDSDTRYSPSFQ
VH1014-072   EVQLVQSGAEVKKPGESLKISCKGSGYSFTSYWIGWVRQMPGKGLEWMGIIYPGDSDTRYSPSFQ
VH1014-163   EVQLVQSGAEVKKPGESLKISCQGSGYRFISYWIGWVRQMPGKGLEWMGRIYPGDSDTRYSPSFQ
VH1014-093   EVQLVQSGAEVKKPGESLKISCKGSGYSFTSYWIGWVRQMPGKGLEWMGRIYPGDSDTRYSPSFQ
VH1014-044   EVQLVQSGAEVKKPGESLKISCKGSGYRFSSYWIGWVRQMPGKGLEWMGSIFPGDSDTRYSPSFQ
Consensus    EVQLVQSGAEVKKPGESLKISCXXSGYXFXXYWIGWVRQMPGKGLEWMGXIXPGDSXTRXXPSFQ

IgHV5-51-1   GQVTISADKSISTAYLQWSSLKASDTAMYYCAR-----------GMDVWGQGTTVTVSS
TH1014-005   GQVTISADKSISTAYLQWTSLKASDTAIYYCARQRGD--YYYFYGMDVWGQGTTVTVSS
TH1014-060   GQVTISADKSIATAYLQWNSLKASDTAMYYCARHAGD--FYYFDGLDVWGQGTTVTVSS
TH1014-106   GQVTISADKSISTAYLQWSSLKASDTAMYYCARLTGDRGFDYYSGMDVWGQGTTVTVSS
VH1014-041   GQVTISADKSISTAYLQWSSLKASDTAMYYCARQKGD--FYYFFGLDVWGQGTAITVSS
VH1014-150   GQVTISADKSISTAYLQWSSLKASDTAMYYCARQAGD--YYYYNGMDVWGQGTTVTVSS
VH1014-067   GQVTISVDKSISTAYLQWSSLKASDTAMYYCARQKGD--YYYHYGLDVWGQGTTVTVSS
VH1014-072   GQVTISADKSISTAYLQWSSLKASDTAMYYCARQKGD--YYYFNGLDVWGQGTTVTVSS
VH1014-163   GQVTISVDKSISTAYLQWSSLKASDTAMYYCARQRGD--YYYFNGLDVWGQGTTVTVSS
VH1014-093   GQVTISADKSITTAYLQWSSLRASDTAMYYCARQRGD--YYYFFGLDIWGQGTTVTVSL
VH1014-044   GQVTISADKSITTAYLQWSSLKASDTAMYYCARQAGD--YYYYNGMDVWGQGTTVTVSS
Consensus    GQVTISXDKSIXTAYLQWXSLXASDTAXYYCARXXXXXXXXXYXXGXDXWGQGTXXTVSX
```

**Figure 1M**

IgHV3-23-1 / IGHJ4-02 – VH alignment

```
IgHV3-23-1    EVQLLESGGGLVQPGGSLRLSCAASGFTFSSYAMSWVRQAPGKGLEWVSAISGSGGSTYYADSVKG
TH1014-006    EVQLLESGGGLVQPGGSLRLSCAASGFTFSNYALIWVRQAPGKGLEWVSIIRGGAGSTYYADSVKG
Consensus     EVQLLESGGGLVQPGGSLRLSCAASGFTFSXYAXXWVRQAPGKGLEWVSXIXGXXGSTYYADSVKG

IgHV3-23-1    RFTISRDNSKNTLYLQMNSLRAEDTAVYYCAK------YFDYWGQGTLVTVSS
TH1014-006    RFTISRDNSKNTLYLQMNSLRAEDTAVYYCAKARIWGPLFDYWGQGTLVTVSS
Consensus     RFTISRDNSKNTLYLQMNSLRAEDTAVYYCAKARIWGPXFDYWGQGTLVTVSS
```

**Figure 1N**

IgHV1-18-1 / IGHJ4-02 – VH alignment

```
IgHV1-18-1    QVQLVQSGAEVKKPGASVKVSCKASGYTFTSYGISWVRQAPGQGLEWMGWISAYNGNTNYAQKLQG
TH1014-059    QVQLVQSGAEVKKPGASVRVPCKASGYTFTRYGISWVRQAPGQGLEWMGWISAYNGKTYYAQKLQG
Consensus     QVQLVQSGAEVKKPGASVXVXCKASGYTFTXYGISWVRQAPGQGLEWMGWISAYNGXTXYAQKLQG

IgHV1-18-1    RVTMTTDTSTSTAYMELRSLRSDDTAVYYCAR---------YFDYWGQGTLVTVSS
TH1014-059    RVTMTTDTSTSTAYMELRSLRSDDTAVYYCARSPLLWFEELYFDYWGQGTLVTVSS
Consensus     RVTMTTDTSTSTAYMELRSLRSDDTAVYYCARSPLLWFEELYFDYWGQGTLVTVSS
```

**Figure 1O**

IgHV1-69-4 / IGHJ4-02 – VH alignment

```
IgHV1-69-4    QVQLVQSGAEVKKPGSSVKVSCKASGGTFSSYAISWVRQAPGQGLEWMGRIIPILGIANYAQKFQG
TH1014-111    QVQLVQSGAEVKKPGSSVKVSCKASGGTFSSYGISWVRQAPGPGLEWMGRIIPILGIANYAQKFQG
Consensus     QVQLVQSGAEVKKPGSSVKVSCKASGGTFSSYXISWVRQAPGXGLEWMGRIIPILGIANYAQKFQG

IgHV1-69-4    RVTITADKSTSTAYMELSSLRSEDTAVYYCAR------YFDYWGQGTLVTVSS
TH1014-111    RVTITADKSTNTAYMELSSLRSEDTAVYYCARDQEYSSNWYYWGQGTLVTVSS
Consensus     RVTITADKSTXTAYMELSSLRSEDTAVYYCARDQEYSSXXXYWGQGTLVTVSS
```

**Figure 2A**

IgKV1-12-01 / IGKJ5-01 – VL alignment (Group 1)

```
IgKV1-12-01  DIQMTQSPSSVSASVGDRVTITCRASQGISSWLAWYQQKPGKAPKLLIYAASSLQSGVPSRFSGSG
VL1014-050   DIQMTQSPSSVSASVGDRVTITCRASQGISSWLAWYQHKPGKAPKLLIYAASILQSGVPSRFSGSG
VL1014-084   DIQMTQSPSSVSASVGDRVTITCRASQGISSWLAWYQHKPGKAPKLLIYVASILQSGVPSRFSGSG
VL1014-049   DIQMTQSPSSVSASVGDRVTITCRASQGISSWLAWYQHKPGKAPKLLIYAASILQSGVPSRFSGSG
VL1014-051   DIQMTQSPSSVSASVGDRVTITCRASQGISSWLAWYQHKPGKAPKLLIYAASILQSGVPSRFSGSG
VL1014-055   DIQMTQSPSSVSASVGDRVTITCRASQGISSWLAWYQHKPGKAPKLLIYAASILQSGVPSRFSGSG
Consensus    DIQMTQSPSSVSASVGDRVTITCRASQGISSWLAWYQXKPGKAPKLLIYXASXLQSGVPSRFSGSG

IgKV1-12-01  SGTDFTLTISSLQPEDFATYYCQQANSFPITFGQGTRLEIK
VL1014-050   SGTDFTLTISSLQPEDFATYYCQQANSFPITFGQGTRLEIK
VL1014-084   SGTDFTLTISSLQPEDFATYYCQQANSFPLTFGGGTKVEIK
VL1014-049   SGTDFTLTISSLRPEDFATYYCQQANSFPITFGQGTRLEIK
VL1014-051   SGTDFTLTISSLRPEDFATYYCQQANSFPITFGQGTRLEIK
VL1014-055   SGTDFTLTISSLRPEDFATYYCQQANSFPITFGQGTRLEIK
Consensus    SGTDFTLTISSLXPEDFATYYCQQANSFPXTFGXGTRXEIK
```

**Figure 2B**

IgKV3-11-01 / IGKJ1-01 – VL alignment (Group 1)

```
IgKV3-11-01  EIVLTQSPATLSLSPGERATLSCRASQSVSSYLAWYQQKPGQAPRLLIYDASNRATGIPARFSGSG
VL1014-169   EIVLTQSPATLSLSPGERATLSCRASQSVSSYLAWYQQKPGQAPRLLIYDASNRATGIPARFSGSG
VL1014-124   EIVLTQSPATLSLSPGERATLSCRASQSVSSYLAWYQQKPGQAPRLLIYDASNRATGIPARFSGSG
VL1014-161   EIVLTQSPATLSLSPGERATLSCRASQSVSSYLAWYQQKPGQAPRLLIYDASNRATGIPARFSGSG
VL1014-123   EIVLTQSPATLSLSPGERATLSCRASQSVSSYLAWYQQKPGQAPRLLIYDTSNRATGIPARFSGSG
Consensus    EIVLTQSPATLSLSPGERATLSCRASQSVSSYLAWYQQKPGQAPRLLIYDXSNRATGIPARFSGSG

IgKV3-11-01  SGTDFTLTISSLEPEDFAVYYCQQRSNWPWTFGQGTKVEIK
VL1014-169   SGTDFTLTISSLEPEDFAVYYCQQRSNWPRTFGQGTKVEIK
VL1014-124   SGTDFTLTISSLEPEDFAVYYCQQRSNWPRTFGQGTKVEIK
VL1014-161   SGTDFTLTISSLEPEDFAVYYCQQRSNWPRTFGQGTKVEIK
VL1014-123   SGTDFTLTISSLEPEDFAVYYCQQRSHWPRTFGQGTKVEIK
Consensus    SGTDFTLTISSLEPEDFAVYYCQQRSXWPRTFGQGTKVEIK
```

**Figure 2C**

IgKV1D-16-01 / IGKJ5-01– VL alignment (Group 2, No. 1)

```
IgKV1D-16 …  DIQMTQSPSSLSASVGDRVTITCRASQGISSWLAWYQQKPEKAPKSLIYAASSLQSGVPSRFSGSG
VL1014-025   DIQMTQSPSSLSASVGDRVTITCRASQGISRWLAWYQQKPEKAPKSLIYAASSLRSGVPSRFSGSG
VL1014-001   DIQMTQSPSSLSASVGDRVTITCRASQGISSWLAWYQQKPEKAPKSLIFAASSLQSGVPSRFSGSG
VL1014-019   DIQMTQSPSSLSASVGDRVTITCRASQGISRWLAWYQQKPEKAPKSLIYAASSLRSGVPSRFSGSG
VL1014-143   DIQMTQSPSSLSASVGDRVTITCRASQGISSWLAWYQQKPEKAPKSLIYAASRLQSGVPSRFSGSG
VL1014-021   DIQMTQSPSSLSASVGDRVTITCRASQGISSWLAWYQQKPEKAPKSLIYAASSLQSGVPSRFSGSG
VL1014-027   DIQMTQSPSSLSASVGDRVTITCRASQGISSWLAWYQQKPEKAPKSLIYAASSLQSGVPSRFSGSG
Consensus    DIQMTQSPSSLSASVGDRVTITCRASQGISXWLAWYQQKPEKAPKSLIXAASXLXSGVPSRFSGSG

IgKV1D-16 …  SGTDFTLTISSLQPEDFATYYCQQYNSYPITFGQGTRLEIK
VL1014-025   SGTDFTLTISSLQPEDFATYYCQQYNSYPITFGQGTRLEIK
VL1014-001   SGTDFTLTISSLQPEDFATYYCQQYISFPITFGQGTRLEIK
VL1014-019   SGTDFTLTISSLQPEDFATYYCQQYNSYPITFGQGTRLEIK
VL1014-143   SGTDFTLTISSLQPEDFATYYCQQYNSYPITFGQGTRLEIK
VL1014-021   SGTDFTLTISSLQPEDFATYYCQQYNSYPITFGQGTRLEIK
VL1014-027   SGTDFTLTISSLQPEDFATYYCQQYNSYPITFGQGTRLEIK
Consensus    SGTDFTLTISSLQPEDFATYYCQQYNSXPITFGQGTRLEIK
```

**Figure 2D**

IgKV1D-16-01 / IGKJ1-01– VL alignment (Group 2, No. 2)

```
IgKV1D-16 …  DIQMTQSPSSLSASVGDRVTITCRASQGISSWLAWYQQKPEKAPKSLIYAASSLQSGVPSRFSGSG
VL1014-091   DIQMTQSPSSLSASVGDRVTITCRASQGISSWLVWYQQKPEKAPKSLIYAASSLQSGVPSRFSGSG
VL1014-032   DIQMTQSPSSLSASVGDRVTITCRASQGISSWLAWYQQKPEKAPKSLIYATFRLQSGVPSRFSGSG
VL1014-035   DIQMTQSPSSLSASVGDRVTITCRASQGISSWLAWYQQKPEKAPKSLIYATFRLQSGVPSRFSGSG
VL1014-036   DIQMTQSPSSLSASVGDRVTITCRASQGISSWLTWYQQKPEKAPKSLIYAASRLQSGVPSRFSGSG
VL1014-054   DIQMTQSPSSLSASVGDRVTITCRASQGISSWLAWYQQKPEKAPKSLIYAASSLQSGVPSRFSGSG
VL1014-094   DIQMTQSPSSLSASVGDRVTITCRASQGISSWLVWYQQKPEKAPKSLIYAASSLQSGVPSRFSGSG
Consensus    DIQMTQSPSSLSASVGDRVTITCRASQGISSWLXWYQQKPEKAPKSLIYAXXXLQSGVPSRFSGSG

IgKV1D-16 …  SGTDFTLTISSLQPEDFATYYCQQYNSYPWTFGQGTKVEIK
VL1014-091   SGTDFTLTISSLQPEDFATYYCQQYNSFPPTFGQGTKVEIK
VL1014-032   SGTDFTLTISSLQPEDFATYYCQQYNSFPPTFGQGTKVEIK
VL1014-035   SGTDFTLTISSLQPEDFATYYCQQYNSFPPTFGQGTKVEIK
VL1014-036   SGTDFTLTISSLQPEDFATYYCQQYNSFPPTFGQGTKVEIK
VL1014-054   SGTDFTLTISSLQPEDFATYYCQQYNSFPPTFGGTKVEIK
VL1014-094   SGTDFTLTISSLQPEDFATYYCQQYNSFPPTFGQGTKVEIK
Consensus    SGTDFTLTISSLQPEDFATYYCQQYNSFPPTFGXGTKVEIK
```

**Figure 2E**

IgKV1D-16-01 / IGKJ2-01 – VL alignment (Group 3a)

```
IgKV1D-16 …  DIQMTQSPSSLSASVGDRVTITCRASQGISSWLAWYQQKPEKAPKSLIYAASSLQSGVPSRFSGSG
VL1014-098   DIQMTQSPSSLSASVGDRVTITCRASQGISSWLAWYQQKPEKAPKSLIYAASSLQSGVPSRFSGSG
VL1014-100   DIQMTQSPSSLSASVGDRVTITCRASQGISSWLAWYQQKPEKAPKSLIYAASSLQSGVPSRFSGSG
VL1014-105   DIQMTQSPSSLSASVGDRVTITCRASQGISSWLAWYQQKPEKAPKSLIYAASSLQSGVPSRFSGSG
VL1014-125   DIQMTQSPSSLSASVGDRVTITCRASQGINSWLAWYQQKPEKAPKSLIYAASSLQSGVPSRFSGSG
VL1014-162   DIQMTQSPSSLSASVGDRVTITCRASQGISSWLAWYQQKPEKAPKSLIYAASSLQSGVPSRFSGSG
Consensus    DIQMTQSPSSLSASVGDRVTITCRASQGIXSWLAWYQQKPEKAPKSLIYAASSLQSGVPSRFSGSG

IgKV1D-16 …  SGTDFTLTISSLQPEDFATYYCQQYNSYPYTFGQGTKLEIK
VL1014-098   SGTDFTLTISSLQPEDFATYYCQQYNSYPYTFGQGTKLEIK
VL1014-100   SGTDFTLTISSLQPEDFATYYCQQYNSYPYTFGQGTKLEIK
VL1014-105   SGTDFTLTISSLQPEDFATYYCQQYNSYPYTFGQGTKLEIK
VL1014-125   SGTDFTLTISSLQPEDFATYYCQQYNSYPYTFGQGTKLEIK
VL1014-162   SGTDFTLTISSLQPEDFATYYCQQYNSYPLTFGGGTKVEIK
Consensus    SGTDFTLTISSLQPEDFATYYCQQYNSYPXTFGXGTKXEIK
```

**Figure 2F**

IgKV1D-16-01 / IGKJ5-01 – VL alignment (Group 3b)

```
IgKV1D-16 …  DIQMTQSPSSLSASVGDRVTITCRASQGISSWLAWYQQKPEKAPKSLIYAASSLQSGVPSRFSGSG
VL1014-153   DIQMTQSPSSLSASVGDRVTITCRASQGISSWLAWYQQKPEKAPKSLIYDASSLQSGVPSRFSGSG
VL1014-152   DIQMTQSPSSLSASVGDRVTITCRASQGINSWLAWYQQKPEKAPKSLIYDASSLQSGVPSRFSGSG
VL1014-166   DIQMTQSPSSLSASVGDRVTITCRASQGISNWLAWYQQKPEKAPKSLIYDASSLQSGVPSRFSGSG
VL1014-167   DIQMTQSPSSLSASVGDRVTITCRASQGISNWLAWYQQKPEKAPKSLIYDASSLQSGVPSRFSGSG
VL1014-160   DIQMTQSPSSLSASVGDRVTITCRASQDISSWLAWYQQKPEKAPKSLIYAASSLQSGVPSRFSGSG
VL1014-033   DIQMTQSPSSLSASVGDRVTITCRASQGISSWLAWYQQKPEKAPKSLIYAASSLQSGVPSRFSGSG
Consensus    DIQMTQSPSSLSASVGDRVTITCRASQGIXXWLAWYQQKPEKAPKSLIYXASSLQSGVPSRFSGSG

IgKV1D-16 …  SGTDFTLTISSLQPEDFATYYCQQYNSYPITFGQGTRLEIK
VL1014-153   YGTDFSLTISSLQPEDFAIYYCQQYKSYPITFGQGTRLEIK
VL1014-152   SGTDFTLTISSLQPENFATYYCQQYNSYPITFGQGTRLEIK
VL1014-166   SGTDFTLTISSLQPEDFATYYCQQYNSYPITFGQGTRLEIK
VL1014-167   SGTDFTLTISSLQPEDFATYYCQQYNSYPITFGQGTRLEIK
VL1014-160   SGTDFTLTISSLQPEDFATYYCQQYNSYPITFGQGTRLEIK
VL1014-033   SGTDFTLTISSLQPEDFATYYCQQYNSYPITFGQGTRLEIK
Consensus    XGTDFXLTISSLQPEXFAXYYCQQYXSYPITFGQGTRLEIK
```

**Figure 2G**

IgKV3-20-01 / IGKJ4-01 – VL alignment

```
IgKV3-20-01  EIVLTQSPGTLSLSPGERATLSCRASQSVSSSYLAWYQQKPGQAPRLLIYGASSRATGIPDRFSGS
VL1014-005   EIVLTQSPGTLSLSPGERATLSCRASQSVSSSYLAWYQQKPGQVPRLLIYGASSRATGIPDRFSGS
VL1014-059   EIVLTQSPGTLSLSPGERATLSCRASQSVSSTYLAWYQQKPGQAPRLLIYGASSRATGIPDRFSGS
VL1014-060   EIVLTQSPGTLSLSPGERATLSCRASQSVSSSYLAWYQQKPGQAPRLLIYGASSRATGIPDRFSGS
VL1014-106   EIVLTQSPGTLSLSPGERATLSCRASQSVSSSYLAWYQQKPGQAPRLLIYGASSRATGIPDRFSGS
VL1014-111   EIVLTQSPGTLSLSPGERATLSCRASQSVRSSYLAWYQQKPGQAPRLLIYGASSRATGIPDRFSGS
VL1014-041   EIVLTQSPGTLSLSPGERATLSCRASQSVSSSYLAWYQQKPGQAPRLLIYGASSRATGIPDRFSGS
VL1014-150   EIVLTQSPGTLSLSPGERATLSCRASQSVSSSYLTWYQQKPGQAPRLLIYGASSRATGIPDRFSGS
VL1014-067   EIVLTQSPGTLSLSPGERATLSCRASQSVSSSYLAWYQQKPGQAPRLLIYGASSRATGIPDRFSGS
VL1014-072   EIVLTQSPGTLSLSPGERATLSCRASQSVSSSYLAWYQQKPGQAPRLLIYGASSRATGIPDRFSGS
VL1014-163   EIVLTQSPGTLSLSPGERATLSCRASQSVSSSYLAWYQQKPGQAPRLLIYGASSRATGIPDRFSGS
VL1014-093   EIVLTQSPGTLSLSPGERATLSCRASQSVSSSYLAWYQQKPGQAPRLLIYGASSRATGIPDRFSGS
VL1014-044   EIVLTQSPGTLSLSPGERATLSCRASQSVSSSYLAWYQQKPGQAPRLLIYGASSRATGIPDRFSGS
Consensus    EIVLTQSPGTLSLSPGERATLSCRASQSVXSXYLXWYQQKPGQXPRLLIYGASSRATGIPDRFSGS

IgKV3-20-01  GSGTDFTLTISRLEPEDFAVYYCQQYGSSP-LTFGGGTKVEIK
VL1014-005   GSGTDFTLTISRLEPEDFAVYYCQQYGSS--LTFGGGTKVEIK
VL1014-059   GSGTDFTLTISRLEPEDFAVYYCQQYGTS-LFTFGPGTKVDIK
VL1014-060   GSGTDFTLTISRLEPEDFAVYYCQQYGSSPITFGQGTRLEIK
VL1014-106   GSGTDFTLTISRLEPEDFAVYYCQQYGSS--FTFGPGTKVDIK
VL1014-111   GSGTDFTLTISRLEPEDFAVYYCQLYGSSP--TFGPGTKVDIK
VL1014-041   GSGTDFTLTISRLEPEDFAVYYCQQYGSS--LTFGGGTKVEIK
VL1014-150   GSGTDFTLTISRLEPEDFAVYYCQQYGSS--LTFGGGTKVEIK
VL1014-067   GSGTDFTLTISRLEPEDFAVYYCQQYGSSPRLTFGGGTKVEIK
VL1014-072   GSGTDFTLTISRLEPEDFAVYYCQQYGSSPRLTFGGGTKVEIK
VL1014-163   GSGTDFTLTISRLEPEDFAVYYCQQYGSS--LTFGGGTKVEIK
VL1014-093   GSGTDFTLTISRLEPEDFAVYYCQQYGSS--LTFGGGTKVEIK
VL1014-044   GSGTDFTLTISRLEPEDFAVYYCQQYGSS--LTFGGGTKVEIK
Consensus    GSGTDFTLTISRLEPEDFAVYYCQXYGXSXXXTFGXGTXXXIK
```

**Figure 2H**

<u>IgKV3-11-01 / IGKJ4-01 – VL alignment</u>

```
IgKV3-11-01  EIVLTQSPATLSLSPGERATLSCRASQSVSSYLAWYQQKPGQAPRLLIYDASNRATGIPARFSGS
VL1014-006   EIVLTQSPATLSLSPGERATLSCRASQSVSSYLAWYQQKPGQAPRLLIYDASNRATGIPARFSGS
Consensus    EIVLTQSPATLSLSPGERATLSCRASQSVSSYLAWYQQKPGQAPRLLIYDASNRATGIPARFSGS

IgKV3-11-01  GSGTDFTLTISSLEPEDFAVYYCQQRSNWPPLTFGGGTKVEIK
VL1014-006   GSGTDFTLTISSLEPEDFAVYYCQQRSNWPPLTFGGGTKVEIK
Consensus    GSGTDFTLTISSLEPEDFAVYYCQQRSNWPPLTFGGGTKVEIK
```

**Figure 3A**

Binding to AU565

| clone name | EC$_{50}$ µg/mL |
|---|---|
| PC1014-025 | 0.580 |
| PC1014-050 | 0.722 |
| PC1014-084 | 0.491 |
| PC1014-091 | 0.965 |
| PC1014-098 | 0.336 |
| PC1014-127 | 2.290 |
| Herceptin | 0.547 |
| isotype-control | - |

**Figure 3B**

Binding to AU565

| clone name | EC$_{50}$ µg/mL |
|---|---|
| PC1014-129 | 0.578 |
| PC1014-132 | 0.911 |
| PC1014-153 | 0.626 |
| PC1014-159 | 1.300 |
| PC1014-169 | 0.908 |
| Herceptin | 0.547 |
| isotype-control | - |

**Figure 3C**

Binding to A431

| | clone name | EC$_{50}$ µg/mL |
|---|---|---|
| ● | PC1014-025 | 0.128 |
| ○ | PC1014-050 | 1.135 |
| ■ | PC1014-084 | 0.498 |
| ▣ | PC1014-091 | 0.633 |
| ▲ | PC1014-098 | 0.101 |
| △ | PC1014-127 | 0.270 |
| ▼ | Herceptin | 0.845 |
| ▽ | isotype-control | - |

**Figure 3D**

Binding to A431

| | clone name | EC$_{50}$ µg/mL |
|---|---|---|
| ● | PC1014-129 | 0.316 |
| ○ | PC1014-132 | 0.068 |
| ■ | PC1014-153 | 0.244 |
| ▣ | PC1014-159 | 0.855 |
| ▲ | PC1014-169 | 0.415 |
| ▼ | Herceptin | 0.845 |
| ▽ | isotype-control | - |

**Figure 3E**

Binding to AU565

| | clone name | EC$_{50}$ μg/mL |
|---|---|---|
| ● | PC1014-005 | 0.818 |
| ○ | PC1014-006 | 0.304 |
| ▱ | PC1014-059 | 1.309 |
| ▲ | PC1014-060 | 2.678 |
| ▼ | PC1014-106 | 1.548 |
| ▽ | PC1014-111 | 0.915 |
| ◆ | Herceptin | 0.547 |
| ◇ | isotype-control | – |

**Figure 3F**

Binding to A431

| | clone name | EC$_{50}$ μg/mL |
|---|---|---|
| ● | PC1014-005 | 0.789 |
| ○ | PC1014-006 | 0.106 |
| ▱ | PC1014-059 | 0.213 |
| ▲ | PC1014-060 | 1.982 |
| ▼ | PC1014-106 | 1.664 |
| ▽ | PC1014-111 | 0.996 |
| ◆ | Herceptin | 0.845 |
| ◇ | isotype-control | – |

**Figure 4A**

**Figure 4B**

**Figure 5A**

**Figure 5B**

**Figure 6**

**Figure 7**

**Figure 8A**

**Figure 8B**

**Figure 8C**

**Figure 8D**

**Figure 9A**

**Figure 9B**

**Figure 9C**

**Figure 9D**

**Figure 9E**

**Figure 9F**

**Figure 10**

**Figure 11A**

**Figure 11B**

**Figure 12**

**Figure 13**

**Figure 14**

**Figure 15**

**Figure 16A**

**NCI-N87 model**
therapeutic treatment starting 8 days after tumorinjection

Legend:
- HuMab-HepC
- HuMab 091
- HuMab 084
- HuMab 169
- △ treatment 40 mg/kg
- △ treatment 10 mg/kg

Y-axis: Tumorsize (mm3)
X-axis: Days after treatment initiation

**Figure 16B**

**Survival**

Legend:
- HuMab-HepC
- HuMab 091
- HuMab 084
- HuMab 169

Y-axis: Percent survival
X-axis: Time (days after tumor inoculation)

**Figure 16C**

NCI-N87 model
therapeutic treatment starting 8 days after tumorinjection

**Figure 16D**

Survival

**Figure 17A**

**Figure 17B**

**Figure 18A**

**Figure 18B**

**Figure 19A**

**Controls**

**Figure 19B**

**005-ITL x 169-K409R**

**Figure 19C**

**025-ITL x 005-K409R**

**Figure 19D**

**025-ITL x 169-K409R**

- ◇· IgG1-025-ITL
- -△· IgG1-169-K409R
- ■ IgG1-025-ITL + IgG1-169-K409R
- ● IgG1-025-ITL x IgG1-169-K409R

**Figure 19E**

**153-ITL x 005-K409R**

- ◇· IgG1-153-ITL
- -△· IgG1-005-K409R
- ■ IgG1-153-ITL + IgG1-005-K409R
- ● IgG1-153-ITL x IgG1-005-K409R

**Figure 19F**

**153-ITL x 169-K409R**

- ◇· IgG1-153-ITL
- -△· IgG1-169-K409R
- ■ IgG1-153-ITL + IgG1-169-K409R
- ● IgG1-153-ITL x IgG1-169-K409R

**Figure 19G**

153-ITL x 153-N297Q-K409R

**Figure 20**

Mean (with SEM) day 41

**Figure 21A**

NCI-N87
(Mean & SEM)

Tumor volume ($mm^3$)

Time (days after tumor inoculation)

- —○— IgG1-b12
- —□— IgG1-153-K409R
- —△— IgG1-169
- —●— IgG1-169 + IgG1-153
- ◇ Herceptin
- ▲ IgG1-153-ITL x IgG1-169-K409R

**Figure 21B**

Kaplan -Meier
NCI-N87

Mice tumor <400 $mm^3$ (%)

Time (days after tumor inoculation)

- IgG1-b12
- IgG1-153
- IgG1-169
- IgG1-153 + IgG1-169
- IgG1-153-ITL x IgG1-169-K409R
- Herceptin

**Figure 22A**

| | |
|---|---|
| ■ | IgG4-7D8 x IgG4-2F8 |
| ▼ | IgG4-7D8 x IgG1-2F8-ITL |
| □ | IgG4-7D8 x IgG1-2F8-IT |
| ● | IgG4-7D8 x IgG1-2F8-IL |
| ○ | IgG4-7D8 x IgG1-2F8-TL |

**Figure 22B**

| | |
|---|---|
| ■ | IgG4-7D8 x IgG4-2F8 |
| ▨ | IgG4-7D8-CPPC x IgG4-2F8 |
| ▼ | IgG4-7D8-CPPC x IgG1-2F8-ITL |
| □ | IgG4-7D8-CPPC x IgG1-2F8-IT |
| ● | IgG4-7D8-CPPC x IgG1-2F8-IL |
| ○ | IgG4-7D8-CPPC x IgG1-2F8-TL |

**Figure 22C**

| | |
|---|---|
| ■ | IgG1-2F8-F405L+IgG4-7D8 |
| ○ | IgG1-2F8-F405L+IgG4-7D8-CPPC |

**Figure 23A**

**Figure 23B**

**Figure 24A**

Positive Control
IgG1-2F8-F405AxIgG1-7D8-K409R
IgG1-2F8-F405DxIgG1-7D8-K409R
IgG1-2F8-F405ExIgG1-7D8-K409R
IgG1-2F8-F405HxIgG1-7D8-K409R
IgG1-2F8-F405IxIgG1-7D8-K409R
IgG1-2F8-F405KxIgG1-7D8-K409R
IgG1-2F8-F405LxIgG1-7D8-K409R
IgG1-2F8-F405MxIgG1-7D8-K409R
IgG1-2F8-F405NxIgG1-7D8-K409R
IgG1-2F8-F405QxIgG1-7D8-K409R
IgG1-2F8-F405SxIgG1-7D8-K409R
IgG1-2F8-F405TxIgG1-7D8-K409R
IgG1-2F8-F405VxIgG1-7D8-K409R
IgG1-2F8-F405WxIgG1-7D8-K409R
IgG1-2F8-F405YxIgG1-7D8-K409R
IgG4-2F8xIgG4-7D8
IgG1-2F8-F405GxIgG1-7D8-K409R
IgG1-2F8-F405RxIgG1-7D8-K409R
IgG1-2F8xIgG1-7D8-K409R

**Figure 24B**

**Figure 25A**

- ■ Positive Control
- ● IgG1-2F8-Y407AxIgG1-7D8-K409R
- ◐ IgG1-2F8-Y407GxIgG1-7D8-K409R
- ◐ IgG1-2F8-Y407LxIgG1-7D8-K409R
- ◐ IgG1-2F8-Y407MxIgG1-7D8-K409R
- ○ IgG1-2F8-Y407NxIgG1-7D8-K409R
- ○ IgG1-2F8-Y407WxIgG1-7D8-K409R

- △ IgG1-2F8-Y407HxIgG1-7D8-K409R
- △ IgG1-2F8-Y407IxIgG1-7D8-K409R
- △ IgG1-2F8-Y407VxIgG1-7D8-K409R

- ▣ IgG4-2F8xIgG4-7D8

- ◇ IgG1-2F8-Y407DxIgG1-7D8-K409R
- ◆ IgG1-2F8-Y407E x IgG1-7D8-K409
- ▼ IgG1-2F8-Y407FxIgG1-7D8-K409R
- ▽ IgG1-2F8-Y407KxIgG1-7D8-K409R
- ▽ IgG1-2F8-Y407QxIgG1-7D8-K409R
- ▽ IgG1-2F8-Y407RxIgG1-7D8-K409R
- ▽ IgG1-2F8-Y407TxIgG1-7D8-K409R
- ▽ IgG1-2F8-Y407SxIgG1-7D8-K409R

- ✕ IgG1-2F8xIgG1-7D8-K409R

**Figure 25B**

**Figure 26A**

| | |
|---|---|
| ■ | Positive Control |
| ● | IgG1-2F8-L368AxIgG1-7D8-K409R |
| ● | IgG1-2F8-L368DxIgG1-7D8-K409R |
| ● | IgG1-2F8-L368ExIgG1-7D8-K409R |
| ○ | IgG1-2F8-L368GxIgG1-7D8-K409R |
| ● | IgG1-2F8-L368HxIgG1-7D8-K409R |
| ○ | IgG1-2F8-L368IxIgG1-7D8-K409R |
| ○ | IgG1-2F8-L368NxIgG1-7D8-K409R |
| ◆ | IgG1-2F8-L368QxIgG1-7D8-K409R |
| ◆ | IgG1-2F8-L368RxIgG1-7D8-K409R |
| ◇ | IgG1-2F8-L368SxIgG1-7D8-K409R |
| ◆ | IgG1-2F8-L368TxIgG1-7D8-K409R |
| ◇ | IgG1-2F8-L368VxIgG1-7D8-K409R |
| ◇ | IgG1-2F8-L368WxIgG1-7D8-K409R |
| △ | IgG1-2F8-L368KxIgG1-7D8-K409R |
| □ | IgG4-2F8xIgG4-7D8 |
| ▽ | IgG1-2F8-L368FxIgG1-7D8-K409R |
| ▼ | IgG1-2F8-L368MxIgG1-7D8-K409R |
| ▽ | IgG1-2F8-L368YxIgG1-7D8-K409R |
| ✕ | IgG1-2F8xIgG1-7D8-K409R |

**Figure 26B**

**Figure 27A**

Legend:
- Positive Control
- IgG4-2F8xIgG4-7D8
- IgG1-2F8-K370WxIgG1-7D8-K409R
- IgG1-2F8-K370AxIgG1-7D8-K409R
- IgG1-2F8-K370DxIgG1-7D8-K409R
- IgG1-2F8-K370ExIgG1-7D8-K409R
- IgG1-2F8-K370FxIgG1-7D8-K409R
- IgG1-2F8-K370GxIgG1-7D8-K409R
- IgG1-2F8-K370HxIgG1-7D8-K409R
- IgG1-2F8-K370IxIgG1-7D8-K409R
- IgG1-2F8-K370LxIgG1-7D8-K409R
- IgG1-2F8-K370MxIgG1-7D8-K409R
- IgG1-2F8-K370NxIgG1-7D8-K409R
- IgG1-2F8-K370QxIgG1-7D8-K409R
- IgG1-2F8-K370RxIgG1-7D8-K409R
- IgG1-2F8-K370SxIgG1-7D8-K409R
- IgG1-2F8-K370TxIgG1-7D8-K409R
- IgG1-2F8-K370VxIgG1-7D8-K409R
- IgG1-2F8-K370YxIgG1-7D8-K409R
- IgG1-2F8xIgG1-7D8-K409R

**Figure 27B**

**Figure 28A**

| | |
|---|---|
| ■ | Positive Control |
| ● | IgG1-2F8-D399AxIgG1-7D8-K409R |
| ◕ | IgG1-2F8-D399FxIgG1-7D8-K409R |
| ◑ | IgG1-2F8-D399HxIgG1-7D8-K409R |
| ◔ | IgG1-2F8-D399IxIgG1-7D8-K409R |
| ○ | IgG1-2F8-D399KxIgG1-7D8-K409R |
| ◆ | IgG1-2F8-D399LxIgG1-7D8-K409R |
| ◈ | IgG1-2F8-D399MxIgG1-7D8-K409R |
| ◇ | IgG1-2F8-D399NxIgG1-7D8-K409R |
| ◈ | IgG1-2F8-D399RxIgG1-7D8-K409R |
| ◇ | IgG1-2F8-D399SxIgG1-7D8-K409R |
| ◇ | IgG1-2F8-D399TxIgG1-7D8-K409R |
| ◕ | IgG1-2F8-D399GxIgG1-7D8-K409R |
| ▲ | IgG1-2F8-D399WxIgG1-7D8-K409R |
| △ | IgG1-2F8-D399YxIgG1-7D8-K409R |
| □ | IgG4-2F8xIgG4-7D8 |
| ▽ | IgG1-2F8-D399ExIgG1-7D8-K409R |
| ▽ | IgG1-2F8-D399QxIgG1-7D8-K409R |
| ▽ | IgG1-2F8-D399VxIgG1-7D8-K409R |
| ✕ | IgG1-2F8xIgG1-7D8-K409R |

**Figure 28B**

**Figure 29A**

**Figure 29B**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9955367 A
- WO 2006116107 A
- WO 2005117973 A
- WO 2008031531 A
- WO 2009154651 A
- WO 2009068628 A
- WO 2007059782 A
- WO 0243478 A
- WO 2009097006 A
- US 2009317869 A
- WO 2010001251 A
- EP 2011056388 W
- WO 11131746 A
- WO 2008119353 A
- WO 2011131746 A
- US 5731168 A
- EP 1870459 A, Chugai
- WO 2009089004 A, Amgen
- WO 2007110205 A, Merck
- US 6077 A
- US 835 A
- WO 0070087 A
- WO 0046147 A
- US 5589466 A
- US 5973972 A
- US 5545806 A
- US 5569825 A
- US 5625126 A
- US 5633425 A
- US 5789650 A
- US 5877397 A
- US 5661016 A
- US 5814318 A
- US 5874299 A
- US 5770429 A
- US 5545807 A
- WO 9824884 A
- WO 9425585 A
- WO 931227 A
- WO 9222645 A
- WO 9203918 A
- WO 0109187 A

- WO 0114424 A
- US 5733743 A
- US 4946778 A
- WO 2006033386 A, Kirin
- WO 2008145142 A, Genmab
- US 5635483 A
- US 5780588 A
- US 5663149 A
- US 20050238649 A
- US 4681581 A
- US 4735210 A
- US 5101827 A
- US 5102990 A
- US 35500 A
- US 5648471 A
- US 5697902 A
- US 4766106 A
- US 4179337 A
- US 4495285 A
- US 4609546 A
- WO 02083180 A
- WO 2004043493 A
- WO 2007018431 A
- WO 2007089149 A
- WO 2009017394 A
- WO 201062171 A, Syntarga BV
- US 6989452 B, Medarex
- US 6214345 B
- WO 2004010957 A
- US 7659241 B
- US 7829531 B
- US 7851437 B
- US 11833028 B
- US 7498298 B
- US 11833954 B
- WO 2005081711 A
- US 6440735 B
- US 6713055 B
- US 6949245 B
- US 7244826 B
- US 7632924 B
- WO 02100348 A
- WO 04035607 A

### Non-patent literature cited in the description

- **GRAUS-PORTA et al.** *Embo J,* 1997, vol. 16, 1647-1655
- **TAO et al.** *J Cell Sci,* 2008, vol. 121, 3207-3217

- **RIESE ; STERN.** *Bioessays,* 1998, vol. 20, 41-48
- **HUANG et al.** *Expert Opin Biol Ther,* 2009, vol. 9, 97-110
- **REESE et al.** *Stem Cells,* 1997, vol. 15, 1-8
- **ANDRECHEK et al.** *Proc Natl Acad Sci U S A,* 2000, vol. 97, 3444-3449
- **SLAMON et al.** *Science,* 1987, vol. 235, 177-182
- **GARCIA DE PALAZZO et al.** *Int J Biol Markers,* 1993, vol. 8, 233-239
- **ROSS et al.** *Oncologist,* 2003, vol. 8, 307-325
- **OSMAN et al.** *J Urol,* 2005, vol. 174, 2174-2177
- **KAPITANOVIC et al.** *Gastroenterology,* 1997, vol. 112, 1103-1113
- **TURKEN et al.** *Neoplasma,* 2003, vol. 50, 257-261
- **OSHIMA et al.** *Int J Biol Markers,* 2001, vol. 16, 250-254
- **CHO et al.** *Nature,* 2003, vol. 421, 756-760
- **WEHRMAN et al.** *Proc Natl Acad Sci U S A,* 2006, vol. 103, 19063-19068
- **SCHMITZ et al.** *Exp Cell Res,* 2009, vol. 315, 659-670
- **NAHTA ; ESTEVA.** *Oncogene,* 2007, vol. 26, 3637-3643
- **DINH et al.** *Clin Adv Hematol Oncol,* 2007, vol. 5, 707-717
- **FRANKLIN et al.** *Cancer Cell,* 2004, vol. 5, 317-328
- **HUGHES et al.** *Mol Cancer Ther,* 2009, vol. 8, 1885-1892
- **PEDERSEN et al.** *Mol Cancer Res,* 2009, vol. 7, 275-284
- **EMDE et al.** *Oncogene,* 2011, vol. 30, 1631-1642
- **SPIRIDON et al.** *Clin Cancer res,* 2002, vol. 8, 1720-1730
- **BASELGA et al.** *J Clin Oncol,* 2010, vol. 28, 1138-1144
- **BURRIS et al.** *J Clin Oncol,* 2011, vol. 29, 398-405
- **LEWIS PHILLIPS et al.** *Cancer Res,* 2008, vol. 68, 9280-9290
- **PEREZ et al.** *Abstract BA3, European Society for Medical Oncology meeting,* 2010
- **BEN-KASUS et al.** *PNAS,* 2009, vol. 106, 3294-9
- **BAULIDA et al.** *J Biol Chem,* 1996, vol. 271, 5251-5257
- **PEDERSEN NM et al.** *Mol Cancer Res,* 2009, vol. 7, 275-84
- **MULLER ; KONTERMANN.** *BioDrugs,* 2010, vol. 24, 89-98
- **JONES et al.** *Lancet Oncol,* 2009, vol. 10, 1179-1187
- **KIEWE et al.** *Clin Cancer Res,* 2006, vol. 12, 3085-3091
- **BAEUERLE ; REINHARDT.** *Cancer Research,* 2009, vol. 96, 4941
- **BARGOU et al.** *Science,* 2008, vol. 321, 974-976
- **MOORE et al.** *Blood,* 2011, vol. 117, 4542-4551
- **BAEUERLE et al.** *Current opinion in Molecular Therapeutics,* 2009, vol. 11, 22-30
- **FRIEDMAN et al.** *Biotechnol Appl Biochem.,* 21 August 2009, vol. 54 (2), 121-31
- **ROBINSON et al.** *Br.J.Cancer,* 2008, vol. 99, 1415-25
- Fundamental Immunology. Raven Press, 1989
- **CHOTHIA ; LESK.** *J. Mol. Biol.,* 1987, vol. 196, 901-917
- **BROCHET X.** *Nucl Acids Res.,* 2008, vol. 36, W503-508
- **LEFRANC MP.** *Nucleic Acids Research,* 1999, vol. 27, 209-212
- **KABAT et al.** Sequences of Proteins of Immunological Interest. National Institutes of Health, 1991
- **WARD et al.** *Nature,* 1989, vol. 341, 544-546
- **HOLT et al.** *Trends Biotechnol.,* November 2003, vol. 21 (11), 484-90
- **REVETS et al.** *Expert Opin Biol Ther.,* January 2005, vol. 5 (1), 111-24
- **BIRD et al.** *Science,* 1988, vol. 242, 423-426
- **HUSTON et al.** *PNAS USA,* 1988, vol. 85, 5879-5883
- **BOLT S et al.** *Eur J Immunol,* 1993, vol. 23, 403-411
- **OGANESYAN.** *Acta Crys.,* 2008, vol. D64, 700-704
- **SHIELDS et al.** *JBC,* 2001, vol. 276, 6591-6604
- **E. MEYERS ; W. MILLER.** *Comput. Appl. Biosci,* 1988, vol. 4, 11-17
- **NEEDLEMAN ; WUNSCH.** *J. Mol. Biol.,* 1970, vol. 48, 444-453
- **VAN BERKEL et al.** *Biotechnol. Bioeng.,* 2010, vol. 105, 350
- **YAMANE-OHNUKI et al.** *Biotechnol. Bioeng,* 2004, vol. 87, 614
- **UMAÑA et al.** *Nature Biotech,* 1999, vol. 17, 176
- **NATSUME et al.** *Cancer Sci.,* 2009, vol. 100, 2411
- **VAN DER NEUT-KOLFSCHOTEN et al.** *Science,* 14 September 2007, vol. 317 (5844), 1554-7
- **MARVIN ; ZHU.** *Acta Pharmacol Sin,* 2005, vol. 26, 649

- **SYKES ; JOHNSTON.** *Nat Biotech,* 1997, vol. 17, 355-59
- **SCHAKOWSKI et al.** *Mol Ther,* 2001, vol. 3, 793-800
- **BENVENISTY ; RESHEF.** *PNAS USA,* 1986, vol. 83, 9551-55
- **WIGLER et al.** *Cell,* 1978, vol. 14, 725
- **CORARO ; PEARSON.** *Somatic Cell Genetics,* 1981, vol. 7, 603
- **VAN HEEKE ; SCHUSTER.** *J Biol Chem,* 1989, vol. 264, 5503-5509
- Current Protocols in Molecular Biology. Greene Publishing and Wiley InterScience, 1987
- **GRANT et al.** *Methods in Enzymol,* 1987, vol. 153, 516-544
- **BEBBINGTON.** *Biotechnology (NY),* 1992, vol. 10, 169-175
- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **LONBERG, N. et al.** *Nature,* 1994, vol. 368, 856-859
- **LONBERG, N.** *Handbook of Experimental Pharmacology,* 1994, vol. 113, 49-101
- **LONBERG, N. ; HUSZAR, D.** *Intern. Rev. Immunol.,* 1995, vol. 13, 65-93
- **HARDING, F. ; LONBERG, N.** *Ann. N.Y. Acad. Sci,* 1995, vol. 764, 536-546
- **TAYLOR, L. et al.** *Nucleic Acids Research,* 1992, vol. 20, 6287-6295
- **CHEN, J. et al.** *International Immunology,* 1993, vol. 5, 647-656
- **TUAILLON et al.** *J. Immunol.,* 1994, vol. 152, 2912-2920
- **TAYLOR, L. et al.** *International Immunology,* 1994, vol. 6, 579-591
- **FISHWILD, D. et al.** *Nature Biotechnology,* 1996, vol. 14, 845-851
- **CHEN et al.** *EMBO J.,* 1993, vol. 12, 821-830
- **FISHWILD et al.** *Nature Biotechnology,* 1996, vol. 14, 845-851
- **CHEN et al.** *EMBO J.,* 1993, vol. 12, 811-820
- **HOOGENBOOM et al.** *J. Mol. Biol.,* 1991, vol. 227, 381
- **VAUGHAN et al.** *Nature Biotech,* 1996, vol. 14, 309
- **HANES ; PLUCTHAU.** *PNAS USA,* 1997, vol. 94, 4937-4942
- **PARMLEY ; SMITH.** *Gene,* 1988, vol. 73, 305-318
- **SCOTT.** *TIBS,* 1992, vol. 17, 241-245
- **CWIRLA et al.** *PNAS USA,* 1990, vol. 87, 6378-6382
- **RUSSEL et al.** *Nucl. Acids Research,* 1993, vol. 21, 1081-1085
- **HOGENBOOM et al.** *Immunol. Reviews,* 1992, vol. 130, 43-68
- **CHISWELL ; MCCAFFERTY.** *TIBTECH,* 1992, vol. 10, 80-84
- **EVANS et al.** *J. Immunol. Meth.,* 1995, vol. 184, 123-38
- **PLUCKTHUN.** The Pharmacology of Monoclonal Antibodies. Springer-Verlag, 1994, vol. 113, 269-315
- **MCCAFFERTY et al.** *Nature,* 1990, vol. 348, 552-554
- **VAN DER NEUT KOLFSCHOTEN et al.** *Science,* 2007, vol. 317 (5844), 1554-7
- **DALL'ACQUA WF et al.** *J Immunol.,* 2006, vol. 177 (2), 1129-1138
- **HEZAREH M.** *J Virol.,* 2001, vol. 75 (24), 12161-12168
- **PASTAN et al.** *Cell,* 1986, vol. 47, 641
- **GOLDENBERG, CALIF.** *A Cancer Journal for Clinicians,* 1994, vol. 44 (43
- **WOYKE et al.** *Antimicrob. Agents and Chemother.,* 2001, vol. 45 (12), 3580-3584
- **PETTIT et al.** *Antimicrob. Agents and Chemother.,* 1998, vol. 42, 2961-2965
- **SENTER et al.** *Proceedings of the American Association for Cancer Research,* 28 March 2004, vol. 45
- Cancer Chemotherapy and Biotherapy. Lippincott Raven, 1996, 655-686
- **HUNTER et al.** *Nature,* 1962, vol. 144, 945
- **DAVID et al.** *Biochemistry,* 1974, vol. 13, 1014
- **PAIN et al.** *J. Immunol. Meth.,* 1981, vol. 40, 219
- **NYGREN, J.** *Histochem. and Cytochem.,* 1982, vol. 30, 407
- **OSAMU KANEMITSU.** Antibody Engineering Handbook. Chijin Shokan, 1994
- **DUBOWCHIK ; WALKER.** Pharm. Therapeutics. 1999, vol. 83, 67-123
- Remington: The Science and Practice of Pharmacy. Mack Publishing Co, 1995
- **KOZAK, M.** *Gene,* 1999, vol. 234 (2), 187-208
- **LONZA BIOLOGICS ; BEBBINGTON, C.R. et al.** *Biotechnology (N Y),* 1992, vol. 10 (2), 169-75
- **BARNES, L.M. et al.** *Cytotechnology,* 2000, vol. 32 (2), 109-123
- Current Protocols in Immunology. John Wiley & Sons, Inc, 2006
- **ASLANIDIS, C. ; P.J. DE JONG.** *Nucleic Acids Res,* 1990, vol. 18 (20), 6069-74
- **LEFRANC MP. et al.** *Nucleic Acids Research,* 1999, vol. 27, 209-212
- **BROCHET X.** *Nucl. Acids Res.,* 2008, vol. 36, W503-508
- **JUNTILLA et al.** *Cancer Cell,* 2009, vol. 15 (5), 353-355
- **FRANKLIN MC.** *Cancer Cell,* 2004
- **LANDGRAF R.** *BCR,* 2007

- **LARSEN SS et al.** *Breast Cancer Res Treat,* 2000, vol. 58, 41-56
- **AGUS DB. et al.** *Cancer Cell,* 2002, vol. 2, 127-137
- **KREITMAN RJ.** *BioDrugs,* 2009, vol. 23 (1), 1-13
- **LEFRANC MP et al.** *Nucleic Acids Research,* 1999, vol. 27, 209-212
- **BARBAS, CF.** *J Mol Biol.,* 05 April 1993, vol. 230 (3), 812-23
- **MAZOR Y. et al.** *J. Immunol. Methods,* 2007, vol. 321, 41-59
- **KUO SR. et al.** *Bioconjugate Chem. 2009,* 2009, vol. 20, 1975-1982
- **KREITMAN RJ.** *BioDrugs,* 2009, vol. 23, 1-13
- **JUNTILLA TT. et al.** *Cancer Cell,* 2009, vol. 15, 429-440